# EUROPEAN PATENT APPLICATION

(11) **EP 4 847 618 A2**
(43) Date of publication of application: **02.12.2026**
(21) Application number: 26177152.1
(22) Date of filing: 07.05.2026
(51) Int. Cl.: C12N 15/113, A61K 31/7088

(54) **SIRNA, DERIVATIVE, AND CONJUGATE FOR TARGETED REGULATION OF FXI GENE EXPRESSION AND USE THEREOF**

(30) Priority: 08.05.2025 CN 202510593222; 13.11.2025 CN 202511659072
(71) Applicant: Beijing Youcare Kechuang Pharmaceutical Technology Co., Ltd., Daxing District, Beijing 100176 (CN)
(72) Inventor: HUANG, Zeao, Beijing, 100176 (CN); SONG, Gengshen, Beijing, 100176 (CN); YANG, Shuo, Beijing, 100176 (CN); XU, Chen, Beijing, 100176 (CN); JI, Guangshen, Beijing, 100176 (CN); YU, Fei, Beijing, 100176 (CN); WU, Yucheng, Beijing, 100176 (CN); TIAN, Zhikang, Beijing, 100176 (CN)
(74) Representative: Dehns

(57) **Abstract**

The present disclosure provides siRNA, derivative, and conjugate for targeted regulation of coagulation factor XI (FXI) gene expression, and use thereof. In the present disclosure, a series of siRNAs were designed based on the messenger ribonucleic acid (mRNA) sequence of FXI and were subjected to alternating modifications and modifications using a set of specific modification templates. Results from cell-based and animal experiments demonstrate that some oligonucleotide sequences with alternating modifications and specific template modifications can significantly inhibit FXI gene expression and may be used for the development of anticoagulants.

## Description

The present application claims priority to Chinese Patent Application No. 2025105932227 filed on May 8, 2025 and Chinese Patent Application No. 2025116590721 filed on November 13, 2025. The contents of the above Chinese Patent Applications are incorporated herein by reference in their entirety.

### TECHNICAL FIELD

The present disclosure belongs to the field of biomedicine, and specifically relates to siRNAs, derivatives, and conjugates for targeted regulation of FXI gene expression, and the use thereof, for example, in regulating FXI gene expression or in anticoagulation.

### BACKGROUND

The coagulation cascade is accomplished through the combined actions of the intrinsic coagulation pathway and the extrinsic coagulation pathway. Specifically, the intrinsic coagulation pathway includes coagulation factors FXI and FXII. In the classical model, FXII is activated upon contact with collagen, RNA, or polyphosphates. Activated FXII (FXIIa) subsequently activates downstream FXI to produce FXIa. Through downstream coagulation factors such as FIX and FX, thrombin is ultimately generated via the common pathway. Thrombin initiates fibrin formation and simultaneously activates FXI, forming a positive feedback loop that amplifies the coagulation process. Thrombosis is primarily triggered through the intrinsic pathway.

The extrinsic coagulation pathway is typically initiated by tissue factor (TF), which activates FVII and subsequently activates the common pathway, playing a crucial role in hemostasis. When vascular injury occurs, FVII or FVIIa in the blood binds to tissue factor (TF) originating from outside the blood vessel. The FVIIa-TF complex converts FX into FXa, after which FXa converts a limited amount of prothrombin into thrombin. During hemostasis, FXI is converted into FXIa by thrombin. FXIa then promotes clot consolidation by activating FIX and its downstream pathways, but its overall role is relatively minor. Overall, FXI, a key molecule in the intrinsic coagulation pathway, plays a significant role in regulating the physiological process of thrombosis, but is scarcely involved in pathological processes such as bleeding, which are primarily regulated by the extrinsic pathway. Therefore, FXI can serve as a novel target for antithrombotic therapy.

Among currently marketed anticoagulant drugs, vitamin K antagonists (VKAs) primarily inhibit prothrombin and FX, while heparin and direct oral anticoagulants (DOACs) primarily inhibit FXa and thrombin. These drugs may inhibit thrombosis while suppressing hemostatic functions associated with the extrinsic coagulation pathway, thereby posing a certain risk of bleeding during treatment. In contrast, the target FXI in this study plays a minor role in hemostasis but a fundamental role in thrombosis. Therefore, it can effectively separate the processes of hemostasis and thrombosis, thereby reduce the risk of bleeding while providing anticoagulant effects. Furthermore, fully modified siRNA drugs conjugated with GalNAc exhibit excellent long-acting properties, enabling administration once every six months while maintaining sustained efficacy, which will significantly improve patient compliance.

### SUMMARY

### Problem to be solved by the present disclosure:

Among anticoagulants commonly used in clinical practice, vitamin K antagonists (VKAs) primarily inhibit prothrombin and FX, while heparin and direct oral anticoagulants (DOACs) primarily inhibit FXa and thrombin. These drugs may inhibit thrombosis while suppressing hemostatic functions associated with the extrinsic coagulation pathway, thereby posing a certain risk of bleeding during treatment. In addition, many anticoagulant small-molecule inhibitors under development require daily administration, resulting in low patient compliance. Furthermore, for patients with impaired renal function, drugs metabolized through the kidneys have a significant impact on renal function. Therefore, there is an urgent need to develop anticoagulant drugs that can reduce the risk of bleeding while improving patient compliance.

### Solution:

In the present disclosure, a series of siRNA sequences are designed based on the FXI mRNA sequence, and specific template modifications are introduced, followed by conjugation with N-acetylgalactosamine (GalNAc).

In the present disclosure, by modifying the designed siRNA sequences, certain alternately modified and specifically modified sequences that significantly inhibit FXI gene expression are identified.

In one aspect, the present disclosure provides an siRNA duplex comprising an oligonucleotide duplex formed by pairing of a sense strand and an antisense strand.

In another aspect, the present disclosure provides a conjugate for reducing the expression of FXI, comprising the siRNA duplex described above and a conjugate moiety attached thereto.

In another aspect, the present disclosure provides a nucleic acid-protein complex comprising a duplex region of the siRNA duplex described above or an antisense strand of the duplex region, and a nuclease.

In another aspect, the present disclosure provides a recombinant vector comprising a nucleic acid molecule encoding the siRNA duplex described above.

In some embodiments, the vector backbone of the recombinant vector is selected from recombinant virus-derived circular RNA vectors, tRNA scaffolds, rRNA scaffolds, and chimeric tRNA/pre-miRNA vectors.

In another aspect, the present disclosure provides a recombinant cell that synthesizes and secretes the siRNA duplex described above.

In some embodiments, the recombinant cell is selected from *Rhodovulum sulfidophilum* and ribonuclease III-deficient *Corynebacterium glutamicum.*

In another aspect, the present disclosure provides a method of preparing an siRNA duplex, comprising culturing the recombinant cell described above or chemical synthesis.

In another aspect, the present disclosure provides a pharmaceutical composition comprising the siRNA duplex described above, the conjugate described above, or the nucleic acid-protein complex described above, and a pharmaceutically acceptable carrier.

In another aspect, the present disclosure provides a method of inhibiting FXI gene expression, comprising contacting the siRNA duplex described above, the conjugate described above, the nucleic acid-protein complex described above, or the pharmaceutical composition described above with a target cell.

In some embodiments, the method is for non-diagnostic or non-therapeutic purposes.

In some embodiments, the method is performed *in vivo* or *in vitro.*

In another aspect, the present disclosure provides the use of the siRNA duplex described above, the conjugate described above, the nucleic acid-protein complex described above, or the pharmaceutical composition described above in the manufacture of a medicament for treating a disease associated with FXI gene expression.

The disease associated with FXI gene expression is selected from diseases caused by overexpression of FXI protein, pathogenic mutations in the FXI gene, abnormal metabolism of FXI protein, and abnormal interactions between FXI and another substance.

In some embodiments, the disease associated with FXI gene expression is selected from thrombotic diseases, including deep vein thrombosis, venous or arterial thrombosis, ischemic stroke, pulmonary embolism, myocardial infarction, stroke, Alzheimer's disease, and thrombosis associated with chronic kidney disease or end-stage renal disease.

In another aspect, the present disclosure provides the siRNA duplex described above, the conjugate described above, the nucleic acid-protein complex described above, or the pharmaceutical composition described above for use in treating a disease associated with FXI gene expression, such as prevention of stroke in atrial fibrillation, anticoagulation during dialysis in end-stage renal disease, and anticoagulation following knee replacement surgery.

In another aspect, the present disclosure provides a method of treating a disease associated with FXI gene expression, comprising administering to a subject in need thereof an effective amount of the siRNA duplex described above, the conjugate described above, the nucleic acid-protein complex described above, or the pharmaceutical composition described above.

In another aspect, the present disclosure provides the use of the siRNA duplex described above, the conjugate described above, the nucleic acid-protein complex described above, or the pharmaceutical composition described above in the manufacture of a medicament for treating cardiovascular and cerebrovascular diseases.

In another aspect, the present disclosure provides the siRNA duplex described above, the conjugate described above, the nucleic acid-protein complex described above, or the pharmaceutical composition described above for use in treating cardiovascular and cerebrovascular diseases.

In another aspect, the present disclosure provides a method of treating cardiovascular and cerebrovascular diseases, comprising administering to a subject in need thereof an effective amount of the siRNA duplex described above, the conjugate described above, the nucleic acid-protein complex described above, or the pharmaceutical composition described above.

Advantages of the present disclosure:
The beneficial effects achieved by the present disclosure include, but are not limited to, the following:
(1) By adopting alternating 2'-fluoro and 2'-O-methyl modifications together with the template modifications disclosed herein, the sequences can significantly inhibit the expression level of the target gene FXI in HepG2 cells. The inhibition rate is significantly higher than that of the highly active sequences disclosed in existing patents.
(2) The siRNA conjugate of the present disclosure, conjugated with the GalNAc conjugate moiety G101, exhibits a high inhibition rate against the expression of the target gene FXI in mice, with a duration of at least 49 days.
(3) The siRNA conjugate of the present disclosure, conjugated with the GalNAc conjugate moiety G101, exhibits a strong inhibitory effect on the expression and activity of the target gene FXI and a prolongation effect on APTT (activated partial thromboplastin time) in cynomolgus monkeys, with a duration of at least 84 days.
(4) Certain core sequences, when employing various modification templates, all exhibit relatively high inhibition rates against the target gene FXI.

### DETAILED DESCRIPTION OF PREFERRED EMBODIMENTS

To facilitate understanding of the present disclosure, certain terms are first defined. In addition, it should be noted that whenever a value or a range of values for a parameter is recited, it is intended that intermediate values and ranges between the recited values are also intended to be encompassed within the scope of the present disclosure.

As used herein, the articles "a" and "an" refer to one or more than one *(i.e.,* at least one) of the grammatical object of the article. By way of example, "an element" refers to one element or more than one element, such as multiple elements.

The term "including" is used herein to mean, and is used interchangeably with, the phrase "including, but not limited to."

The term "or" is used herein to mean, and is used interchangeably with, the term "and/or", unless the context clearly indicates otherwise.

As used herein, the term "about" or "approximately", as applied to one or more target values, refers to a value that is similar to the stated reference value. In certain embodiments, unless otherwise specified or clearly evident from the context, the term "approximately" or "about" refers to a value within 20%, 19%, 18%, 17%, 16%, 15%, 14%, 13%, 12%, 11%, 10%, 9%, 8%, 7%, 6%, 5%, 4%, 3%, 2%, 1%, or less of the stated reference value in either direction (greater than or less than) (unless such a range would exceed 100% of the possible value).

As used herein, "FXI" refers to the gene encoding coagulation factor XI or the protein expressed by the gene.

The term "FXI gene" may refer to a wild-type FXI gene or a mutant FXI gene containing sequence variations. Numerous sequence variations in the FXI gene have been identified and can be found in databases such as NCBI dbSNP and UniProt (see, for example, ncbi.nlm.nih.gov/snp).

"G", "C", "A", and "U" each generally represent nucleotides containing guanine, cytosine, adenine, and uracil as their bases, respectively. "T" and "dT" are used interchangeably herein and refer to a deoxyribonucleotide in which the nucleobase is thymine, such as deoxyribothymine, 2'-deoxythymidine, or thymidine. However, it should be understood that the term "ribonucleotide", "nucleotide", or "deoxyribonucleotide" may also refer to a modified nucleotide (as described in further detail below) or a surrogate replacement moiety. Those skilled in the art will readily appreciate that guanine, cytosine, adenine, and uracil may be replaced by other moieties without substantially altering the base pairing properties of an oligonucleotide (including a nucleotide comprising such a replacement moiety). For example, without limitation, a nucleotide containing inosine as its base may pair with a nucleotide containing adenine, cytosine, or uracil. Hence, nucleotides containing uracil, guanine, or adenine may be replaced in the nucleotide sequences of the present disclosure by a nucleotide containing, for example, inosine. Sequences containing such replacement moieties are applicable to, but are not limited to, double-stranded ribonucleic acids, double-stranded ribonucleic acid derivatives, double-stranded ribonucleic acid conjugates, pharmaceutical compositions, and methods of the present disclosure.

The terms "complementary", "fully complementary", and "substantially complementary", as used herein, may refer to base pairing between the sense and antisense strands of an siRNA, or between the antisense strand of an siRNA and a target sequence, as understood from the context in which they are used. In some embodiments of the present disclosure, a first nucleotide sequence may be considered complementary to a second nucleotide sequence if the two sequences exhibit at least 70%, 75%, 80%, 85%, 90%, 95%, 96%, 97%, 98%, or 99% sequence complementarity. In an exemplary embodiment, 18 out of 20 nucleobases in the first nucleotide sequence pair with a corresponding region of the second nucleotide sequence, thereby achieving 90% complementarity. The terms "double-stranded ribonucleic acid", "double-stranded RNA (dsRNA) molecule", "dsRNA", and "RNA duplex" are used interchangeably and refer to a complex of ribonucleic acid molecules having a double-stranded structure, comprising two antiparallel, complementary or substantially complementary nucleic acid strands, which possess "sense" and "antisense" orientations relative to a target gene, such as the FXI gene. In some examples, double-stranded ribonucleic acid (dsRNA) triggers degradation of a target RNA, such as mRNA, through a post-transcriptional gene silencing mechanism (referred to herein as RNA interference or RNAi).

As used herein, the terms "siRNA duplex" and "double-stranded RNAi agent" in some cases also typically refer to the technical meaning as defined above. As is well known in the art, the terms "siRNA duplex", "double-stranded RNAi agent", "RNAi agent", "small interfering ribonucleic acid", or "siRNA" refer to small RNAi molecules. These are a class of double-stranded RNA molecules, also known in the art as short interfering RNA or silencing RNA. siRNA typically comprises a sense strand (also referred to as a passenger strand) and an antisense strand (also referred to as a guide strand), each of which is 17 to 30 nucleotides in length, typically 19 to 25 nucleotides in length, wherein the antisense strand is complementary (*e.g*., at least 95% complementary, such as fully complementary) to a target nucleic acid (suitably a mature mRNA sequence), and the sense strand is complementary to the antisense strand, such that the sense and antisense strands form a duplex or duplex region. The sense and antisense strands of an siRNA may form a blunt-ended duplex, or may form a duplex with a 3' overhang (also referred to as a protruding end), for example, of 1, 2, or 3 nucleotides in length, similar to products generated by Dicer, which can form substrates for RISC *in vivo.* Effective extended forms of Dicer substrates have been described in US 8,349,809 and US 8,513,207, which are incorporated herein by reference. In some examples, both the sense strand and the antisense strand have a 3' overhang of two nucleotides in length. Accordingly, the duplex region may be, for example, 17, 18, 19, 20, 21, 22, 23, 24, or 25 nucleotides in length, such as 19, 20, 21, 22, or 23 nucleotides in length.

Moreover, as used herein, "siRNA" in some cases also refers to a "core sequence". As used herein, the term "core sequence" in some cases specifically refers to an siRNA duplex in which each nucleotide in the double-stranded ribonucleic acid is an unmodified nucleotide, and it also appears in the context as "motif" or "siRNA motif". Accordingly, in the present disclosure, the terms "siRNA", "core sequence", "motif", and "siRNA motif" are used interchangeably, and their meanings also include the corresponding nucleotide arrangement of the siRNA duplex to which they refer. In the present disclosure, those skilled in the art can clearly understand the precise technical meaning of these terms based on the context. Furthermore, the 5'-terminal nucleotide of the antisense strand of the motif may be linked to a 5'-phosphate group or a 5'-phosphate derivative, or may not be linked to a 5'-phosphate group or a 5'-phosphate derivative.

As used herein, the term "siRNA derivative" refers to a double-stranded ribonucleic acid comprising at least one modified nucleotide, and in some cases also appears as a "double-stranded ribonucleic acid derivative". In the present disclosure, the siRNA motif is modified using different modification patterns to prepare corresponding siRNA derivatives. For example, in some embodiments, the motif is modified using an alternating modification pattern, thereby obtaining an alternately modified siRNA derivative. In other embodiments, the motif is modified using a specific modification template, thereby obtaining an siRNA derivative with the specific modification template. In still other embodiments, the motif is modified using the anti-off-target modification pattern described herein, thereby obtaining an siRNA derivative with anti-off-target modifications. In some cases, multiple different modification patterns may be simultaneously applied to the same siRNA motif, thereby obtaining corresponding siRNA derivatives with multiple modification patterns.

As used herein, the term "siRNA conjugate" refers to a double-stranded ribonucleic acid conjugate or a conjugate of a double-stranded ribonucleic acid derivative obtained by attaching a conjugate moiety to a double-stranded ribonucleic acid or a double-stranded ribonucleic acid derivative. Preferably, the term "siRNA conjugate" refers to a conjugate of a double-stranded ribonucleic acid derivative.

As used herein, the term "siRNA" in some cases not only refers to the unmodified siRNA duplex (or siRNA motif) described above, but may also refer to the corresponding siRNA derivative and/or siRNA conjugate. For example, in contexts involving, but not limited to, therapeutic methods or therapeutic agents, "siRNA" broadly refers to at least one of an siRNA motif, an siRNA derivative, and/or an siRNA conjugate. Those skilled in the art can clearly understand the specific technical meaning based on the context.

The term "antisense strand" refers to the strand of a double-stranded ribonucleic acid (*e.g*., the RNA duplex described herein) that includes a region substantially complementary to a target sequence. As used herein, the term "region of complementarity" refers to a region on the antisense strand that is substantially complementary to a sequence defined herein (*e.g*., a target sequence). When the region of complementarity is not fully complementary to the target sequence, mismatches may occur in the internal or terminal regions of the molecule. Typically, mismatches are most tolerated in the terminal regions, for example, within 5, 4, 3, 2, or 1 nucleotides from the 5' and/or 3' ends.

As used herein, the term "sense strand" refers to the strand of a double-stranded ribonucleic acid that includes a region substantially complementary to the antisense strand (as defined herein).

The term "alternating modification" refers to a modification pattern in which nucleotides are modified with 2'-O-methyl (2'-OMe) and 2'-fluoro (2'-F) alternately according to the nucleotide arrangement of a double-stranded ribonucleic acid. For example, for the antisense strand of an siRNA, odd-numbered positions (*i.e.,* positions 1, 3, 5, 7, 11, 13, 15, 17, 19, 21, and 23) are modified with 2'-O-methyl, while even-numbered positions (*i.e.,* positions 2, 4, 6, 8, 10, 12, 14, 16, 18, 20, and 22) are modified with 2'-fluoro. For the sense strand complementary to the antisense strand, the positions corresponding to those modified with 2'-O-methyl on the antisense strand are modified with 2'-fluoro, while the positions corresponding to those modified with 2'-fluoro on the antisense strand are modified with 2'-O-methyl on the sense strand.

For RNA interference (RNAi), inhibition of a target gene is achieved by loading the antisense strand of an siRNA into the AGO2 protein to form an RNA-induced silencing complex (RISC), which then cleaves the mRNA transcript of the gene. Loading of the RNA-induced silencing complex (RISC) requires phosphorylation at the 5' end of the antisense strand (5'-phosphate). 5' phosphorylation may occur naturally in cells through the action of cleavage and polyadenylation factor I subunit 1 (C1p1), or may be introduced through chemical synthesis. The term "natural 5' phosphorylation" or "simple direct 5' phosphorylation" refers to phosphorylation at the 5' end of the antisense strand of siRNA occurring in the intracellular environment, rather than being introduced through chemical synthesis.

As used herein, the term "conjugate moiety" refers to a GalNAc derivative attached to an oligonucleotide. In some cases, the conjugate moiety comprises a targeting moiety (also referred to as a ligand), and optionally a linker; for example, a GalNAc derivative attached to an oligonucleotide via a divalent, trivalent, or tetravalent branched linker arm, respectively; for another example, a GalNAc derivative attached to an oligonucleotide via a monovalent linker arm. In most cases, the terms "ligand" and "conjugate moiety" have meanings well known in the art.

As used herein, the term "inhibiting" is used interchangeably with "reducing", "silencing", "downregulating", "suppressing", and other similar terms, and includes any level of inhibition. As used herein, the term "regulating" in some cases refers to "inhibiting", and those skilled in the art can clearly understand the intended meaning based on the context.

As used herein, the phrase "inhibiting expression of FXI" includes inhibition of expression of any FXI gene (*e.g.,* a mouse FXI gene, a rat FXI gene, a monkey FXI gene, or a human FXI gene) as well as variants (*e.g*., naturally occurring variants) or mutants of the FXI gene. Thus, the FXI gene may be a wild-type FXI gene, a mutant FXI gene, or a transgenic FXI gene in the context of genetically manipulated cells, cell populations, or organisms.

"Inhibiting FXI gene expression" includes any level of inhibition of the FXI gene, *e.g.,* at least partial inhibition of FXI gene expression, such as inhibition of at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, or at least about 99%.

FXI gene expression may be assessed based on the level of any variable associated with FXI gene expression, such as FXI mRNA levels or FXI protein levels. Inhibition of FXI gene expression may be assessed by a reduction in the absolute or relative level of one or more of these variables compared to a control level. The control level may be any type of control level utilized in the art, *e.g.,* a pre-dose baseline level or a level determined from a similar subject, cell, or sample that is untreated or treated with a control (*e.g.,* a buffer-only control or an inert control).

As used herein, the term "regulating" in some cases may carry the same meaning as "inhibiting". Accordingly, "regulating LPA gene expression" may mean "inhibiting LPA gene expression." Those skilled in the art can clearly understand the specific technical meaning based on the context.

As used herein, a "patient" or "subject" is intended to include humans or non-human animals, preferably mammals, such as monkeys. More preferably, the subject or patient is a human.

As used herein, an "FXI-associated disease" is intended to include any disease associated with the FXI gene or protein. The disease may be caused, for example, by overproduction of the FXI protein, by mutations in the FXI gene, by abnormal cleavage of the FXI protein, or by abnormal interaction between FXI and other proteins or other endogenous or exogenous substances. Exemplary FXI-associated diseases include thrombotic diseases, including deep vein thrombosis, venous or arterial thrombosis, ischemic stroke, pulmonary embolism, myocardial infarction, stroke, Alzheimer's disease, and thrombosis associated with chronic kidney disease or end-stage renal disease.

As used herein, a "therapeutically effective amount" is intended to include an amount of an RNAi agent that, when administered to a patient for treating an FXI-associated disease, is sufficient to treat the disease (*e.g.,* by attenuating, ameliorating, or maintaining the existing disease or one or more symptoms of the disease). The "therapeutically effective amount" may vary depending on the RNAi agent, how the agent is administered, the disease and its severity, medical history, age, weight, family history, genetic makeup, stage of pathological processes mediated by the expression of FXI, type of prior or concomitant therapy (if any), and other individual characteristics of the patient to be treated.

As used herein, a "prophylactically effective amount" is intended to include an amount of an RNAi agent that, when administered to a subject who has not yet experienced or exhibited symptoms of an FXI-associated disease, but who may be susceptible to the disease, is sufficient to prevent or ameliorate the disease or one or more symptoms of the disease. Amelioration of the disease includes slowing the progression of the disease or reducing the severity of later-onset disease. The "prophylactically effective amount" may vary depending on the RNAi agent, how the agent is administered, degree of risk of the disease, medical history, age, weight, family history, genetic makeup, type of prior or concomitant therapy (if any), and other individual characteristics of the patient to be treated.

The "therapeutically effective amount" or "prophylactically effective amount" also includes an amount of an RNAi agent that produces a desired local or systemic effect at a reasonable benefit/risk ratio applicable to any treatment. The RNAi agent used in the methods of the present disclosure may be administered in an amount sufficient to achieve a reasonable benefit/risk ratio applicable to such treatment.

As used herein, the term "sample" includes a collection of similar fluids, cells, or tissues isolated from a subject, as well as fluids, cells, or tissues present in the subject. Examples of biological fluids include blood, serum and serous fluid, plasma, cerebrospinal fluid, ocular fluid, lymph, urine, saliva, *etc.* Tissue samples may include samples from tissues, organs, or localized regions. For example, samples may be derived from specific organs, parts of organs, or fluids or cells within these organs. In certain examples, samples may be derived from the liver (*e.g.,* the entire liver or certain segments of the liver, or certain types of cells in the liver, such as hepatocytes). In a preferred example, a "sample derived from a subject" refers to blood or plasma drawn from the subject. In other examples, a "sample derived from a subject" refers to liver tissue (or subcomponents thereof) derived from the subject.

As used herein, unless otherwise specified, when referring to any nucleotide position on any strand of an siRNA motif, siRNA derivative, siRNA conjugate, siRNA duplex, *etc.,* the positions are indicated in the 5' to 3' direction.

In one aspect, the present disclosure provides an siRNA duplex for targeted regulation of FXI gene expression, comprising a sense strand and an antisense strand that form a reverse complementary duplex region, wherein the antisense strand comprises at least 15, 16, 17, 18, or 19 consecutive nucleotides complementary to a target sequence having the nucleotide sequence TTTCAGGATGATTTTCTTATATCAAGT (SEQ ID NO: 773) or ACTTCAGTTTCTGGTGAATGT (SEQ ID NO: 774).

The target sequence described above is derived from *Homo sapiens* coagulation factor XI (F11), transcript variant 1, with NCBI accession number NM_000128.4.

In some embodiments, the antisense strand comprises a fragment of at least 15, 16, 17, 18, or 19 consecutive nucleotides from any one of the sequences of SEQ ID NOs: 114 to 118, 121 to 123, and 139, or a modified fragment thereof.

In some embodiments, the reverse complementary duplex region is 17 to 21 bp in length, such as 20 or 21 bp in length.

In some embodiments, the sense strand comprises a fragment of at least 15, 16, 17, 18, or 19 consecutive nucleotides from any one of the sequences of SEQ ID NOs: 8 to 12, 15 to 17, and 33, or a modified fragment thereof.

In some embodiments, the sense strand and the antisense strand each independently comprise 19 to 23 nucleotides; preferably, the sense strand comprises 19 to 21 nucleotides, and the antisense strand comprises 21 to 23 nucleotides.

In some embodiments, the siRNA duplex comprises any one oligonucleotide duplex selected from the following pairings of a sense strand and an antisense strand, or a combination of at least two thereof, optionally wherein the sense strand and the antisense strand each independently comprise at least one modified nucleotide:
(1) the sense strand has the sequence of SEQ ID NO: 8 or a fragment thereof; and the antisense strand has the sequence of SEQ ID NO: 114 or a fragment thereof;
(2) the sense strand has the sequence of SEQ ID NO: 9 or a fragment thereof; and the antisense strand has the sequence of SEQ ID NO: 115 or a fragment thereof;
(3) the sense strand has the sequence of SEQ ID NO: 10 or a fragment thereof; and the antisense strand has the sequence of SEQ ID NO: 116 or a fragment thereof;
(4) the sense strand has the sequence of SEQ ID NO: 11 or a fragment thereof; and the antisense strand has the sequence of SEQ ID NO: 117 or a fragment thereof;
(5) the sense strand has the sequence of SEQ ID NO: 12 or a fragment thereof; and the antisense strand has the sequence of SEQ ID NO: 118 or a fragment thereof;
(6) the sense strand has the sequence of SEQ ID NO: 15 or a fragment thereof; and the antisense strand has the sequence of SEQ ID NO: 121 or a fragment thereof;
(7) the sense strand has the sequence of SEQ ID NO: 16 or a fragment thereof; and the antisense strand has the sequence of SEQ ID NO: 122 or a fragment thereof;
(8) the sense strand has the sequence of SEQ ID NO: 17 or a fragment thereof; and the antisense strand has the sequence of SEQ ID NO: 123 or a fragment thereof;
(9) the sense strand has the sequence of SEQ ID NO: 33 or a fragment thereof; and the antisense strand has the sequence of SEQ ID NO: 139 or a fragment thereof.

In some embodiments, the siRNA duplex is an RNAi agent for inhibiting FXI gene expression.

In some embodiments, the sense strand differs from any one of the sequences of SEQ ID NOs: 8 to 12, 15 to 17, and 33 by 1 to 3 nucleotides.

In some embodiments, the antisense strand differs from any one of the sequences of SEQ ID NOs: 114 to 118, 121 to 123, and 139 by 1 to 3 nucleotides.

In some embodiments, the number of nucleotides in the sense strand is either the same as or different from the number of nucleotides in the antisense strand.

In some embodiments, the sense strand comprises 19 nucleotides and the antisense strand comprises 19 nucleotides.

In some embodiments, the sense strand comprises 19 nucleotides and the antisense strand comprises 21 nucleotides.

In some embodiments, the sense strand comprises 20 nucleotides and the antisense strand comprises 20 nucleotides.

In some embodiments, the sense strand comprises 20 nucleotides and the antisense strand comprises 22 nucleotides.

In some embodiments, the sense strand comprises 21 nucleotides and the antisense strand comprises 21 nucleotides.

In some embodiments, the sense strand comprises 21 nucleotides and the antisense strand comprises 23 nucleotides.

In some embodiments, the sense strand comprises 23 nucleotides and the antisense strand comprises 23 nucleotides.

In some embodiments, the sense strand and the antisense strand each independently comprise at least one modified nucleotide.

In some embodiments, the at least one modified nucleotide is any one selected from the group consisting of a deoxy-nucleotide, a 3'-deoxy-thymidine (dT) nucleotide, a 2'-O-methyl modified nucleotide, a 2'-fluoro-modified nucleotide, a 2'-deoxy-modified nucleotide, a locked nucleotide, an unlocked nucleotide, a conformationally restricted nucleotide, a constrained ethyl nucleotide, an abasic nucleotide, a 2'-amino-modified nucleotide, a 2'-O-allyl-modified nucleotide, a 2'-C-alkyl-modified nucleotide, a 2'-hydroxy-modified nucleotide, a 2'-methoxyethyl-modified nucleotide, a 2'-O-alkyl-modified nucleotide, a morpholino nucleotide, a phosphoramidate, a nucleotide comprising a non-natural base, a tetrahydropyran-modified nucleotide, a 1,5-anhydrohexitol-modified nucleotide, a cyclohexenyl-modified nucleotide, a nucleotide comprising a phosphorothioate group, a nucleotide comprising a methylphosphonate group, a nucleotide comprising a 5'-phosphate group, and a nucleotide comprising a 5'-phosphate mimic, or a combination of at least two thereof.

In some embodiments, at least one strand comprises a 3' overhang of at least one nucleotide.

In some embodiments, at least one strand comprises a 3' overhang of at least two nucleotides.

In some embodiments, the duplex region of the siRNA duplex is 15 to 30 base pairs in length.

In some embodiments, the duplex region of the siRNA duplex is 17 to 25 base pairs in length.

In some embodiments, the duplex region of the siRNA duplex is 19 to 23 base pairs in length.

In some embodiments, the duplex region of the siRNA duplex is 21 base pairs in length.

In some embodiments, each strand has 15 to 30 nucleotides.

In some embodiments, each strand has 19 to 25 nucleotides.

In some embodiments, all the modification of nucleotides on the sense and antisense strands are at the 2' position of the ribose of the nucleotide.

In some embodiments, the modification at the 2' position of the ribose of the nucleotide is any one selected from 2'-O-methyl modification, 2'-methoxyethyl modification, 2'-fluoro modification, 2'-benzyloxy modification, 2'-methylcarbonylamino modification, and 2'-pyridinylmethoxy modification, or a combination thereof.

In some embodiments, each of the modification at the 2' position of the ribose of the nucleotide is selected from a combination of 2'-O-methyl modification and 2'-fluoro modification.

In some embodiments, each of the modification at the 2' position of the ribose of the nucleotide is selected from an alternating combination of 2'-O-methyl modification and 2'-fluoro modification.

In some embodiments, each of the chemical modification at the 2' position of the ribose of the nucleotide is as follows: on the antisense strand, all odd-numbered positions are modified with 2'-O-methyl, and all even-numbered positions are modified with 2'-fluoro; on the sense strand, positions corresponding to 2'-O-methyl modifications on the antisense strand are modified with 2'-fluoro, and positions corresponding to 2'-fluoro modifications on the antisense strand are modified with 2'-O-methyl.

In some embodiments, when the sense strand is an odd number of nucleotides in length, each of the modification pattern at the 2' position of the ribose of the nucleotide is as follows: on the sense strand, all odd-numbered positions are modified with 2'-fluoro, and all even-numbered positions are modified with 2'-O-methyl; and on the antisense strand, all odd-numbered positions are modified with 2'-O-methyl, and all even-numbered positions are modified with 2'-fluoro.

In some embodiments, when the sense strand is an even number of nucleotides in length, each of the modification pattern at the 2' position of the ribose of the nucleotide is as follows: on the sense strand, all odd-numbered positions are modified with 2'-O-methyl, and all even-numbered positions are modified with 2'-fluoro; and on the antisense strand, all odd-numbered positions are modified with 2'-O-methyl, and all even-numbered positions are modified with 2'-fluoro.

In some embodiments, nucleotides are linked to one another via a 3',5'-phosphodiester bond.

In some embodiments, nucleotides are linked to one another via a 3',5'-phosphorothioate bond.

In some embodiments, there is a 3',5'-phosphorothioate bond between nucleotides at positions 1 to 2 from the 3' and/or 5' ends of the sense strand and/or the antisense strand of the siRNA derivative. For example, in some embodiments, a stereochemically pure 3',5'-phosphorothioate bond is formed. In some embodiments, one, two, or three 3',5'-phosphorothioate bonds may be present among nucleotides at positions 1 to 4 from the 5' end of the sense strand and/or the antisense strand, and one, two, or three 3',5'-phosphorothioate bonds may be present among nucleotides at positions 1 to 4 from the 3' end of the antisense strand.

In some embodiments, a 3',5'-phosphorothioate linkage is introduced between nucleotides at positions 1 and 2 and between nucleotides at positions 2 and 3 from the 5' end of the sense strand; a 3',5'-phosphorothioate linkage is introduced between nucleotides at positions 1 and 2 and between nucleotides at positions 2 and 3 from the 5' end of the antisense strand, and a 3',5'-phosphorothioate linkage is introduced between nucleotides at positions 1 and 2 and between nucleotides at positions 2 and 3 from the 3' end of the antisense strand. In some specific embodiments, the sense strand and/or the antisense strand have alternating 2'-F and 2'-OMe modifications.

In some embodiments, the siRNA duplex comprises any one oligonucleotide duplex selected from the following pairings of a sense strand and an antisense strand, or a combination of at least two thereof:
(1) the sense strand has the sequence of SEQ ID NO: 220; the antisense strand has the sequence of SEQ ID NO: 326;
(2) the sense strand has the sequence of SEQ ID NO: 221; the antisense strand has the sequence of SEQ ID NO: 327;
(3) the sense strand has the sequence of SEQ ID NO: 222; the antisense strand has the sequence of SEQ ID NO: 328;
(4) the sense strand has the sequence of SEQ ID NO: 223; the antisense strand has the sequence of SEQ ID NO: 329;
(5) the sense strand has the sequence of SEQ ID NO: 224; the antisense strand has the sequence of SEQ ID NO: 330;
(6) the sense strand has the sequence of SEQ ID NO: 227; the antisense strand has the sequence of SEQ ID NO: 333;
(7) the sense strand has the sequence of SEQ ID NO: 228; the antisense strand has the sequence of SEQ ID NO: 334;
(8) the sense strand has the sequence of SEQ ID NO: 229; the antisense strand has the sequence of SEQ ID NO: 335;
(9) the sense strand has the sequence of SEQ ID NO: 245; the antisense strand has the sequence of SEQ ID NO: 351.

In some embodiments, the oligonucleotides have alternating 2'-fluoro and 2'-O-methyl modifications or template modifications.

In some embodiments, phosphorylation of the 5' carbon atom of the nucleotide glycoside at the 5' end of the modified antisense strand includes, but is not limited to, the following 5'-phosphorylation groups: 5'-vinylphosphonate group (5'-E-VP), 5'-methylphosphonate group (5'-MP), 5'-C-methylphosphonate group, 5'-phosphorothioate group (5'-PS), and 5'-phosphate group (5'-P). The structures of the modified nucleotides are as follows: 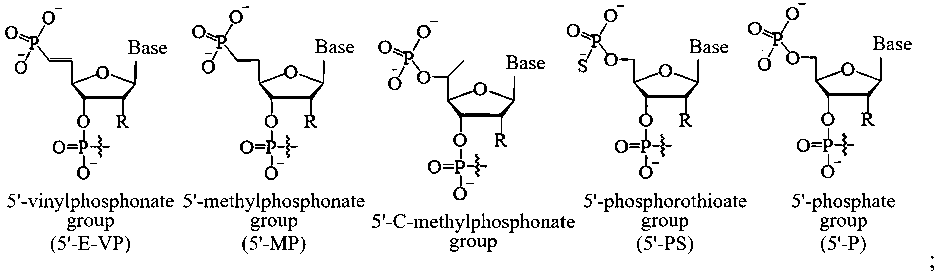
wherein R is hydrogen, hydroxyl, amino, C₁₋₄ alkyl, aryl, C₁₋₄ alkoxy, C₁₋₄ alkylcarbonylamino, or halogen;
the base is any one selected from adenine, guanine, cytosine, thymine, and uracil.

In some embodiments, the antisense strand of the siRNA derivative has any one of modification patterns A to H as follows:

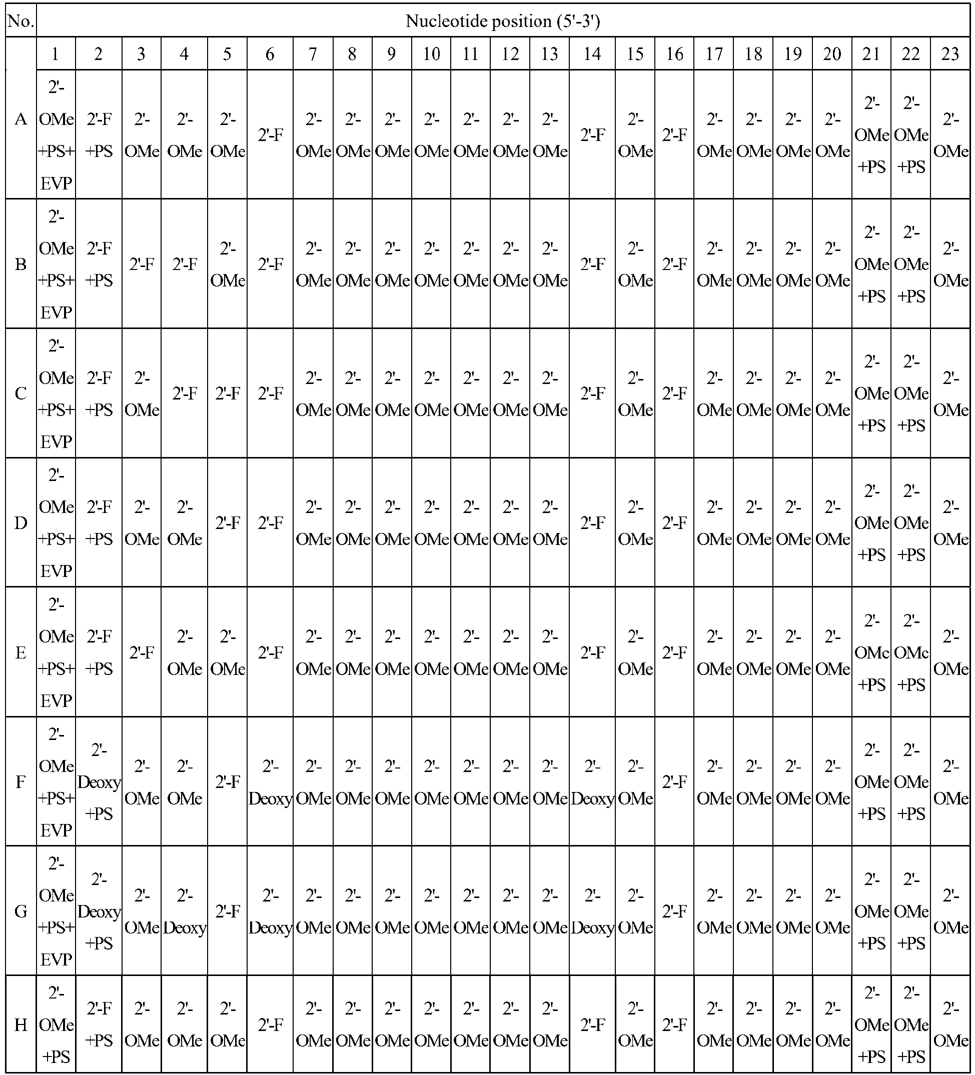

and/or, the sense strand adopts any one of modification patterns a to c as follows: 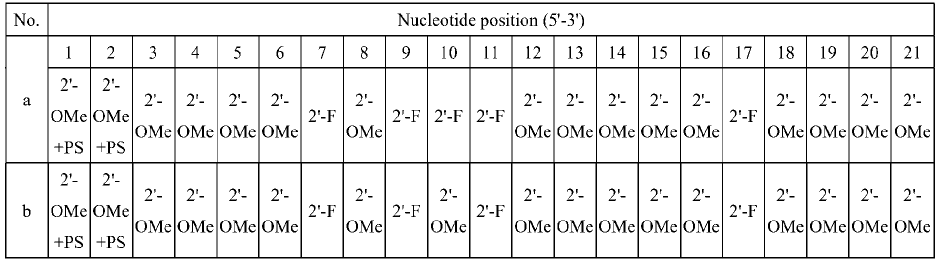 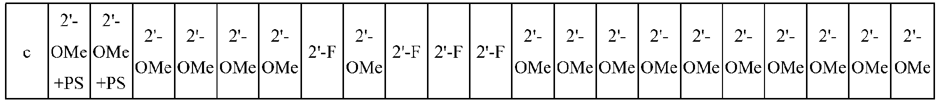
wherein 2'-OMe represents a 2'-O-methyl modification; 2'-F represents a 2'-fluoro modification; 2'-Deoxy represents a 2'-deoxy modification; PS indicates that, starting from the 5' end, the nucleotide at the position is linked to the adjacent nucleotide on the right via a phosphorothioate bond; EVP represents a 5'-vinyl-(E)-phosphonate modification.

In some embodiments, the antisense strand is modified according to modification pattern A, and the sense strand is modified according to modification pattern a.

In some embodiments, the antisense strand is modified according to modification pattern B, and the sense strand is modified according to modification pattern a.

In some embodiments, the antisense strand is modified according to modification pattern C, and the sense strand is modified according to modification pattern a.

In some embodiments, the antisense strand is modified according to modification pattern C, and the sense strand is modified according to modification pattern b.

In some embodiments, the antisense strand is modified according to modification pattern D, and the sense strand is modified according to modification pattern b.

In some embodiments, the antisense strand is modified according to modification pattern E, and the sense strand is modified according to modification pattern b.

In some embodiments, the antisense strand is modified according to modification pattern F, and the sense strand is modified according to modification pattern b.

In some embodiments, the antisense strand is modified according to modification pattern G, and the sense strand is modified according to modification pattern b.

In some embodiments, the antisense strand is modified according to modification pattern H, and the sense strand is modified according to modification pattern c.

In some embodiments, the antisense strand of the siRNA derivative has any one of modification patterns A' to G' as follows:

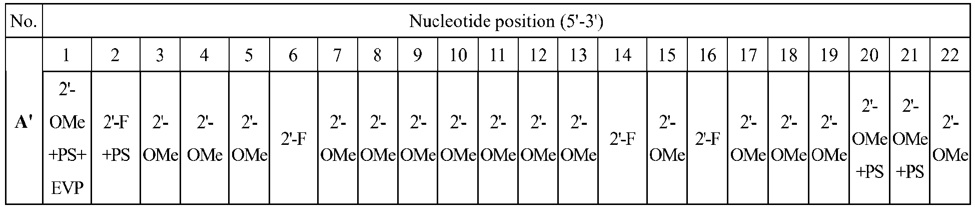

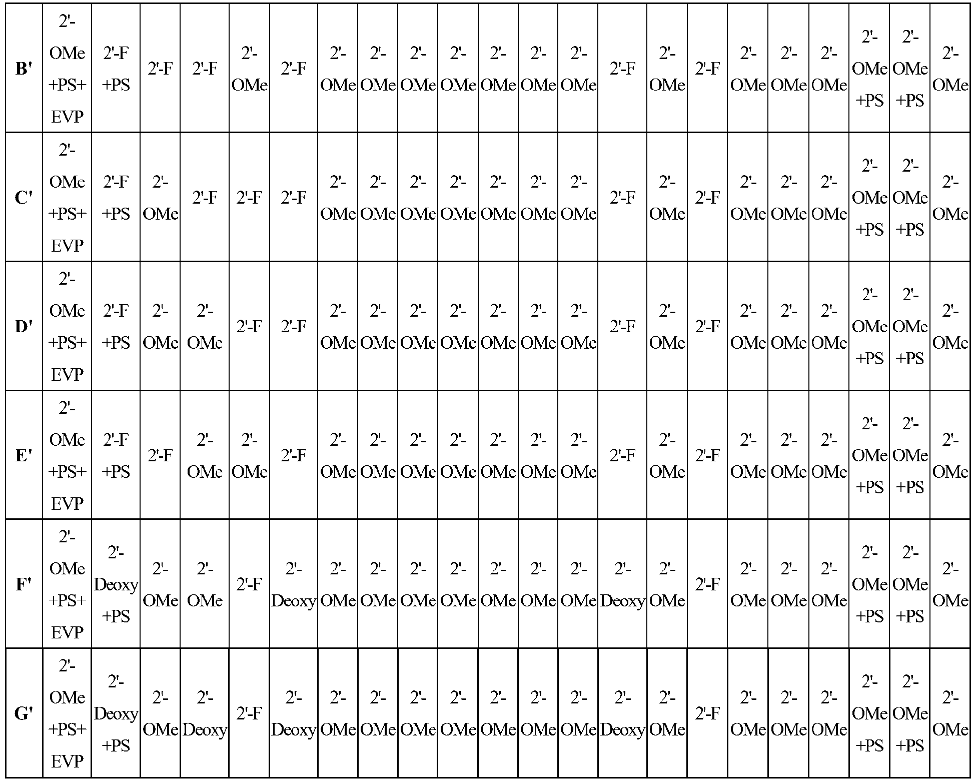

and/or, the sense strand adopts modification pattern a' or b' as follows: 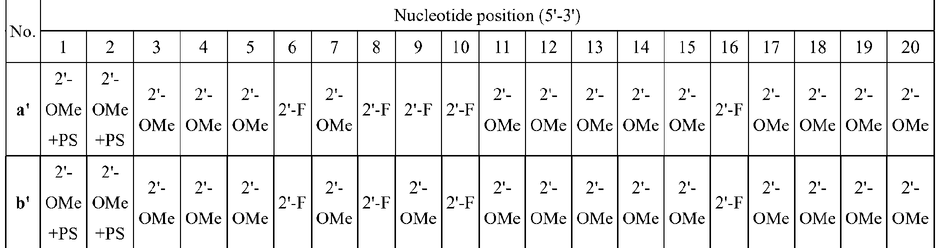
wherein 2'-OMe represents a 2'-O-methyl modification; 2'-F represents a 2'-fluoro modification; 2'-Deoxy represents a 2'-deoxy modification; PS indicates that, starting from the 5' end, the nucleotide at the position is linked to the adjacent nucleotide on the right via a phosphorothioate bond; EVP represents a 5'-vinyl-(E)-phosphonate modification.

In some embodiments, the antisense strand is modified according to modification pattern A', and the sense strand is modified according to modification pattern a'.

In some embodiments, the antisense strand is modified according to modification pattern B', and the sense strand is modified according to modification pattern a'.

In some embodiments, the antisense strand is modified according to modification pattern C', and the sense strand is modified according to modification pattern a'.

In some embodiments, the antisense strand is modified according to modification pattern C', and the sense strand is modified according to modification pattern b'.

In some embodiments, the antisense strand is modified according to modification pattern D', and the sense strand is modified according to modification pattern b'.

In some embodiments, the antisense strand is modified according to modification pattern E', and the sense strand is modified according to modification pattern b'.

In some embodiments, the antisense strand is modified according to modification pattern F', and the sense strand is modified according to modification pattern b'.

In some embodiments, the antisense strand is modified according to modification pattern G', and the sense strand is modified according to modification pattern b'.

In some embodiments, the siRNA duplex comprises any one oligonucleotide duplex selected from the following pairings of a sense strand and an antisense strand, or a combination of at least two thereof:
(1) the sense strand has the sequence of SEQ ID NO: 426; and the antisense strand has the sequence of SEQ ID NO: 552;
(2) the sense strand has the sequence of SEQ ID NO: 427; and the antisense strand has the sequence of SEQ ID NO: 553;
(3) the sense strand has the sequence of SEQ ID NO: 429; and the antisense strand has the sequence of SEQ ID NO: 555;
(4) the sense strand has the sequence of SEQ ID NO: 430; and the antisense strand has the sequence of SEQ ID NO: 556;
(5) the sense strand has the sequence of SEQ ID NO: 431; and the antisense strand has the sequence of SEQ ID NO: 557;
(6) the sense strand has the sequence of SEQ ID NO: 435; and the antisense strand has the sequence of SEQ ID NO: 561;
(7) the sense strand has the sequence of SEQ ID NO: 439; and the antisense strand has the sequence of SEQ ID NO: 565;
(8) the sense strand has the sequence of SEQ ID NO: 440; and the antisense strand has the sequence of SEQ ID NO: 566;
(9) the sense strand has the sequence of SEQ ID NO: 442; and the antisense strand has the sequence of SEQ ID NO: 568;
(10) the sense strand has the sequence of SEQ ID NO: 443; and the antisense strand has the sequence of SEQ ID NO: 569;
(11) the sense strand has the sequence of SEQ ID NO: 444; and the antisense strand has the sequence of SEQ ID NO: 570;
(12) the sense strand has the sequence of SEQ ID NO: 445; and the antisense strand has the sequence of SEQ ID NO: 571;
(13) the sense strand has the sequence of SEQ ID NO: 446; and the antisense strand has the sequence of SEQ ID NO: 572;
(14) the sense strand has the sequence of SEQ ID NO: 447; and the antisense strand has the sequence of SEQ ID NO: 573;
(15) the sense strand has the sequence of SEQ ID NO: 448; and the antisense strand has the sequence of SEQ ID NO: 574;
(16) the sense strand has the sequence of SEQ ID NO: 449; and the antisense strand has the sequence of SEQ ID NO: 575;
(17) the sense strand has the sequence of SEQ ID NO: 450; and the antisense strand has the sequence of SEQ ID NO: 576;
(18) the sense strand has the sequence of SEQ ID NO: 454; and the antisense strand has the sequence of SEQ ID NO: 580;
(19) the sense strand has the sequence of SEQ ID NO: 455; and the antisense strand has the sequence of SEQ ID NO: 581;
(20) the sense strand has the sequence of SEQ ID NO: 456; and the antisense strand has the sequence of SEQ ID NO: 582;
(21) the sense strand has the sequence of SEQ ID NO: 468; and the antisense strand has the sequence of SEQ ID NO: 594;
(22) the sense strand has the sequence of SEQ ID NO: 471; and the antisense strand has the sequence of SEQ ID NO: 597;
(23) the sense strand has the sequence of SEQ ID NO: 477; and the antisense strand has the sequence of SEQ ID NO: 603;
(24) the sense strand has the sequence of SEQ ID NO: 478; and the antisense strand has the sequence of SEQ ID NO: 604;
(25) the sense strand has the sequence of SEQ ID NO: 479; and the antisense strand has the sequence of SEQ ID NO: 605;
(26) the sense strand has the sequence of SEQ ID NO: 480; and the antisense strand has the sequence of SEQ ID NO: 606;
(27) the sense strand has the sequence of SEQ ID NO: 529; and the antisense strand has the sequence of SEQ ID NO: 655;
(28) the sense strand has the sequence of SEQ ID NO: 530; and the antisense strand has the sequence of SEQ ID NO: 656;
(29) the sense strand has the sequence of SEQ ID NO: 532; and the antisense strand has the sequence of SEQ ID NO: 658.

In some embodiments, the nucleotides at positions 2 to 8 from the 5' end of the antisense strand are each independently a modified nucleotide, wherein the modified nucleotide is UNA or GNA; or, the nucleotides at positions 2 to 8 from the 5' end of the antisense strand are each independently DNA, wherein the structures of UNA and GNA are as follows: 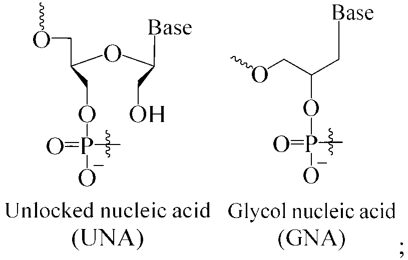 wherein the base is any one selected from adenine, guanine, cytosine, thymine, and uracil.

In some embodiments, the nucleotide at the 3' end of the sense strand or the antisense strand is an inverted modified nucleotide, or the nucleotide at position 6 or 7 from the 5' end of the sense strand is an inverted modified nucleotide, wherein the modified nucleotide is selected from dAinV, dTinV, dGinV, and dCinV having the following structures:

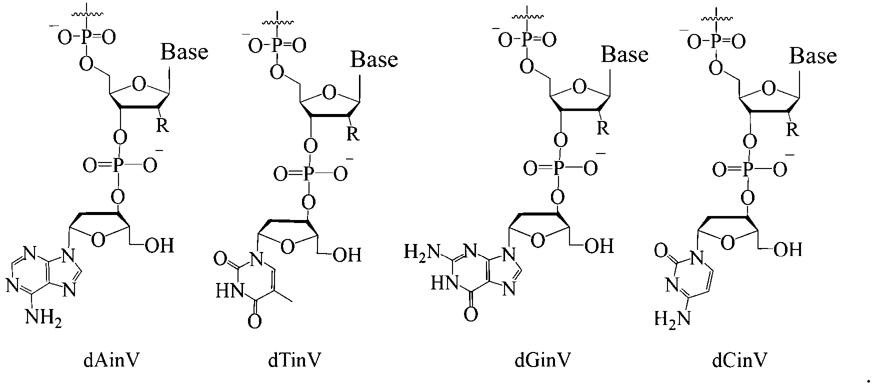

In some embodiments, the siRNA duplex comprises any one oligonucleotide duplex selected from the following pairings of a sense strand and an antisense strand, or a combination of at least two thereof:
(1) the sense strand has the sequence of SEQ ID NO: 677; and the antisense strand has the sequence of SEQ ID NO: 686;
(2) the sense strand has the sequence of SEQ ID NO: 678; and the antisense strand has the sequence of SEQ ID NO: 687;
(3) the sense strand has the sequence of SEQ ID NO: 683; and the antisense strand has the sequence of SEQ ID NO: 693;
(4) the sense strand has the sequence of SEQ ID NO: 680; and the antisense strand has the sequence of SEQ ID NO: 689.

The double-stranded ribonucleic acid or double-stranded ribonucleic acid derivative of the present disclosure may optionally be linked to one or more conjugate moieties to form a double-stranded ribonucleic acid conjugate. The conjugate moiety may be attached to the sense strand, the antisense strand, or both strands at the 3' end, the 5' end, or both ends. For example, the conjugate moiety may be linked to the sense strand. In a preferred example, the conjugate moiety is linked to the 3' end of the sense strand. In one example, the conjugate moiety has an arbitrary GalNAc structure.

The present disclosure provides an siRNA conjugate comprising an siRNA duplex described herein and a conjugate moiety linked to the siRNA duplex.

In some embodiments, the conjugate moiety is linked to the 3' end or 5' end of the sense strand, preferably to the 3' end.

In some embodiments, the conjugate moiety comprises one or more GalNAc or derivatives thereof attached via a bivalent or trivalent branched linker arm.

Typically, the conjugate moiety comprises at least one pharmaceutically acceptable targeting moiety, or further comprises a linker, wherein the siRNA, the linker, and the targeting moiety are sequentially linked. In some embodiments, the number of targeting moieties is 1 to 6. In some embodiments, the number of targeting moieties is 2 to 4. In some embodiments, the number of targeting moieties is 3. The conjugate moiety may be covalently or non-covalently linked to the siRNA molecule, wherein the linkage site may be located at the 3' end or 5' end of the sense strand, at the 5' end of the antisense strand, or within the internal sequence of the siRNA. In some embodiments, the linkage site is located at the 3' end of the sense strand of the siRNA.

In some embodiments, the pharmaceutically acceptable targeting moiety may be a conventional ligand in the field of siRNA delivery, such as various ligands described in WO2009082607A2, the contents of which are incorporated herein by reference in their entirety.

In some embodiments, each ligand is independently selected from a ligand capable of binding to a cell surface receptor. In some embodiments, at least one ligand is a ligand capable of binding to a hepatocyte surface receptor. In some embodiments, at least one ligand is a ligand capable of binding to a mammalian cell surface receptor. In some embodiments, at least one ligand is a ligand capable of binding to a human hepatocyte surface receptor. In some embodiments, at least one ligand is a ligand capable of binding to a hepatocyte surface asialoglycoprotein receptor (ASGPR). The types of these ligands are well known to those skilled in the art, and their general function is to specifically bind to receptors on the surface of target cells, thereby mediating the delivery of siRNA conjugated to the ligand into the target cells.

In some embodiments, the pharmaceutically acceptable targeting moiety in the siRNA conjugate may be galactose or N-acetylgalactosamine, wherein the galactose or N-acetylgalactosamine molecule may be monovalent, divalent, trivalent, or tetravalent. That is, when the siRNA molecule forms an siRNA conjugate with a conjugate moiety containing a galactose or N-acetylgalactosamine molecule as the targeting moiety, the molar ratio of the siRNA molecule to the galactose or N-acetylgalactosamine molecule in the siRNA conjugate is 1:1, 1:2, 1:3, or 1:4. In some embodiments, when the siRNA is conjugated with a conjugate moiety containing N-acetylgalactosamine, the N-acetylgalactosamine molecule is trivalent.

The targeting moiety may be linked to the siRNA molecule via a suitable linker, and those skilled in the art may select an appropriate linker based on the specific type of targeting moiety. The types of these linkers and targeting moieties, as well as the modes of attachment to siRNA may be found in WO2015006740A2, the contents of which are incorporated herein by reference in their entirety.

In some embodiments, the structure of the conjugate moiety is, for example, as follows: 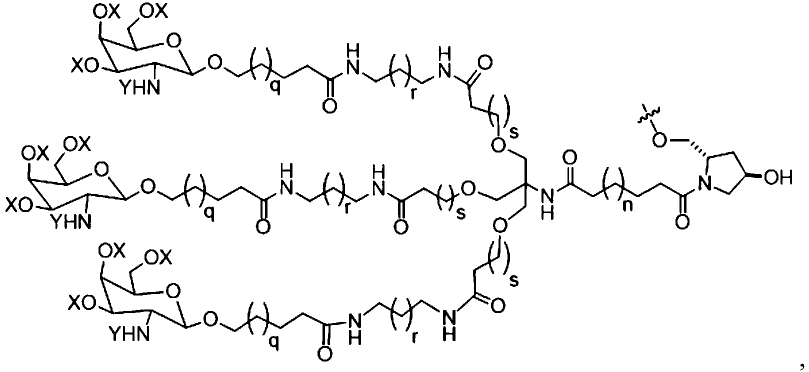 wherein X is a hydroxyl protecting group or H, the hydroxyl protecting group being selected from acetyl, benzoyl, and isobutyryl; Y is an amino protecting group or H, the amino protecting group being selected from formyl, acetyl, propionyl, n-butyryl, and isobutyryl; n is an integer from 0 to 20; q, r, and s are independently integers from 1 to 7.

In some embodiments, the structure of the conjugate moiety is, for example, as follows:

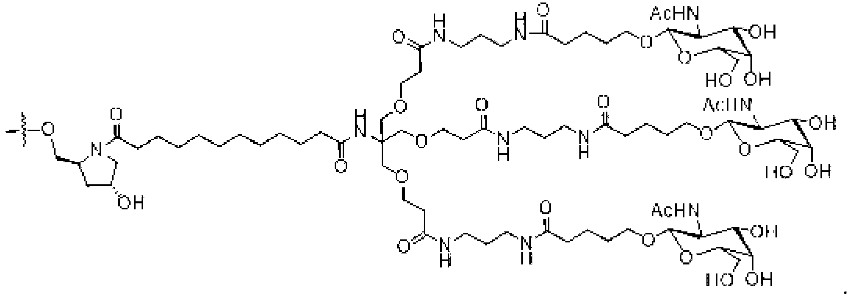

In some embodiments, the structure of the conjugate moiety is, for example, as follows: 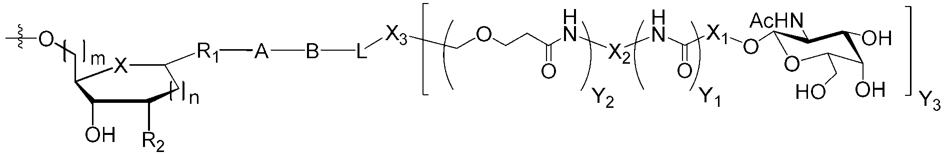
wherein X is oxygen, -N(Y)-, or sulfur;
Y is C₁₋₄ alkyl or C₆₋₁₀ aryl;
R₁ is oxygen or sulfur;
R₂ is hydrogen, -NH₂, C₁₋₄ alkyl, C₆₋₁₀ aryl, C₁₋₄ alkoxy, or halogen;
A is -(CH₂)ₐ-, -(CH₂CH₂O)_{b}-, -((CH₂)_{c}NHCO)_{d}-, or -((CH₂)_{c}CONH)_{d}-, wherein a is an integer from 1 to 15, b is an integer from 1 to 7, c is an integer from 1 to 7, and d is an integer from 1 to 5;
B is -(CH₂)ₑ-, wherein e is an integer from 0 to 7;
L is -CONH- or -NHCO-;
X₁ is -(CH₂)_{f}- or -(CH₂CH₂O)_{f}CH₂-, wherein f is an integer from 1 to 5;
X₂ is -(CH₂)_{g}-, wherein g is an integer from 1 to 6;
X₃ is oxygen or sulfur;
Y₁ is 0 or 1;
Y₂ is 0, 1, or 2;
when Y₃ is 1, X₄ is CH₂; when Y₃ is 2, X₄ is CH; when Y₃ is 3, X₃ is carbon;
m is an integer from 0 to 4;
n is an integer from 0 to 4.

In some embodiments, the conjugate moiety has, for example, any one of the following structures: 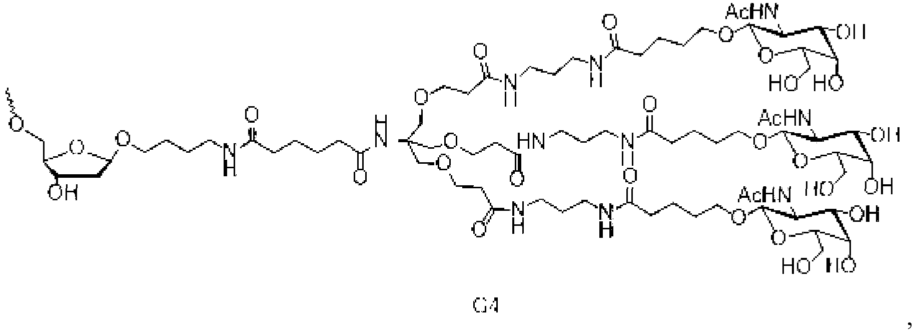 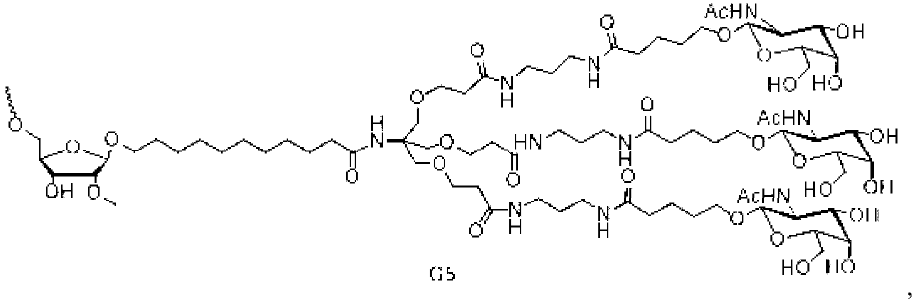 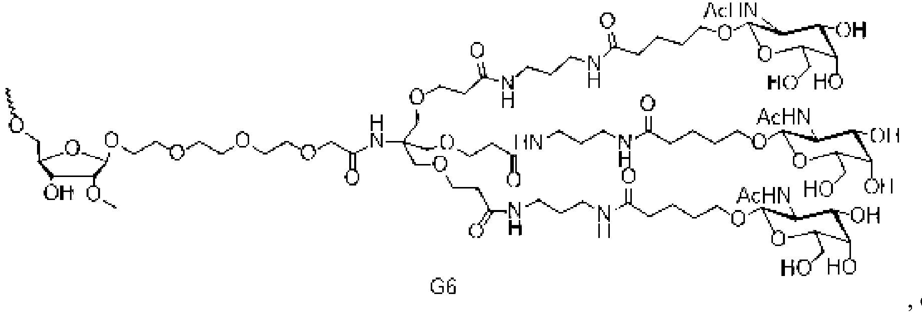 or

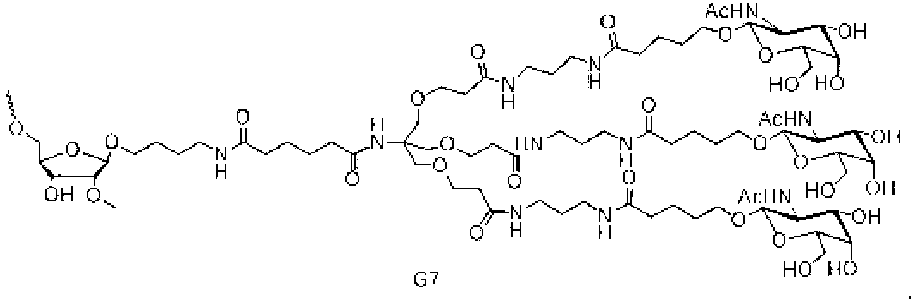

In some embodiments, the siRNA conjugate has, for example, any one of the following structures: 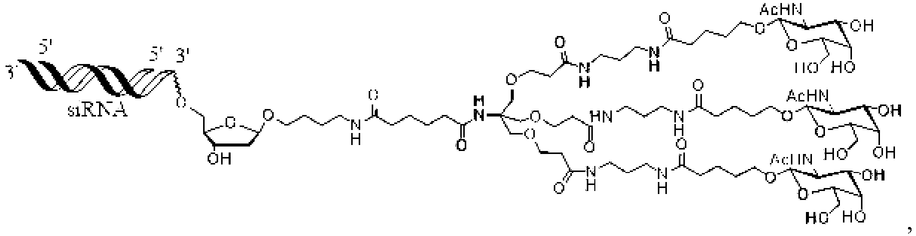 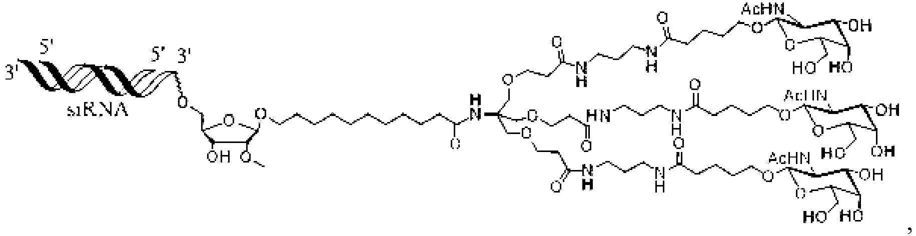 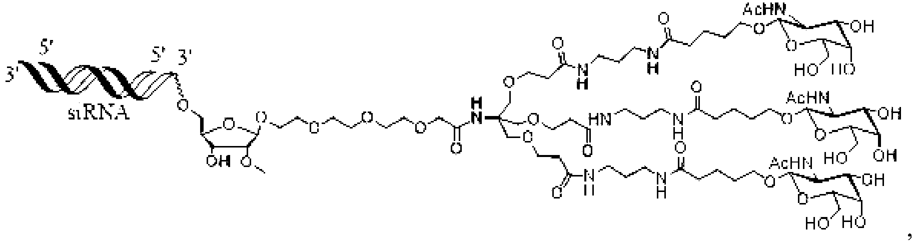 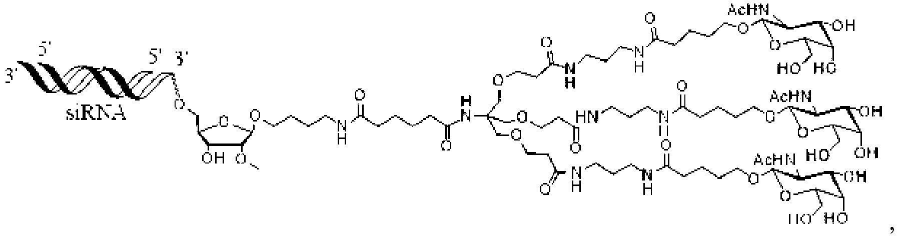 and

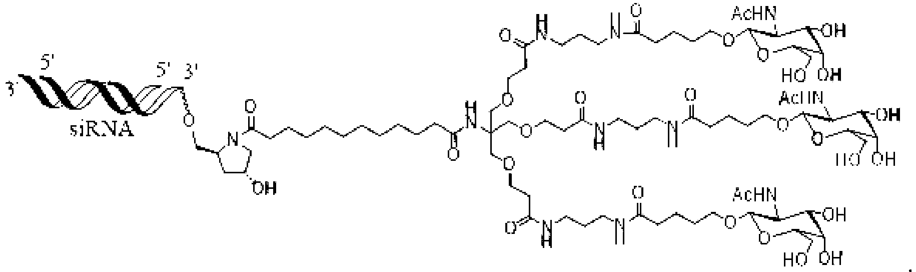

In some embodiments, the conjugate moiety has, for example, the following structure: 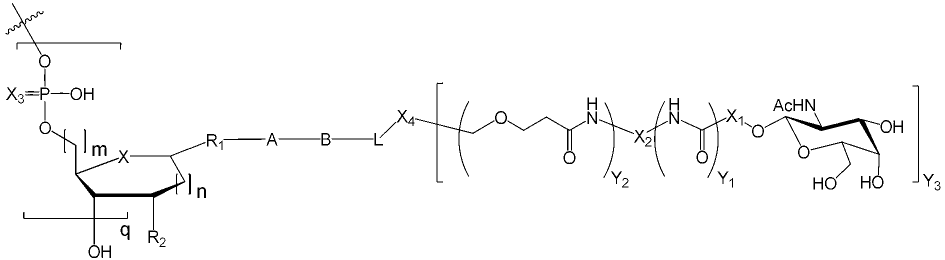
wherein X is oxygen, -N(Y)-, or sulfur;
Y is C₁₋₄ alkyl or C₆₋₁₀ aryl;
R₁ is oxygen or sulfur;
R₂ is hydrogen, -NH₂, C₁₋₄ alkyl, C₆₋₁₀ aryl, C₁₋₄ alkoxy, or halogen;
A is -(CH₂)ₐ-, -(CH₂CH₂O)_{b}-, -((CH₂)_{c}NHCO)_{d}-, or -((CH₂)_{c}CONH)_{d}-, wherein a is an integer from 1 to 15, b is an integer from 1 to 7, c is an integer from 1 to 7, and d is an integer from 1 to 5;
B is -(CH₂)ₑ-, wherein e is an integer from 0 to 7;
L is -CONH- or -NHCO-;
X₁ is -(CH₂)_{f}- or -(CH₂CH₂O)_{f}CH₂-, wherein f is an integer from 1 to 5;
X₂ is -(CH₂)_{g}-, wherein g is an integer from 1 to 6;
X₃ is oxygen or sulfur;
Y₁ is 0 or 1;
Y₂ is 0, 1, or 2;
when Y₃ is 1, X₄ is CH₂; when Y₃ is 2, X₄ is CH; when Y₃ is 3, X₃ is carbon;
m is an integer from 0 to 4;
n is an integer from 0 to 4;
q is an integer from 0 to 4.

In some embodiments, the conjugate moiety has, for example, any one of the following structures: 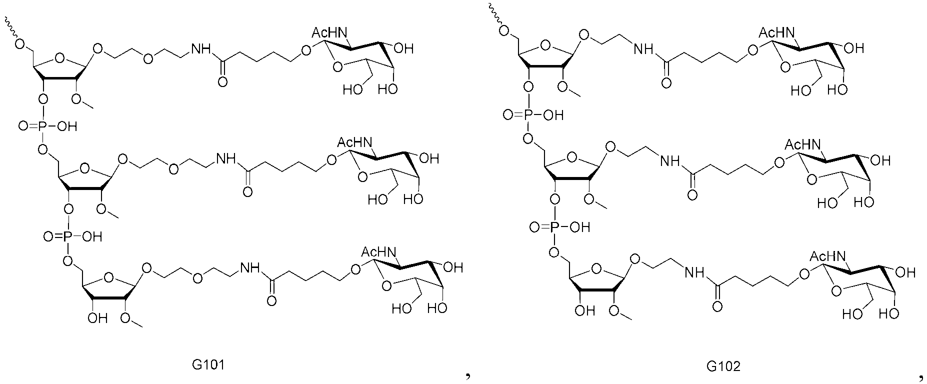 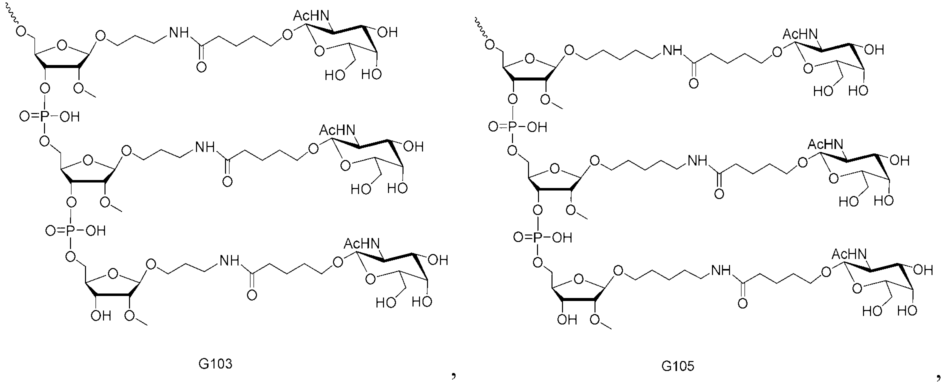 and

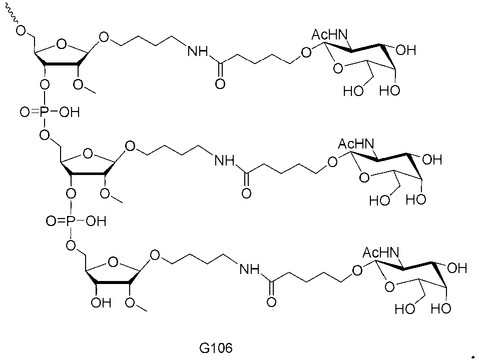

In some embodiments, the siRNA conjugate of the present disclosure has, for example, any one of the following structures: 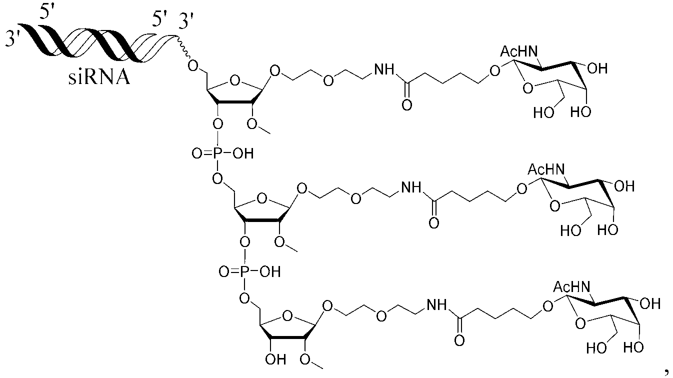 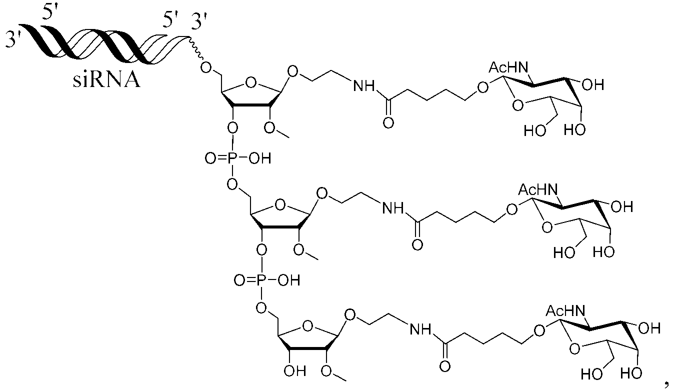 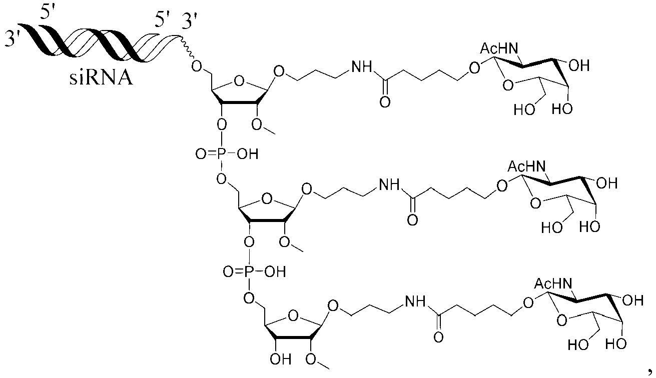 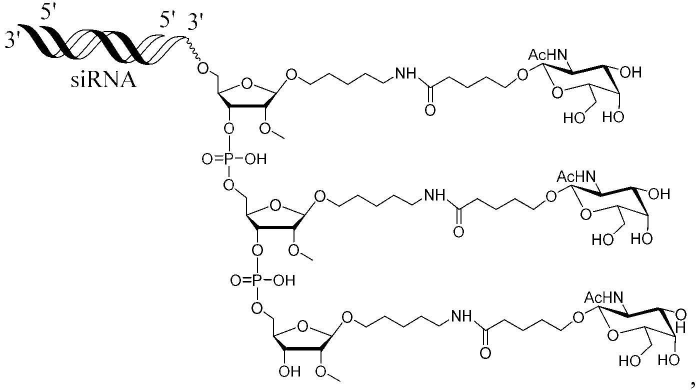 or

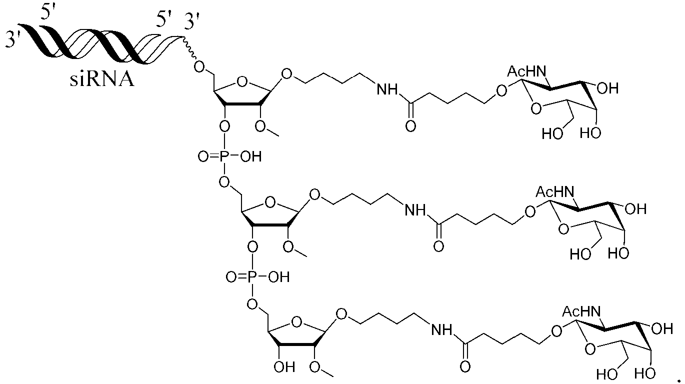

In some embodiments, the conjugate moiety is G101.

In some embodiments, the siRNA conjugate comprises any one oligonucleotide duplex selected from the following pairings of a sense strand and an antisense strand, or a combination of at least two thereof:
(1) the sense strand has the sequence of SEQ ID NO: 695; and the antisense strand has the sequence of SEQ ID NO: 732;
(2) the sense strand has the sequence of SEQ ID NO: 706; and the antisense strand has the sequence of SEQ ID NO: 743;
(3) the sense strand has the sequence of SEQ ID NO: 707; and the antisense strand has the sequence of SEQ ID NO: 744;
(4) the sense strand has the sequence of SEQ ID NO: 708; and the antisense strand has the sequence of SEQ ID NO: 745;
(5) the sense strand has the sequence of SEQ ID NO: 710; and the antisense strand has the sequence of SEQ ID NO: 747;
(6) the sense strand has the sequence of SEQ ID NO: 713; and the antisense strand has the sequence of SEQ ID NO: 750;
(7) the sense strand has the sequence of SEQ ID NO: 714; and the antisense strand has the sequence of SEQ ID NO: 751;
(8) the sense strand has the sequence of SEQ ID NO: 715; and the antisense strand has the sequence of SEQ ID NO: 752;
(9) the sense strand has the sequence of SEQ ID NO: 719; and the antisense strand has the sequence of SEQ ID NO: 756;
(10) the sense strand has the sequence of SEQ ID NO: 720; and the antisense strand has the sequence of SEQ ID NO: 757;
(11) the sense strand has the sequence of SEQ ID NO: 721; and the antisense strand has the sequence of SEQ ID NO: 758;
(12) the sense strand has the sequence of SEQ ID NO: 722; and the antisense strand has the sequence of SEQ ID NO: 759;
(13) the sense strand has the sequence of SEQ ID NO: 723; and the antisense strand has the sequence of SEQ ID NO: 760;
(14) the sense strand has the sequence of SEQ ID NO: 724; and the antisense strand has the sequence of SEQ ID NO: 761;
(15) the sense strand has the sequence of SEQ ID NO: 725; and the antisense strand has the sequence of SEQ ID NO: 762;
(16) the sense strand has the sequence of SEQ ID NO: 728; and the antisense strand has the sequence of SEQ ID NO: 765;
(17) the sense strand has the sequence of SEQ ID NO: 729; and the antisense strand has the sequence of SEQ ID NO: 766.

The present disclosure also provides a nucleic acid-protein complex comprising a duplex region of the siRNA duplex or siRNA conjugate described above or an antisense strand of the duplex region, and a nuclease.

In the present disclosure, the term "nucleic acid-protein complex" refers to an RNA-induced silencing complex (RISC) formed by binding of siRNA to Argonaute protein (AGO). The siRNA duplex is then unwound into a sense strand and an antisense strand. The sense strand is degraded, while the antisense strand (guide strand) directs the RISC to bind to a target mRNA homologous to the siRNA via base pairing. The RISC possesses nuclease activity, and the siRNA-guided RISC cleaves the homologous single-stranded mRNA, resulting in loss of mRNA function, *i.e.,* preventing translation into protein and thereby achieving gene "silencing".

The present disclosure also provides a recombinant vector comprising a nucleic acid molecule encoding the siRNA described herein.

In some embodiments, the vector backbone of the recombinant vector is selected from recombinant virus-derived circular RNA vectors, tRNA scaffolds, rRNA scaffolds, and chimeric tRNA/pre-miRNA vectors.

The present disclosure also provides a recombinant cell comprising the siRNA or recombinant vector described above.

In some embodiments, the recombinant cell is selected from *Rhodovulum sulfidophilum* and ribonuclease III-deficient *Corynebacterium glutamicum.*

As used herein, the term "recombinant vector" preferably refers to a vector comprising a regulatory sequence operably linked to a nucleotide sequence encoding the sense strand contained in the nucleic acid molecule of the present disclosure. The term "recombinant cell" refers to a cell into which at least one recombinant vector capable of expressing the nucleic acid molecule or at least one strand of the nucleic acid molecule has been introduced.

The present disclosure also provides a method of preparing the siRNA described herein, comprising culturing the recombinant cell described above, or directly obtaining the siRNA by means of chemical synthesis and mixing.

The present disclosure also provides a pharmaceutical composition comprising the siRNA duplex or the corresponding siRNA conjugate, and a pharmaceutically acceptable carrier.

In one example, provided herein is a pharmaceutical composition comprising the siRNA described herein and a pharmaceutically acceptable carrier. The siRNA-containing pharmaceutical composition may be used for treating a disease or disorder associated with the expression or activity of the FXI gene, such as hypertension. Such pharmaceutical compositions are formulated based on the mode of delivery. One example is a composition formulated for systemic administration via parenteral delivery, *e.g.,* via subcutaneous (s.c.) injection. Another example is a composition formulated for direct delivery into the brain parenchyma, *e.g.,* via infusion into the brain, such as by continuous pump infusion.

The pharmaceutical composition comprising the siRNA of the present disclosure may be, for example, a solution with or without a buffer, or a composition comprising a pharmaceutically acceptable carrier. Such compositions include, for example, aqueous or crystalline compositions, liposomal formulations, micellar formulations, emulsions, and gene therapy vectors.

In the method of the present disclosure, the siRNA may be administered in a solution. A free siRNA may be administered in an unbuffered solution, *e.g.,* in saline or in water. Alternatively, the free siRNA may also be administered in a suitable buffered solution. The buffered solution may comprise acetate, citrate, prolamin, carbonate, or phosphate, or any combination thereof. In a preferred example, the buffered solution is phosphate-buffered saline (PBS). The pH and osmolarity of the buffered solution comprising the siRNA may be adjusted such that it is suitable for administration to a subject.

In some examples, the buffered solution further comprises an agent for controlling the osmolarity of the solution, such that the osmolarity is maintained at a desired value, *e.g.,* at the physiological value of human plasma. Solutes that may be added to the buffered solution to control the osmolarity include, but are not limited to, proteins, peptides, amino acids, non-metabolized polymers, vitamins, ions, sugars, metabolites, organic acids, lipids, or salts. In some examples, the agent for controlling the osmolarity of the solution is a salt. In certain examples, the agent for controlling the osmolarity of the solution is sodium chloride or potassium chloride.

The pharmaceutical composition of the present disclosure may be administered at a dose sufficient to inhibit FXI gene expression. Generally, suitable doses of the siRNA of the present disclosure range from about 0.001 to about 200.0 mg/kg body weight per day, typically from about 1 to 50 mg/kg body weight per day. For example, the siRNA (*e.g*., dsRNA) may be administered at a single dose of about 0.01, 0.05, 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, 5, 5.1, 5.2, 5.3, 5.4, 5.5, 5.6, 5.7, 5.8, 5.9, 6, 6.1, 6.2, 6.3, 6.4, 6.5, 6.6, 6.7, 6.8, 6.9, 7, 7.1, 7.2, 7.3, 7.4, 7.5, 7.6, 7.7, 7.8, 7.9, 8, 8.1, 8.2, 8.3, 8.4, 8.5, 8.6, 8.7, 8.8, 8.9, 9, 9.1, 9.2, 9.3, 9.4, 9.5, 9.6, 9.7, 9.8, 9.9, 10, 10.5, 11, 11.5, 12, 12.5, 13, 13.5, 14, 14.5, 15, 15.5, 16, 16.5, 17, 17.5, 18, 18.5, 19, 19.5, 20, 20.5, 21, 21.5, 22, 22.5, 23, 23.5, 24, 24.5, 25, 25.5, 26, 26.5, 27, 27.5, 28, 28.5, 29, 29.5, 30, 31, 32, 33, 34, 34, 35, 36, 37, 38, 39, 40, 41, 42, 43, 44, 45, 46, 47, 48, 49, or about 50 mg/kg.

The pharmaceutical composition may be administered once daily, or multiple times at various intervals ranging from 1 to 365 days, or the siRNA may be administered as two, three, or more sub-doses at appropriate intervals within a year, or even using continuous infusion or delivery via a controlled-release formulation. In such cases, the amount of siRNA in each sub-dose must be correspondingly reduced in order to achieve the total daily dose. Dosage units may also be compounded for delivery over several days, *e.g.,* using a conventional sustained-release formulation that provides sustained release of siRNA over a period of several days. Sustained-release formulations are well known in the art and are particularly useful for delivering agents to specific sites, and thus may be used with the agents of the present disclosure. In such examples, the dosage unit contains a corresponding plurality of daily doses.

In other examples, a single dose of the pharmaceutical composition may be long-acting, such that subsequent doses are administered at intervals of no more than 3, 4, or 5 days, or no more than 1, 2, 3, or 4 weeks. In some examples of the present disclosure, a single dose of the pharmaceutical composition of the present disclosure is administered once weekly. In other examples of the present disclosure, a single dose of the pharmaceutical composition of the present disclosure is administered once every two months.

Those skilled in the art will appreciate that certain factors may influence the dosage and dosing schedule required for effective treatment of a subject, including, but not limited to, the severity of the disease or disorder, prior treatments, the general health and/or age of the subject, and other existing diseases. Furthermore, treating a subject with a therapeutically effective amount of the composition may involve a single treatment or a series of treatments. As described elsewhere herein, the effective dose and *in vivo* half-life for each siRNA encompassed by the present disclosure may be estimated using conventional methods or on the basis of *in vivo* testing using suitable animal models.

The pharmaceutical composition of the present disclosure may be administered in various ways depending upon whether local or systemic treatment is desired and upon the area to be treated. Administration may be topical *(e.g.,* via a transdermal patch); pulmonary; *e.g.,* via inhalation or insufflation of a powder or aerosol, including via a nebulizer; intratracheal; intranasal; epidermal and transdermal, oral, or parenteral. Parenteral administration includes intravenous, intra-arterial, subcutaneous, intraperitoneal, or intramuscular injection or infusion; subdermal administration, *e.g.,* via an implantable device; or intracranial administration, *e.g.,* intraparenchymal, intrathecal, or intraventricular administration.

The siRNA for use in the compositions and methods of the present disclosure may be formulated for delivery in membranous molecular assemblies, such as liposomes or micelles. As used herein, the term "liposome" refers to a vesicle composed of amphipathic lipids arranged in at least one lipid bilayer (*e.g.,* one bilayer or a plurality of bilayers), having an external membrane formed by lipophilic materials and an internal aqueous compartment. The lipophilic material separates the internal aqueous compartment from the external aqueous environment, which typically does not contain the siRNA composition (although in some instances it may). Liposomes are useful for transferring and delivering active ingredients to sites of action. Because liposomal membranes are structurally similar to biological membranes, when liposomes are applied to a tissue, the liposomal bilayer fuses with the bilayer of the cell membrane. As the fusion of liposomes with cells proceeds, the internal aqueous contents, including the siRNA, are delivered into the cell, where the siRNA can specifically bind to a target RNA and mediate RNA interference (RNAi).

Liposomes containing siRNA may be prepared by a variety of methods. In one example, a lipid component of the liposome is dissolved in a detergent such that micelles are formed with the lipid component. For example, the lipid component may be an amphipathic cationic lipid or a lipid conjugate. The detergent may have a high critical micelle concentration and may be nonionic. Exemplary detergents include cholate, CHAPS, octyl glucoside, deoxycholate, and lauroyl sarcosinate. The siRNA formulation is then added to the micelles containing the lipid component. The cationic groups on the lipid interact with the siRNA and condense around the siRNA to form liposomes. After condensation, the detergent is removed, *e.g.,* by dialysis, to obtain the corresponding liposomal formulation of the siRNA.

The siRNA, such as the siRNA duplex of the present disclosure, may be fully encapsulated in a lipid formulation (*e.g*., a lipid nanoparticle (LNP) or another nucleic acid-lipid particle).

As used herein, the term "LNP" refers to a stable nucleic acid-lipid particle. The LNP comprises a cationic lipid, a non-cationic lipid, and a lipid that prevents aggregation of the particles (*e.g.,* a PEG-lipid conjugate). LNPs are extremely useful for synthetic applications, as they exhibit a prolonged circulation lifetime following intravenous (i.v.) injection and accumulate at distal sites (*e.g.,* sites physically separated from the site of administration).

In one example, the mass ratio of lipid to siRNA duplex is about 1:1 to about 50:1, about 1:1 to about 25:1, about 3:1 to about 15:1, about 4:1 to about 10:1, about 5:1 to about 9:1, or about 6:1 to about 9:1.

In some preferred embodiments, the lipid nanoparticle comprises a cationic lipid, a neutral lipid, a structured lipid, and a polymer-conjugated lipid.

In some preferred embodiments, the cationic lipid is a compound having the structure of formula (I), or an N-oxide, a solvate, a pharmaceutically acceptable salt, or a stereoisomer thereof, wherein G₁ is C₁₋₆ alkylene; G₂ is C₂₋₈ alkylene; G₃ is C₁₋₃ alkylene; L₁ is C₆₋₁₅ linear alkyl; L₂ is C₁₂₋₂₅ branched alkyl; for example, YK-009 having the structure of formula (I-I) (see patent CN114044741B, the contents of which are incorporated herein by reference in their entirety, particularly including the general formulas and specific compounds described therein).

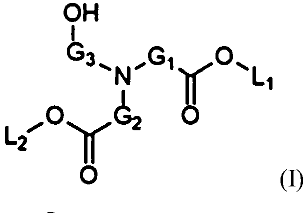

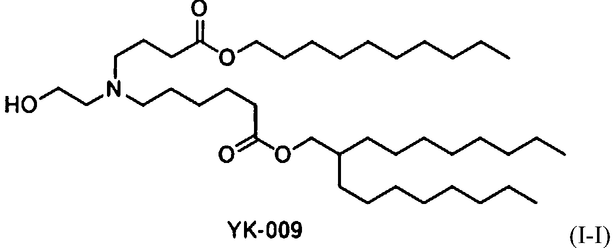

In some preferred embodiments, the cationic lipid is a compound having the structure of formula (II), or an N-oxide, a solvate, a pharmaceutically acceptable salt, or a stereoisomer thereof, wherein G₁ is C₂₋₈ alkylene; G₂ is C₂₋₈ alkylene; L₁ is -C(O)O- or -OC(O)-; L₂ is -C(O)O- or - OC(O)-; R₁ is C₆₋₂₅ linear or branched alkyl; R₂ is C₆₋₂₅ linear or branched alkyl; G₃ is HO(CH₂)₂- or HO(CH₂)₃-; G₄ is HO(CH₂)₂- or HO(CH₂)₃-; L is (CH₂)₂-, -(CH₂)₃-, or -(CH₂)₄-; for example, YK-401 having the structure of formula (II-I), YK-402 having the structure of formula (II-II), or YK-407 having the structure of formula (II-III) (see patent CN115784921B, the contents of which are incorporated herein by reference in their entirety, particularly including the general formulas and specific compounds described therein).

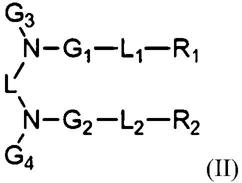

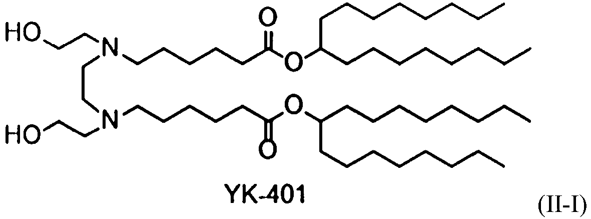

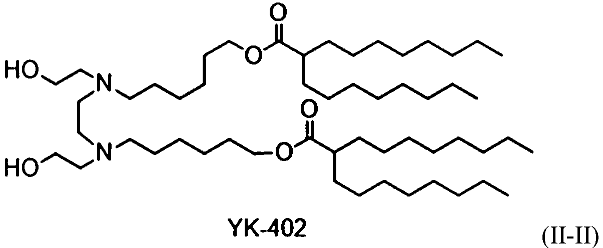

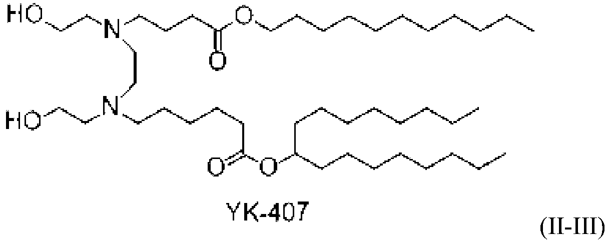

In some preferred embodiments, the cationic lipid is a compound having the structure of formula (III), or an N-oxide, a solvate, a pharmaceutically acceptable salt, or a stereoisomer thereof, wherein G₁ is C₁₋₆ alkylene; G₂ is C₂₋₈ alkylene; R₁ is C₆₋₂₀ linear or branched alkyl; R₂ is C₁₂₋₂₅ branched alkyl; G₃ is HO(CH₂)₂N(CH₃)(CH₂)₂-, HO(CH₂)₂N(CH₂CH₃)(CH₂)₂-, (HO(CH₂)₂)₂N(CH₂)₂-, CH₃O(CH₂)₂N(CH₃)(CH₂)₂-, (CH₃)₂N(CH₂)₃SC(O)O(CH₂)₂-, (CH₃)₂N(CH₂)₃SC(O)-, CH₃NH(CH₂)₂N(CH₃)(CH₂)₂-, or CH₃CH₂NH(CH₂)₂-; for example, YK-201 having the structure of formula (III-I) or YK-202 having the structure of formula (III-II) (see patent CN115677518B, the contents of which are incorporated herein by reference in their entirety, particularly including the general formulas and specific compounds described therein).

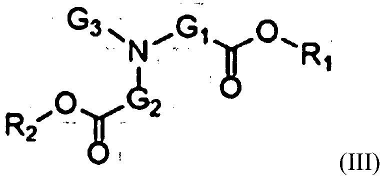

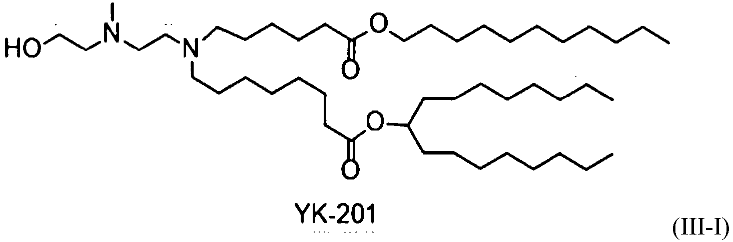

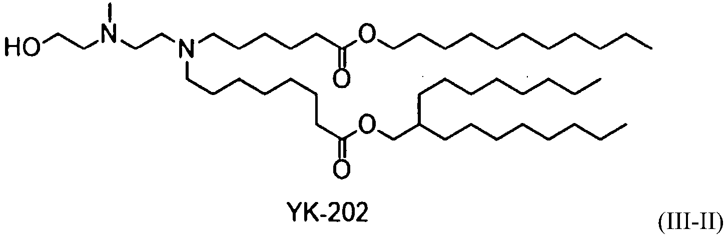

In some preferred embodiments, the cationic lipid is a compound having the structure of formula (IV), or an N-oxide, a solvate, a pharmaceutically acceptable salt, or a stereoisomer thereof, wherein G₁ is C₁₋₈ alkylene; G₂ is C₂₋₈ alkylene; R₁ is C₆₋₂₅ linear or branched alkyl; R₂ is C₁₂₋₂₅ linear or branched alkyl; G₃ is HO(CH₂)₂N(R₃)CH₂CH(OH)CH₂-, wherein R₃ is -CH₃, - CH₂CH₃, or -CH₂CH₂OH; for example, YK-305 having the structure of formula (IV-I) or YK-310 having the structure of formula (IV-II) (see patent CN115745820B, the contents of which are incorporated herein by reference in their entirety, particularly including the general formulas and specific compounds described therein).

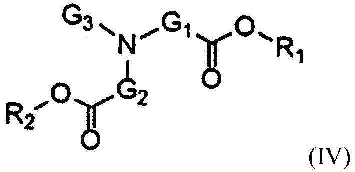

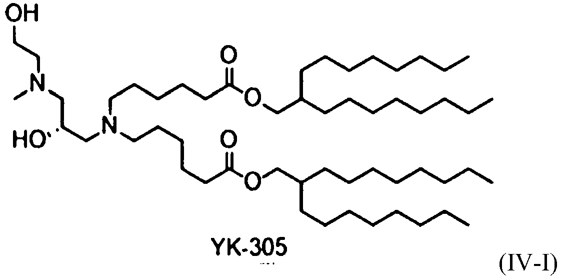

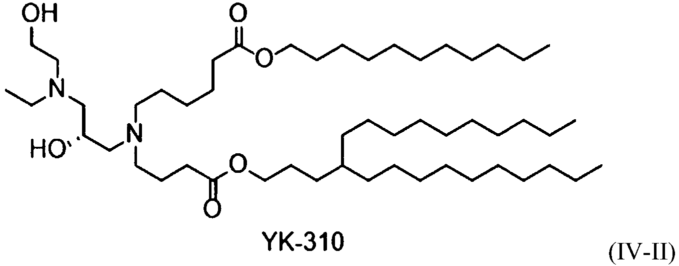

In some preferred embodiments, the cationic lipid is a compound having the structure of formula (V), or an N-oxide, a solvate, a pharmaceutically acceptable salt, or a stereoisomer thereof, wherein G¹ and G² are each independently unsubstituted C₆-C₁₀ alkylene; G³ is unsubstituted C₁-C₁₂ alkylene; R¹ and R² are each independently C₆-C₂₄ alkyl or C₆-C₂₄ alkenyl; R³ is OR⁵, N, - C(=O)OR⁴, -OC(=O)R⁴, or -NR⁵C(=O)R⁴; R⁴ is C₁-C₁₂ hydrocarbyl; and R⁵ is H or C₁-C₆ hydrocarbyl; for example, ALC0315 having the structure of formula (V-I) (see patent CN108368028B, the contents of which are incorporated herein by reference in their entirety, particularly including the general formulas and specific compounds described therein).

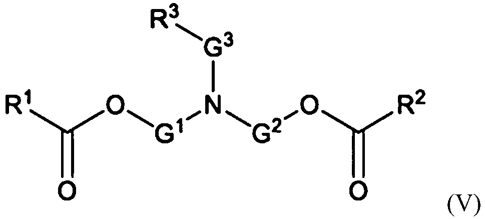

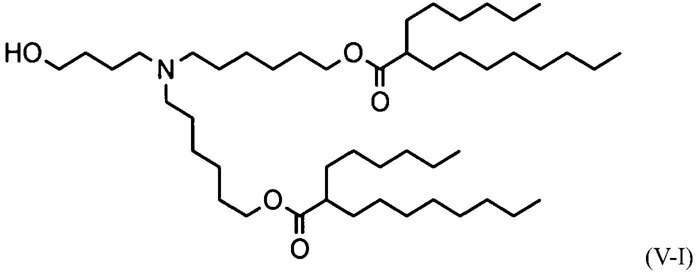

In some preferred embodiments, the cationic lipid is a compound having the structure of formula (VI), or an N-oxide, a solvate, a pharmaceutically acceptable salt, or a stereoisomer thereof, wherein R₄ is selected from -(CH₂)ₙQ and -(CH₂)ₙCHQR; Q is selected from the group consisting of -OR, -OH, -O(CH₂)ₙN(R)₂, -OC(O)R, -CX₃, -CN, -N(R)C(O)R, -N(H)C(O)R, - N(R)S(O)₂R, -N(H)S(O)₂R, -N(R)C(O)N(R)₂, -N(H)C(O)N(R)₂, -N(H)C(O)N(H)(R), - N(R)C(S)N(R)₂, -N(H)C(S)N(R)₂, -N(H)C(S)N(H)(R), -N(R)S(O)₂R₈, and heterocycle; n is 1, 2, or 3; for example, SM102 having the structure of formula (VI-I) (see patent CN110520409A, the contents of which are incorporated herein by reference in their entirety, particularly including the general formulas and specific compounds described therein).

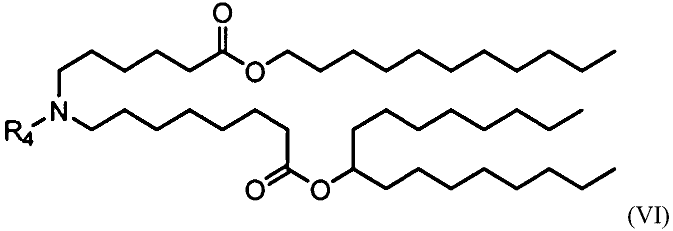

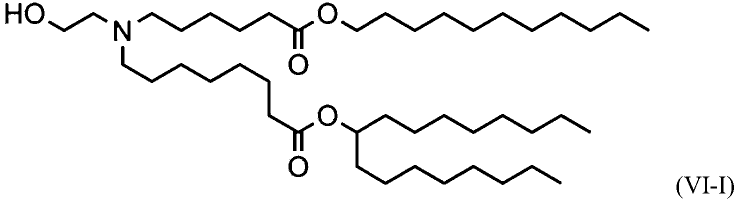

In some preferred embodiments, the cationic lipid is a compound having the structure of formula (VII), or an N-oxide, a solvate, a pharmaceutically acceptable salt, or a stereoisomer thereof (see patent CN102625696B, DLIN-MC3-DMA (as shown in formula (VII)), the contents of which are incorporated herein by reference in their entirety, particularly including the general formulas and specific compounds described therein).

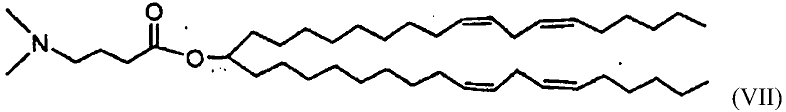

In some more preferred embodiments, the cationic lipid is any one selected from the group consisting of YK-009, YK-407, YK-305, ALC0315, SM102, and DLIN-MC3-DMA, or a combination of at least two thereof.

In some preferred embodiments, the molar ratio of the cationic lipid to the neutral lipid is 1:1 to 10:1.

In some preferred embodiments, the molar ratio of the cationic lipid to the structured lipid is 1:1 to 5:1.

In some preferred embodiments, the molar ratio of the cationic lipid, the neutral lipid, the structured lipid, and the polymer-conjugated lipid is (25 to 65):(5 to 25):(25 to 70):(0.5 to 5).

In some preferred embodiments, the molar ratio of the cationic lipid, the neutral lipid, the structured lipid, and the polymer-conjugated lipid is (25 to 65):(5 to 25):(25 to 45):(0.5 to 5).

In some more preferred embodiments, the molar ratio of the cationic lipid, the neutral lipid, the structured lipid, and the polymer-conjugated lipid is 50:10:38.5:1.5 or 49:10:39.5:1.5.

In some preferred embodiments, the neutral lipid is any one selected from the group consisting of phosphatidylcholine, phosphatidylethanolamine, sphingomyelin, ceramide, sterols, and derivatives thereof, or a combination of at least two thereof.

In some more preferred embodiments, the neutral lipid is any one selected from the group consisting of 1,2-dilinoleoyl-sn-glycero-3-phosphocholine (DLPC), 1,2-dimyristoyl-sn-glycerophosphocholine (DMPC), 1,2-dioleoyl-sn-glycero-3-phosphocholine (DOPC), 1,2-dipalmitoylsn-glycero-3-phosphocholine (DPPC), 1,2-distearoyl-sn-glycero-3-phosphocholine (DSPC), 1,2-diundecanoyl-sn-glycero-phosphocholine (DUPC), 1-palmitoyl-2-oleoyl-sn-glycero-3-phosphocholine (POPC), 1,2-di-O-octadecenyl-sn-glycero-3-phosphocholine (18:0 Diether PC), 1-oleoyl-2-cholesterylhemisuccinoyl-sn-glycero-3-phosphocholine (OChemsPC), 1-hexadecylsn-glycero-3-phosphocholine (C16 Lyso PC), 1,2-dilinolenoyl-sn-glycero-3-phosphocholine, 1,2-diarachidonoyl-sn-glycero-3-phosphocholine, 1,2-didocosahexaenoyl-sn-glycero-3-phosphocholine, 1,2-dioleoyl-sn-glycero-3-phosphoethanolamine (DOPE), 1,2-diphytanoyl-sn-glycero-3-phosphoethanolamine (ME 16.0 PE), 1,2-distearoyl-sn-glycero-3-phosphoethanolamine, 1,2-dilinoleoyl-sn-glycero-3-phosphoethanolamine, 1,2-dilinolenoyl-sn-glycero-3-phosphoethanolamine, 1,2-diarachidonoyl-sn-glycero-3-phosphoethanolamine, 1,2-didocosahexaenoyl-sn-glycero-3-phosphoethanolamine, 1,2-dioleoyl-sn-glycero-3-phospho-rac-(1-glycerol) sodium salt (DOPG), dipalmitoyl phosphatidylglycerol (DPPG), palmitoyl oleoyl phosphatidylethanolamine (POPE), distearoyl-phosphatidyl-ethanolamine (DSPE), dipalmitoyl phosphatidylethanolamine (DPPE), dimyristoyl phosphoethanolamine (DMPE), 1-stearoyl-2-oleoyl-stearoylethanolamine (SOPE), 1-stearoyl-2-oleoyl-phosphatidylcholine (SOPC), sphingomyelin, phosphatidylcholine, phosphatidylethanolamine, phosphatidylserine, phosphatidylinositol, phosphatidic acid, palmitoyl oleoyl phosphatidylcholine, lysophosphatidylcholine, lysophosphatidylethanolamine (LPE), and mixtures thereof, or a combination of at least two thereof.

In some more preferred embodiments, the neutral lipid is DOPE and/or DSPC.

In some preferred embodiments, the structured lipid is any one selected from the group consisting of cholesterol, nonsterols, sitosterol, ergosterol, campesterol, stigmasterol, brassinosterol, tomatidine, ursolic acid, α-tocopherol, and corticosteroids, or a combination of at least two thereof.

In some more preferred embodiments, the structured lipid is cholesterol.

In some preferred embodiments, the polymer-conjugated lipid is any one selected from the group consisting of PEG-modified phosphatidylethanolamine, PEG-modified phosphatidic acid, PEG-modified ceramide, PEG-modified dialkylamine, PEG-modified diacylglycerol, and PEG-modified dialkylglycerol, or a combination of at least two thereof.

In some more preferred embodiments, the polymer-conjugated lipid is any one selected from the group consisting of distearoyl phosphatidylethanolamine polyethylene glycol 2000 (DSPE-PEG2000), dimyristoylglycero-3-methoxypolyethylene glycol 2000 (DMG-PEG2000), and methoxypolyethylene glycol ditetradecylacetamide (ALC-0159), or a combination of at least two thereof.

Examples of pharmaceutical compositions of the present disclosure include, but are not limited to, aqueous formulations, emulsion formulations, and liposome-containing formulations. These compositions may be derived from a variety of components, examples of which include, but are not limited to, preformed liquids, self-emulsifying solids, and self-emulsifying semisolids. For example, formulations targeting the liver are preferred when treating hepatic disorders (*e.g*., hepatocellular carcinoma).

The pharmaceutical formulations of the present disclosure (which may conveniently be presented in unit dosage forms) may be prepared using conventional techniques well known in the pharmaceutical industry. Such techniques include the step of bringing into association the active ingredients with the pharmaceutical carrier(s) or excipient(s). Generally, the formulations are prepared by uniformly and intimately bringing into association the active ingredients with a liquid carrier or a finely divided solid carrier or both, and then, if necessary, shaping the product.

The compositions of the present disclosure may be formulated into any of a variety of possible dosage forms, including, but not limited to, tablets, capsules, gelatin capsules, liquid syrups, soft capsules, suppositories, and enemas. The compositions of the present disclosure may also be formulated as suspensions in aqueous, non-aqueous, or mixed media. Aqueous suspensions may further comprise substances that increase the viscosity of the suspension, including, for example, sodium carboxymethylcellulose, sorbitol, and/or dextran. The suspension may also comprise a stabilizer.

Certain compositions of the present disclosure further incorporate a carrier compound into the formulation. As used herein, the term "carrier compound" or "carrier" may refer to a nucleic acid or analog thereof that is inert *(i.e.,* lacks biological activity per se) but is recognized as a nucleic acid during *in vivo* processes that reduce the bioavailability of a biologically active nucleic acid, for example by degrading the biologically active nucleic acid or facilitating its removal from circulation. Co-administration of a nucleic acid and a carrier compound (typically the latter in excess) may result in a substantial reduction in the amount of nucleic acid recovered in the liver, kidney, or other extracirculatory reservoirs, presumably due to competition between the carrier compound and the nucleic acid for common receptors. For example, when co-administered with polyinosinic acid, dextran sulfate, polycytidylic acid, or 4-acetamido-4'-isothiocyano-stilbene-2,2'-disulfonic acid, the recovery of partially phosphorothioated dsRNA in hepatic tissue may be reduced (Miyao et al., DsRNA Res. Dev., 1995, 5:115-121; Takakura et al., DsRNA & Nucl. Acid Drug Dev., 1996, 6:177-183).

In contrast to carrier compounds, a "pharmaceutical carrier" or "excipient" refers to a pharmaceutically acceptable solvent, suspending agent, or any other pharmaceutically inert vehicle for delivering one or more nucleic acids to an animal. The excipient may be liquid or solid and is selected with reference to the intended mode of administration in order to provide the desired bulk, consistency, and other properties, when combined with the nucleic acid and other components of a particular pharmaceutical composition Typical pharmaceutical carriers include, but are not limited to, binders (*e.g*., pregelatinized corn starch, polyvinylpyrrolidone, or hydroxypropyl methylcellulose); fillers (*e.g.,* lactose and other sugars, microcrystalline cellulose, pectin, gelatin, calcium sulfate, ethyl cellulose, polyacrylates, or calcium hydrogen phosphate); lubricants (*e.g*., magnesium stearate, talc, silica, colloidal silicon dioxide, stearic acid, metallic stearate, hydrogenated vegetable oil, corn starch, polyethylene glycol, sodium benzoate, or sodium acetate); disintegrants (*e.g.,* starch or sodium starch glycolate); and wetting agents *(e.g.,* sodium lauryl sulfate).

Pharmaceutically acceptable organic or inorganic excipients suitable for non-parenteral administration that do not deleteriously react with nucleic acids may also be used to formulate the compositions of the present disclosure. Suitable pharmaceutically acceptable carriers include, but are not limited to, water, saline solutions, alcohols, polyethylene glycol, gelatin, lactose, amylose, magnesium stearate, talc, silicic acid, viscous paraffin, hydroxymethyl cellulose, and polyvinylpyrrolidone.

Formulations suitable for topical administration of nucleic acids may include sterile or non-sterile aqueous solutions or non-aqueous solutions in common solvents such as alcohols, or nucleic acid solutions in liquid or solid oil bases. These solutions may further include buffers, diluents, and other suitable additives. Pharmaceutically acceptable organic or inorganic excipients suitable for non-parenteral administration that do not deleteriously react with nucleic acids may be used.

Suitable pharmaceutically acceptable excipients include, but are not limited to, water, saline solutions, alcohols, polyethylene glycol, gelatin, lactose, amylose, magnesium stearate, talc, silicic acid, viscous paraffin, hydroxymethyl cellulose, and polyvinylpyrrolidone.

The present disclosure also provides methods of treating or preventing diseases and disorders that can be modulated by downregulating the FXI gene expression. Examples include thrombotic diseases, including deep vein thrombosis, venous or arterial thrombosis, ischemic stroke, pulmonary embolism, myocardial infarction, stroke, Alzheimer's disease, and thrombosis associated with chronic kidney disease or end-stage renal disease.

The siRNA of the present disclosure may be administered to a subject using any route of administration known in the art, including, but not limited to, subcutaneous, intravenous, intramuscular, intraocular, intrabronchial, intrapleural, intraperitoneal, intra-arterial, lymphatic, or cerebrospinal administration, and any combination thereof. In a preferred example, the agents are administered subcutaneously.

In another example, the siRNA is administered in combination with an additional therapeutic agent. The siRNA and the additional therapeutic agent may be co-administered in the same composition, *e.g.,* parenterally, or the additional therapeutic agent may be administered as part of a separate composition or by another method described herein.

Examples of additional therapeutic agents include agents known for treating thrombotic diseases or cardiovascular and cerebrovascular diseases, including, for example, anticoagulants, antiplatelet agents, thrombolytic agents for acute myocardial infarction and pulmonary embolism, and other agents. Specifically, the anticoagulants include warfarin, heparin, low-molecular-weight heparins (*e.g.,* enoxaparin), and direct oral anticoagulants (*e.g.,* dabigatran, rivaroxaban, apixaban, edoxaban); the antiplatelet agents include aspirin, clopidogrel, ticagrelor, and prasugrel; the thrombolytic agents for acute myocardial infarction and pulmonary embolism include alteplase, tenecteplase, and streptokinase; the other agents include fondaparinux sodium and bivalirudin.

In one example, the siRNA is administered to a patient, followed by administration of the additional therapeutic agent (or vice versa). In another example, the siRNA and the additional therapeutic agent are administered simultaneously.

The following examples are provided to illustrate the present disclosure but are not intended to limit the scope thereof. Unless otherwise specified, the technical methods used in the examples are conventional methods well known to those skilled in the art, and the raw materials used are commercially available products.

The abbreviations for nucleotides used herein are as follows:
A = adenosine-3'-phosphate
Am = 2'-O-methyladenosine-3'-phosphate
Ams = 2'-O-methyladenosine-3'-phosphorothioate
Af = 2'-fluoroadenosine-3'-phosphate
Afs = 2'-fluoroadenosine-3'-phosphorothioate
dA = 2'-deoxyadenosine-3'-phosphate
dAs = 2'-deoxyadenosine-3'-phosphorothioate
G = guanosine-3'-phosphate
Gm = 2'-O-methylguanosine-3'-phosphate
Gms = 2'-O-methylguanosine-3'-phosphorothioate
Gf = 2'-fluoroguanosine-3'-phosphate
Gfs = 2'-fluoroguanosine-3'-phosphorothioate
dG = 2'-deoxyguanosine-3'-phosphate
dGs = 2'-deoxyguanosine-3'-phosphorothioate
C = cytidine-3'-phosphate
Cm = 2'-O-methylcytidine-3'-phosphate
Cms = 2'-O-methylcytidine-3'-phosphorothioate
Cf = 2'-fluorocytidine-3'-phosphate
Cfs = 2'-fluorocytidine-3'-phosphorothioate
dC = 2'-deoxycytidine-3'-phosphate
dCs = 2'-deoxycytidine-3'-phosphorothioate
U = uridine-3'-phosphate
Um = 2'-O-methyluridine-3'-phosphate
Ums = 2'-O-methyluridine-3'-phosphorothioate
Uf = 2'-fluorouridine-3'-phosphate
Ufs = 2'-fluorouridine-3'-phosphorothioate
dU = 2'-deoxyuridine-3'-phosphate
dUs = 2'-deoxyuridine-3'-phosphorothioate
AmsEVP = 5'-vinyl-(E)-phosphonate-2'-O-methyladenosine-3'-phosphorothioate
UmsEVP = 5'-vinyl-(E)-phosphonate-2'-O-methyluridine-3'-phosphorothioate
dAinV = (3'-3' linkage) 2'-deoxyadenosine-3'-phosphate
dTinV = (3'-3' linkage) 2'-deoxythymidine-3'-phosphate
Agna = adenosine-glycol nucleic acid
Cgna = cytidine-glycol nucleic acid
Ggna = guanosine-glycol nucleic acid
Tgna = thymidine-glycol nucleic acid
Ugna = uridine-glycol nucleic acid

The code rules for siRNA motifs (or motifs), siRNA derivatives, and siRNA conjugates used herein are illustrated as follows:
The motif refers to an unmodified RNA sequence. Sequences in which the sense and antisense strands are 20 and 22 nucleotides in length are distinguished from sequences in which the sense and antisense strands are 21 and 23 nucleotides in length by adding the suffix "s" after the position code, such as compound B00211s.

Sequences with alternating 2'-O-methyl (2'-OMe) and 2'-fluoro (2'-F) modifications are designated by adding the suffix "-AL" after the motif code. For example, the code for the alternately modified B00211 is B00211-AL.

The siRNA derivatives such as those listed in Table 7 of Example 1 involve the use of the modification templates DV25P, DV26P, DV27P, DV29P, DV32P, DV34P, DV39P, DV40P, DV25SP, DV26SP, DV27SP, DV29SP, DV32SP, DV34SP, DV39SP, and DV40SP disclosed herein. The corresponding coding rule involves changing the first letter of the motif code for the siRNA derivative from "B" to "C", followed by addition of the name of the corresponding template for the siRNA derivative after the code. For example, the sequence B00211 modified using the template DV25P is designated as C00211-DV25P.

The siRNA derivatives such as those listed in Table 8 of Example 1 involve the use of the modification templates disclosed herein and may further incorporate anti-off-target and/or inverted modifications. The corresponding coding rule involves changing the first letter of the motif code for the siRNA derivative from "B" to "C", followed by sequential addition of the names of the corresponding template and/or anti-off-target/inverted modifications (if present) after the code. For example, for motif B00211, a derivative obtained using the template DV25P and incorporating the inverted modification InV is designated as C00211-DV25PInVG101.

The siRNA conjugates such as those listed in Table 8 of Example 1 involve the use of the modification templates disclosed herein and may further incorporate anti-off-target or inverted modifications; for siRNA derivatives with an inverted modification on the sense strand, the conjugate moiety is attached to the 5' end of the sense strand; for siRNA derivatives without an inverted modification, the conjugate moiety is attached to the 3' end of the sense strand. The coding rule involves changing the first letter of the motif code from "B" to "D," followed by sequential addition of the names of the corresponding template and anti-off-target/inverted modifications (if present) after the code, and concluding with the name of the conjugate moiety such as "G103" or "G101". For example, for motif B00211, a conjugate obtained using the template DV25P and incorporating the inverted modification InV along with attachment of the conjugate moiety G101 is designated as D00211-DV25PInVG101. See the table below for examples of codes.

Examples of codes for siRNA duplexes (motifs, derivatives, and conjugates) in the present disclosure

| Type of siRNA duplex | siRNA motif | Alternately modified siRNA derivative | Template-modified siRNA derivative | Template-modified and inverted modified siRNA derivative | Template-modified and anti-off-target modified siRNA conjugate |
|---|---|---|---|---|---|
| Examples of codes | B00211 | B00211-AL | C00211-DV25P | C00211-DV25PInV | D00211-DV25PInVG101 |

### Examples

Additional objectives, features, and advantages of the present disclosure will become apparent from the following detailed description. However, it should be understood that the detailed description and specific examples, although indicating specific embodiments of the present disclosure, are provided for illustrative purposes only, since various changes and modifications made within the spirit and scope of the present disclosure will become apparent to those skilled in the art upon reading the detailed description.

Unless otherwise specified, the experimental techniques and methods used in the following examples are conventional methods. For example, experimental methods for which specific conditions are not indicated in the following examples are typically performed under conventional conditions, such as those described in Sambrook et al., Molecular Cloning: A Laboratory Manual (New York: Cold Spring Harbor Laboratory Press, 1989), or according to the conditions recommended by the respective manufacturers. Unless otherwise specified, the materials and reagents used in the examples are commercially available.

Those skilled in the art will appreciate that in various examples of the present disclosure, when the test substance in an experiment is an siRNA conjugate, the experimental data and results (including, but not limited to, inhibition rate, IC₅₀, and IC₄₀) can accordingly reflect the inhibition rate, IC₅₀, IC₄₀, and other parameters of the corresponding siRNA derivative of the siRNA conjugate. There is no obstacle to understanding for those skilled in the art.

### Example 1: Sequence design

### 1. Core sequences

A total of 106 siRNA motifs were designed based on the human FXI mRNA sequence (NM_000128.4) (Table 1). Specifically, APC represents the core sequence siFXIg1 of the most active compound siFXIg1M1SP disclosed in CN113227376B, and was used as a positive control in this study. ANC represents a nonsense sequence and was used as a negative control.

**Table 1: siRNA motifs**

| siRNA ID | Sense strand sequence (5'-3') | SEQ ID NO: | Antisense strand sequence (5'-3') | SEQ ID NO: |
|---|---|---|---|---|
| B00202 | CUUAUUAAGAAUUGCAGCAAA | 1 | UUUGCUGCAAUUCUUAAUAAGGG | 107 |
| B00203 | UUAUUAAGAAUUGCAGCAAGU | 2 | ACUUGCUGCAAUUCUUAAUAAGG | 108 |
| B00204 | AACAAGGUCUUUUCAGGAUGA | 3 | UCAUCCUGAAAAGACCUUGUUGG | 109 |
| B00205s | AAGGUCUUUUCAGGAUGAUU | 4 | AAUCAUCCUGAAAAGACCUUGU | 110 |
| B00206s | GGUCUUUUCAGGAUGAUUUU | 5 | AAAAUCAUCCUGAAAAGACCUU | 111 |
| B00207s | UCUUUUCAGGAUGAUUUUCU | 6 | AGAAAAUCAUCCUGAAAAGACC | 112 |
| B00208s | UUUUCAGGAUGAUUUUCUUA | 7 | UAAGAAAAUCAUCCUGAAAAGA | 113 |
| B00209 | UUUCAGGAUGAUUUUCUUAUA | 8 | UAUAAGAAAAUCAUCCUGAAAAG | 114 |
| B00210 | UUCAGGAUGAUUUUCUUAUAU | 9 | AUAUAAGAAAAUCAUCCUGAAAA | 115 |
| B00211 | CAGGAUGAUUUUCUUAUAUCA | 10 | UGAUAUAAGAAAAUCAUCCUGAA | 116 |
| B00211s | AGGAUGAUUUUCUUAUAUCA | 11 | UGAUAUAAGAAAAUCAUCCUGA | 117 |
| B00212 | AGGAUGAUUUUCUUAUAUCAA | 12 | UUGAUAUAAGAAAAUCAUCCUGA | 118 |
| B00213 | AUGAUUUUCUUAUAUCAAGUA | 13 | UACUUGAUAUAAGAAAAUCAUCC | 119 |
| B00214 | UUUUCAGGAUGAUUUUCUUAU | 14 | AUAAGAAAAUCAUCCUGAAAAGA | 120 |
| B00215 | UCAGGAUGAUUUUCUUAUAUA | 15 | UAUAUAAGAAAAUCAUCCUGAAA | 121 |
| B00216 | GGAUGAUUUUCUUAUAUCAAA | 16 | UUUGAUAUAAGAAAAUCAUCCUG | 122 |
| B00217s | AUGAUUUUCUUAUAUCAAGU | 17 | ACUUGAUAUAAGAAAAUCAUCC | 123 |
| B00301 | AUCAAGUGGUACAUUUCAUUU | 18 | AAAUGAAAUGUACCACUUGAUAU | 124 |
| B00302 | UUUCUUAUAUCAAGUGGUACA | 19 | UGUACCACUUGAUAUAAGAAAAU | 125 |
| B00303 | UAUAUCAAGUGGUACAUUUCA | 20 | UGAAAUGUACCACUUGAUAUAAG | 126 |
| B00304 | AUAUCAAGUGGUACAUUUCAU | 21 | AUGAAAUGUACCACUUGAUAUAA | 127 |
| B00305 | CUUAUAUCAAGUGGUACAUUU | 22 | AAAUGUACCACUUGAUAUAAGAA | 128 |
| B00306 | UCAAGUGGUACAUUUCAUUUU | 23 | AAAAUGAAAUGUACCACUUGAUA | 129 |
| B00307 | GUGGUACAUUUCAUUUUAUUU | 24 | AAAUAAAAUGAAAUGUACCACUU | 130 |
| B00308 | UGGUACAUUUCAUUUUAUUUA | 25 | UAAAUAAAAUGAAAUGUACCACU | 131 |
| B00309 | UACAUUUCAUUUUAUUUACUU | 26 | AAGUAAAUAAAAUGAAAUGUACC | 132 |
| B00310 | CAUUUCAUUUUAUUUACUUCA | 27 | UGAAGUAAAUAAAAUGAAAUGUA | 133 |
| B00311 | UCAUUUUAUUUACUUCAGUUU | 28 | AAACUGAAGUAAAUAAAAUGAAA | 134 |
| B00312 | AGUGGUACAUUUCAUUUUAUU | 29 | AAUAAAAUGAAAUGUACCACUUG | 135 |
| B00401 | CAGUUUCUGGUGAAUGUGUGA | 30 | UCACACAUUCACCAGAAACUGAA | 136 |
| B00402 | UUAUUUACUUCAGUUUCUGGU | 31 | ACCAGAAACUGAAGUAAAUAAAA | 137 |
| B00403 | UUACUUCAGUUUCUGGUGAAU | 32 | AUUCACCAGAAACUGAAGUAAAU | 138 |
| B00404 | ACUUCAGUUUCUGGUGAAUGU | 33 | ACAUUCACCAGAAACUGAAGUAA | 139 |
| B00405 | UUCAGUUUCUGGUGAAUGUGU | 34 | ACACAUUCACCAGAAACUGAAGU | 140 |
| B00406s | UGGUGAAUGUGUGACUCAGU | 35 | ACUGAGUCACACAUUCACCAGA | 141 |
| B00407s | UGAAUGUGUGACUCAGUUGU | 36 | ACAACUGAGUCACACAUUCACC | 142 |
| B00408 | AAUGUGUGACUCAGUUGUUGA | 37 | UCAACAACUGAGUCACACAUUCA | 143 |
| B00409 | AUGUGUGACUCAGUUGUUGAA | 38 | UUCAACAACUGAGUCACACAUUC | 144 |
| B00410 | UACUUCAGUUUCUGGUGAAUA | 39 | UAUUCACCAGAAACUGAAGUAAA | 145 |
| B00701 | ACCCAAGAUGUUUACUCUUCA | 40 | UGAAGAGUAAACAUCUUGGGUGG | 146 |
| B00702 | UGCACUUACCACCCAAGAUGU | 41 | ACAUCUUGGGUGGUAAGUGCAGA | 147 |
| B00703 | CACUUACCACCCAAGAUGUUU | 42 | AAACAUCUUGGGUGGUAAGUGCA | 148 |
| B00704 | ACCACCCAAGAUGUUUACUCU | 43 | AGAGUAAACAUCUUGGGUGGUAA | 149 |
| B00705 | CCACCCAAGAUGUUUACUCUU | 44 | AAGAGUAAACAUCUUGGGUGGUA | 150 |
| B00706 | CCCAAGAUGUUUACUCUUCAA | 45 | UUGAAGAGUAAACAUCUUGGGUG | 151 |
| B00707 | CAAGAUGUUUACUCUUCACUU | 46 | AAGUGAAGAGUAAACAUCUUGGG | 152 |
| B00708 | AAGAUGUUUACUCUUCACUUU | 47 | AAAGUGAAGAGUAAACAUCUUGG | 153 |
| B00709 | GAUGUUUACUCUUCACUUUCA | 48 | UGAAAGUGAAGAGUAAACAUCUU | 154 |
| B02601 | UUGCUUGUUUUUUACCUUCUU | 49 | AAGAAGGUAAAAAACAAGCAACC | 155 |
| B02602 | AUCCCGGUUGCUUGUUUUUUA | 50 | UAAAAAACAAGCAACCGGGAUGA | 156 |
| B02603 | UCCCGGUUGCUUGUUUUUUAA | 51 | UUAAAAAACAAGCAACCGGGAUG | 157 |
| B02604 | CCGGUUGCUUGUUUUUUACCU | 52 | AGGUAAAAAACAAGCAACCGGGA | 158 |
| B02605s | GGUUGCUUGUUUUUUACCUU | 53 | AAGGUAAAAAACAAGCAACCGG | 159 |
| B02606 | UGCUUGUUUUUUACCUUCUUU | 54 | AAAGAAGGUAAAAAACAAGCAAC | 160 |
| B02607 | CUUGUUUUUUACCUUCUUUUA | 55 | UAAAAGAAGGUAAAAAACAAGCA | 161 |
| B02608 | GUUUUUUACCUUCUUUUCCCA | 56 | UGGGAAAAGAAGGUAAAAAACAA | 162 |
| B02609 | UUACCUUCUUUUCCCAGGAAU | 57 | AUUCCUGGGAAAAGAAGGUAAAA | 163 |
| B05001 | UUAAAUCAAUCUGAAAUAAAA | 58 | UUUUAUUUCAGAUUGAUUUAAAA | 164 |
| B05002s | CAUUUUAAAUCAAUCUGAAA | 59 | UUUCAGAUUGAUUUAAAAUGCC | 165 |
| B05003 | UUUAAAUCAAUCUGAAAUAAA | 60 | UUUAUUUCAGAUUGAUUUAAAAU | 166 |
| B05004 | AAAUCAAUCUGAAAUAAAAGA | 61 | UCUUUUAUUUCAGAUUGAUUUAA | 167 |
| B05005 | AUCAAUCUGAAAUAAAAGAGA | 62 | UCUCUUUUAUUUCAGAUUGAUUU | 168 |
| B05006 | UCAAUCUGAAAUAAAAGAGGA | 63 | UCCUCUUUUAUUUCAGAUUGAUU | 169 |
| B05007 | AAUCUGAAAUAAAAGAGGACA | 64 | UGUCCUCUUUUAUUUCAGAUUGA | 170 |
| B05008 | UCUGAAAUAAAAGAGGACACA | 65 | UGUGUCCUCUUUUAUUUCAGAUU | 171 |
| B05201 | AUAAUCCAUGAUCAGUAUAAA | 66 | UUUAUACUGAUCAUGGAUUAUUA | 172 |
| B05202 | GGGGUUCAAGAAAUAAUAAUA | 67 | UAUUAUUAUUUCUUGAACCCCAA | 173 |
| B05203 | GGGUUCAAGAAAUAAUAAUCA | 68 | UGAUUAUUAUUUCUUGAACCCCA | 174 |
| B05204 | UCAAGAAAUAAUAAUCCAUGA | 69 | UCAUGGAUUAUUAUUUCUUGAAC | 175 |
| B05205 | CAAGAAAUAAUAAUCCAUGAU | 70 | AUCAUGGAUUAUUAUUUCUUGAA | 176 |
| B05206 | AGAAAUAAUAAUCCAUGAUCA | 71 | UGAUCAUGGAUUAUUAUUUCUUG | 177 |
| B05207 | AUAAUAAUCCAUGAUCAGUAU | 72 | AUACUGAUCAUGGAUUAUUAUUU | 178 |
| B05208 | UAAUCCAUGAUCAGUAUAAAA | 73 | UUUUAUACUGAUCAUGGAUUAUU | 179 |
| B06401 | UACAGAGGACAUAAAAUAACA | 74 | UGUUAUUUUAUGUCCUCUGUAUC | 180 |
| B06402s | GAAGAGAUACAGAGGACAUA | 75 | UAUGUCCUCUGUAUCUCUUCUG | 181 |
| B06403 | GAAGAGAUACAGAGGACAUAA | 76 | UUAUGUCCUCUGUAUCUCUUCUG | 182 |
| B06404 | AAGAGAUACAGAGGACAUAAA | 77 | UUUAUGUCCUCUGUAUCUCUUCU | 183 |
| B06405 | AGAGAUACAGAGGACAUAAAA | 78 | UUUUAUGUCCUCUGUAUCUCUUC | 184 |
| B06406 | AGAUACAGAGGACAUAAAAUA | 79 | UAUUUUAUGUCCUCUGUAUCUCU | 185 |
| B06407 | GAUACAGAGGACAUAAAAUAA | 80 | UUAUUUUAUGUCCUCUGUAUCUC | 186 |
| B06408 | CAGAGGACAUAAAAUAACCCA | 81 | UGGGUUAUUUUAUGUCCUCUGUA | 187 |
| B06409 | AGGACAUAAAAUAACCCAUAA | 82 | UUAUGGGUUAUUUUAUGUCCUCU | 188 |
| B06410 | GACAUAAAAUAACCCAUAAGA | 83 | UCUUAUGGGUUAUUUUAUGUCCU | 189 |
| B06411 | AUAAAAUAACCCAUAAGAUGA | 84 | UCAUCUUAUGGGUUAUUUUAUGU | 190 |
| B07401 | UGGAUUCUGGAGAAAACUCAA | 85 | UUGAGUUUUCUCCAGAAUCCAGU | 191 |
| B07402 | UGGACUGGAUUCUGGAGAAAA | 86 | UUUUCUCCAGAAUCCAGUCCACG | 192 |
| B07403 | GACUGGAUUCUGGAGAAAACU | 87 | AGUUUUCUCCAGAAUCCAGUCCA | 193 |
| B07404s | UGGAUUCUGGAGAAAACUCA | 88 | UGAGUUUUCUCCAGAAUCCAGU | 194 |
| B07405s | UUCUGGAGAAAACUCAAGCA | 89 | UGCUUGAGUUUUCUCCAGAAUC | 195 |
| B07406 | UCUGGAGAAAACUCAAGCAGU | 90 | ACUGCUUGAGUUUUCUCCAGAAU | 196 |
| B07407 | CUGGAGAAAACUCAAGCAGUA | 91 | UACUGCUUGAGUUUUCUCCAGAA | 197 |
| B07408 | UGGAGAAAACUCAAGCAGUGU | 92 | ACACUGCUUGAGUUUUCUCCAGA | 198 |
| B07501 | CUCAAGCAGUGUGAAUGGGUU | 93 | AACCCAUUCACACUGCUUGAGUU | 199 |
| B07502 | GAGAAAACUCAAGCAGUGUGA | 94 | UCACACUGCUUGAGUUUUCUCCA | 200 |
| B07503 | AGAAAACUCAAGCAGUGUGAA | 95 | UUCACACUGCUUGAGUUUUCUCC | 201 |
| B07504 | GAAAACUCAAGCAGUGUGAAU | 96 | AUUCACACUGCUUGAGUUUUCUC | 202 |
| B07505 | AAACUCAAGCAGUGUGAAUGA | 97 | UCAUUCACACUGCUUGAGUUUUC | 203 |
| B07506 | UCAAGCAGUGUGAAUGGGUUA | 98 | UAACCCAUUCACACUGCUUGAGU | 204 |
| B07901 | CACAAGUUGUUAUGUCCAAAA | 99 | UUUUGGACAUAACAACUUGUGAC | 205 |
| B07902 | AAGAAAACAAACUGUCACAAA | 100 | UUUGUGACAGUUUGUUUUCUUCU | 206 |
| B07903 | ACAAACUGUCACAAGUUGUUA | 101 | UAACAACUUGUGACAGUUUGUUU | 207 |
| B07904 | CAAACUGUCACAAGUUGUUAU | 102 | AUAACAACUUGUGACAGUUUGUU | 208 |
| B07905 | AACUGUCACAAGUUGUUAUGU | 103 | ACAUAACAACUUGUGACAGUUUG | 209 |
| B07906 | UCACAAGUUGUUAUGUCCAAA | 104 | UUUGGACAUAACAACUUGUGACA | 210 |
| B07907 | CAAGUUGUUAUGUCCAAAACU | 105 | AGUUUUGGACAUAACAACUUGUG | 211 |
| B07908 | AAGUUGUUAUGUCCAAAACUA | 106 | UAGUUUUGGACAUAACAACUUGU | 212 |
| APC | AUUUCUGGGUAUUCUUUCA | 775 | UGAAAGAAUACCCAGAAAUCG | 776 |
| ANC | GGUUCUGUACUUAUUGUCA | 777 | AGUGACAAUAAGUACAGAACC | 778 |

### 2. siRNA derivatives

### 2.1 Alternating 2'-fluoro and 2'-O-methyl modifications

To enhance the inhibitory activity and stability, the siRNA motifs listed in Table 1 were modified with alternating 2'-O-methyl (2'-OMe) and 2'-fluoro (2'-F) groups, and 3',5'-phosphorothioate linkages were introduced between nucleotides at the 5' end and/or the 3' end. The rules for alternating modifications in the present disclosure are as follows: For sequences in which the sense strand has an odd length, on the sense strand of the corresponding alternately modified siRNA derivative, nucleotides at odd-numbered positions are modified with 2'-F and nucleotides at even-numbered positions are modified with 2'-OMe. For sequences in which the sense strand has an even length, on the sense strand of the corresponding alternately modified siRNA derivative, nucleotides at odd-numbered positions are modified with 2'-OMe and nucleotides at even-numbered positions are modified with 2'-F; on the antisense strand, nucleotides at odd-numbered positions are modified with 2'-OMe and nucleotides at even-numbered positions are modified with 2'-F. Additionally, a 3',5'-phosphorothioate linkage is introduced between nucleotides at positions 1 and 2 and between nucleotides at positions 2 and 3 from the 5' end of the sense strand; a 3',5'-phosphorothioate linkage is introduced between nucleotides at positions 1 and 2 and between nucleotides at positions 2 and 3 from the 5' end of the antisense strand, and a 3',5'-phosphorothioate linkage is introduced between nucleotides at positions 1 and 2 and between nucleotides at positions 2 and 3 from the 3' end of the antisense strand. The alternately modified siRNA derivatives designed according to the rule are designated by adding the suffix "-AL" after the code of the original core sequence. The alternately modified siRNA derivatives were derived from motifs B00202 to B00217, B00302 to B00312, B00401 to B00410, B00701 to B00709, B02601 to B02609, B05001 to B05008, B05201 to B05208, B06401 to B06411, B07401 to B07408, B07501 to B07506, and B07901 to B07908 listed in Table 1 using alternating 2'-fluoro and 2'-O-methyl modifications. The siRNA derivative designated APC-AL, derived from the most active siRNA motif siFXIg1M1SP disclosed in CN113227376B using alternating 2'-fluoro and 2'-O-methyl modifications, was used as a positive control in this study. The siRNA derivative designated ANC-AL, derived from the siRNA motif ANC listed in Table 1 using alternating 2'-fluoro and 2'-O-methyl modifications, was used as a negative control in this study.

**Table 2: siRNA derivatives with alternating 2'-fluoro and 2'-O-methyl modifications**

| siRNA ID | Sense strand sequence (5'-3') | SEQ ID NO: | Antisense strand sequence (5'-3') | SEQ ID NO: |
|---|---|---|---|---|
| B00202-AL | Cfs-Ums-Uf-Am-Uf-Um-Af-Am-Gf- Am-Af-Um-Uf-Gm-Cf-Am-Gf-Cm-Af- Am-Af | 213 | Ums-Ufs-Um-Gf-Cm-Uf-Gm-Cf-Am- Af-Um-Uf-Cm-Uf-Um-Af-Am-Uf-Am- Af-Gms-Gfs-Gm | 319 |
| B00203-AL | Ufs-Ums-Af-Um-Uf-Am-Af-Gm-Af- Am-Uf-Um-Gf-Cm-Af-Gm-Cf-Am-Af- Gm-Uf | 214 | Ams-Cfs-Um-Uf-Gm-Cf-Um-Gf-Cm- Af-Am-Uf-Um-Cf-Um-Uf-Am-Af-Um- Af-Ams-Gfs-Gm | 320 |
| B00204-AL | Afs-Ams-Cf-Am-Af-Gm-Gf-Um-Cf- Um-Uf-Um-Uf-Cm-Af-Gm-Gf-Am-Uf- Gm-Af | 215 | Ums-Cfs-Am-Uf-Cm-Cf-Um-Gf-Am- Af-Am-Af-Gm-Af-Cm-Cf-Um-Uf-Gm- Uf-Ums-Gfs-Gm | 321 |
| B00205sAL | Ams-Afs-Gm-Gf-Um-Cf-Um-Uf-Um- Uf-Cm-Af-Gm-Gf-Am-Uf-Gm-Af-Um- Uf | 216 | Ams-Afs-Um-Cf-Am-Uf-Cm-Cf-Um- Gf-Am-Af-Am-Af-Gm-Af-Cm-Cf-Um- Ufs-Gms-Uf | 322 |
| B00206sAL | Gms-Gfs-Um-Cf-Um-Uf-Um-Uf-Cm- Af-Gm-Gf-Am-Uf-Gm-Af-Um-Uf-Um- Uf | 217 | Ams-Afs-Am-Af-Um-Cf-Am-Uf-Cm- Cf-Um-Gf-Am-Af-Am-Af-Gm-Af-Cm- Cfs-Ums-Uf | 323 |
| B00207sAL | Ums-Cfs-Um-Uf-Um-Uf-Cm-Af-Gm- Gf-Am-Uf-Gm-Af-Um-Uf-Um-Uf-Cm- Uf | 218 | Ams-Gfs-Am-Af-Am-Af-Um-Cf-Am- Uf-Cm-Cf-Um-Gf-Am-Af-Am-Af-Gm- Afs-Cms-Cf | 324 |
| B00208sAL | Ums-Ufs-Um-Uf-Cm-Af-Gm-Gf-Am- Uf-Gm-Af-Um-Uf-Um-Uf-Cm-Uf-Um- Af | 219 | Ums-Afs-Am-Gf-Am-Af-Am-Af-Um- Cf-Am-Uf-Cm-Cf-Um-Gf-Am-Af-Am- Afs-Gms-Af | 325 |
| B00209-AL | Ufs-Ums-Uf-Cm-Af-Gm-Gf-Am-Uf- Gm-Af-Um-Uf-Um-Uf-Cm-Uf-Um-Af- Um-Af | 220 | Ums-Afs-Um-Af-Am-Gf-Am-Af-Am- Af-Um-Cf-Am-Uf-Cm-Cf-Um-Gf-Am- Af-Ams-Afs-Gm | 326 |
| B00210-AL | Ufs-Ums-Cf-Am-Gf-Gm-Af-Um-Gf- Am-Uf-Um-Uf-Um-Cf-Um-Uf-Am-Uf- Am-Uf | 221 | Ams-Ufs-Am-Uf-Am-Af-Gm-Af-Am- Af-Am-Uf-Cm-Af-Um-Cf-Cm-Uf-Gm- Af-Ams-Afs-Am | 327 |
| B00211-AL | Cfs-Ams-Gf-Gm-Af-Um-Gf-Am-Uf- Um-Uf-Um-Cf-Um-Uf-Am-Uf-Am-Uf- Cm-Af | 222 | Ums-Gfs-Am-Uf-Am-Uf-Am-Af-Gm- Af-Am-Af-Am-Uf-Cm-Af-Um-Cf-Cm- Uf-Gms-Afs-Am | 328 |
| B00211sAL | Ams-Gfs-Gm-Af-Um-Gf-Am-Uf-Um- Uf-Um-Cf-Um-Uf-Am-Uf-Am-Uf-Cm- Af | 223 | Ums-Gfs-Am-Uf-Am-Uf-Am-Af-Gm- Af-Am-Af-Am-Uf-Cm-Af-Um-Cf-Cm- Ufs-Gms-Af | 329 |
| B00212-AL | Afs-Gms-Gf-Am-Uf-Gm-Af-Um-Uf- Um-Uf-Cm-Uf-Um-Af-Um-Af-Um-Cf- Am-Af | 224 | Ums-Ufs-Gm-Af-Um-Af-Um-Af-Am- Gf-Am-Af-Am-Af-Um-Cf-Am-Uf-Cm- Cf-Ums-Gfs-Am | 330 |
| B00213-AL | Afs-Ums-Gf-Am-Uf-Um-Uf-Um-Cf- Um-Uf-Am-Uf-Am-Uf-Cm-Af-Am-Gf- Um-Af | 225 | Ums-Afs-Cm-Uf-Um-Gf-Am-Uf-Am- Uf-Am-Af-Gm-Af-Am-Af-Am-Uf-Cm- Af-Ums-Cfs-Cm | 331 |
| B00214-AL | Ufs-Ums-Uf-Um-Cf-Am-Gf-Gm-Af- Um-Gf-Am-Uf-Um-Uf-Um-Cf-Um-Uf- Am-Uf | 226 | Ams-Ufs-Am-Af-Gm-Af-Am-Af-Am- Uf-Cm-Af-Um-Cf-Cm-Uf-Gm-Af-Am- Af-Ams-Gfs-Am | 332 |
| B00215-AL | Ufs-Cms-Af-Gm-Gf-Am-Uf-Gm-Af- Um-Uf-Um-Uf-Cm-Uf-Um-Af-Um-Af- Um-af | 227 | Ums-Afs-Um-Af-Um-Af-Am-Gf-Am- Af-Am-Af-Um-Cf-Am-Uf-Cm-Cf-Um- Gf-Ams-Afs-Am | 333 |
| B00216-AL | Gfs-Gms-Af-Um-Gf-Am-Uf-Um-Uf- Um-Cf-Um-Uf-Am-Uf-Am-Uf-Cm-Af- Am-af | 228 | Ums-Ufs-Um-Gf-Am-Uf-Am-Uf-Am- Af-Gm-Af-Am-Af-Am-Uf-Cm-Af-Um- Cf-Cms-Ufs-Gm | 334 |
| B00217sAL | Ams-Ufs-Gm-Af-Um-Uf-Um-Uf-Cm- Uf-Um-Af-Um-Af-Um-Cf-Am-Af-Gm- Uf | 229 | Ams-Cfs-Um-Uf-Gm-Af-Um-Af-Um- Af-Am-Gf-Am-Af-Am-Af-Um-Cf-Am- Ufs-Cms-Cf | 335 |
| B00301-AL | Afs-Ums-Cf-Am-Af-Gm-Uf-Gm-Gf- Um-Af-Cm-Af-Um-Uf-Um-Cf-Am-Uf- Um-Uf | 230 | Ams-Afs-Am-Uf-Gm-Af-Am-Af-Um- Gf-Um-Af-Cm-Cf-Am-Cf-Um-Uf-Gm- Af-Ums-Afs-Um | 336 |
| B00302-AL | Ufs-Ums-Uf-Cm-Uf-Um-Af-Um-Af- Um-Cf-Am-Af-Gm-Uf-Gm-Gf-Um-Af- Cm-Af | 231 | Ums-Gfs-Um-Af-Cm-Cf-Am-Cf-Um- Uf-Gm-Af-Um-Af-Um-Af-Am-Gf-Am- Af-Ams-Afs-Um | 337 |
| B00303-AL | Ufs-Ams-Uf-Am-Uf-Cm-Af-Am-Gf- Um-Gf-Gm-Uf-Am-Cf-Am-Uf-Um-Uf- Cm-Af | 232 | Ums-Gfs-Am-Af-Am-Uf-Gm-Uf-Am- Cf-Cm-Af-Cm-Uf-Um-Gf-Am-Uf-Am- Uf-Ams-Afs-Gm | 338 |
| B00304-AL | Afs-Ums-Af-Um-Cf-Am-Af-Gm-Uf- Gm-Gf-Um-Af-Cm-Af-Um-Uf-Um-Cf- Am-Uf | 233 | Ams-Ufs-Gm-Af-Am-Af-Um-Gf-Um- Af-Cm-Cf-Am-Cf-Um-Uf-Gm-Af-Um- Af-Ums-Afs-Am | 339 |
| B00305-AL | Cfs-Ums-Uf-Am-Uf-Am-Uf-Cm-Af- Am-Gf-Um-Gf-Gm-Uf-Am-Cf-Am-Uf- Um-Uf | 234 | Ams-Afs-Am-Uf-Gm-Uf-Am-Cf-Cm- Af-Cm-Uf-Um-Gf-Am-Uf-Am-Uf-Am- Af-Gms-Afs-Am | 340 |
| B00306-AL | Ufs-Cms-Af-Am-Gf-Um-Gf-Gm-Uf- Am-Cf-Am-Uf-Um-Uf-Cm-Af-Um-Uf- Um-Uf | 235 | Ams-Afs-Am-Af-Um-Gf-Am-Af-Am- Uf-Gm-Uf-Am-Cf-Cm-Af-Cm-Uf-Um- Gf-Ams-Ufs-Am | 341 |
| B00307-AL | 1 Gfs-Ums-Gf-Gm-Uf-Am-Cf-Am-Uf- Um-Uf-Cm-Af-Um-Uf-Um-Uf-Am-Uf- Um-Uf | 236 | Ams-Afs-Am-Uf-Am-Af-Am-Af-Um- Gf-Am-Af-Am-Uf-Gm-Uf-Am-Cf-Cm- Af-Cms-Ufs-Um | 342 |
| B00308-AL | 1 Ufs-Gms-Gf-Um-Af-Cm-Af-Um-Uf- Um-Cf-Am-Uf-Um-Uf-Um-Af-Um-Uf- Um-Af | 237 | Ums-Afs-Am-Af-Um-Af-Am-Af-Am- Uf-Gm-Af-Am-Af-Um-Gf-Um-Af-Cm- Cf-Ams-Cfs-Um | 343 |
| B00309-AL | Ufs-Ams-Cf-Am-Uf-Um-Uf-Cm-Af- Um-Uf-Um-Uf-Am-Uf-Um-Uf-Am-Cf- Um-Uf | 238 | Ams-Afs-Gm-Uf-Am-Af-Am-Uf-Am- Af-Am-Af-Um-Gf-Am-Af-Am-Uf-Gm- Uf-Ams-Cfs-Cm | 344 |
| B00310-AL | Cfs-Ams-Uf-Um-Uf-Cm-Af-Um-Uf- Um-Uf-Am-Uf-Um-Uf-Am-Cf-Um-Uf- Cm-Af | 239 | Ums-Gfs-Am-Af-Gm-Uf-Am-Af-Am- Uf-Am-Af-Am-Af-Um-Gf-Am-Af-Am- Uf-Gms-Ufs-Am | 345 |
| B00311-AL | Ufs-Cms-Af-Um-Uf-Um-Uf-Am-Uf- Um-Uf-Am-Cf-Um-Uf-Cm-Af-Gm-Uf- Um-Uf | 240 | Ams-Afs-Am-Cf-Um-Gf-Am-Af-Gm- Uf-Am-Af-Am-Uf-Am-Af-Am-Af-Um- Gf-Ams-Afs-Am | 346 |
| B00312-AL | Afs-Uf-Gm-Gf-Um-Af-Cm-Af-Um-Uf- Um-Cf-Am-Uf-Um-Uf-Um-Af-Um-Uf | 241 | Ams-Afs-Um-Af-Am-Af-Am-Uf-Gm- Af-Am-Af-Um-Gf-Um-Af-Cm-Cf-Am- Cf-Ums-Ufs-Gm | 347 |
| B00401-AL | Cfs-Ams-Gf-Um-Uf-Um-Cf-Um-Gf- Gm-Uf-Gm-Af-Am-Uf-Gm-Uf-Gm-Uf- Gm-Af | 242 | Ums-Cfs-Am-Cf-Am-Cf-Am-Uf-Um- Cf-Am-Cf-Cm-Af-Gm-Af-Am-Af-Cm- Uf-Gms-Afs-Am | 348 |
| B00402-AL | Ufs-Ums-Af-Um-Uf-Um-Af-Cm-Uf- Um-Cf-Am-Gf-Um-Uf-Um-Cf-Um-Gf- Gm-Uf | 243 | Ams-Cfs-Cm-Af-Gm-Af-Am-Af-Cm- Uf-Gm-Af-Am-Gf-Um-Af-Am-Af-Um- Af-Ams-Afs-Am | 349 |
| B00403-AL | Ufs-Ums-Af-Cm-Uf-Um-Cf-Am-Gf- Um-Uf-Um-Cf-Um-Gf-Gm-Uf-Gm-Af- Am-Uf | 244 | Ams-Ufs-Um-Cf-Am-Cf-Cm-Af-Gm- Af-Am-Af-Cm-Uf-Gm-Af-Am-Gf-Um- Af-Ams-Afs-Um | 350 |
| B00404-AL | Afs-Cms-Uf-Um-Cf-Am-Gf-Um-Uf- Um-Cf-Um-Gf-Gm-Uf-Gm-Af-Am-Uf- Gm-Uf | 245 | Ams-Cfs-Am-Uf-Um-Cf-Am-Cf-Cm- Af-Gm-Af-Am-Af-Cm-Uf-Gm-Af-Am- Gf-Ums-Afs-Am | 351 |
| B00405-AL | Ufs-Ums-Cf-Am-Gf-Um-Uf-Um-Cf- Um-Gf-Gm-Uf-Gm-Af-Am-Uf-Gm-Uf- Gm-Uf | 246 | Ams-Cfs-Am-Cf-Am-Uf-Um-Cf-Am- Cf-Cm-Af-Gm-Af-Am-Af-Cm-Uf-Gm- Af-Ams-Gfs-Um | 352 |
| B00406sAL | Ums-Gfs-Gm-Uf-Gm-Af-Am-Uf-Gm- Uf-Gm-Uf-Gm-Af-Cm-Uf-Cm-Af-Gm- Uf | 247 | Ams-Cfs-Um-Gf-Am-Gf-Um-Cf-Am- Cf-Am-Cf-Am-Uf-Um-Cf-Am-Cf-Cm- Afs-Gms-Af | 353 |
| B00407sAL | Ums-Gfs-Am-Af-Um-Gf-Um-Gf-Um- Gf-Am-Cf-Um-Cf-Am-Gf-Um-Uf-Gm- Uf | 248 | Ams-Cfs-Am-Af-Cm-Uf-Gm-Af-Gm- Uf-Cm-Af-Cm-Af-Cm-Af-Um-Uf-Cm- Afs-Cms-Cf | 354 |
| B00408-AL | Afs-Ams-Uf-Gm-Uf-Gm-Uf-Gm-Af- Cm-Uf-Cm-Af-Gm-Uf-Um-Gf-Um-Uf- Gm-Af | 249 | Ums-Cfs-Am-Af-Cm-Af-Am-Cf-Um- Gf-Am-Gf-Um-Cf-Am-Cf-Am-Cf-Am- Uf-Ums-Cfs-Am | 355 |
| B00409-AL | Afs-Ums-Gf-Um-Gf-Um-Gf-Am-Cf- Um-Cf-Am-Gf-Um-Uf-Gm-Uf-Um-Gf- Am-Af | 250 | Ums-Ufs-Cm-Af-Am-Cf-Am-Af-Cm- Uf-Gm-Af-Gm-Uf-Cm-Af-Cm-Af-Cm- Af-Ums-Ufs-Cm | 356 |
| B00410-AL | Ufs-Cf-Um-Uf-Cm-Af-Gm-Uf-Um-Uf- Cm-Uf-Gm-Gf-Um-Gf-Am-Af-Um-af | 251 | Ums-Afs-Um-Uf-Cm-Af-Cm-Cf-Am- Gf-Am-Af-Am-Cf-Um-Gf-Am-Af-Gm- Uf-Ams-Afs-Am | 357 |
| B00701-AL | Afs-Cms-Cf-Cm-Af-Am-Gf-Am-Uf- Gm-Uf-Um-Uf-Am-Cf-Um-Cf-Um-Uf- Cm-Af | 252 | Ums-Gfs-Am-Af-Gm-Af-Gm-Uf-Am- Af-Am-Cf-Am-Uf-Cm-Uf-Um-Gf-Gm- Gf-Ums-Gfs-Gm | 358 |
| B00702-AL | Ufs-Gms-Cf-Am-Cf-Um-Uf-Am-Cf- Cm-Af-Cm-Cf-Cm-Af-Am-Gf-Am-Uf- Gm-Uf | 253 | Ams-Cfs-Am-Uf-Cm-Uf-Um-Gf-Gm- Gf-Um-Gf-Gm-Uf-Am-Af-Gm-Uf-Gm- Cf-Ams-Gfs-Am | 359 |
| B00703-AL | Cfs-Ams-Cf-Um-Uf-Am-Cf-Cm-Af- Cm-Cf-Cm-Af-Am-Gf-Am-Uf-Gm-Uf- Um-Uf | 254 | Ams-Afs-Am-Cf-Am-Uf-Cm-Uf-Um- Gf-Gm-Gf-Um-Gf-Gm-Uf-Am-Af-Gm- Uf-Gms-Cfs-Am | 360 |
| B00704-AL | Afs-Cms-Cf-Am-Cf-Cm-Cf-Am-Af- Gm-Af-Um-Gf-Um-Uf-Um-Af-Cm-Uf- Cm-Uf | 255 | Ams-Gfs-Am-Gf-Um-Af-Am-Af-Cm- Af-Um-Cf-Um-Uf-Gm-Gf-Gm-Uf-Gm- Gf-Ums-Afs-Am | 361 |
| B00705-AL | Cfs-Cms-Af-Cm-Cf-Cm-Af-Am-Gf- Am-Uf-Gm-Uf-Um-Uf-Am-Cf-Um-Cf- Um-Uf | 256 | Ams-Afs-Gm-Af-Gm-Uf-Am-Af-Am- Cf-Am-Uf-Cm-Uf-Um-Gf-Gm-Gf-Um- Gf-Gms-Ufs-Am | 362 |
| B00706-AL | Cfs-Cms-Cf-Am-Af-Gm-Af-Um-Gf- Um-Uf-Um-Af-Cm-Uf-Cm-Uf-Um-Cf- Am-Af | 257 | Ums-Ufs-Gm-Af-Am-Gf-Am-Gf-Um- Af-Am-Af-Cm-Af-Um-Cf-Um-Uf-Gm- Gf-Gms-Ufs-Gm | 363 |
| B00707-AL | Cfs-Ams-Af-Gm-Af-Um-Gf-Um-Uf- Um-Af-Cm-Uf-Cm-Uf-Um-Cf-Am-Cf- Um-Uf | 258 | Ams-Afs-Gm-Uf-Gm-Af-Am-Gf-Am- Gf-Um-Af-Am-Af-Cm-Af-Um-Cf-Um- Uf-Gms-Gfs-Gm | 364 |
| B00708-AL | Afs-Ams-Gf-Am-Uf-Gm-Uf-Um-Uf- Am-Cf-Um-Cf-Um-Uf-Cm-Af-Cm-Uf- Um-Uf | 259 | Ams-Afs-Am-Gf-Um-Gf-Am-Af-Gm- Af-Gm-Uf-Am-Af-Am-Cf-Am-Uf-Cm- Uf-Ums-Gfs-Gm | 365 |
| B00709-AL | Gfs-Ams-Uf-Gm-Uf-Um-Uf-Am-Cf- Um-Cf-Um-Uf-Cm-Af-Cm-Uf-Um-Uf- Cm-Af | 260 | Ums-Gfs-Am-Af-Am-Gf-Um-Gf-Am- Af-Gm-Af-Gm-Uf-Am-Af-Am-Cf-Am- Uf-Cms-Ufs-Um | 366 |
| B02601-AL | Ufs-Ums-Gf-Cm-Uf-Um-Gf-Um-Uf- Um-Uf-Um-Uf-Am-Cf-Cm-Uf-Um-Cf- Um-Uf | 261 | Ams-Afs-Gm-Af-Am-Gf-Gm-Uf-Am- Af-Am-Af-Am-Af-Cm-Af-Am-Gf-Cm- Af-Ams-Cfs-Cm | 367 |
| B02602-AL | Afs-Ums-Cf-Cm-Cf-Gm-Gf-Um-Uf- Gm-Cf-Um-Uf-Gm-Uf-Um-Uf-Um-Uf- Um-Af | 262 | Ums-Afs-Am-Af-Am-Af-Am-Cf-Am- Af-Gm-Cf-Am-Af-Cm-Cf-Gm-Gf-Gm- Af-Ums-Gfs-Am | 368 |
| B02603-AL | Ufs-Cms-Cf-Cm-Gf-Gm-Uf-Um-Gf- Cm-Uf-Um-Gf-Um-Uf-Um-Uf-Um-Uf- Am-Af | 263 | Ums-Ufs-Am-Af-Am-Af-Am-Af-Cm- Af-Am-Gf-Cm-Af-Am-Cf-Cm-Gf-Gm- Gf-Ams-Ufs-Gm | 369 |
| B02604-AL | Cfs-Cms-Gf-Gm-Uf-Um-Gf-Cm-Uf- Um-Gf-Um-Uf-Um-Uf-Um-Uf-Am-Cf- Cm-Uf | 264 | Ams-Gfs-Gm-Uf-Am-Af-Am-Af-Am- Af-Cm-Af-Am-Gf-Cm-Af-Am-Cf-Cm- Gf-Gms-Gfs-Am | 370 |
| B02605sAL | Gms-Gfs-Um-Uf-Gm-Cf-Um-Uf-Gm- Uf-Um-Uf-Um-Uf-Um-Af-Cm-Cf-Um- Uf | 265 | Ams-Afs-Gm-Gf-Um-Af-Am-Af-Am- Af-Am-Cf-Am-Af-Gm-Cf-Am-Af-Cm- Cfs-Gms-Gf | 371 |
| B02606-AL | Ufs-Gms-Cf-Um-Uf-Gm-Uf-Um-Uf- Um-Uf-Um-Af-Cm-Cf-Um-Uf-Cm-Uf- Um-Uf | 266 | Ams-Afs-Am-Gf-Am-Af-Gm-Gf-Um- Af-Am-Af-Am-Af-Am-Cf-Am-Af-Gm- Cf-Ams-Afs-Cm | 372 |
| B02607-AL | Cfs-Ums-Uf-Gm-Uf-Um-Uf-Um-Uf- Um-Af-Cm-Cf-Um-Uf-Cm-Uf-Um-Uf- Um-Af | 267 | Ums-Afs-Am-Af-Am-Gf-Am-Af-Gm- Gf-Um-Af-Am-Af-Am-Af-Am-Cf-Am- Af-Gms-Cfs-Am | 373 |
| B02608-AL | Gfs-Ums-Uf-Um-Uf-Um-Uf-Am-Cf- Cm-Uf-Um-Cf-Um-Uf-Um-Uf-Cm-Cf- Cm-Af | 268 | Ums-Gfs-Gm-Gf-Am-Af-Am-Af-Gm- Af-Am-Gf-Gm-Uf-Am-Af-Am-Af-Am- Af-Cms-Afs-Am | 374 |
| B02609-AL | Ufs-Ums-Af-Cm-Cf-Um-Uf-Cm-Uf- Um-Uf-Um-Cf-Cm-Cf-Am-Gf-Gm-Af- Am-Uf | 269 | Ams-Ufs-Um-Cf-Cm-Uf-Gm-Gf-Gm- Af-Am-Af-Am-Gf-Am-Af-Gm-Gf-Um- Af-Ams-Afs-Am | 375 |
| B05001-AL | 1 Ufs-Ums-Af-Am-Af-Um-Cf-Am-Af- Um-Cf-Um-Gf-Am-Af-Am-Uf-Am-Af- Am-Af | 270 | Ums-Ufs-Um-Uf-Am-Uf-Um-Uf-Cm- Af-Gm-Af-Um-Uf-Gm-Af-Um-Uf-Um- Af-Ams-Afs-Am | 376 |
| B05002sAL | Cms-Afs-Um-Uf-Um-Uf-Am-Af-Am- Uf-Cm-Af-Am-Uf-Cm-Uf-Gm-Af-Am- Af | 271 | Ums-Ufs-Um-Cf-Am-Gf-Am-Uf-Um- Gf-Am-Uf-Um-Uf-Am-Af-Am-Af-Um- Gfs-Cms-Cf | 377 |
| B05003-AL | Ufs-Ums-Uf-Am-Af-Am-Uf-Cm-Af- Am-Uf-Cm-Uf-Gm-Af-Am-Af-Um-Af- Am-Af | 272 | Ums-Ufs-Um-Af-Um-Uf-Um-Cf-Am- Gf-Am-Uf-Um-Gf-Am-Uf-Um-Uf-Am- Af-Ams-Afs-Um | 378 |
| B05004-AL | 1 Afs-Ams-Af-Um-Cf-Am-Af-Um-Cf- Um-Gf-Am-Af-Am-Uf-Am-Af-Am-Af- Gm-Af | 273 | Ums-Cfs-Um-Uf-Um-Uf-Am-Uf-Um- Uf-Cm-Af-Gm-Af-Um-Uf-Gm-Af-Um- Uf-Ums-Afs-Am | 379 |
| B05005-AL | Afs-Ums-Cf-Am-Af-Um-Cf-Um-Gf- Am-Af-Am-Uf-Am-Af-Am-Af-Gm-Af- Gm-Af | 274 | Ums-Cfs-Um-Cf-Um-Uf-Um-Uf-Am- Uf-Um-Uf-Cm-Af-Gm-Af-Um-Uf-Gm- Af-Ums-Ufs-Um | 380 |
| B05006-AL | Ufs-Cms-Af-Am-Uf-Cm-Uf-Gm-Af- Am-Af-Um-Af-Am-Af-Am-Gf-Am-Gf- Gm-Af | 275 | Ums-Cfs-Cm-Uf-Cm-Uf-Um-Uf-Um- Af-Um-Uf-Um-Cf-Am-Gf-Am-Uf-Um- Gf-Ams-Ufs-Um | 381 |
| B05007-AL | Afs-Ams-Uf-Cm-Uf-Gm-Af-Am-Af- Um-Af-Am-Af-Am-Gf-Am-Gf-Gm-Af- Cm-Af | 276 | Ums-Gfs-Um-Cf-Cm-Uf-Cm-Uf-Um- Uf-Um-Af-Um-Uf-Um-Cf-Am-Gf-Am- Uf-Ums-Gfs-Am | 382 |
| B05008-AL | Ufs-Cms-Uf-Gm-Af-Am-Af-Um-Af- Am-Af-Am-Gf-Am-Gf-Gm-Af-Cm-Af- Cm-Af | 277 | Ums-Gfs-Um-Gf-Um-Cf-Cm-Uf-Cm- Uf-Um-Uf-Um-Af-Um-Uf-Um-Cf-Am- Gf-Ams-Ufs-Um | 383 |
| B05201-AL | Afs-Ums-Af-Am-Uf-Cm-Cf-Am-Uf- Gm-Af-Um-Cf-Am-Gf-Um-Af-Um-Af- Am-Af | 278 | Ums-Ufs-Um-Af-Um-Af-Cm-Uf-Gm- Af-Um-Cf-Am-Uf-Gm-Gf-Am-Uf-Um- Af-Ums-Ufs-Am | 384 |
| B05202-AL | Gfs-Gms-Gf-Gm-Uf-Um-Cf-Am-Af- Gm-Af-Am-Af-Um-Af-Am-Uf-Am-Af- Um-Af | 279 | Ums-Afs-Um-Uf-Am-Uf-Um-Af-Um- Uf-Um-Cf-Um-Uf-Gm-Af-Am-Cf-Cm- Cf-Cms-Afs-Am | 385 |
| B05203-AL | Gfs-Gms-Gf-Um-Uf-Cm-Af-Am-Gf- Am-Af-Am-Uf-Am-Af-Um-Af-Am-Uf- Cm-Af | 280 | Ums-Gfs-Am-Uf-Um-Af-Um-Uf-Am- Uf-Um-Uf-Cm-Uf-Um-Gf-Am-Af-Cm- Cf-Cms-Cfs-Am | 386 |
| B05204-AL | Ufs-Cms-Af-Am-Gf-Am-Af-Am-Uf- Am-Af-Um-Af-Am-Uf-Cm-Cf-Am-Uf- Gm-Af | 281 | Ums-Cfs-Am-Uf-Gm-Gf-Am-Uf-Um- Af-Um-Uf-Am-Uf-Um-Uf-Cm-Uf-Um- Gf-Ams-Afs-Cm | 387 |
| B05205-AL | Cfs-Ams-Af-Gm-Af-Am-Af-Um-Af- Am-Uf-Am-Af-Um-Cf-Cm-Af-Um-Gf- Am-Uf | 282 | Ams-Ufs-Cm-Af-Um-Gf-Gm-Af-Um- Uf-Am-Uf-Um-Af-Um-Uf-Um-Cf-Um- Uf-Gms-Afs-Am | 388 |
| B05206-AL | Afs-Gms-Af-Am-Af-Um-Af-Am-Uf- Am-Af-Um-Cf-Cm-Af-Um-Gf-Am-Uf- Cm-Af | 283 | Ums-Gfs-Am-Uf-Cm-Af-Um-Gf-Gm- Af-Um-Uf-Am-Uf-Um-Af-Um-Uf-Um- Cf-Ums-Ufs-Gm | 389 |
| B05207-AL | Afs-Ums-Af-Am-Uf-Am-Af-Um-Cf- Cm-Af-Um-Gf-Am-Uf-Cm-Af-Gm-Uf- Am-Uf | 284 | Ams-Ufs-Am-Cf-Um-Gf-Am-Uf-Cm- Af-Um-Gf-Gm-Af-Um-Uf-Am-Uf-Um- Af-Ums-Ufs-Um | 390 |
| B05208-AL | Ufs-Ams-Af-Um-Cf-Cm-Af-Um-Gf- Am-Uf-Cm-Af-Gm-Uf-Am-Uf-Am-Af- Am-Af | 285 | Ums-Ufs-Um-Uf-Am-Uf-Am-Cf-Um- Gf-Am-Uf-Cm-Af-Um-Gf-Gm-Af-Um- Uf-Ams-Ufs-Um | 391 |
| B06401-AL | Ufs-Ams-Cf-Am-Gf-Am-Gf-Gm-Af- Cm-Af-Um-Af-Am-Af-Am-Uf-Am-Af- Cm-Af | 286 | Ums-Gfs-Um-Uf-Am-Uf-Um-Uf-Um- Af-Um-Gf-Um-Cf-Cm-Uf-Cm-Uf-Gm- Uf-Ams-Ufs-Cm | 392 |
| B06402sAL | Gms-Afs-Am-Gf-Am-Gf-Am-Uf-Am- Cf-Am-Gf-Am-Gf-Gm-Af-Cm-Af-Um- Af | 287 | Ums-Afs-Um-Gf-Um-Cf-Cm-Uf-Cm- Uf-Gm-Uf-Am-Uf-Cm-Uf-Cm-Uf-Um- Cfs-Ums-Gf | 393 |
| B06403-AL | Gfs-Ams-Af-Gm-Af-Gm-Af-Um-Af- Cm-Af-Gm-Af-Gm-Gf-Am-Cf-Am-Uf- Am-Af | 288 | Ums-Ufs-Am-Uf-Gm-Uf-Cm-Cf-Um- Cf-Um-Gf-Um-Af-Um-Cf-Um-Cf-Um- Uf-Cms-Ufs-Gm | 394 |
| B06404-AL | Afs-Ams-Gf-Am-Gf-Am-Uf-Am-Cf- Am-Gf-Am-Gf-Gm-Af-Cm-Af-Um-Af- Am-Af | 289 | Ums-Ufs-Um-Af-Um-Gf-Um-Cf-Cm- Uf-Cm-Uf-Gm-Uf-Am-Uf-Cm-Uf-Cm- Uf-Ums-Cfs-Um | 395 |
| B06405-AL | Afs-Gms-Af-Gm-Af-Um-Af-Cm-Af- Gm-Af-Gm-Gf-Am-Cf-Am-Uf-Am-Af- Am-Af | 290 | Ums-Ufs-Um-Uf-Am-Uf-Gm-Uf-Cm- Cf-Um-Cf-Um-Gf-Um-Af-Um-Cf-Um- Cf-Ums-Ufs-Cm | 396 |
| B06406-AL | Afs-Gms-Af-Um-Af-Cm-Af-Gm-Af- Gm-Gf-Am-Cf-Am-Uf-Am-Af-Am-Af- Um-Af | 291 | Ums-Afs-Um-Uf-Um-Uf-Am-Uf-Gm- Uf-Cm-Cf-Um-Cf-Um-Gf-Um-Af-Um- Cf-Ums-Cfs-Um | 397 |
| B06407-AL | Gfs-Ams-Uf-Am-Cf-Am-Gf-Am-Gf- Gm-Af-Cm-Af-Um-Af-Am-Af-Am-Uf- Am-Af | 292 | Ums-Ufs-Am-Uf-Um-Uf-Um-Af-Um- Gf-Um-Cf-Cm-Uf-Cm-Uf-Gm-Uf-Am- Uf-Cms-Ufs-Cm | 398 |
| B06408-AL | Cfs-Ams-Gf-Am-Gf-Gm-Af-Cm-Af- Um-Af-Am-Af-Am-Uf-Am-Af-Cm-Cf- Cm-Af | 293 | Ums-Gfs-Gm-Gf-Um-Uf-Am-Uf-Um- Uf-Um-Af-Um-Gf-Um-Cf-Cm-Uf-Cm- Uf-Gms-Ufs-Am | 399 |
| B06409-AL | Afs-Gms-Gf-Am-Cf-Am-Uf-Am-Af- Am-Af-Um-Af-Am-Cf-Cm-Cf-Am-Uf- Am-Af | 294 | Ums-Ufs-Am-Uf-Gm-Gf-Gm-Uf-Um- Af-Um-Uf-Um-Uf-Am-Uf-Gm-Uf-Cm- Cf-Ums-Cfs-Um | 400 |
| B06410-AL | Gfs-Ams-Cf-Am-Uf-Am-Af-Am-Af- Um-Af-Am-Cf-Cm-Cf-Am-Uf-Am-Af- Gm-Af | 295 | Ums-Cfs-Um-Uf-Am-Uf-Gm-Gf-Gm- Uf-Um-Af-Um-Uf-Um-Uf-Am-Uf-Gm- Uf-Cms-Cfs-Um | 401 |
| B06411-AL | Afs-Ums-Af-Am-Af-Am-Uf-Am-Af- Cm-Cf-Cm-Af-Um-Af-Am-Gf-Am-Uf- Gm-Af | 296 | Ums-Cfs-Am-Uf-Cm-Uf-Um-Af-Um- Gf-Gm-Gf-Um-Uf-Am-Uf-Um-Uf-Um- Af-Ums-Gfs-Um | 402 |
| B07401-AL | Ufs-Gms-Gf-Am-Uf-Um-Cf-Um-Gf- Gm-Af-Gm-Af-Am-Af-Am-Cf-Um-Cf- Am-Af | 297 | Ums-Ufs-Gm-Af-Gm-Uf-Um-Uf-Um- Cf-Um-Cf-Cm-Af-Gm-Af-Am-Uf-Cm- Cf-Ams-Gfs-Um | 403 |
| B07402-AL | Ufs-Gms-Gf-Am-Cf-Um-Gf-Gm-Af- Um-Uf-Cm-Uf-Gm-Gf-Am-Gf-Am-Af- Am-Af | 298 | Ums-Ufs-Um-Uf-Cm-Uf-Cm-Cf-Am- Gf-Am-Af-Um-Cf-Cm-Af-Gm-Uf-Cm- Cf-Ams-Cfs-Gm | 404 |
| B07403-AL | Gfs-Ams-Cf-Um-Gf-Gm-Af-Um-Uf- Cm-Uf-Gm-Gf-Am-Gf-Am-Af-Am-Af- Cm-Uf | 299 | Ams-Gfs-Um-Uf-Um-Uf-Cm-Uf-Cm- Cf-Am-Gf-Am-Af-Um-Cf-Cm-Af-Gm- Uf-Cms-Cfs-Am | 405 |
| B07404sAL | Ums-Gfs-Gm-Af-Um-Uf-Cm-Uf-Gm- Gf-Am-Gf-Am-Af-Am-Af-Cm-Uf-Cm- Af | 300 | Ums-Gfs-Am-Gf-Um-Uf-Um-Uf-Cm- Uf-Cm-Cf-Am-Gf-Am-Af-Um-Cf-Cm- Afs-Gms-Uf | 406 |
| B07405sAL | Ums-Ufs-Cm-Uf-Gm-Gf-Am-Gf-Am- Af-Am-Af-Cm-Uf-Cm-Af-Am-Gf-Cm- Af | 301 | Ums-Gfs-Cm-Uf-Um-Gf-Am-Gf-Um- Uf-Um-Uf-Cm-Uf-Cm-Cf-Am-Gf-Am- Afs-Ums-Cf | 407 |
| B07406-AL | Ufs-Cms-Uf-Gm-Gf-Am-Gf-Am-Af- Am-Af-Cm-Uf-Cm-Af-Am-Gf-Cm-Af- Gm-Uf | 302 | Ams-Cfs-Um-Gf-Cm-Uf-Um-Gf-Am- Gf-Um-Uf-Um-Uf-Cm-Uf-Cm-Cf-Am- Gf-Ams-Afs-Um | 408 |
| B07407-AL | Cfs-Ums-Gf-Gm-Af-Gm-Af-Am-Af- Am-Cf-Um-Cf-Am-Af-Gm-Cf-Am-Gf- Um-Af | 303 | Ums-Afs-Cm-Uf-Gm-Cf-Um-Uf-Gm- Af-Gm-Uf-Um-Uf-Um-Cf-Um-Cf-Cm- Af-Gms-Afs-Am | 409 |
| B07408-AL | Ufs-Gms-Gf-Am-Gf-Am-Af-Am-Af- Cm-Uf-Cm-Af-Am-Gf-Cm-Af-Gm-Uf- Gm-Uf | 304 | Ams-Cfs-Am-Cf-Um-Gf-Cm-Uf-Um- Gf-Am-Gf-Um-Uf-Um-Uf-Cm-Uf-Cm- Cf-Ams-Gfs-Am | 410 |
| B07501-AL | Cfs-Ums-Cf-Am-Af-Gm-Cf-Am-Gf- Um-Gf-Um-Gf-Am-Af-Um-Gf-Gm-Gf- Um-Uf | 305 | Ams-Afs-Cm-Cf-Cm-Af-Um-Uf-Cm- Af-Cm-Af-Cm-Uf-Gm-Cf-Um-Uf-Gm- Af-Gms-Ufs-Um | 411 |
| B07502-AL | Gfs-Ams-Gf-Am-Af-Am-Af-Cm-Uf- Cm-Af-Am-Gf-Cm-Af-Gm-Uf-Gm-Uf- Gm-Af | 306 | Ums-Cfs-Am-Cf-Am-Cf-Um-Gf-Cm- Uf-Um-Gf-Am-Gf-Um-Uf-Um-Uf-Cm- Uf-Cms-Cfs-Am | 412 |
| B07503-AL | Afs-Gms-Af-Am-Af-Am-Cf-Um-Cf- Am-Af-Gm-Cf-Am-Gf-Um-Gf-Um-Gf- Am-Af | 307 | Ums-Ufs-Cm-Af-Cm-Af-Cm-Uf-Gm- Cf-Um-Uf-Gm-Af-Gm-Uf-Um-Uf-Um- Cf-Ums-Cfs-Cm | 413 |
| B07504-AL | Gfs-Ams-Af-Am-Af-Cm-Uf-Cm-Af- Am-Gf-Cm-Af-Gm-Uf-Gm-Uf-Gm-Af- Am-Uf | 308 | Ams-Ufs-Um-Cf-Am-Cf-Am-Cf-Um- Gf-Cm-Uf-Um-Gf-Am-Gf-Um-Uf-Um- Uf-Cms-Ufs-Cm | 414 |
| B07505-AL | Afs-Ams-Af-Cm-Uf-Cm-Af-Am-Gf- Cm-Af-Gm-Uf-Gm-Uf-Gm-Af-Am-Uf- Gm-Af | 309 | Ums-Cfs-Am-Uf-Um-Cf-Am-Cf-Am- Cf-Um-Gf-Cm-Uf-Um-Gf-Am-Gf-Um- Uf-Ums-Ufs-Cm | 415 |
| B07506-AL | Ufs-Cms-Af-Am-Gf-Cm-Af-Gm-Uf- Gm-Uf-Gm-Af-Am-Uf-Gm-Gf-Gm-Uf- Um-Af | 310 | Ums-Afs-Am-Cf-Cm-Cf-Am-Uf-Um- Cf-Am-Cf-Am-Cf-Um-Gf-Cm-Uf-Um- Gf-Ams-Gfs-Um | 416 |
| B07901-AL | Cfs-Ams-Cf-Am-Af-Gm-Uf-Um-Gf- Um-Uf-Am-Uf-Gm-Uf-Cm-Cf-Am-Af- Am-Af | 311 | Ums-Ufs-Um-Uf-Gm-Gf-Am-Cf-Am- Uf-Am-Af-Cm-Af-Am-Cf-Um-Uf-Gm- Uf-Gms-Afs-Cm | 417 |
| B07902-AL | Afs-Ams-Gf-Am-Af-Am-Af-Cm-Af- Am-Af-Cm-Uf-Gm-Uf-Cm-Af-Cm-Af- Am-Af | 312 | Ums-Ufs-Um-Gf-Um-Gf-Am-Cf-Am- Gf-Um-Uf-Um-Gf-Um-Uf-Um-Uf-Cm- Uf-Ums-Cfs-Um | 418 |
| B07903-AL | Afs-Cms-Af-Am-Af-Cm-Uf-Gm-Uf- Cm-Af-Cm-Af-Am-Gf-Um-Uf-Gm-Uf- Um-Af | 313 | Ums-Afs-Am-Cf-Am-Af-Cm-Uf-Um- Gf-Um-Gf-Am-Cf-Am-Gf-Um-Uf-Um- Gf-Ums-Ufs-Um | 419 |
| B07904-AL | Cfs-Ams-Af-Am-Cf-Um-Gf-Um-Cf- Am-Cf-Am-Af-Gm-Uf-Um-Gf-Um-Uf- Am-Uf | 314 | Ams-Ufs-Am-Af-Cm-Af-Am-Cf-Um- Uf-Gm-Uf-Gm-Af-Cm-Af-Gm-Uf-Um- Uf-Gms-Ufs-Um | 420 |
| B07905-AL | Afs-Ams-Cf-Um-Gf-Um-Cf-Am-Cf- Am-Af-Gm-Uf-Um-Gf-Um-Uf-Am-Uf- Gm-Uf | 315 | Ams-Cfs-Am-Uf-Am-Af-Cm-Af-Am- Cf-Um-Uf-Gm-Uf-Gm-Af-Cm-Af-Gm- Uf-Ums-Ufs-Gm | 421 |
| B07906-AL | Ufs-Cms-Af-Cm-Af-Am-Gf-Um-Uf- Gm-Uf-Um-Af-Um-Gf-Um-Cf-Cm-Af- Am-Af | 316 | Ums-Ufs-Um-Gf-Gm-Af-Cm-Af-Um- Af-Am-Cf-Am-Af-Cm-Uf-Um-Gf-Um- Gf-Ams-Cfs-Am | 422 |
| B07907-AL | Cfs-Ams-Af-Gm-Uf-Um-Gf-Um-Uf- Am-Uf-Gm-Uf-Cm-Cf-Am-Af-Am-Af- Cm-Uf | 317 | Ams-Gfs-Um-Uf-Um-Uf-Gm-Gf-Am- Cf-Am-Uf-Am-Af-Cm-Af-Am-Cf-Um- Uf-Gms-Ufs-Gm | 423 |
| B07908-AL | Afs-Ams-Gf-Um-Uf-Gm-Uf-Um-Af- Um-Gf-Um-Cf-Cm-Af-Am-Af-Am-Cf- Um-Af | 318 | Ums-Afs-Gm-Uf-Um-Uf-Um-Gf-Gm- Af-Cm-Af-Um-Af-Am-Cf-Am-Af-Cm- Uf-Ums-Gfs-Um | 424 |
| APC-AL | Afs-Ums-Uf-Um-Cf-Um-Gf-Gm-Gf- Um-Af-Um-Uf-Cm-Uf-Um-Uf-Cm-Af | 779 | Ums-Gfs-Am-Af-Am-Gf-Am-Af-Um- Af-Cm-Cf-Cm-Af-Gm-Af-Am-Af-Ums- Cfs-Gm | 780 |
| ANC-AL | Gfs-Gms-Uf-Um-Cf-Um-Gf-Um-Af- Cm-Uf-Um-Af-Um-Uf-Gm-Uf-Cm-Af | 781 | Ams-Gfs-Um-Gf-Am-Cf-Am-Af-Um- Af-Am-Gf-Um-Af-Cm-Af-Gm-Af-Ams- Cfs-Cm | 782 |

### 2.2 Template-modified siRNA derivatives

### 2.2.1 Modification templates

The modification templates disclosed herein include DV25P, DV26P, DV27P, DV29P, DV32P, DV34P, DV39P, DV40P, DV25SP, DV26SP, DV27SP, DV29SP, DV32SP, DV34SP, DV39SP, and DV40SP. DV22 represents a modification template disclosed in the prior art, corresponding to the Advanced ESC template, in which fluoro modifications are introduced at positions 2, 6, 14, and 16 of the antisense strand and positions 7, 9, 10, and 11 of the sense strand. The modification templates are shown in Tables 3 to 6. The modification rules are as follows:
For siRNA motifs in which the sense and antisense strands are 21 and 23 nucleotides in length, respectively, the antisense and sense strands were modified according to any one of the modification patterns shown in Tables 3 and 4, respectively.

For siRNA motifs in which the sense and antisense strands are 20 and 22 nucleotides in length, respectively, the antisense and sense strands were modified according to any one of the modification patterns shown in Tables 5 and 6, respectively.

The siRNA modification template, in which the antisense strand was modified according to modification pattern A and the sense strand was modified according to modification pattern a, was designated as DV25P.

The siRNA modification template, in which the antisense strand was modified according to modification pattern B and the sense strand was modified according to modification pattern a, was designated as DV26P.

The siRNA modification template, in which the antisense strand was modified according to modification pattern C and the sense strand was modified according to modification pattern a, was designated as DV27P.

The siRNA modification template, in which the antisense strand was modified according to modification pattern C and the sense strand was modified according to modification pattern b, was designated as DV29P.

The siRNA modification template, in which the antisense strand was modified according to modification pattern D and the sense strand was modified according to modification pattern b, was designated as DV32P.

The siRNA modification template, in which the antisense strand was modified according to modification pattern E and the sense strand was modified according to modification pattern b, was designated as DV34P.

The siRNA modification template, in which the antisense strand was modified according to modification pattern F and the sense strand was modified according to modification pattern b, was designated as DV39P.

The siRNA modification template, in which the antisense strand was modified according to modification pattern G and the sense strand was modified according to modification pattern b, was designated as DV40P.

The modification template DV22 disclosed in the prior art, in which the antisense strand was modified according to modification pattern H and the sense strand was modified according to modification pattern c.

The siRNA modification template, in which the antisense strand was modified according to modification pattern A' and the sense strand was modified according to modification pattern a', was designated as DV25SP.

The siRNA modification template, in which the antisense strand was modified according to modification pattern B' and the sense strand was modified according to modification pattern a', was designated as DV26SP.

The siRNA modification template, in which the antisense strand was modified according to modification pattern C' and the sense strand was modified according to modification pattern a', was designated as DV27SP.

The siRNA modification template, in which the antisense strand was modified according to modification pattern C' and the sense strand was modified according to modification pattern b', was designated as DV29SP.

The siRNA modification template, in which the antisense strand was modified according to modification pattern D' and the sense strand was modified according to modification pattern b', was designated as DV32SP.

The siRNA modification template, in which the antisense strand was modified according to modification pattern E' and the sense strand was modified according to modification pattern b', was designated as DV34SP.

The siRNA modification template, in which the antisense strand was modified according to modification pattern F' and the sense strand was modified according to modification pattern b', was designated as DV39SP.

The siRNA modification template, in which the antisense strand was modified according to modification pattern G' and the sense strand was modified according to modification pattern b', was designated as DV40SP.

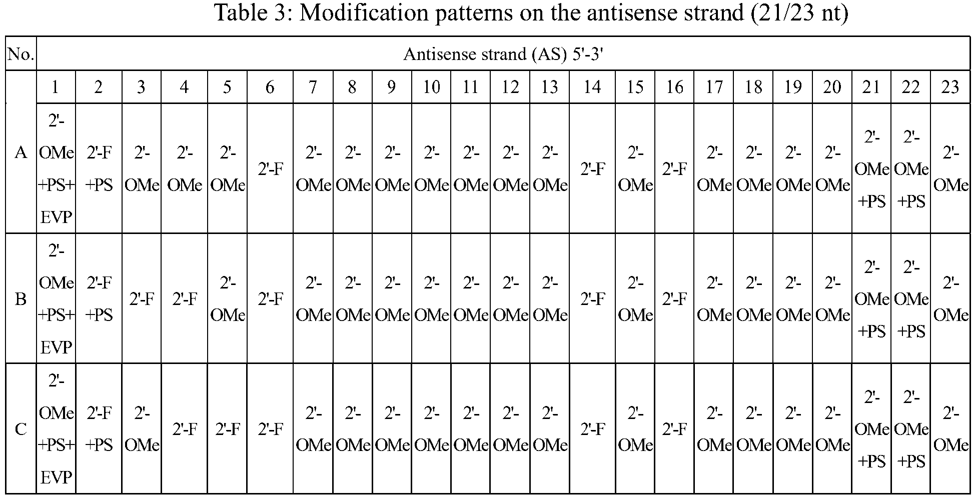

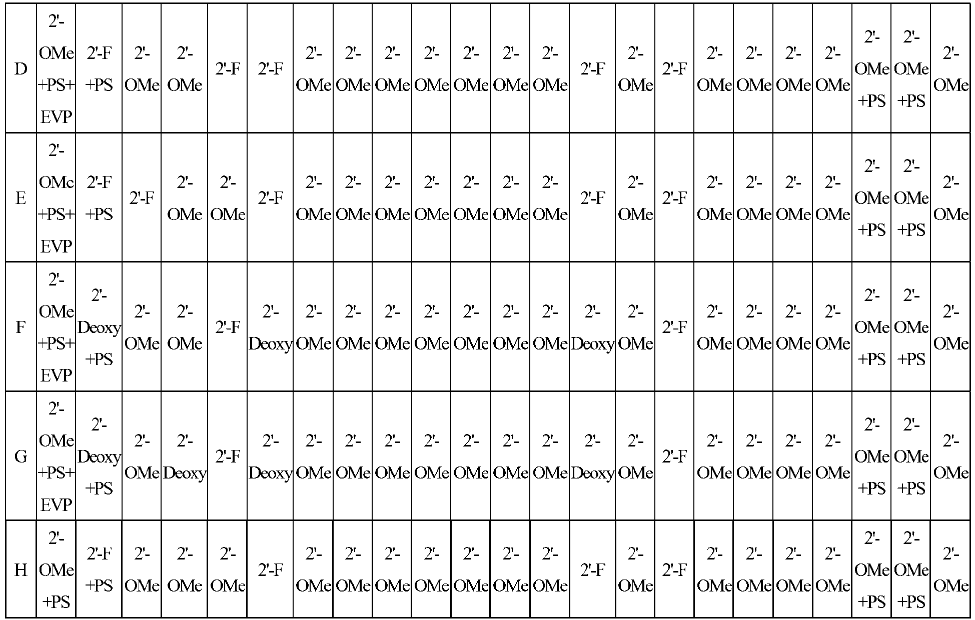

wherein 2'-OMe represents a 2'-O-methyl modification; 2'-F represents a 2'-fluoro modification; 2'-Deoxy represents a 2'-deoxy modification; PS indicates that, starting from the 5' end, the nucleotide at the position is linked to the adjacent nucleotide on the right via a phosphorothioate bond; EVP represents a 5'-vinyl-(E)-phosphonate. 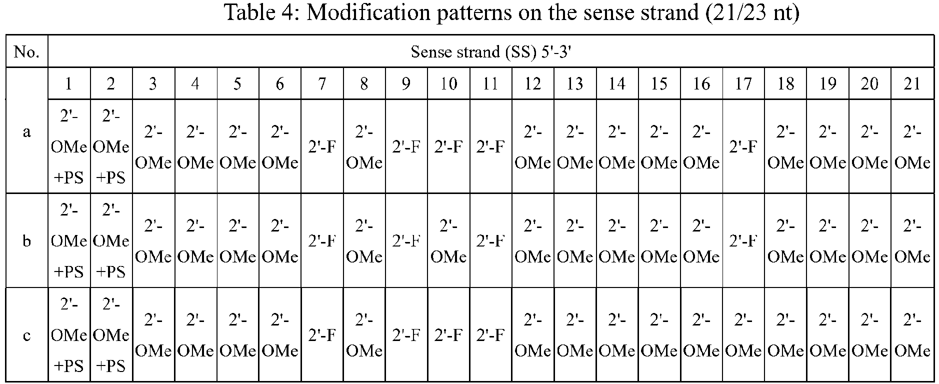
wherein 2'-OMe represents a 2'-O-methyl modification; 2'-F represents a 2'-fluoro modification; 2'-Deoxy represents a 2'-deoxy modification; PS indicates that, starting from the 5' end, the nucleotide at the position is linked to the adjacent nucleotide on the right via a phosphorothioate bond; EVP represents a 5'-vinyl-(E)-phosphonate. 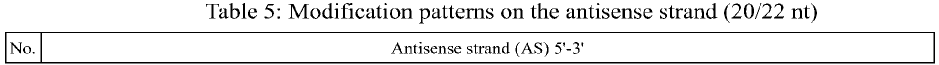 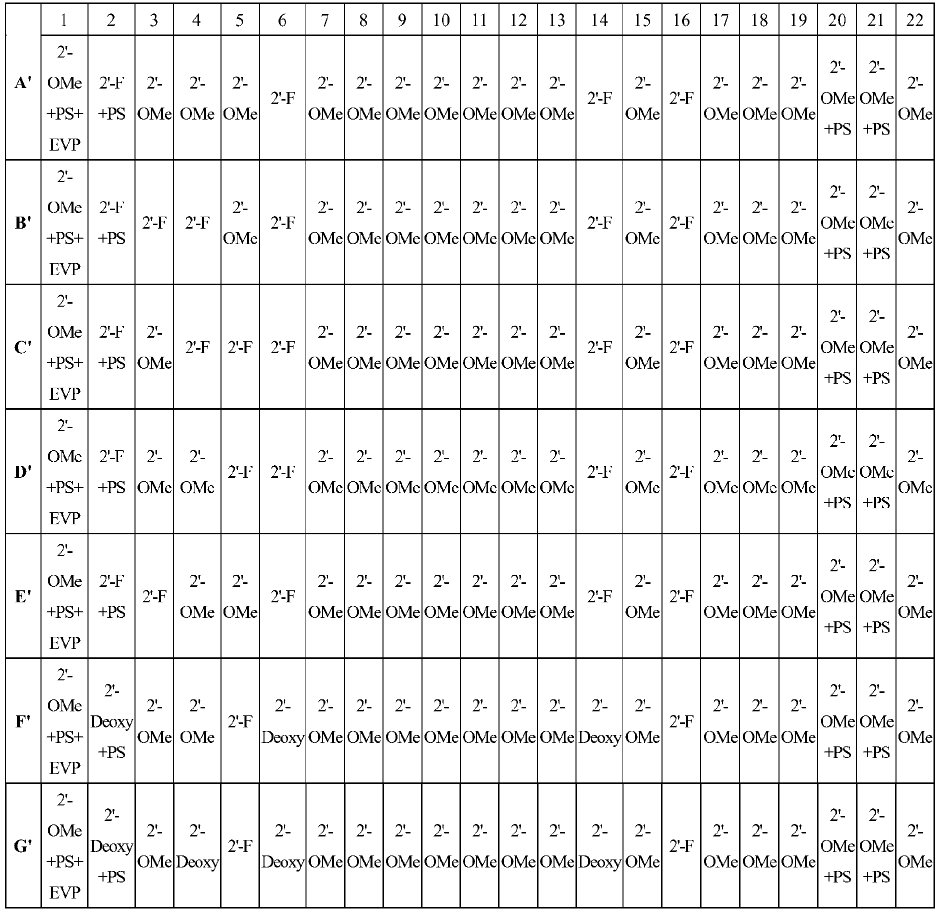
wherein 2'-OMe represents a 2'-O-methyl modification; 2'-F represents a 2'-fluoro modification; 2'-Deoxy represents a 2'-deoxy modification; PS indicates that, starting from the 5' end, the nucleotide at the position is linked to the adjacent nucleotide on the right via a phosphorothioate bond; EVP represents a 5'-vinyl-(E)-phosphonate. 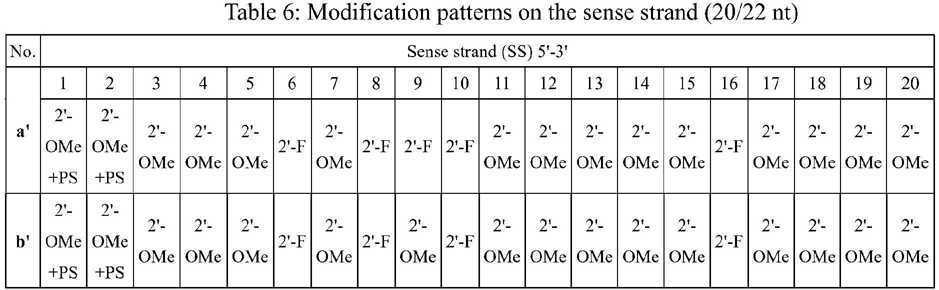
wherein 2'-OMe represents a 2'-O-methyl modification; 2'-F represents a 2'-fluoro modification; PS indicates that, starting from the 5' end, the nucleotide at the position is linked to the adjacent nucleotide on the right via a phosphorothioate bond; EVP represents a 5'-vinyl-(E)-phosphonate.

### 2.2.2 Template-modified siRNA derivatives disclosed herein

In this experiment, the siRNA motifs B00209, B00210, B00211, B00211s, B00212, B00214, B00215, B00216, B00217s, B00301, B00304, B00307, B00311, B00312, B00403, B00404, B00410, and B05202 were modified using the modification templates described in Section 2.2.1, as shown in Table 7. Natural 5' phosphorylation or simple direct 5' phosphorylation may undergo dephosphorylation in cells. Directly 5'-phosphorylated oligonucleotide strands may undergo 90% dephosphorylation after 2 hours of circulation in blood and complete loss after 24 hours. A 5'-E-VP phosphorylation design was adopted, in which E-vinylphosphonate replaces the bridging oxygen, thereby improving phosphorylation and stability. In the modification templates disclosed herein, the antisense strand incorporates the 5'-E-VP phosphorylation design. In Table 7, the sequence designated APC-SL corresponds to the most active compound siFXIg1-M1SP disclosed in CN113227376B, which was modified using the modification template described in the patent and was used as a positive control in this example.

**Table 7: siRNA derivatives**

| siRNA ID | siRNA ID | SEQ ID NO of antisense motif | Antisense modification pattern | Antisense strand (5'-3') | SEQ ID NO of antisense strand | SEO ID NO of sense motif | Sense modification pattern | Sense strand (5'-3') | SEQ ID NO of sense strand |
|---|---|---|---|---|---|---|---|---|---|
| | C00209-DV22 | 8 | H | Ums-Afs-Um-Am- Am-Gf-Am-Am- Am-Am-Um-Cm- Am-Uf-Cm-Cf- Um-Gm-Am-Am- Ams-Ams-Gm | 551 | 114 | c | Ums-Ums-Um- Cm-Am-Gm-Gf- Am-Uf-Gf-Af- Um-Um-Um-Um- Cm-Um-Um-Am- Um-Am | 425 |
| | C00209-DV25P | 8 | A | UmsEVP-Afs-Um- Am-Am-Gf-Am- Am-Am-Am-Um- Cm-Am-Uf-Cm- Cf-Um-Gm-Am- Am-Ams-Ams-Gm | 552 | 114 | a | Ums-Ums-Um- Cm-Am-Gm-Gf- Am-Uf-Gf-Af- Um-Um-Um-Um- Cm-Uf-Um-Am- Um-Am | 426 |
| | C00209-DV26P | 8 | B | UmsEVP-Afs-Uf- Af-Am-Gf-Am- Am-Am-Am-Um- Cm-Am-Uf-Cm- Cf-Um-Gm-Am- Am-Ams-Ams-Gm | 553 | 114 | a | Ums-Ums-Um- Cm-Am-Gm-Gf- Am-Uf-Gf-Af- Um-Um-Um-Um- Cm-Uf-Um-Am- Um-Am | 427 |
| B00209 | C00209-DV27P | 8 | C | UmsEVP-Afs-Um- Af-Af-Gf-Am-Am- Am-Am-Um-Cm- Am-Uf-Cm-Cf- Um-Gm-Am-Am- Ams-Ams-Gm | 554 | 114 | a | Ums-Ums-Um- Cm-Am-Gm-Gf- Am-Uf-Gf-Af- Um-Um-Um-Um- Cm-Uf-Um-Am- Um-Am | 428 |
| | C00209-DV29P | 8 | C | UmsEVP-Afs-Um- Af-Af-Gf-Am-Am- Am-Am-Um-Cm- Am-Uf-Cm-Cf- Um-Gm-Am-Am- Ams-Ams-Gm | 555 | 114 | b | Ums-Ums-Um- Cm-Am-Gm-Gf- Am-Uf-Gm-Af- Um-Um-Um-Um- Cm-Uf-Um-Am- Um-Am | 429 |
| | C00209-DV32P | 8 | D | UmsEVP-Afs-Um- Am-Af-Gf-Am- Am-Am-Am-Um- Cm-Am-Uf-Cm- Cf-Um-Gm-Am- Am-Ams-Ams-Gm | 556 | 114 | b | Ums-Ums-Um- Cm-Am-Gm-Gf- Am-Uf-Gm-Af- Um-Um-Um-Um- Cm-Uf-Um-Am- Um-Am | 430 |
| | C00209-DV34P | 8 | E | UmsEVP-Afs-Uf- Am-Am-Gf-Am- Am-Am-Um- | 557 | 114 | b | Ums-Ums-Um- Cm-Am-Gm-Gf- Am-Uf-Gm-Af- | 431 |
| | | | | Cm-Am-Uf-Cm- Cf-Um-Gm-Am- Am-Ams-Ams-Gm | | | | Um-Um-Um-Um- Cm-Uf-Um-Am- Um-Am | |
| | C00209-DV39P | 8 | F | UmsEVP-dAs-Um- Am-Af-dG-Am- Am-Am-Am-Um- Cm-Am-dU-Cm- Cf-Um-Gm-Am- Am-Ams-Ams-Gm | 558 | 114 | b | Ums-Ums-Um- Cm-Am-Gm-Gf- Am-Uf-Gm-Af- Um-Um-Um-Um- Cm-Uf-Um-Am- Um-Am | 432 |
| | C00209-DV40P | 8 | G | UmsEVP-dAs-Um- dA-Af-dG-Am- Am-Am-Am-Um- Cm-Am-dU-Cm- Cf-Um-Gm-Am- Am-Ams-Ams-Gm | 559 | 114 | b | Ums-Ums-Um- Cm-Am-Gm-Gf- Am-Uf-Gm-Af- Um-Um-Um-Um- dC-Uf-Um-Am- Um-Am | 433 |
| | C00210-DV22 | 9 | H | Ams-Ufs-Am-Um- Am-Af-Gm-Am- Am-Am-Am-Um- Cm-Af-Um-Cf- Cm-Um-Gm-Am- Ams-Ams-Am | 560 | 115 | c | Ums-Ums-Cm- Am-Gm-Gm-Af- Um-Gf-Af-Uf- Um-Um-Um-Cm- Um-Um-Am-Um- Am-Um | 434 |
| | C00210-DV25P | 9 | A | AmsEVP-Ufs-Am- Um-Am-Af-Gm- Am-Am-Am-Am- Um-Cm-Af-Um- Cf-Cm-Um-Gm- Am-Ams-Ams-Am | 561 | 115 | a | Ums-Ums-Cm- Am-Gm-Gm-Af- Um-Gf-Af-Uf- Um-Um-Um-Cm- Um-Uf-Am-Um- Am-Um | 435 |
| B00210 | C00210-DV26P | 9 | B | AmsEVP-Ufs-Af- Uf-Am-Af-Gm- Am-Am-Am-Am- Um-Cm-Af-Um- Cf-Cm-Um-Gm- Am-Ams-Ams-Am | 562 | 115 | a | Ums-Ums-Cm- Am-Gm-Gm-Af- Um-Gf-Af-Uf- Um-Um-Um-Cm- Um-Uf-Am-Um- Am-Um | 436 |
| | C00210-DV27P | 9 | C | AmsEVP-Ufs-Am- Uf-Af-Af-Gm-Am- Am-Am-Am-Um- Cm-Af-Um-Cf- Cm-Um-Gm-Am- Ams-Ams-Am | 563 | 115 | a | Ums-Ums-Cm- Am-Gm-Gm-Af- Um-Gf-Af-Uf- Um-Um-Um-Cm- Um-Uf-Am-Um- Am-Um | 437 |
| | C00210-DV29P | 9 | C | AmsEVP-Ufs-Am- Uf-Af-Af-Gm-Am- Am-Am-Am-Um- Cm-Af-Um-Cf- Cm-Um-Gm-Am- Ams-Ams-Am | 564 | 115 | b | Ums-Ums-Cm- Am-Gm-Gm-Af- Um-Gf-Am-Uf- Um-Um-Um-Cm- Um-Uf-Am-Um- Am-Um | 438 |
| | C00210-DV32P | 9 | D | AmsEVP-Ufs-Am- Um-Af-Af-Gm- Am-Am-Am-Am- Um-Cm-Af-Um- Cf-Cm-Um-Gm- Am-Ams-Ams-Am | 565 | 115 | b | Ums-Ums-Cm- Am-Gm-Gm-Af- Um-Gf-Am-Uf- Um-Um-Um-Cm- Um-Uf-Am-Um- Am-Um | 439 |
| | C00210-DV34P | 9 | E | AmsEVP-Ufs-Af- Um-Am-Af-Gm- Am-Am-Am-Am- Um-Cm-Af-Um- Cf-Cm-Um-Gm- Am-Ams-Ams-Am | 566 | 115 | b | Ums-Ums-Cm- Am-Gm-Gm-Af- Um-Gf-Am-Uf- Um-Um-Um-Cm- Um-Uf-Am-Um- Am-Um | 440 |
| | C00211-DV22 | 10 | H | Ums-Gfs-Am-Um- Am-Uf-Am-Am- Gm-Am-Am-Am- Am-Uf-Cm-Af- Um-Cm-Cm-Um- Gms-Ams-Am | 567 | 116 | c | Cms-Ams-Gm- Gm-Am-Um-Gf- Am-Uf-Uf-Uf- Um-Cm-Um-Um- Am-Um-Am-Um- Cm-Am | 441 |
| | C00211-DV25P | 10 | A | UmsEVP-Gfs-Am- Um-Am-Uf-Am- Am-Gm-Am-Am- Am-Am-Uf-Cm- Af-Um-Cm-Cm- Um-Gms-Ams-Am | 568 | 116 | a | Cms-Ams-Gm- Gm-Am-Um-Gf- Am-Uf-Uf-Uf- Um-Cm-Um-Um- Am-Uf-Am-Um- Cm-Am | 442 |
| B00211 | C00211-DV26P | 10 | B | UmsEVP-Gfs-Af- Uf-Am-Uf-Am- Am-Gm-Am-Am- Am-Am-Uf-Cm- Af-Um-Cm-Cm- Um-Gms-Ams-Am | 569 | 116 | a | Cms-Ams-Gm- Gm-Am-Um-Gf- Am-Uf-Uf-Uf- Um-Cm-Um-Um- Am-Uf-Am-Um- Cm-Am | 443 |
| | C00211-DV27P | 10 | C | UmsEVP-Gfs-Am- Uf-Af-Uf-Am-Am- Gm-Am-Am-Am- Am-Uf-Cm-Af- Um-Cm-Cm-Um- Gms-Ams-Am | 570 | 116 | a | Cms-Ams-Gm- Gm-Am-Um-Gf- Am-Uf-Uf-Uf- Um-Cm-Um-Um- Am-Uf-Am-Um- Cm-Am | 444 |
| | C00211-DV29P | 10 | C | UmsEVP-Gfs-Am- Uf-Af-Uf-Am-Am- Gm-Am-Am-Am- Am-Uf-Cm-Af- Um-Cm-Cm-Um- Gms-Ams-Am | 571 | 116 | b | Cms-Ams-Gm- Gm-Am-Um-Gf- Am-Uf-Um-Uf- Um-Cm-Um-Um- Am-Uf-Am-Um- Cm-Am | 445 |
| | C00211-DV32P | 10 | D | UmsEVP-Gfs-Am- Um-Af-Uf-Am- Am-Gm-Am-Am- Am-Am-Uf-Cm- | 572 | 116 | b | Cms-Ams-Gm- Gm-Am-Um-Gf- Am-Uf-Um-Uf- Um-Cm-Um-Um- | 446 |
| | | | | Af-Um-Cm-Cm- Um-Gms-Ams-Am | | | | Am-Uf-Am-Um- Cm-Am | |
| | C00211-DV34P | 10 | E | UmsEVP-Gfs-Af- Um-Am-Uf-Am- Am-Gm-Am-Am- Am-Am-Uf-Cm- Af-Um-Cm-Cm- Um-Gms-Ams-Am | 573 | 116 | b | Cms-Ams-Gm- Gm-Am-Um-Gf- Am-Uf-Um-Uf- Um-Cm-Um-Um- Am-Uf-Am-Um- Cm-Am | 447 |
| | C00211-DV39P | 10 | F | UmsEVP-dGs-Am- Um-Af-dU-Am- Am-Gm-Am-Am- Am-Am-dU-Cm- Af-Um-Cm-Cm- Um-Gms-Ams-Am | 574 | 116 | b | Cms-Ams-Gm- Gm-Am-Um-Gf- Am-Uf-Um-Uf- Um-Cm-Um-Um- Am-Uf-Am-Um- Cm-Am | 448 |
| | C00211-DV40P | 10 | G | UmsEVP-dGs-Am- dU-Af-dU-Am- Am-Gm-Am-Am- Am-Am-dU-Cm- Af-Um-Cm-Cm- Um-Gms-Ams-Am | 575 | 116 | b | Cms-Ams-Gm- Gm-Am-Um-Gf- Am-Uf-Um-Uf- Um-Cm-Um-Um- dA-Uf-Am-Um- Cm-Am | 449 |
| B00211s | C00211s - DV25SP | 11 | A' | UmsEVP-Gfs-Am- Um-Am-Uf-Am- Am-Gm-Am-Am- Am-Am-Uf-Cm- Af-Um-Cm-Cm- Ums-Gms-Am | 576 | 117 | a' | Ams-Gms-Gm- Am-Um-Gf-Am- Uf-Uf-Uf-Um- Cm-Um-Um-Am- Uf-Am-Um-Cm- Am | 450 |
| B00212 | C00212-DV22 | 12 | H | Ums-Ufs-Gm-Am- Um-Af-Um-Am- Am-Gm-Am-Am- Am-Af-Um-Cf- Am-Um-Cm-Cm- Ums-Gms-Am | 577 | 118 | c | Ams-Gms-Gm- Am-Um-Gm-Af- Um-Uf-Uf-Uf- Cm-Um-Um-Am- Um-Am-Um-Cm- Am-Am | 451 |
| | C00212-DV25P | 12 | A | UmsEVP-Ufs-Gm- Am-Um-Af-Um- Am-Am-Gm-Am- Am-Am-Af-Um- Cf-Am-Um-Cm- Cm-Ums-Gms-Am | 578 | 118 | a | Ams-Gms-Gm- Am-Um-Gm-Af- Um-Uf-Uf-Uf- Cm-Um-Um-Am- Um-Af-Um-Cm- Am-Am | 452 |
| | C00212-DV26P | 12 | B | UmsEVP-Ufs-Gf- Af-Um-Af-Um- Am-Am-Gm-Am- Am-Am-Af-Um- Cf-Am-Um-Cm- Cm-Ums-Gms-Am | 579 | 118 | a | Ams-Gms-Gm- Am-Um-Gm-Af- Um-Uf-Uf-Uf- Cm-Um-Um-Am- Um-Af-Um-Cm- Am-Am | 453 |
| | C00212-DV27P | 12 | C | UmsEVP-Ufs-Gm- Af-Uf-Af-Um-Am- | 580 | 118 | a | Ams-Gms-Gm- Am-Um-Gm-Af- | 454 |
| | | | | Am-Gm-Am-Am- Am-Af-Um-Cf- Am-Um-Cm-Cm- Ums-Gms-Am | | | | Um-Uf-Uf-Uf- Cm-Um-Um-Am- Um-Af-Um-Cm- Am-Am | |
| | C00212-DV29P | 12 | C | UmsEVP-Ufs-Gm- Af-Uf-Af-Um-Am- Am-Gm-Am-Am- Am-Af-Um-Cf- Am-Um-Cm-Cm- Ums-Gms-Am | 581 | 118 | b | Ams-Gms-Gm- Am-Um-Gm-Af- Um-Uf-Um-Uf- Cm-Um-Um-Am- Um-Af-Um-Cm- Am-Am | 455 |
| | C00212-DV32P | 12 | D | UmsEVP-Ufs-Gm- Am-Uf-Af-Um- Am-Am-Gm-Am- Am-Am-Af-Um- Cf-Am-Um-Cm- Cm-Ums-Gms-Am | 582 | 118 | b | Ams-Gms-Gm- Am-Um-Gm-Af- Um-Uf-Um-Uf- Cm-Um-Um-Am- Um-Af-Um-Cm- Am-Am | 456 |
| | C00212-DV34P | 12 | E | UmsEVP-Ufs-Gf- Am-Um-Af-Um- Am-Am-Gm-Am- Am-Am-Af-Um- Cf-Am-Um-Cm- Cm-Ums-Gms-Am | 583 | 118 | b | Ams-Gms-Gm- Am-Um-Gm-Af- Um-Uf-Um-Uf- Cm-Um-Um-Am- Um-Af-Um-Cm- Am-Am | 457 |
| | C00214-DV22 | 14 | H | Ams-Ufs-Am-Am- Gm-Af-Am-Am- Am-Um-Cm-Am- Um-Cf-Cm-Uf- Gm-Am-Am-Am- Ams-Gms-Am | 584 | 120 | c | Ums-Ums-Um- Um-Cm-Am-Gf- Gm-Af-Uf-Gf- Am-Um-Um-Um- Um-Cm-Um-Um- Am-Um | 458 |
| B00214 | C00214-DV25P | 14 | A | AmsEVP-Ufs-Am- Am-Gm-Af-Am- Am-Am-Um-Cm- Am-Um-Cf-Cm- Uf-Gm-Am-Am- Am-Ams-Gms-Am | 585 | 120 | a | Ums-Ums-Um- Um-Cm-Am-Gf- Gm-Af-Uf-Gf- Am-Um-Um-Um- Um-Cf-Um-Um- Am-Um | 459 |
| | C00214-DV26P | 14 | B | AmsEVP-Ufs-Af- Af-Gm-Af-Am- Am-Am-Um-Cm- Am-Um-Cf-Cm- Uf-Gm-Am-Am- Am-Ams-Gms-Am | 586 | 120 | a | Ums-Ums-Um- Um-Cm-Am-Gf- Gm-Af-Uf-Gf- Am-Um-Um-Um- Um-Cf-Um-Um- Am-Um | 460 |
| | C00214-DV27P | 14 | C | AmsEVP-Ufs-Am- Af-Gf-Af-Am-Am- Am-Um-Cm-Am- Um-Cf-Cm-Uf- Gm-Am-Am-Am- Ams-Gms-Am | 587 | 120 | a | Ums-Ums-Um- Um-Cm-Am-Gf- Gm-Af-Uf-Gf- Am-Um-Um-Um- Um-Cf-Um-Um- Am-Um | 461 |
| | C00214-DV29P | 14 | C | AmsEVP-Ufs-Am- Af-Gf-Af-Am-Am- Am-Um-Cm-Am- Um-Cf-Cm-Uf- Gm-Am-Am-Am- Ams-Gms-Am | 588 | 120 | b | Ums-Ums-Um- Um-Cm-Am-Gf- Gm-Af-Um-Gf- Am-Um-Um-Um- Um-Cf-Um-Um- Am-Um | 462 |
| | C00214-DV32P | 14 | D | AmsEVP-Ufs-Am- Am-Gf-Af-Am- Am-Am-Um-Cm- Am-Um-Cf-Cm- Uf-Gm-Am-Am- Am-Ams-Gms-Am | 589 | 120 | b | Ums-Ums-Um- Um-Cm-Am-Gf- Gm-Af-Um-Gf- Am-Um-Um-Um- Um-Cf-Um-Um- Am-Um | 463 |
| | C00214-DV34P | 14 | E | AmsEVP-Ufs-Af- Am-Gm-Af-Am- Am-Am-Um-Cm- Am-Um-Cf-Cm- Uf-Gm-Am-Am- Am-Ams-Gms-Am | 590 | 120 | b | Ums-Ums-Um- Um-Cm-Am-Gf- Gm-Af-Um-Gf- Am-Um-Um-Um- Um-Cf-Um-Um- Am-Um | 464 |
| | C00215-DV22 | 15 | H | UUms-Afs-Um- Am-Um-Af-Am- Gm-Am-Am-Am- Am-Um-Cf-Am- Uf-Cm-Cm-Um- Gm-Ams-Ams-Am | 591 | 121 | c | Ums-Cms-Am- Gm-Gm-Am-Uf- Gm-Af-Uf-Uf- Um-Um-Cm-Um- Um-Am-Um-Am- Um-am | 465 |
| | C00215-DV25P | 15 | A | UmsEVP-Afs-Um- Am-Um-Af-Am- Gm-Am-Am-Am- Am-Um-Cf-Am- Uf-Cm-Cm-Um- Gm-Ams-Ams-Am | 592 | 121 | a | Ums-Cms-Am- Gm-Gm-Am-Uf- Gm-Af-Uf-Uf- Um-Um-Cm-Um- Um-Af-Um-Am- Um-am | 466 |
| B00215 | C00215-DV26P | 15 | B | UmsEVP-Afs-Uf- Af-Um-Af-Am- Gm-Am-Am-Am- Am-Um-Cf-Am- Uf-Cm-Cm-Um- Gm-Ams-Ams-Am | 593 | 121 | a | Ums-Cms-Am- Gm-Gm-Am-Uf- Gm-Af-Uf-Uf- Um-Um-Cm-Um- Um-Af-Um-Am- Um-am | 467 |
| | C00215-DV27P | 15 | C | UmsEVP-Afs-Um- Af-Uf-Af-Am-Gm- Am-Am-Am-Am- Um-Cf-Am-Uf- Cm-Cm-Um-Gm- Ams-Ams-Am | 594 | 121 | a | Ums-Cms-Am- Gm-Gm-Am-Uf- Gm-Af-Uf-Uf- Um-Um-Cm-Um- Um-Af-Um-Am- Um-am | 468 |
| | C00215-DV29P | 15 | C | UmsEVP-Afs-Um- Af-Uf-Af-Am-Gm- Am-Am-Am-Am- Um-Cf-Am-Uf- | 595 | 121 | b | Ums-Cms-Am- Gm-Gm-Am-Uf- Gm-Af-Um-Uf- Um-Um-Cm-Um- | 469 |
| | | | | Cm-Cm-Um-Gm- Ams-Ams-Am | | | | Um-Af-Um-Am- Um-Am | |
| | C00215-DV32P | 15 | D | UmsEVP-Afs-Um- Am-Uf-Af-Am- Gm-Am-Am-Am- Am-Um-Cf-Am- Uf-Cm-Cm-Um- Gm-Ams-Ams-Am | 596 | 121 | b | Ums-Cms-Am- Gm-Gm-Am-Uf- Gm-Af-Um-Uf- Um-Um-Cm-Um- Um-Af-Um-Am- Um-Am | 470 |
| | C00215-DV34P | 15 | E | UmsEVP-Afs-Uf- Am-Um-Af-Am- Gm-Am-Am-Am- Am-Um-Cf-Am- Uf-Cm-Cm-Um- Gm-Ams-Ams-Am | 597 | 121 | b | Ums-Cms-Am- Gm-Gm-Am-Uf- Gm-Af-Um-Uf- Um-Um-Cm-Um- Um-Af-Um-Am- Um-Am | 471 |
| | C00215-DV39P | 15 | F | UmsEVP-dAs-Um- Am-Uf-dA-Am- Gm-Am-Am-Am- Am-Um-dC-Am- Uf-Cm-Cm-Um- Gm-Ams-Ams-Am | 598 | 121 | b | Ums-Cms-Am- Gm-Gm-Am-Uf- Gm-Af-Um-Uf- Um-Um-Cm-Um- Um-Af-Um-Am- Um-Am | 472 |
| | C00215-DV40P | 15 | G | UmsEVP-dAs-Um- dA-Uf-dA-Am- Gm-Am-Am-Am- Am-Um-dC-Am- Uf-Cm-Cm-Um- Gm-Ams-Ams-Am | 599 | 121 | b | Ums-Cms-Am- Gm-Gm-Am-Uf- Gm-Af-Um-Uf- Um-Um-Cm-Um- dU-Af-Um-Am- Um-Am | 473 |
| | C00216-DV22 | 16 | H | Ums-Ufs-Um-Gm- Am-Uf-Am-Um- Am-Am-Gm-Am- Am-Af-Am-Uf- Cm-Am-Um-Cm- Cms-Ums-Gm | 600 | 122 | c | Gms-Gms-Am- Um-Gm-Am-Uf- Um-Uf-Uf-Cf- Um-Um-Am-Um- Am-Um-Cm-Am- Am-Am | 474 |
| B00216 | C00216-DV25P | 16 | A | UmsEVP-Ufs-Um- Gm-Am-Uf-Am- Um-Am-Am-Gm- Am-Am-Af-Am- Uf-Cm-Am-Um- Cm-Cms-Ums-Gm | 601 | 122 | a | Gms-Gms-Am- Um-Gm-Am-Uf- Um-Uf-Uf-Cf- Um-Um-Am-Um- Am-Uf-Cm-Am- Am-Am | 475 |
| | C00216-DV26P | 16 | B | UmsEVP-Ufs-Uf- Gf-Am-Uf-Am- Um-Am-Am-Gm- Am-Am-Af-Am- Uf-Cm-Am-Um- Cm-Cms-Ums-Gm | 602 | 122 | a | Gms-Gms-Am- Um-Gm-Am-Uf- Um-Uf-Uf-Cf- Um-Um-Am-Um- Am-Uf-Cm-Am- Am-Am | 476 |
| | C00216-DV27P | 16 | C | UmsEVP-Ufs-Um- Gf-Af-Uf-Am-Um- | 603 | 122 | a | Gms-Gms-Am- Um-Gm-Am-Uf- | 477 |
| | | | | Am-Am-Gm-Am- Am-Af-Am-Uf- Cm-Am-Um-Cm- Cms-Ums-Gm | | | | Um-Uf-Uf-Cf- Um-Um-Am-Um- Am-Uf-Cm-Am- Am-Am | |
| | C00216-DV29P | 16 | C | UmsEVP-Ufs-Um- Gf-Af-Uf-Am-Um- Am-Am-Gm-Am- Am-Af-Am-Uf- Cm-Am-Um-Cm- Cms-Ums-Gm | 604 | 122 | b | Gms-Gms-Am- Um-Gm-Am-Uf- Um-Uf-Um-Cf- Um-Um-Am-Um- Am-Uf-Cm-Am- Am-Am | 478 |
| | C00216-DV32P | 16 | D | UmsEVP-Ufs-Um- Gm-Af-Uf-Am- Um-Am-Am-Gm- Am-Am-Af-Am- Uf-Cm-Am-Um- Cm-Cms-Ums-Gm | 605 | 122 | b | Gms-Gms-Am- Um-Gm-Am-Uf- Um-Uf-Um-Cf- Um-Um-Am-Um- Am-Uf-Cm-Am- Am-Am | 479 |
| | C00216-DV34P | 16 | E | UmsEVP-Ufs-Uf- Gm-Am-Uf-Am- Um-Am-Am-Gm- Am-Am-Af-Am- Uf-Cm-Am-Um- Cm-Cms-Ums-Gm | 606 | 122 | b | Gms-Gms-Am- Um-Gm-Am-Uf- Um-Uf-Um-Cf- Um-Um-Am-Um- Am-Uf-Cm-Am- Am-Am | 480 |
| B00217s | C00217s - DV32SP | 17 | D' | AmsEVP-Cfs-Um- Um-Gf-Af-Um- Am-Um-Am-Am- Gm-Am-Af-Am- Af-Um-Cm-Am- Ums-Cms-Cm | 607 | 123 | b' | Ams-Ums-Gm- Am-Um-Uf-Um- Uf-Cm-Uf-Um- Am-Um-Am-Um- Cf-Am-Am-Gm- Um | 481 |
| B00301 | C00301-DV22 | 18 | H | Ams-Afs-Am-Um- Gm-Af-Am-Am- Um-Gm-Um-Am- Cm-Cf-Am-Cf- Um-Um-Gm-Am- Ums-Ams-Um | 608 | 124 | c | Ams-Ums-Cm- Am-Am-Gm-Uf- Gm-Gf-Uf-Af- Cm-Am-Um-Um- Um-Cm-Am-Um- Um-Um | 482 |
| | C00301-DV25P | 18 | A | AmsEVP-Afs-Am- Um-Gm-Af-Am- Am-Um-Gm-Um- Am-Cm-Cf-Am- Cf-Um-Um-Gm- Am-Ums-Ams-Um | 609 | 124 | a | Ams-Ums-Cm- Am-Am-Gm-Uf- Gm-Gf-Uf-Af- Cm-Am-Um-Um- Um-Cf-Am-Um- Um-Um | 483 |
| | C00301-DV26P | 18 | B | AmsEVP-Afs-Af- Uf-Gm-Af-Am- Am-Um-Gm-Um- Am-Cm-Cf-Am- Cf-Um-Um-Gm- Am-Ums-Ams-Um | 610 | 124 | a | Ams-Ums-Cm- Am-Am-Gm-Uf- Gm-Gf-Uf-Af- Cm-Am-Um-Um- Um-Cf-Am-Um- Um-Um | 484 |
| | C00301-DV27P | 18 | C | AmsEVP-Afs-Am- Uf-Gf-Af-Am-Am- Um-Gm-Um-Am- Cm-Cf-Am-Cf- Um-Um-Gm-Am- Ums-Ams-Um | 611 | 124 | a | Ams-Ums-Cm- Am-Am-Gm-Uf- Gm-Gf-Uf-Af- Cm-Am-Um-Um- Um-Cf-Am-Um- Um-Um | 485 |
| | C00301-DV29P | 18 | C | AmsEVP-Afs-Am- Uf-Gf-Af-Am-Am- Um-Gm-Um-Am- Cm-Cf-Am-Cf- Um-Um-Gm-Am- Ums-Ams-Um | 612 | 124 | b | Ams-Ums-Cm- Am-Am-Gm-Uf- Gm-Gf-Um-Af- Cm-Am-Um-Um- Um-Cf-Am-Um- Um-Um | 486 |
| | C00301-DV32P | 18 | D | AmsEVP-Afs-Am- Um-Gf-Af-Am- Am-Um-Gm-Um- Am-Cm-Cf-Am- Cf-Um-Um-Gm- Am-Ums-Ams-Um | 613 | 124 | b | Ams-Ums-Cm- Am-Am-Gm-Uf- Gm-Gf-Um-Af- Cm-Am-Um-Um- Um-Cf-Am-Um- Um-Um | 487 |
| | C00301-DV34P | 18 | E | AmsEVP-Afs-Af- Um-Gm-Af-Am- Am-Um-Gm-Um- Am-Cm-Cf-Am- Cf-Um-Um-Gm- Am-Ums-Ams-Um | 614 | 124 | b | Ams-Ums-Cm- Am-Am-Gm-Uf- Gm-Gf-Um-Af- Cm-Am-Um-Um- Um-Cf-Am-Um- Um-Um | 488 |
| | C00304-DV22 | 21 | H | Ams-Ufs-Gm-Am- Am-Af-Um-Gm- Um-Am-Cm-Cm- Am-Cf-Um-Uf- Gm-Am-Um-Am- Ums-Ams-Am | 615 | 127 | c | Ams-Ums-Am- Um-Cm-Am-Af- Gm-Uf-Gf-Gf- Um-Am-Cm-Am- Um-Um-Um-Cm- Am-Um | 489 |
| B00304 | C00304-DV25P | 21 | A | AmsEVP-Ufs-Gm- Am-Am-Af-Um- Gm-Um-Am-Cm- Cm-Am-Cf-Um- Uf-Gm-Am-Um- Am-Ums-Ams-Am | 616 | 127 | a | Ams-Ums-Am- Um-Cm-Am-Af- Gm-Uf-Gf-Gf- Um-Am-Cm-Am- Um-Uf-Um-Cm- Am-Um | 490 |
| | C00304-DV26P | 21 | B | AmsEVP-Ufs-Gf- Af-Am-Af-Um- Gm-Um-Am-Cm- Cm-Am-Cf-Um- Uf-Gm-Am-Um- Am-Ums-Ams-Am | 617 | 127 | a | Ams-Ums-Am- Um-Cm-Am-Af- Gm-Uf-Gf-Gf- Um-Am-Cm-Am- Um-Uf-Um-Cm- Am-Um | 491 |
| | C00304-DV27P | 21 | C | AmsEVP-Ufs-Gm- Af-Af-Af-Um-Gm- Um-Am-Cm-Cm- Am-Cf-Um-Uf- | 618 | 127 | a | Ams-Ums-Am- Um-Cm-Am-Af- Gm-Uf-Gf-Gf- Um-Am-Cm-Am- | 492 |
| | | | | Gm-Am-Um-Am- Ums-Ams-Am | | | | Um-Uf-Um-Cm- Am-Um | |
| | C00304-DV29P | 21 | C | AmsEVP-Ufs-Gm- Af-Af-Af-Um-Gm- Um-Am-Cm-Cm- Am-Cf-Um-Uf- Gm-Am-Um-Am- Ums-Ams-Am | 619 | 127 | b | Ams-Ums-Am- Um-Cm-Am-Af- Gm-Uf-Gm-Gf- Um-Am-Cm-Am- Um-Uf-Um-Cm- Am-Um | 493 |
| | C00304-DV32P | 21 | D | AmsEVP-Ufs-Gm- Am-Af-Af-Um- Gm-Um-Am-Cm- Cm-Am-Cf-Um- Uf-Gm-Am-Um- Am-Ums-Ams-Am | 620 | 127 | b | Ams-Ums-Am- Um-Cm-Am-Af- Gm-Uf-Gm-Gf- Um-Am-Cm-Am- Um-Uf-Um-Cm- Am-Um | 494 |
| | C00304-DV34P | 21 | E | AmsEVP-Ufs-Gf- Am-Am-Af-Um- Gm-Um-Am-Cm- Cm-Am-Cf-Um- Uf-Gm-Am-Um- Am-Ums-Ams-Am | 621 | 127 | b | Ams-Ums-Am- Um-Cm-Am-Af- Gm-Uf-Gm-Gf- Um-Am-Cm-Am- Um-Uf-Um-Cm- Am-Um | 495 |
| | C00307-DV22 | 24 | H | Ams-Afs-Am-Um- Am-Af-Am-Am- Um-Gm-Am-Am- Am-Uf-Gm-Uf- Am-Cm-Cm-Am- Cms-Ums-Um | 622 | 130 | c | Gms-Ums-Gm- Gm-Um-Am-Cf- Am-Uf-Uf-Uf- Cm-Am-Um-Um- Um-Um-Am-Um- Um-Um | 496 |
| B00307 | C00307-DV25P | 24 | A | AmsEVP-Afs-Am- Um-Am-Af-Am- Am-Um-Gm-Am- Am-Am-Uf-Gm- Uf-Am-Cm-Cm- Am-Cms-Ums-Um | 623 | 130 | a | Gms-Ums-Gm- Gm-Um-Am-Cf- Am-Uf-Uf-Uf- Cm-Am-Um-Um- Um-Uf-Am-Um- Um-Um | 497 |
| | C00307-DV26P | 24 | B | AmsEVP-Afs-Af- Uf-Am-Af-Am- Am-Um-Gm-Am- Am-Am-Uf-Gm- Uf-Am-Cm-Cm- Am-Cms-Ums-Um | 624 | 130 | a | Gms-Ums-Gm- Gm-Um-Am-Cf- Am-Uf-Uf-Uf- Cm-Am-Um-Um- Um-Uf-Am-Um- Um-Um | 498 |
| | C00307-DV27P | 24 | C | AmsEVP-Afs-Am- Uf-Af-Af-Am-Am- Um-Gm-Am-Am- Am-Uf-Gm-Uf- Am-Cm-Cm-Am- Cms-Ums-Um | 625 | 130 | a | Gms-Ums-Gm- Gm-Um-Am-Cf- Am-Uf-Uf-Uf- Cm-Am-Um-Um- Um-Uf-Am-Um- Um-Um | 499 |
| | C00307-DV29P | 24 | C | AmsEVP-Afs-Am- Uf-Af-Af-Am-Am- | 626 | 130 | b | Gms-Ums-Gm- Gm-Um-Am-Cf- | 500 |
| | | | | Um-Gm-Am-Am- Am-Uf-Gm-Uf- Am-Cm-Cm-Am- Cms-Ums-Um | | | | Am-Uf-Um-Uf- Cm-Am-Um-Um- Um-Uf-Am-Um- Um-Um | |
| | C00307-DV32P | 24 | D | AmsEVP-Afs-Am- Um-Af-Af-Am- Am-Um-Gm-Am- Am-Am-Uf-Gm- Uf-Am-Cm-Cm- Am-Cms-Ums-Um | 627 | 130 | b | Gms-Ums-Gm- Gm-Um-Am-Cf- Am-Uf-Um-Uf- Cm-Am-Um-Um- Um-Uf-Am-Um- Um-Um | 501 |
| | C00307-DV34P | 24 | E | AmsEVP-Afs-Af- Um-Am-Af-Am- Am-Um-Gm-Am- Am-Am-Uf-Gm- Uf-Am-Cm-Cm- Am-Cms-Ums-Um | 628 | 130 | b | Gms-Ums-Gm- Gm-Um-Am-Cf- Am-Uf-Um-Uf- Cm-Am-Um-Um- Um-Uf-Am-Um- Um-Um | 502 |
| | C00307-DV39P | 24 | F | AmsEVP-dAs-Am- Um-Af-dA-Am- Am-Um-Gm-Am- Am-Am-dU-Gm- Uf-Am-Cm-Cm- Am-Cms-Ums-Um | 629 | 130 | b | Gms-Ums-Gm- Gm-Um-Am-Cf- Am-Uf-Um-Uf- Cm-Am-Um-Um- Um-Uf-Am-Um- Um-Um | 503 |
| | C00307-DV40P | 24 | G | AmsEVP-dAs-Am- dU-Af-dA-Am- Am-Um-Gm-Am- Am-Am-dU-Gm- Uf-Am-Cm-Cm- Am-Cms-Ums-Um | 630 | 130 | b | Gms-Ums-Gm- Gm-Um-Am-Cf- Am-Uf-Um-Uf- Cm-Am-Um-Um- dU-Uf-Am-Um- Um-Um | 504 |
| B00311 | C00311-DV22 | 28 | H | Ams-Afs-Am-Cm- Um-Gf-Am-Am- Gm-Um-Am-Am- Am-Uf-Am-Af- Am-Am-Um-Gm- Ams-Ams-Am | 631 | 134 | c | Ums-Cms-Am- Um-Um-Um-Uf- Am-Uf-Uf-Uf- Am-Cm-Um-Um- Cm-Am-Gm-Um- Um-Um | 505 |
| | C00311-DV25P | 28 | A | AmsEVP-Afs-Am- Cm-Um-Gf-Am- Am-Gm-Um-Am- Am-Am-Uf-Am- Af-Am-Am-Um- Gm-Ams-Ams-Am | 632 | 134 | a | Ums-Cms-Am- Um-Um-Um-Uf- Am-Uf-Uf-Uf- Am-Cm-Um-Um- Cm-Af-Gm-Um- Um-Um | 506 |
| | C00311-DV26P | 28 | B | AmsEVP-Afs-Af- Cf-Um-Gf-Am- Am-Gm-Um-Am- Am-Am-Uf-Am- Af-Am-Am-Um- Gm-Ams-Ams-Am | 633 | 134 | a | Ums-Cms-Am- Um-Um-Um-Uf- Am-Uf-Uf-Uf- Am-Cm-Um-Um- Cm-Af-Gm-Um- Um-Um | 507 |
| | C00311-DV27P | 28 | C | AmsEVP-Afs-Am- Cf-Uf-Gf-Am-Am- Gm-Um-Am-Am- Am-Uf-Am-Af- Am-Am-Um-Gm- Ams-Ams-Am | 634 | 134 | a | Ums-Cms-Am- Um-Um-Um-Uf- Am-Uf-Uf-Uf- Am-Cm-Um-Um- Cm-Af-Gm-Um- Um-Um | 508 |
| | C00311-DV29P | 28 | C | AmsEVP-Afs-Am- Cf-Uf-Gf-Am-Am- Gm-Um-Am-Am- Am-Uf-Am-Af- Am-Am-Um-Gm- Ams-Ams-Am | 635 | 134 | b | Ums-Cms-Am- Um-Um-Um-Uf- Am-Uf-Um-Uf- Am-Cm-Um-Um- Cm-Af-Gm-Um- Um-Um | 509 |
| | C00311-DV32P | 28 | D | AmsEVP-Afs-Am- Cm-Uf-Gf-Am- Am-Gm-Um-Am- Am-Am-Uf-Am- Af-Am-Am-Um- Gm-Ams-Ams-Am | 636 | 134 | b | Ums-Cms-Am- Um-Um-Um-Uf- Am-Uf-Um-Uf- Am-Cm-Um-Um- Cm-Af-Gm-Um- Um-Um | 510 |
| | C00311-DV34P | 28 | E | AmsEVP-Afs-Af- Cm-Um-Gf-Am- Am-Gm-Um-Am- Am-Am-Uf-Am- Af-Am-Am-Um- Gm-Ams-Ams-Am | 637 | 134 | b | Ums-Cms-Am- Um-Um-Um-Uf- Am-Uf-Um-Uf- Am-Cm-Um-Um- Cm-Af-Gm-Um- Um-Um | 511 |
| B00312 | C00312-DV22 | 29 | H | Ams-Afs-Um-Am- Am-Af-Am-Um- Gm-Am-Am-Am- Um-Gf-Um-Af- Cm-Cm-Am-Cm- Ums-Ums-Gm | 638 | 135 | c | Ams-Gms-Um- Gm-Gm-Um-Af- Cm-Af-Uf-Uf- Um-Cm-Am-Um- Um-Um-Um-Am- Um-Um | 512 |
| | C00312-DV25P | 29 | A | AmsEVP-Afs-Um- Am-Am-Af-Am- Um-Gm-Am-Am- Am-Um-Gf-Um- Af-Cm-Cm-Am- Cm-Ums-Ums-Gm | 639 | 135 | a | Ams-Gms-Um- Gm-Gm-Um-Af- Cm-Af-Uf-Uf- Um-Cm-Am-Um- Um-Uf-Um-Am- Um-Um | 513 |
| | C00312-DV26P | 29 | B | AmsEVP-Afs-Uf- Af-Am-Af-Am- Um-Gm-Am-Am- Am-Um-Gf-Um- Af-Cm-Cm-Am- Cm-Ums-Ums-Gm | 640 | 135 | a | Ams-Gms-Um- Gm-Gm-Um-Af- Cm-Af-Uf-Uf- Um-Cm-Am-Um- Um-Uf-Um-Am- Um-Um | 514 |
| | C00312-DV27P | 29 | C | AmsEVP-Afs-Um- Af-Af-Af-Am-Um- Gm-Am-Am-Am- Um-Gf-Um-Af- | 641 | 135 | a | Ams-Gms-Um- Gm-Gm-Um-Af- Cm-Af-Uf-Uf- Um-Cm-Am-Um- | 515 |
| | | | | Cm-Cm-Am-Cm- Ums-Ums-Gm | | | | Um-Uf-Um-Am- Um-Um | |
| | C00312-DV29P | 29 | C | AmsEVP-Afs-Um- Af-Af-Af-Am-Um- Gm-Am-Am-Am- Um-Gf-Um-Af- Cm-Cm-Am-Cm- Ums-Ums-Gm | 642 | 135 | b | Ams-Gms-Um- Gm-Gm-Um-Af- Cm-Af-Um-Uf- Um-Cm-Am-Um- Um-Uf-Um-Am- Um-Um | 516 |
| | C00312-DV32P | 29 | D | AmsEVP-Afs-Um- Am-Af-Af-Am- Um-Gm-Am-Am- Am-Um-Gf-Um- Af-Cm-Cm-Am- Cm-Ums-Ums-Gm | 643 | 135 | b | Ams-Gms-Um- Gm-Gm-Um-Af- Cm-Af-Um-Uf- Um-Cm-Am-Um- Um-Uf-Um-Am- Um-Um | 517 |
| | C00312-DV34P | 29 | E | AmsEVP-Afs-Uf- Am-Am-Af-Am- Um-Gm-Am-Am- Am-Um-Gf-Um- Af-Cm-Cm-Am- Cm-Ums-Ums-Gm | 644 | 135 | b | Ams-Gms-Um- Gm-Gm-Um-Af- Cm-Af-Um-Uf- Um-Cm-Am-Um- Um-Uf-Um-Am- Um-Um | 518 |
| | C00403-DV22 | 32 | H | Ams-Ufs-Um-Cm- Am-Cf-Cm-Am- Gm-Am-Am-Am- Cm-Uf-Gm-Af- Am-Gm-Um-Am- Ams-Ams-Um | 645 | 138 | c | Ums-Ums-Am- Cm-Um-Um-Cf- Am-Gf-Uf-Uf- Um-Cm-Um-Gm- Gm-Um-Gm-Am- Am-Um | 519 |
| B00403 | C00403-DV25P | 32 | A | AmsEVP-Ufs-Um- Cm-Am-Cf-Cm- Am-Gm-Am-Am- Am-Cm-Uf-Gm- Af-Am-Gm-Um- Am-Ams-Ams-Um | 646 | 138 | a | Ums-Ums-Am- Cm-Um-Um-Cf- Am-Gf-Uf-Uf- Um-Cm-Um-Gm- Gm-Uf-Gm-Am- Am-Um | 520 |
| | C00403-DV26P | 32 | B | AmsEVP-Ufs-Uf- Cf-Am-Cf-Cm- Am-Gm-Am-Am- Am-Cm-Uf-Gm- Af-Am-Gm-Um- Am-Ams-Ams-Um | 647 | 138 | a | Ums-Ums-Am- Cm-Um-Um-Cf- Am-Gf-Uf-Uf- Um-Cm-Um-Gm- Gm-Uf-Gm-Am- Am-Um | 521 |
| | C00403-DV27P | 32 | C | AmsEVP-Ufs-Um- Cf-Af-Cf-Cm-Am- Gm-Am-Am-Am- Cm-Uf-Gm-Af- Am-Gm-Um-Am- Ams-Ams-Um | 648 | 138 | a | Ums-Ums-Am- Cm-Um-Um-Cf- Am-Gf-Uf-Uf- Um-Cm-Um-Gm- Gm-Uf-Gm-Am- Am-Um | 522 |
| | C00403-DV29P | 32 | C | AmsEVP-Ufs-Um- Cf-Af-Cf-Cm-Am- | 649 | 138 | b | Ums-Ums-Am- Cm-Um-Um-Cf- | 523 |
| | | | | Gm-Am-Am-Am- Cm-Uf-Gm-Af- Am-Gm-Um-Am- Ams-Ams-Um | | | | Am-Gf-Um-Uf- Um-Cm-Um-Gm- Gm-Uf-Gm-Am- Am-Um | |
| | C00403-DV32P | 32 | D | AmsEVP-Ufs-Um- Cm-Af-Cf-Cm- Am-Gm-Am-Am- Am-Cm-Uf-Gm- Af-Am-Gm-Um- Am-Ams-Ams-Um | 650 | 138 | b | Ums-Ums-Am- Cm-Um-Um-Cf- Am-Gf-Um-Uf- Um-Cm-Um-Gm- Gm-Uf-Gm-Am- Am-Um | 524 |
| | C00403-DV34P | 32 | E | AmsEVP-Ufs-Uf- Cm-Am-Cf-Cm- Am-Gm-Am-Am- Am-Cm-Uf-Gm- Af-Am-Gm-Um- Am-Ams-Ams-Um | 651 | 138 | b | Ums-Ums-Am- Cm-Um-Um-Cf- Am-Gf-Um-Uf- Um-Cm-Um-Gm- Gm-Uf-Gm-Am- Am-Um | 525 |
| | C00403-DV39P | 32 | F | AmsEVP-dUs-Um- Cm-Af-dC-Cm- Am-Gm-Am-Am- Am-Cm-dU-Gm- Af-Am-Gm-Um- Am-Ams-Ams-Um | 652 | 138 | b | Ums-Ums-Am- Cm-Um-Um-Cf- Am-Gf-Um-Uf- Um-Cm-Um-Gm- Gm-Uf-Gm-Am- Am-Um | 526 |
| | C00403-DV40P | 32 | G | AmsEVP-dUs-Um- dC-Af-dC-Cm-Am- Gm-Am-Am-Am- Cm-dU-Gm-Af- Am-Gm-Um-Am- Ams-Ams-Um | 653 | 138 | b | Ums-Ums-Am- Cm-Um-Um-Cf- Am-Gf-Um-Uf- Um-Cm-Um-Gm- dG-Uf-Gm-Am- Am-Um | 527 |
| B00404 | C00404-DV22 | 33 | H | Ams-Cfs-Am-Um- Um-Cf-Am-Cm- Cm-Am-Gm-Am- Am-Af-Cm-Uf- Gm-Am-Am-Gm- Ums-Ams-Am | 654 | 139 | c | Ams-Cms-Um- Um-Cm-Am-Gf- Um-Uf-Uf-Cf- Um-Gm-Gm-Um- Gm-Am-Am-Um- Gm-Um | 528 |
| | C00404-DV25P | 33 | A | AmsEVP-Cfs-Am- Um-Um-Cf-Am- Cm-Cm-Am-Gm- Am-Am-Af-Cm- Uf-Gm-Am-Am- Gm-Ums-Ams-Am | 655 | 139 | a | Ams-Cms-Um- Um-Cm-Am-Gf- Um-Uf-Uf-Cf- Um-Gm-Gm-Um- Gm-Af-Am-Um- Gm-Um | 529 |
| | C00404-DV26P | 33 | B | AmsEVP-Cfs-Af- Uf-Um-Cf-Am- Cm-Cm-Am-Gm- Am-Am-Af-Cm- Uf-Gm-Am-Am- Gm-Ums-Ams-Am | 656 | 139 | a | Ams-Cms-Um- Um-Cm-Am-Gf- Um-Uf-Uf-Cf- Um-Gm-Gm-Um- Gm-Af-Am-Um- Gm-Um | 530 |
| | C00404-DV27P | 33 | C | AmsEVP-Cfs-Am- Uf-Uf-Cf-Am-Cm- Cm-Am-Gm-Am- Am-Af-Cm-Uf- Gm-Am-Am-Gm- Ums-Ams-Am | 657 | 139 | a | Ams-Cms-Um- Um-Cm-Am-Gf- Um-Uf-Uf-Cf- Um-Gm-Gm-Um- Gm-Af-Am-Um- Gm-Um | 531 |
| | C00404-DV29P | 33 | C | AmsEVP-Cfs-Am- Uf-Uf-Cf-Am-Cm- Cm-Am-Gm-Am- Am-Af-Cm-Uf- Gm-Am-Am-Gm- Ums-Ams-Am | 658 | 139 | b | Ams-Cms-Um- Um-Cm-Am-Gf- Um-Uf-Um-Cf- Um-Gm-Gm-Um- Gm-Af-Am-Um- Gm-Um | 532 |
| | C00404-DV32P | 33 | D | AmsEVP-Cfs-Am- Um-Uf-Cf-Am- Cm-Cm-Am-Gm- Am-Am-Af-Cm- Uf-Gm-Am-Am- Gm-Ums-Ams-Am | 659 | 139 | b | Ams-Cms-Um- Um-Cm-Am-Gf- Um-Uf-Um-Cf- Um-Gm-Gm-Um- Gm-Af-Am-Um- Gm-Um | 533 |
| | C00404-DV34P | 33 | E | AmsEVP-Cfs-Af- Um-Um-Cf-Am- Cm-Cm-Am-Gm- Am-Am-Af-Cm- Uf-Gm-Am-Am- Gm-Ums-Ams-Am | 660 | 139 | b | Ams-Cms-Um- Um-Cm-Am-Gf- Um-Uf-Um-Cf- Um-Gm-Gm-Um- Gm-Af-Am-Um- Gm-Um | 534 |
| | C00410-DV22 | 39 | H | Ums-Afs-Um-Um- Cm-Af-Cm-Cm- Am-Gm-Am-Am- Am-Cf-Um-Gf- Am-Am-Gm-Um- Ams-Ams-Am | 661 | 145 | c | Ums-Ams-Cm- Um-Um-Cm-Af- Gm-Uf-Uf-Uf- Cm-Um-Gm-Gm- Um-Gm-Am-Am- Um-am | 535 |
| B00410 | C00410-DV25P | 39 | A | UmsEVP-Afs-Um- Um-Cm-Af-Cm- Cm-Am-Gm-Am- Am-Am-Cf-Um- Gf-Am-Am-Gm- Um-Ams-Ams-Am | 662 | 145 | a | Ums-Ams-Cm- Um-Um-Cm-Af- Gm-Uf-Uf-Uf- Cm-Um-Gm-Gm- Um-Gf-Am-Am- Um-am | 536 |
| | C00410-DV26P | 39 | B | UmsEVP-Afs-Uf- Uf-Cm-Af-Cm- Cm-Am-Gm-Am- Am-Am-Cf-Um- Gf-Am-Am-Gm- Um-Ams-Ams-Am | 663 | 145 | a | Ums-Ams-Cm- Um-Um-Cm-Af- Gm-Uf-Uf-Uf- Cm-Um-Gm-Gm- Um-Gf-Am-Am- Um-am | 537 |
| | C00410-DV27P | 39 | C | UmsEVP-Afs-Um- Uf-Cf-Af-Cm-Cm- Am-Gm-Am-Am- Am-Cf-Um-Gf- | 664 | 145 | a | Ums-Ams-Cm- Um-Um-Cm-Af- Gm-Uf-Uf-Uf- Cm-Um-Gm-Gm- | 538 |
| | | | | Am-Am-Gm-Um- Ams-Ams-Am | | | | Um-Gf-Am-Am- Um-am | |
| | C00410-DV29P | 39 | C | UmsEVP-Afs-Um- Uf-Cf-Af-Cm-Cm- Am-Gm-Am-Am- Am-Cf-Um-Gf- Am-Am-Gm-Um- Ams-Ams-Am | 665 | 145 | b | Ums-Ams-Cm- Um-Um-Cm-Af- Gm-Uf-Um-Uf- Cm-Um-Gm-Gm- Um-Gf-Am-Am- Um-am | 539 |
| | C00410-DV32P | 39 | D | UmsEVP-Afs-Um- Um-Cf-Af-Cm- Cm-Am-Gm-Am- Am-Am-Cf-Um- Gf-Am-Am-Gm- Um-Ams-Ams-Am | 666 | 145 | b | Ums-Ams-Cm- Um-Um-Cm-Af- Gm-Uf-Um-Uf- Cm-Um-Gm-Gm- Um-Gf-Am-Am- Um-am | 540 |
| | C00410-DV34P | 39 | E | UmsEVP-Afs-Uf- Um-Cm-Af-Cm- Cm-Am-Gm-Am- Am-Am-Cf-Um- Gf-Am-Am-Gm- Um-Ams-Ams-Am | 667 | 145 | b | Ums-Ams-Cm- Um-Um-Cm-Af- Gm-Uf-Um-Uf- Cm-Um-Gm-Gm- Um-Gf-Am-Am- Um-am | 541 |
| | C00410-DV39P | 39 | F | UmsEVP-dAs-Um- Um-Cf-dA-Cm- Cm-Am-Gm-Am- Am-Am-dC-Um- Gf-Am-Am-Gm- Um-Ams-Ams-Am | 668 | 145 | b | Ums-Ams-Cm- Um-Um-Cm-Af- Gm-Uf-Um-Uf- Cm-Um-Gm-Gm- Um-Gf-Am-Am- Um-am | 542 |
| | C00410-DV40P | 39 | G | UmsEVP-dAs-Um- dU-Cf-dA-Cm-Cm- Am-Gm-Am-Am- Am-dC-Um-Gf- Am-Am-Gm-Um- Ams-Ams-Am | 669 | 145 | b | Ums-Ams-Cm- Um-Um-Cm-Af- Gm-Uf-Um-Uf- Cm-Um-Gm-Gm- dU-Gf-Am-Am- Um-am | 543 |
| B05202 | C05202-DV22 | 67 | H | Ums-Afs-Um-Um- Am-Uf-Um-Am- Um-Um-Um-Cm- Um-Uf-Gm-Af- Am-Cm-Cm-Cm- Cms-Ams-Am | 670 | 173 | c | Gms-Gms-Gm- Gm-Um-Um-Cf- Am-Af-Gf-Af- Am-Am-Um-Am- Am-Um-Am-Am- Um-Am | 544 |
| | C05202-DV25P | 67 | A | UmsEVP-Afs-Um- Um-Am-Uf-Um- Am-Um-Um-Um- Cm-Um-Uf-Gm- Af-Am-Cm-Cm- Cm-Cms-Ams-Am | 671 | 173 | a | Gms-Gms-Gm- Gm-Um-Um-Cf- Am-Af-Gf-Af- Am-Am-Um-Am- Am-Uf-Am-Am- Um-Am | 545 |
| | C05202-DV26P | 67 | B | UmsEVP-Afs-Uf- Uf-Am-Uf-Um- | 672 | 173 | a | Gms-Gms-Gm- Gm-Um-Um-Cf- | 546 |
| | | | | Am-Um-Um-Um- Cm-Um-Uf-Gm- Af-Am-Cm-Cm- Cm-Cms-Ams-Am | | | | Am-Af-Gf-Af- Am-Am-Um-Am- Am-Uf-Am-Am- Um-Am | |
| | C05202-DV27P | 67 | C | UmsEVP-Afs-Um- Uf-Af-Uf-Um-Am- Um-Um-Um-Cm- Um-Uf-Gm-Af- Am-Cm-Cm-Cm- Cms-Ams-Am | 673 | 173 | a | Gms-Gms-Gm- Gm-Um-Um-Cf- Am-Af-Gf-Af- Am-Am-Um-Am- Am-Uf-Am-Am- Um-Am | 547 |
| | C05202-DV29P | 67 | C | UmsEVP-Afs-Um- Uf-Af-Uf-Um-Am- Um-Um-Um-Cm- Um-Uf-Gm-Af- Am-Cm-Cm-Cm- Cms-Ams-Am | 674 | 173 | b | Gms-Gms-Gm- Gm-Um-Um-Cf- Am-Af-Gm-Af- Am-Am-Um-Am- Am-Uf-Am-Am- Um-Am | 548 |
| | C05202-DV32P | 67 | D | UmsEVP-Afs-Um- Um-Af-Uf-Um- Am-Um-Um-Um- Cm-Um-Uf-Gm- Af-Am-Cm-Cm- Cm-Cms-Ams-Am | 675 | 173 | b | Gms-Gms-Gm- Gm-Um-Um-Cf- Am-Af-Gm-Af- Am-Am-Um-Am- Am-Uf-Am-Am- Um-Am | 549 |
| | C05202-DV34P | 67 | E | UmsEVP-Afs-Uf- Um-Am-Uf-Um- Am-Um-Um-Um- Cm-Um-Uf-Gm- Af-Am-Cm-Cm- Cm-Cms-Ams-Am | 676 | 173 | b | Gms-Gms-Gm- Gm-Um-Um-Cf- Am-Af-Gm-Af- Am-Am-Um-Am- Am-Uf-Am-Am- Um-Am | 550 |

### 2.3 siRNA derivatives with anti-off-target and inverted modifications

The template-modified siRNA derivatives described in Section 2.2.2 were subjected to the following six anti-off-target designs and three inverted modification designs.

To effectively prevent nuclease attack on the ends of the sequence, the siRNA derivative C00211-DV25P was subjected to inverted modifications, in which the linkage at the ends of the oligonucleotide sequence was altered from the natural 3'-5' linkage to 3'-3' and/or 5'-5' linkages. In this study, inverted modification at the 3' end of the sense strand (InV, No. 2 in the table below), inverted modification at the 3' end of the sense strand with overhang removal at the antisense strand (InVs, No. 3 in the table below), and inverted modification at the 3' end of the antisense strand (AInV, No. 4 in the table below) were attempted.

To mitigate off-target effects, the sequence C00211-DV25P was subjected to anti-off-target designs by employing modification strategies such as GNA substitution in the seed region (7+ and 6+, No. 7 to 8 in the table below) and DNA substitution in the seed region (d7B and d7B5, No. 9 to 10 in the table below) described above, and inverted modification at position 6 or 7 in the seed region (6InV and 7InV, No. 5 to 6 in the table below) were also attempted.

**Table 8: siRNA derivatives with anti-off-target and inverted modifications**

| No. | Sequence ID | Sense strand sequence (5'-3') | SEQ ID NO. | Antisense strand sequence (5'-3') | SEQ ID NO. |
|---|---|---|---|---|---|
| 1 | C00211-DV25P | Cms-Ams-Gm-Gm-Am-Um-Gf- Am-Uf-Uf-Uf-Um-Cm-Um-Um- Am-Uf-Am-Um-Cm-Am | 442 | UmsEVP-Gfs-Am-Um-Am-Uf-Am-Am- Gm-Am-Am-Am-Am-Uf-Cm-Af-Um- Cm-Cm-Um-Gms-Ams-Am | 568 |
| 2 | C00211-DV25PInV | Cm-Am-Gm-Gm-Am-Um-Gf- Am-Uf-Uf-Uf-Um-Cm-Um-Um- Am-Uf-Am-Ums-Cms-dAinV | 677 | UmsEVP-Gfs-Am-Um-Am-Uf-Am-Am- Gm-Am-Am-Am-Am-Uf-Cm-Af-Um- Cm-Cm-Um-Gms-Ams-Am | 686 |
| 3 | C00211-DV25PInVs | Cm-Am-Gm-Gm-Am-Um-Gf- Am-Uf-Uf-Uf-Um-Cm-Um-Um- Am-Uf-Am-Ums-Cms-dAinV | 678 | UmsEVP-Gfs-Am-Um-Am-Uf-Am-Am- Gm-Am-Am-Am-Am-Uf-Cm-Af-Um- Cm-Cms-Ums-Gm | 687 |
| 4 | C00211-DV25PAInV | Cms-Ams-Gm-Gm-Am-Um-Gf- Am-Uf-Uf-Uf-Um-Cm-Um-Um- Am-Uf-Am-Um-Cm-Am | 679 | UmsEVP-Gfs-Am-Um-Am-Uf-Am-Am- Gm-Am-Am-Am-Am-Uf-Cm-Af-Um- Cm-Cm-Um-Gms-Ams-dAinV | 688 |
| 5 | C00211-DV25P6InV | Cms-Ams-Gm-Gm-Am-Um-Gf- Am-Uf-Uf-Uf-Um-Cm-Um-Um- Am-Uf-Am-Um-Cm-Am | 680 | UmsEVP-Gfs-Am-Um-Am-dUinV-Am- Am-Gm-Am-Am-Am-Am-Uf-Cm-Af- Um-Cm-Cm-Um-Gms-Ams-Am | 689 |
| 6 | C00211-DV25P7InV | Cms-Ams-Gm-Gm-Am-Um-Gf- Am-Uf-Uf-Uf-Um-Cm-Um-Um- Am-Uf-Am-Um-Cm-Am | 681 | UmsEVP-Gfs-Am-Um-Am-Uf-dAinV- Am-Gm-Am-Am-Am-Am-Uf-Cm-Af- Um-Cm-Cm-Um-Gms-Ams-Am | 690 |
| 7 | C00211-DV25P7+ | Cms-Ams-Gm-Gm-Am-Um-Gf- Am-Uf-Uf-Uf-Um-Cm-Um-Um- Am-Uf-Am-Um-Cm-Am | 682 | UmsEVP-Gfs-Am-Um-Am-Uf-Agna- Am-Gm-Am-Am-Am-Am-Uf-Cm-Af- Um-Cm-Cm-Um-Gms-Ams-Am | 691 |
| 8 | C00211-DV25P6+ | Cms-Ams-Gm-Gm-Am-Um-Gf- Am-Uf-Uf-Uf-Um-Cm-Um-Um- Am-Uf-Am-Um-Cm-Am | 683 | UmsEVP-Gfs-Am-Um-Am-Ugna-Am- Am-Gm-Am-Am-Am-Am-Uf-Cm-Af- Um-Cm-Cm-Um-Gms-Ams-Am | 692 |
| 9 | C00211-DV25Pd7B | Cms-Ams-Gm-Gm-Am-Um-Gf- Am-Uf-Uf-Uf-Um-Cm-Um-dU- Am-Uf-Am-Um-Cm-Am | 684 | UmsEVP-Gfs-Am-Um-Am-Uf-dA-Am- Gm-Am-Am-Am-Am-Uf-Cm-Af-Um- Cm-Cm-Um-Gms-Ams-Am | 693 |
| 10 | C00211-DV25Pd7B5 | Cms-Ams-Gm-Gm-Am-Um-Gf- Am-Uf-Uf-Uf-Um-Cm-Um-dU- Am-Uf-Am-Um-Cm-Am | 685 | UmsEVP-Gfs-Am-Um-dA-Uf-dA-Am- Gm-Am-Am-Am-Am-Uf-Cm-Af-Um- Cm-Cm-Um-Gms-Ams-Am | 694 |

### 2.4 siRNA conjugates

For siRNA derivatives with an inverted modification on the sense strand, the conjugate moiety is attached to the 5' end of the sense strand. For siRNA derivatives without an inverted modification, the conjugate moiety is attached to the 3' end of the sense strand. DPC-SL10 corresponds to the most active compound L10-siFXIg1M1SP disclosed in CN113227376B, which was modified using the modification template described in the patent, with L10 attached to the 3' end of the sense strand, and was used as a positive control in this study. In the sequence, the uppercase letter P indicates that the nucleotide immediately to the left is a 5'-phosphorylated nucleotide.

**Table 9: Conjugate sequences**

| Motif | siRNA ID | Sense strand sequence (5'-3') | SEQ ID NO. | Antisense strand sequence (5'-3') | SEQ ID NO. |
|---|---|---|---|---|---|
| B00209 | D00209-DV26PG101 | Ums-Ums-Um-Cm-Am-Gm-Gf- Am-Uf-Gf-Af-Um-Um-Um-Um- Cm-Uf-Um-Am-Um-Am-G101 | 695 | UmsEVP-Afs-Uf-Af-Am-Gf-Am- Am-Am-Am-Um-Cm-Am-Uf-Cm- Cf-Um-Gm-Am-Am-Ams-Ams-Gm | 732 |
| | D00209-DV26PInVG1 01 | G101-Um-Um-Um-Cm-Am-Gm- Gf-Am-Uf-Gf-Af-Um-Um-Um-Um- Cm-Uf-Um-Ams-Ums-dAinV | 696 | UmsEVP-Afs-Uf-Af-Am-Gf-Am- Am-Am-Am-Um-Cm-Am-Uf-Cm- Cf-Um-Gm-Am-Am-Ams-Ams-Gm | 733 |
| | D00209-DV32Pd7BG 101 | Ums-Ums-Um-Cm-Am-Gm-Gf- Am-Uf-Gm-Af-Um-Um-Um-dT- Cm-Uf-Um-Am-Um-Am-G101 | 697 | UmsEVP-Afs-Um-Am-Af-Gf-dA- Am-Am-Am-Um-Cm-Am-Uf-Cm- Cf-Um-Gm-Am-Am-Ams-Ams-Gm | 734 |
| | D00209-DV32PG101 | Ums-Ums-Um-Cm-Am-Gm-Gf- Am-Uf-Gm-Af-Um-Um-Um-Um- Cm-Uf-Um-Am-Um-Am-G101 | 698 | UmsEVP-Afs-Um-Am-Af-Gf-Am- Am-Am-Am-Um-Cm-Am-Uf-Cm- Cf-Um-Gm-Am-Am-Ams-Ams-Gm | 735 |
| | D00209-DV32PInVG1 01 | G101-Um-Um-Um-Cm-Am-Gm- Gf-Am-Uf-Gm-Af-Um-Um-Um- Um-Cm-Uf-Um-Ams-Ums-dAinV | 699 | UmsEVP-Afs-Um-Am-Af-Gf-Am- Am-Am-Am-Um-Cm-Am-Uf-Cm- Cf-Um-Gm-Am-Am-Ams-Ams-Gm | 736 |
| | D00209-DV34Pd7BG 101 | Ums-Ums-Um-Cm-Am-Gm-Gf- Am-Uf-Gm-Af-Um-Um-Um-dT- Cm-Uf-Um-Am-Um-Am-G101 | 700 | UmsEVP-Afs-Uf-Am-Am-Gf-dA- Am-Am-Am-Um-Cm-Am-Uf-Cm- Cf-Um-Gm-Am-Am-Ams-Ams-Gm | 737 |
| | D00209-DV34PG101 | Ums-Ums-Um-Cm-Am-Gm-Gf- Am-Uf-Gm-Af-Um-Um-Um-Um- Cm-Uf-Um-Am-Um-Am-G101 | 701 | UmsEVP-Afs-Uf-Am-Am-Gf-Am- Am-Am-Am-Um-Cm-Am-Uf-Cm- Cf-Um-Gm-Am-Am-Ams-Ams-Gm | 738 |
| | D00209-DV34PInVG1 01 | G101-Um-Um-Um-Cm-Am-Gm- Gf-Am-Uf-Gm-Af-Um-Um-Um- Um-Cm-Uf-Um-Ams-Ums-dAinV | 702 | UmsEVP-Afs-Uf-Am-Am-Gf-Am- Am-Am-Am-Um-Cm-Am-Uf-Cm- Cf-Um-Gm-Am-Am-Ams-Ams-Gm | 739 |
| B00210 | D00210-DV25PG101 | Ums-Ums-Cm-Am-Gm-Gm-Af- Um-Gf-Af-Uf-Um-Um-Um-Cm- Um-Uf-Am-Um-Am-Um-G101 | 703 | AmsEVP-Ufs-Am-Um-Am-Af-Gm- Am-Am-Am-Am-Um-Cm-Af-Um- Cf-Cm-Um-Gm-Am-Ams-Ams-Am | 740 |
| | D00210-DV25PInVG1 01 | G101-Um-Um-Cm-Am-Gm-Gm- Af-Um-Gf-Af-Uf-Um-Um-Um-Cm- Um-Uf-Am-Ums-Ams-dTinV | 704 | AmsEVP-Ufs-Am-Um-Am-Af-Gm- Am-Am-Am-Am-Um-Cm-Af-Um- Cf-Cm-Um-Gm-Am-Ams-Ams-Am | 741 |
| B00211 | D00211-DV25Pd7BG 101 | Cms-Ams-Gm-Gm-Am-Um-Gf- Am-Uf-Uf-Uf-Um-Cm-Um-dT-Am- Uf-Am-Um-Cm-Am-G101 | 705 | UmsEVP-Gfs-Am-Um-Am-Uf-dA- Am-Gm-Am-Am-Am-Am-Uf-Cm- Af-Um-Cm-Cm-Um-Gms-Ams-Am | 742 |
| | D00211-DV25PG101 | Cms-Ams-Gm-Gm-Am-Um-Gf- Am-Uf-Uf-Uf-Um-Cm-Um-Um- Am-Uf-Am-Um-Cm-Am-G101 | 706 | UmsEVP-Gfs-Am-Um-Am-Uf-Am- Am-Gm-Am-Am-Am-Am-Uf-Cm- Af-Um-Cm-Cm-Um-Gms-Ams-Am | 743 |
| | D00211-DV25PInVG1 01 DV25PInVG1 | G101-Cm-Am-Gm-Gm-Am-Um- Gf-Am-Uf-Uf-Uf-Um-Cm-Um-Um- Am-Uf-Am-Ums-Cms-dAinV | 707 | UmsEVP-Gfs-Am-Um-Am-Uf-Am- Am-Gm-Am-Am-Am-Am-Uf-Cm- Af-Um-Cm-Cm-Um-Gms-Ams-Am | 744 |
| | D00211-DV25PInVsG 101 DV25PInVsG | G101-Cm-Am-Gm-Gm-Am-Um- Gf-Am-Uf-Uf-Uf-Um-Cm-Um-Um- Am-Uf-Am-Ums-Cms-dAinV | 708 | UmsEVP-Gfs-Am-Um-Am-Uf-Am- Am-Gm-Am-Am-Am-Am-Uf-Cm- Af-Um-Cm-Cms-Ums-Gm | 745 |
| | D00211-DV26Pd7BG 101 | Cms-Ams-Gm-Gm-Am-Um-Gf- Am-Uf-Uf-Uf-Um-Cm-Um-dT-Am- Uf-Am-Um-Cm-Am-G101 | 709 | UmsEVP-Gfs-Af-Uf-Am-Uf-dA-Am- Gm-Am-Am-Am-Am-Uf-Cm-Af- Um-Cm-Cm-Um-Gms-Ams-Am | 746 |
| | D00211-DV26PG101 | Cms-Ams-Gm-Gm-Am-Um-Gf- Am-Uf-Uf-Uf-Um-Cm-Um-Um- Am-Uf-Am-Um-Cm-Am-G101 | 710 | UmsEVP-Gfs-Af-Uf-Am-Uf-Am- Am-Gm-Am-Am-Am-Am-Uf-Cm- Af-Um-Cm-Cm-Um-Gms-Ams-Am | 747 |
| | D00211-DV26PInVsG 101 | G101-Cm-Am-Gm-Gm-Am-Um- Gf-Am-Uf-Uf-Uf-Um-Cm-Um-Um- Am-Uf-Am-Ums-Cms-dAinV | 711 | UmsEVP-Gfs-Af-Uf-Am-Uf-Am- Am-Gm-Am-Am-Am-Am-Uf-Cm- Af-Um-Cm-Cms-Ums-Gm | 748 |
| | D00211-DV26PInVG1 01 DV26PInVG1 | G101-Cm-Am-Gm-Gm-Am-Um- Gf-Am-Uf-Uf-Uf-Um-Cm-Um-Um- Am-Uf-Am-Ums-Cms-dAinV | 712 | UmsEVP-Gfs-Af-Uf-Am-Uf-Am- Am-Gm-Am-Am-Am-Am-Uf-Cm- Af-Um-Cm-Cm-Um-Gms-Ams-Am | 749 |
| | D00211-DV34PG101 | Cms-Ams-Gm-Gm-Am-Um-Gf- Am-Uf-Um-Uf-Um-Cm-Um-Um- Am-Uf-Am-Um-Cm-Am-G101 | 713 | UmsEVP-Gfs-Af-Um-Am-Uf-Am- Am-Gm-Am-Am-Am-Am-Uf-Cm- Af-Um-Cm-Cm-Um-Gms-Ams-Am | 750 |
| | D00211-DV34PInVsG 101 | G101-Cm-Am-Gm-Gm-Am-Um- Gf-Am-Uf-Um-Uf-Um-Cm-Um- Um-Am-Uf-Am-Ums-Cms-dAinV | 714 | UmsEVP-Gfs-Af-Um-Am-Uf-Am- Am-Gm-Am-Am-Am-Am-Uf-Cm- Af-Um-Cm-Cms-Ums-Gm | 751 |
| | D00211-DV34PInVG1 01 | G101-Cm-Am-Gm-Gm-Am-Um- Gf-Am-Uf-Um-Uf-Um-Cm-Um- Um-Am-Uf-Am-Ums-Cms-dAinV | 715 | UmsEVP-Gfs-Af-Um-Am-Uf-Am- Am-Gm-Am-Am-Am-Am-Uf-Cm- Af-Um-Cm-Cm-Um-Gms-Ams-Am | 752 |
| | D00211-DV39PG101 | Cms-Ams-Gm-Gm-Am-Um-Gf- Am-Uf-Um-Uf-Um-Cm-Um-Um- Am-Uf-Am-Um-Cm-Am-G101 | 716 | UmsEVP-dGs-Am-Um-Af-dU-Am- Am-Gm-Am-Am-Am-Am-dU-Cm- Af-Um-Cm-Cm-Um-Gms-Ams-Am | 753 |
| | D00211-DV39PInVsG 101 | G101-Cm-Am-Gm-Gm-Am-Um- Gf-Am-Uf-Um-Uf-Um-Cm-Um- Um-Am-Uf-Am-Ums-Cms-dAinV | 717 | UmsEVP-dGs-Am-Um-Af-dU-Am- Am-Gm-Am-Am-Am-Am-dU-Cm- Af-Um-Cm-Cms-Ums-Gm | 754 |
| | D00211-DV39PInVG1 01 | G101-Cm-Am-Gm-Gm-Am-Um- Gf-Am-Uf-Um-Uf-Um-Cm-Um- Um-Am-Uf-Am-Ums-Cms-dAinV | 718 | UmsEVP-dGs-Am-Um-Af-dU-Am- Am-Gm-Am-Am-Am-Am-dU-Cm- Af-Um-Cm-Cm-Um-Gms-Ams-Am | 755 |
| B00211s | D00211s-DV25SPG101 | Ams-Gms-Gm-Am-Um-Gf-Am-Uf- Uf-Uf-Um-Cm-Um-Um-Am-Uf- Am-Um-Cm-Am-G101 | 719 | UmsEVP-Gfs-Am-Um-Am-Uf-Am- Am-Gm-Am-Am-Am-Am-Uf-Cm- Af-Um-Cm-Cm-Ums-Gms-Am | 756 |
| | D00211s-DV25SPInV G101 | G101-Am-Gm-Gm-Am-Um-Gf- Am-Uf-Uf-Uf-Um-Cm-Um-Um- Am-Uf-Am-Ums-Cms-dAinV | 720 | UmsEVP-Gfs-Am-Um-Am-Uf-Am- Am-Gm-Am-Am-Am-Am-Uf-Cm- Af-Um-Cm-Cm-Ums-Gms-Am | 757 |
| B00212 | D00212-DV32PG101 | Ams-Gms-Gm-Am-Um-Gm-Af- Um-Uf-Um-Uf-Cm-Um-Um-Am- Um-Af-Um-Cm-Am-Am-G101 | 721 | UmsEVP-Ufs-Gm-Am-Uf-Af-Um- Am-Am-Gm-Am-Am-Am-Af-Um- Cf-Am-Um-Cm-Cm-Ums-Gms-Am | 758 |
| | D00212-DV32PInVG1 01 | G101-Am-Gm-Gm-Am-Um-Gm- Af-Um-Uf-Um-Uf-Cm-Um-Um- Am-Um-Af-Um-Cms-Ams-dAinV | 722 | UmsEVP-Ufs-Gm-Am-Uf-Af-Um- Am-Am-Gm-Am-Am-Am-Af-Um- Cf-Am-Um-Cm-Cm-Ums-Gms-Am | 759 |
| B00216 | D00216-DV34PG101 | Gms-Gms-Am-Um-Gm-Am-Uf- Um-Uf-Um-Cf-Um-Um-Am-Um- Am-Uf-Cm-Am-Am-Am-G101 | 723 | UmsEVP-Ufs-Uf-Gm-Am-Uf-Am- Um-Am-Am-Gm-Am-Am-Af-Am- Uf-Cm-Am-Um-Cm-Cms-Ums-Gm | 760 |
| | D00216-DV34PInVG1 01 | G101-Gm-Gm-Am-Um-Gm-Am- Uf-Um-Uf-Um-Cf-Um-Um-Am- Um-Am-Uf-Cm-Ams-Ams-dAinV | 724 | UmsEVP-Ufs-Uf-Gm-Am-Uf-Am- Um-Am-Am-Gm-Am-Am-Af-Am- Uf-Cm-Am-Um-Cm-Cms-Ums-Gm | 761 |
| B00217s | D00217s-DV32SPG101 | Ams-Ums-Gm-Am-Um-Uf-Um-Uf- Cm-Uf-Um-Am-Um-Am-Um-Cf- Am-Am-Gm-Um-G101 | 725 | AmsEVP-Cfs-Um-Um-Gf-Af-Um- Am-Um-Am-Am-Gm-Am-Af-Am- Af-Um-Cm-Am-Ums-Cms-Cm | 762 |
| | D00217s-DV32SPInV G101 | G101-Am-Um-Gm-Am-Um-Uf- Um-Uf-Cm-Uf-Um-Am-Um-Am- Um-Cf-Am-Ams-Gms-dTinV | 726 | AmsEVP-Cfs-Um-Um-Gf-Af-Um- Am-Um-Am-Am-Gm-Am-Af-Am- Af-Um-Cm-Am-Ums-Cms-Cm | 763 |
| | D00217s-DV32SPInVs G101 | G101-Am-Um-Gm-Am-Um-Uf- Um-Uf-Cm-Uf-Um-Am-Um-Am- Um-Cf-Am-Ams-Gms-dTinV | 727 | AmsEVP-Cfs-Um-Um-Gf-Af-Um- Am-Um-Am-Am-Gm-Am-Af-Am- Af-Um-Cms-Ams-Um | 764 |
| B00404 | D00404-DV25Pd7BG 101 | Ams-Cms-Um-Um-Cm-Am-Gf- Um-Uf-Uf-Cf-Um-Gm-Gm-dT-Gm- Af-Am-Um-Gm-Um-G101 | 728 | AmsEVP-Cfs-Am-Um-Um-Cf-dA- Cm-Cm-Am-Gm-Am-Am-Af-Cm- Uf-Gm-Am-Am-Gm-Ums-Ams-Am | 765 |
| | D00404-DV25PG101 | Ams-Cms-Um-Um-Cm-Am-Gf- Um-Uf-Uf-Cf-Um-Gm-Gm-Um- Gm-Af-Am-Um-Gm-Um-G101 | 729 | AmsEVP-Cfs-Am-Um-Um-Cf-Am- Cm-Cm-Am-Gm-Am-Am-Af-Cm- Uf-Gm-Am-Am-Gm-Ums-Ams-Am | 766 |
| | D00404-DV25PInVsG 101 DV25PInVsG | G101-Am-Cm-Um-Um-Cm-Am- Gf-Um-Uf-Uf-Cf-Um-Gm-Gm-Um- Gm-Af-Am-Ums-Gms-dTinV | 730 | AmsEVP-Cfs-Am-Um-Um-Cf-Am- Cm-Cm-Am-Gm-Am-Am-Af-Cm- Uf-Gm-Am-Ams-Gms-Um | 767 |
| | D00404-DV25PInVG1 01 | G101-Am-Cm-Um-Um-Cm-Am- Gf-Um-Uf-Uf-Cf-Um-Gm-Gm-Um- Gm-Af-Am-Ums-Gms-dTinV | 731 | AmsEVP-Cfs-Am-Um-Um-Cf-Am- Cm-Cm-Am-Gm-Am-Am-Af-Cm- Uf-Gm-Am-Am-Gm-Ums-Ams-Am | 768 |
| APC | DPC-SL10 | Ams-Ums-Um-Um-Cm-Um-Gf-Gf- Gf-Um-Am-Um-Um-Cm-Um-Um- Um-Cm-Am-L10 | 783 | UmsP-Gfs-Am-Am-Am-Gf-Am-Am- Um-Am-Cm-Cm-Cm-Af-Gm-Af- Am-Am-Ums-Cms-Gm | 784 |
| ANC | ANC-DV25PG101 | Gms-Gms-Um-Um-Cm-Um-Gf- Um-Af-Cf-Uf-Um-Am-Um-Um- Gm-Uf-Cm-Am-G101 | 785 | AmsEVP-Gfs-Um-Gm-Am-Cf-Am- Am-Um-Am-Am-Gm-Um-Af-Cm- Af-Gm-Am-Ams-Cms-Cm | 786 |

### Example 2: Synthesis of siRNA compounds

### 2.1 Synthesis of siRNA derivatives with alternating 2'-F and 2'-OMe modifications

Table 2 shows siRNA derivatives with alternating 2'-F and 2'-OMe modifications. Taking the siRNA with sequence ID B00202-AL as an example, the core sequences are as follows:
Sense strand: 5'-CUUAUUAAGAAUUGCAGCAAA-3' (SEQ ID NO: 1)
Antisense strand: 5'-UUUGCUGCAAUUCUUAAUAAGGG-3' (SEQ ID NO: 107)

Odd-numbered positions of the sense strand and even-numbered positions of the antisense strand are modified with 2'-F, while all other positions are modified with 2'-OMe. Additionally, a 3',5'-phosphorothioate linkage is introduced between nucleotides at positions 1 and 2 and between nucleotides at positions 2 and 3 from the 5' end of the sense strand; a 3',5'-phosphorothioate linkage is introduced between nucleotides at positions 1 and 2 and between nucleotides at positions 2 and 3 from the 5' end of the antisense strand, and a 3',5'-phosphorothioate linkage is introduced between nucleotides at positions 1 and 2 and between nucleotides at positions 2 and 3 from the 3' end of the antisense strand.

Instruments and reagents: 192 P DNA/RNA automatic synthesizer (Tsingke), using a universal solid-phase support consisting of cross-linked polystyrene beads; model: Primer Support 5G Unylinker 350 (manufactured by Cytiva).

### Preparation method:

Nucleotide monomers were dissolved in acetonitrile at a concentration of 0.15 M, including DMT-A-OMe phosphoramidite monomer (Formula 1), DMT-C-OMe phosphoramidite monomer (Formula 2), DMT-G-OMe phosphoramidite monomer (Formula 3), DMT-U-OMe phosphoramidite monomer (Formula 4), DMT-A-F phosphoramidite monomer (Formula 5), DMT-C-F phosphoramidite monomer (Formula 6), DMT-G-F phosphoramidite monomer (Formula 7), and DMT-U-F phosphoramidite monomer (Formula 8).

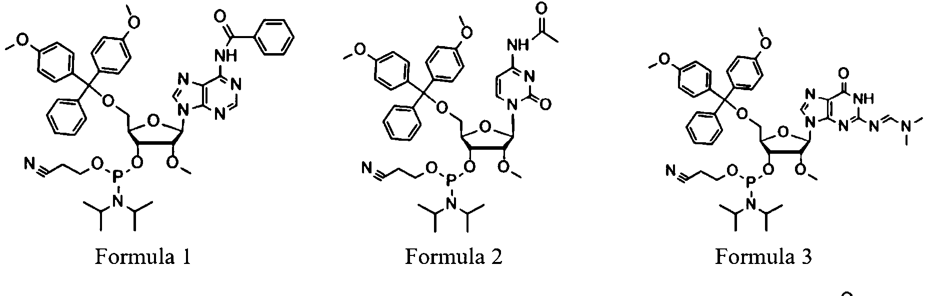

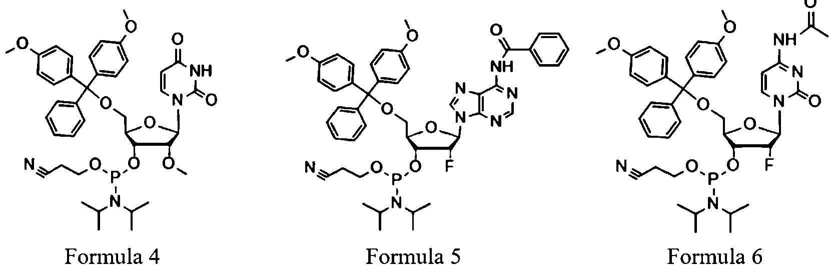

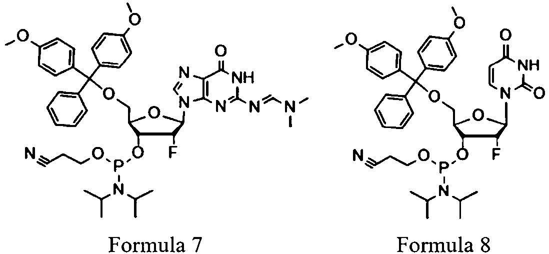

The preparation was carried out according to the following steps:
Nucleotide monomers were sequentially attached in the 3' to 5' direction according to the nucleotide arrangement using the solid-phase phosphoramidite method. The process of attaching each nucleotide monomer involves four steps: deprotection, coupling, oxidation or sulfurization, and hydroxyl protection. Specifically, when two nucleotides are linked via a phosphate linkage, the process of attaching a subsequent nucleotide monomer involves four steps: deprotection, coupling, oxidation, and hydroxyl protection. When two nucleotides are linked via a phosphorothioate linkage, the process of attaching a subsequent nucleotide monomer involves four steps: deprotection, coupling, sulfurization, and hydroxyl protection. The details are as follows:

### (1) Deprotection

3% dichloroacetic acid in toluene was used as a deprotection reagent to remove the DMT protecting group, followed by washing with acetonitrile.

### (2) Coupling

Each nucleotide monomer in acetonitrile was coupled using 0.25 M 5-ethylthio-1H-tetrazole as an activator, followed by washing with acetonitrile.

### (3) Oxidation/sulfurization

Oxidation: 0.05 M iodine in pyridine/water (90/10) was used as an oxidant for oxidation, followed by washing with acetonitrile.

Sulfurization: 3% xanthane hydride in pyridine was used as a sulfurizing agent for sulfurization, followed by washing with acetonitrile.

### (4) Hydroxyl protection

10% acetic anhydride in tetrahydrofuran (CAP A) and tetrahydrofuran/pyridine/methylimidazole (74/10/16, v/v/v) (CAP B) were used as hydroxyl protecting reagents for hydroxyl protection, followed by washing with acetonitrile.

The aforementioned steps were repeated according to the specified nucleotide arrangement to obtain a product with the specific sequence arrangement in either the sense strand or the antisense strand.

(5) 3% dichloroacetic acid in toluene was used as a deprotection reagent to remove the DMT protecting group from the last nucleotide, followed by washing with acetonitrile.

### (6) Ammonolysis and purification

The reacted solid-phase support was transferred to a reactor and treated with concentrated aqueous ammonia (25 to 28%) at 60°C for 12 hours. The system was then cooled to room temperature, and the mixture was transferred to a pressure filter for filtration. The filter cake was washed with a mixture of purified water and ethanol. The filtrates were combined, subjected to column chromatography, concentrated, and lyophilized to obtain 2'-OMe and 2'-F modified single-stranded products.

### (7) Removal of TBDMS protecting groups

The resulting product was treated with DMSO and triethylamine hydrofluoride at 60°C for 2 hours. An aqueous ammonium acetate solution was then added to the reaction mixture and vortexed thoroughly. The resulting mixture was added with anhydrous ethanol, vortexed thoroughly, followed by crystallization at -20°C for 8 to 12 hours. After centrifugation, the supernatant was discarded, and the precipitate was washed with anhydrous ethanol to obtain an unmodified single-stranded product.

### (8) Annealing

The purified sense and antisense strands were mixed at a molar ratio of 1:1, heated to 95°C and maintained for 3 minutes, and slowly cooled to room temperature to form an siRNA duplex.

All other sequences listed in Table 2 were synthesized using the method described above.

### 2.2 Synthesis of template-modified siRNA derivatives disclosed herein

The siRNA sequences described in Section 2.2.2 of Example 1 were synthesized with reference to the method described in Section 2.1 of Example 2, except that the base at the 5' end of the antisense strand (the last base) was synthesized using a monomer containing a phosphonate group at the 5' end, such as vinyl-(E)-phosphonate-A-OMe phosphoramidite monomer (Formula 9) or vinyl-(E)-phosphonate-U-OMe phosphoramidite monomer (Formula 10), the structures of which are as follows:

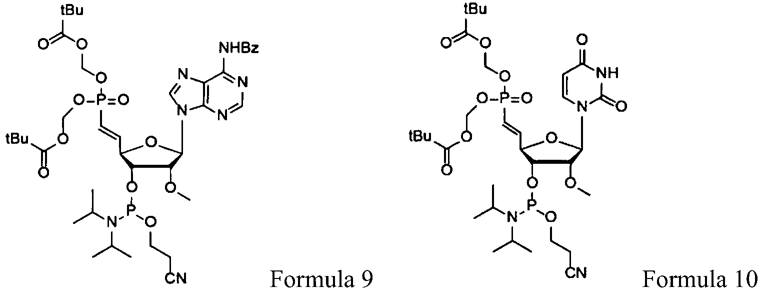

For sequences modified using the template DV39P, the siRNA sequences were synthesized with reference to the method described in Section 2.1 of Example 2, except that nucleotides at positions 2, 6, and 14 from the 5' end of the antisense strand were synthesized using DNA monomers, including DMT-dA phosphoramidite monomer (Formula 11), DMT-dT phosphoramidite monomer (Formula 12), DMT-dC phosphoramidite monomer (Formula 13), or DMT-dG phosphoramidite monomer (Formula 14). For derivatives modified using the template DV40P, nucleotides at positions 2, 6, and 14 from the 5' end of the antisense strand and nucleotides at position 16 from the 5' end of the sense strand were synthesized using any one of the aforementioned four monomers. Exemplary structures of these monomers are as follows:

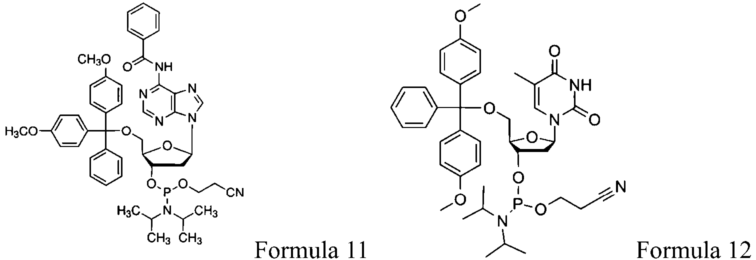

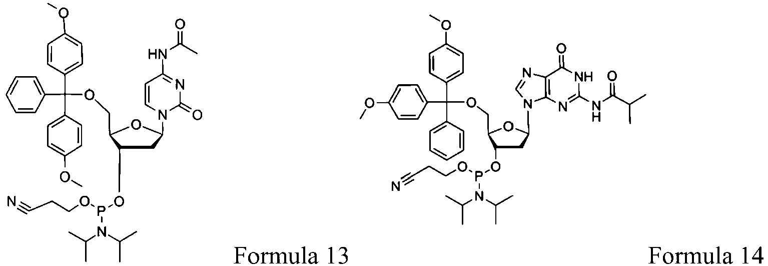

### 2.3 Synthesis of siRNA derivatives with inverted modifications

The siRNA derivatives with inverted modifications were synthesized with reference to the method described in Section 2.1 of Example 2, except that the terminal nucleotides of the sense or antisense strand were synthesized using DNA monomers, including dA(Bz)-CE-Reverse (dAinV), dT(Bz)-CE-Reverse (dTinV), dC(Bz)-CE-Reverse (dCinV), or dG(Bz)-CE-Reverse (dGinV), the structures of which are as follows:

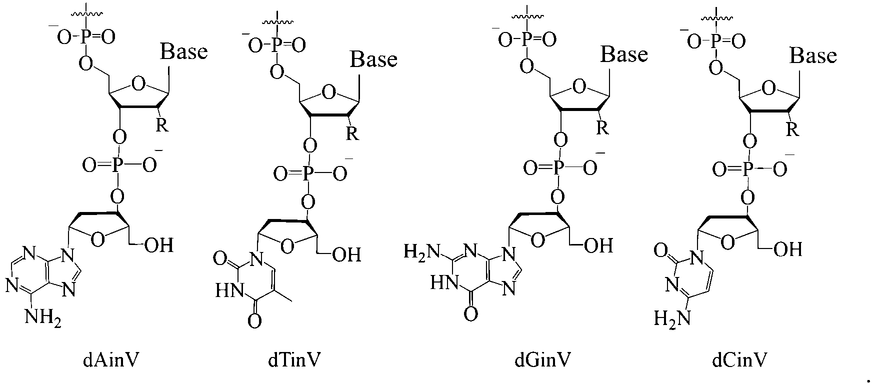

### 2.4 Synthesis of siRNA conjugates

For the conjugates listed in Table 9, in which the GalNAc conjugate moiety G101 (whose structural formula is shown below) is attached to the 3' end of the sense strand, the method of linking the oligonucleotide to G101 was performed according to the preparation method described in Example 3 of patent application CN116854754A.

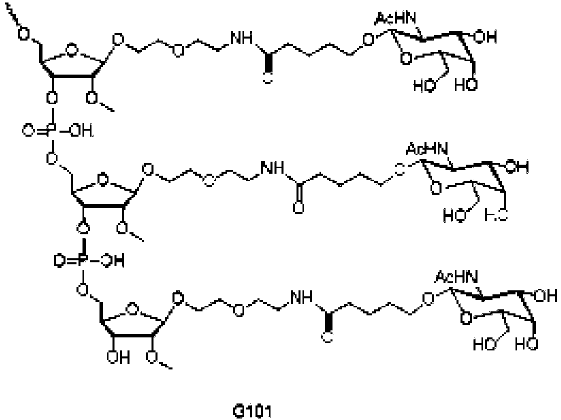

The oligonucleotide and conjugate moiety G101 form a conjugate as shown below:

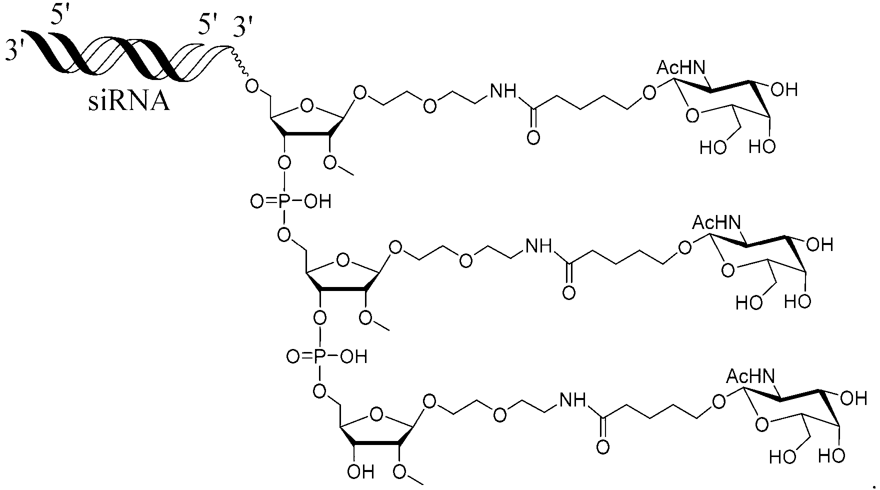

In the sequence ID, G101 indicates that the sequence is attached to the conjugate moiety G101. L10 is the conjugate moiety of the positive control (the most active compound siFXIg1-M1SP disclosed in CN113227376B). L10 indicates that the sequence is attached to the conjugate moiety L10. The structural formula of L10 is as follows:

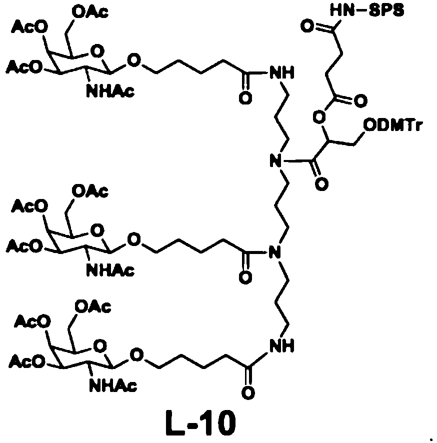

### Example 3: In vitro evaluation of the inhibitory effect of siRNA on FXI gene expression

### 3.1 Inhibitory effect of siRNA motifs and alternating 2'-F and 2'-OMe modified siRNA derivatives on FXI gene expression

The siRNA motifs and alternating 2'-OMe and 2'-F modified siRNA derivatives synthesized in Example 2 were transfected into HepG2 cells via lipid nanoparticles (LNPs). The inhibitory effect of the siRNAs on the target gene FXI was then evaluated using qPCR and ELISA.

### 3.1.1 qPCR assay of inhibition rate of FXI mRNA expression

HepG2 cells were cultured in DMEM culture medium containing 10% fetal bovine serum (supplemented with 100× penicillin and 100× streptomycin at 10 µL/mL), and incubated in a cell incubator at 37°C with 5% CO₂.

Prior to siRNA transfection, Lipofectamine RNAiMAX (Invitrogen, 13778150) was mixed with Opti-MEM at a ratio of 2:98 and vortexed thoroughly to prepare a transfection mixture. The siRNA compounds were diluted to an appropriate concentration using Opti-MEM. For single-concentration transfection, the siRNA working solution was prepared at a concentration of 960 pM. 55 µL of siRNA solution diluted in Opti-MEM was mixed with 55 µL of the transfection mixture at a ratio of 1:1 (v/v).

For the blank control group, 55 µL of the transfection mixture was added to 55 µL of Opti-MEM, vortexed thoroughly, and the mixture was incubated at room temperature for 15 minutes.

The prepared transfection reagent was added to a 24-well cell culture plate (100 µL per well), resulting in a final siRNA concentration of 80 pM per well. Subsequently, 500 µL of cell suspension (at a cell density of 1.5 × 10⁵ cells/mL) was added to each well. After cross-mixing, the plate was incubated in a cell incubator at 37°C with 5% CO₂ for 48 hours.

48 hours after transfection, RNA extraction was first performed according to the instructions of the RNA extraction kit (RNeasy Mini Kit, QIAGEN, 74106). The extracted RNA was then reverse-transcribed into cDNA using the FastKing RT Kit (with gDNase) (TIANGEN, KR116-02). Finally, qPCR amplification was performed using the TB Green^{®} Premix Ex Taq^{™} (Tli RNaseH Plus) kit (Takara, RR420W (L × 5)) to quantify FXI mRNA expression levels. The primers used for qPCR amplification are listed in Table 10. qPCR amplification was performed according to the following program: 95°C for 30 seconds, followed by 40 cycles of 95°C for 5 seconds and 60°C for 34 seconds.

**Table 10: Primers used for qPCR amplification**

| Name | Sequence | SEQ ID NO: |
|---|---|---|
| hGAPDH-F | AGTATGACAACAGCCTCAAG | 769 |
| hGAPDH-R | TCATGAGTCCTTCCACGATA | 770 |
| hFXI-F1 | GTGACCAACGAAGAGTGCCAGA | 771 |
| hFXI-R1 | CCAGACCTCATTGTGTTTGCAGG | 772 |

FXI mRNA expression rate (%) was calculated using the following formula: 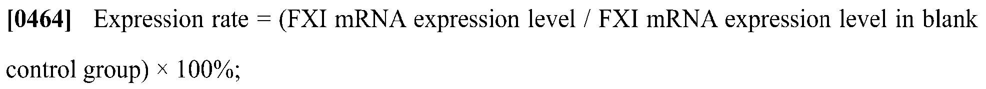 Expression rate = (FXI mRNA expression level / FXI mRNA expression level in blank control group) × 100%;

Inhibition rate of FXI gene expression = 100% - expression rate (%). The results are shown in Table 11.

### 3.1.2 ELISA assay of inhibition rate of FXI protein expression

For siRNAs exhibiting higher inhibition rates in the qPCR assay, the cell culture supernatant collected 48 hours after siRNA transfection was analyzed using the Human Factor XI ELISA Kit (Thermo Fisher, EH118RB), in order to evaluate their inhibitory effect on the target gene FXI at the protein level.

The ELISA data were normalized using the transfection reagent control group as the reference (expression level relative to the transfection reagent control group = FXI protein expression level in each sample / FXI protein expression level in the transfection reagent control group). The results are shown in Table 11.

**Table 11: Inhibition rates of siRNA motifs and alternating 2'-F and 2'-OMe modified siRNA derivatives against the target gene FXI in HepG2 Cells**

| siRNA motif | | Alternating 2'-F and 2'-OMe modified siRNA derivative | | |
|---|---|---|---|---|
| siRNA ID | Inhibition rate (%) (qPCR) | siRNA ID | Inhibition rate (%) (qPCR) | Inhibition rate (%) (ELISA) |
| B00202 | 59 | B00202-AL | 62 | N/A |
| B00203 | 45 | B00203-AL | 56 | N/A |
| B00204 | 64 | B00204-AL | 71 | 58 |
| B00205s | 55 | B00205s-AL | 61 | N/A |
| B00206s | 50 | B00206s-AL | 57 | N/A |
| B00207s | 30 | B00207s-AL | 36 | N/A |
| B00208s | 53 | B00208s-AL | 60 | N/A |
| B00209 | 74 | B00209-AL | 78 | 70 |
| B00210 | 73 | B00210-AL | 76 | 70 |
| B00211 | 74 | B00211-AL | 80 | 69 |
| B00211s | 75 | B00211s-AL | 79 | 70 |
| B00212 | 71 | B00212-AL | 76 | 69 |
| B00213 | 51 | B00213-AL | 64 | N/A |
| B00214 | 65 | B00214-AL | 73 | 64 |
| B00215 | 71 | B00215-AL | 74 | 65 |
| B00216 | 70 | B00216-AL | 75 | 68 |
| B00217s | 69 | B00217s-AL | 74 | 64 |
| B00301 | 63 | B00301-AL | 73 | 69 |
| B00302 | 11 | B00302-AL | 37 | N/A |
| B00303 | 54 | B00303-AL | 65 | N/A |
| B00304 | 68 | B00304-AL | 76 | 63 |
| B00305 | 57 | B00305-AL | 66 | N/A |
| B00306 | 58 | B00306-AL | 65 | N/A |
| B00307 | 70 | B00307-AL | 76 | 71 |
| B00308 | 68 | B00308-AL | 75 | N/A |
| B00309 | 56 | B00309-AL | 65 | N/A |
| B00310 | 63 | B00310-AL | 69 | N/A |
| B00311 | 63 | B00311-AL | 72 | 68 |
| B00312 | 64 | B00312-AL | 70 | 62 |
| B00401 | 36 | B00401-AL | 55 | N/A |
| B00402 | 31 | B00402-AL | 45 | N/A |
| B00403 | 72 | B00403-AL | 79 | 73 |
| B00404 | 66 | B00404-AL | 74 | 60 |
| B00405 | 40 | B00405-AL | 57 | N/A |
| B00406s | -18 | B00406s-AL | 13 | N/A |
| B00407s | -6 | B00407s-AL | 23 | N/A |
| B00408 | 59 | B00408-AL | 68 | 60 |
| B00409 | 22 | B00409-AL | 43 | N/A |
| B00410 | 50 | B00410-AL | 58 | N/A |
| B00701 | 60 | B00701-AL | 72 | 62 |
| B00702 | 13 | B00702-AL | 36 | N/A |
| B00703 | 23 | B00703-AL | 38 | N/A |
| B00704 | 65 | B00704-AL | 75 | 60 |
| B00705 | 56 | B00705-AL | 69 | 59 |
| B00706 | 54 | B00706-AL | 63 | N/A |
| B00707 | 61 | B00707-AL | 71 | 67 |
| B00708 | 53 | B00708-AL | 65 | N/A |
| B00709 | 55 | B00709-AL | 64 | N/A |
| B02601 | 45 | B02601-AL | 54 | N/A |
| B02602 | 39 | B02602-AL | 55 | N/A |
| B02603 | 29 | B02603-AL | 49 | N/A |
| B02604 | 35 | B02604-AL | 53 | N/A |
| B02605s | 46 | B02605s-AL | 54 | N/A |
| B02606 | 40 | B02606-AL | 57 | N/A |
| B02607 | 17 | B02607-AL | 40 | N/A |
| B02608 | 40 | B02608-AL | 53 | N/A |
| B02609 | 7 | B02609-AL | 16 | N/A |
| B05001 | 52 | B05001-AL | 61 | N/A |
| B05002s | 13 | B05002s-AL | 32 | N/A |
| B05003 | -5 | B05003-AL | 10 | N/A |
| B05004 | 14 | B05004-AL | 35 | N/A |
| B05005 | 59 | B05005-AL | 64 | N/A |
| B05006 | 37 | B05006-AL | 50 | N/A |
| B05007 | 23 | B05007-AL | 35 | N/A |
| B05008 | 19 | B05008-AL | 30 | N/A |
| B05201 | 66 | B05201-AL | 73 | 58 |
| B05202 | 66 | B05202-AL | 73 | 59 |
| B05203 | 60 | B05203-AL | 69 | 55 |
| B05204 | 24 | B05204-AL | 34 | N/A |
| B05205 | 9 | B05205-AL | 25 | N/A |
| B05206 | 41 | B05206-AL | 49 | N/A |
| B05207 | 68 | B05207-AL | 68 | 45 |
| B05208 | 14 | B05208-AL | 33 | N/A |
| B06401 | 51 | B06401-AL | 60 | N/A |
| B06402s | 56 | B06402s-AL | 63 | N/A |
| B06403 | 63 | B06403-AL | 66 | N/A |
| B06404 | 35 | B06404-AL | 37 | N/A |
| B06405 | 8 | B06405-AL | 15 | N/A |
| B06406 | 60 | B06406-AL | 61 | N/A |
| B06407 | 62 | B06407-AL | 65 | N/A |
| B06408 | 23 | B06408-AL | 22 | N/A |
| B06409 | 59 | B06409-AL | 57 | N/A |
| B06410 | 43 | B06410-AL | 42 | N/A |
| B06411 | 55 | B06411-AL | 57 | N/A |
| B07401 | 54 | B07401-AL | 61 | N/A |
| B07402 | 30 | B07402-AL | 41 | N/A |
| B07403 | 62 | B07403-AL | 66 | 46 |
| B07404s | 12 | B07404s-AL | 23 | N/A |
| B07405s | -3 | B07405s-AL | 7 | N/A |
| B07406 | 51 | B07406-AL | 59 | N/A |
| B07407 | 51 | B07407-AL | 51 | N/A |
| B07408 | 49 | B07408-AL | 54 | N/A |
| B07501 | 52 | B07501-AL | 58 | N/A |
| B07502 | 39 | B07502-AL | 42 | N/A |
| B07503 | 33 | B07503-AL | 40 | N/A |
| B07504 | 26 | B07504-AL | 32 | N/A |
| B07505 | 34 | B07505-AL | 32 | N/A |
| B07506 | 20 | B07506-AL | 27 | N/A |
| B07901 | 59 | B07901-AL | 65 | N/A |
| B07902 | 55 | B07902-AL | 56 | N/A |
| B07903 | 42 | B07903-AL | 43 | N/A |
| B07904 | 61 | B07904-AL | 60 | N/A |
| B07905 | 61 | B07905-AL | 61 | N/A |
| B07906 | 64 | B07906-AL | 64 | N/A |
| B07907 | 58 | B07907-AL | 55 | N/A |
| B07908 | 39 | B07908-AL | 42 | N/A |
| APC | 56 | APC-AL | 59 | 46 |
| ANC | -11 | ANC-AL | -9 | 5 |

In this example, the inhibition rate represents the average of three independent experiments, as shown in Table 11. Results from the qPCR assay demonstrated that among the 106 siRNA derivatives employing alternating 2'-OMe and 2'-F modifications, 49 exhibited an inhibition rate exceeding 60% against FXI gene expression, 34 exhibited an inhibition rate of 40% to 60%, and 23 exhibited an inhibition rate below 40%, wherein the positive control APC-AL exhibited an inhibition rate of 59%. In the ELISA assay, the positive control APC-AL exhibited an inhibition rate of 46%, while 25 of the 27 selected dominant siRNAs exhibited an inhibition rate higher than that of the positive control.

### 3.2 Inhibitory effect of template-modified siRNA derivatives on FXI gene expression

The template-modified siRNA derivatives disclosed herein synthesized in Example 2 (sequences listed in Table 7) were transfected into HepG2 cells via lipid nanoparticles (LNPs). The inhibitory effect on the target gene FXI was then evaluated using qPCR and ELISA.

The experimental procedure was essentially the same as described in Section 3.1, except that the siRNA transfection concentration was 10 pM. The experimental results are shown in Table 12.

**Table 12: Inhibition rates of template-modified siRNA derivatives in HepG2 Cells**

| siRNA ID | siRNA ID | Inhibition rate (%) (qPCR) | Inhibition rate (%) (ELISA) |
|---|---|---|---|
| B00209 | C00209-DV22 | 44 | 45 |
| | C00209-DV25P | 53 | 53 |
| | C00209-DV26P | 48 | 48 |
| | C00209-DV27P | 42 | 47 |
| | C00209-DV29P | 44 | 49 |
| | C00209-DV32P | 48 | 51 |
| | C00209-DV34P | 44 | 50 |
| | C00209-DV39P | -14 | N/A |
| | C00209-DV40P | 35 | N/A |
| B00210 | C00210-DV22 | 40 | N/A |
| | C00210-DV25P | 42 | 49 |
| | C00210-DV26P | 38 | N/A |
| | C00210-DV27P | 34 | N/A |
| | C00210-DV29P | 34 | N/A |
| | C00210-DV32P | 44 | 38 |
| | C00210-DV34P | 44 | 33 |
| B00211 | C00211-DV22 | 46 | 45 |
| | C00211-DV25P | 50 | 53 |
| | C00211-DV26P | 48 | 51 |
| | C00211-DV27P | 45 | 47 |
| | C00211-DV29P | 49 | 46 |
| | C00211-DV32P | 43 | 50 |
| | C00211-DV34P | 46 | 51 |
| | C00211-DV39P | 46 | 55 |
| | C00211-DV40P | 49 | 58 |
| B00212 | C00212-DV22 | 30 | N/A |
| | C00212-DV25P | 37 | N/A |
| | C00212-DV26P | 32 | N/A |
| | C00212-DV27P | 44 | 50 |
| | C00212-DV29P | 44 | 45 |
| | C00212-DV32P | 45 | 46 |
| | C00212-DV34P | 30 | N/A |
| B00214 | C00214-DV22 | 25 | N/A |
| | C00214-DV25P | 33 | N/A |
| | C00214-DV26P | 40 | N/A |
| | C00214-DV27P | 17 | N/A |
| | C00214-DV29P | 19 | N/A |
| | C00214-DV32P | 19 | N/A |
| | C00214-DV34P | 21 | N/A |
| B00215 | C00215-DV22 | 29 | N/A |
| | C00215-DV25P | 34 | N/A |
| | C00215-DV26P | 37 | N/A |
| | C00215-DV27P | 42 | 39 |
| | C00215-DV29P | 37 | N/A |
| | C00215-DV32P | 42 | 31 |
| | C00215-DV34P | 43 | 32 |
| | C00215-DV39P | 28 | N/A |
| | C00215-DV40P | 24 | N/A |
| B00216 | C00216-DV22 | 33 | N/A |
| | C00216-DV25P | 38 | N/A |
| | C00216-DV26P | 39 | N/A |
| | C00216-DV27P | 41 | 50 |
| | C00216-DV29P | 41 | 50 |
| | C00216-DV32P | 42 | 51 |
| | C00216-DV34P | 44 | 53 |
| B00301 | C00301-DV22 | 30 | N/A |
| | C00301-DV25P | 28 | N/A |
| | C00301-DV26P | 42 | 43 |
| | C00301-DV27P | 27 | N/A |
| | C00301-DV29P | 31 | N/A |
| | C00301-DV32P | 25 | N/A |
| | C00301-DV34P | 33 | N/A |
| B00304 | C00304-DV22 | 28 | N/A |
| | C00304-DV25P | 38 | N/A |
| | C00304-DV26P | 38 | N/A |
| | C00304-DV27P | 29 | N/A |
| | C00304-DV29P | 21 | N/A |
| | C00304-DV32P | 21 | N/A |
| | C00304-DV34P | 29 | N/A |
| B00307 | C00307-DV22 | 36 | N/A |
| | C00307-DV25P | 40 | 42 |
| | C00307-DV26P | 36 | N/A |
| | C00307-DV27P | 40 | N/A |
| | C00307-DV29P | 40 | N/A |
| | C00307-DV32P | 41 | 43 |
| | C00307-DV34P | 36 | N/A |
| | C00307-DV39P | 14 | N/A |
| | C00307-DV40P | 1 | N/A |
| B00311 | C00311-DV22 | 24 | N/A |
| | C00311-DV25P | 4 | N/A |
| | C00311-DV26P | 3 | N/A |
| | C00311-DV27P | -6 | N/A |
| | C00311-DV29P | 4 | N/A |
| | C00311-DV32P | 31 | N/A |
| | C00311-DV34P | 31 | N/A |
| B00312 | C00312-DV22 | 35 | N/A |
| | C00312-DV25P | 42 | 35 |
| | C00312-DV26P | 43 | 35 |
| | C00312-DV27P | 39 | N/A |
| | C00312-DV29P | 34 | N/A |
| | C00312-DV32P | 30 | N/A |
| | C00312-DV34P | 23 | N/A |
| B00403 | C00403-DV22 | 28 | N/A |
| | C00403-DV25P | 25 | N/A |
| | C00403-DV26P | 22 | N/A |
| | C00403-DV27P | 19 | N/A |
| | C00403-DV29P | 30 | N/A |
| | C00403-DV32P | 35 | N/A |
| | C00403-DV34P | 31 | N/A |
| | C00403-DV39P | 10 | N/A |
| | C00403-DV40P | 14 | N/A |
| B00404 | C00404-DV22 | 38 | N/A |
| | C00404-DV25P | 48 | 44 |
| | C00404-DV26P | 47 | 39 |
| | C00404-DV27P | 41 | 40 |
| | C00404-DV29P | 49 | 43 |
| | C00404-DV32P | 41 | 29 |
| | C00404-DV34P | 33 | N/A |
| B00410 | C00410-DV22 | 15 | N/A |
| | C00410-DV25P | 26 | N/A |
| | C00410-DV26P | 21 | N/A |
| | C00410-DV27P | 8 | N/A |
| | C00410-DV29P | 1 | N/A |
| | C00410-DV32P | 6 | N/A |
| | C00410-DV34P | 4 | N/A |
| | C00410-DV39P | -11 | N/A |
| | C00410-DV40P | -2 | N/A |
| B05202 | C05202-DV22 | 24 | N/A |
| | C05202-DV25P | 26 | N/A |
| | C05202-DV26P | 24 | N/A |
| | C05202-DV27P | 29 | N/A |
| | C05202-DV29P | 27 | N/A |
| | C05202-DV32P | 21 | N/A |
| | C05202-DV34P | 33 | N/A |
| APC | APC-SL | 54 | 43 |

As shown in Table 12, the siRNA derivatives provided in the present disclosure exhibited an excellent inhibitory effect on FXI gene expression. Furthermore, compared to the Advanced ESC template DV22 disclosed in the prior art, the template-modified siRNA derivatives of the present disclosure exhibit a stronger inhibitory effect on gene expression.

### 3.3 Inhibitory effect of siRNA derivatives with inverted and anti-off-target modifications on FXI gene expression

The siRNA derivatives with inverted and anti-off-target modifications synthesized in Example 2 (sequences listed in Table 8) were transfected into HepG2 cells via lipid nanoparticles (LNPs) at concentrations of 1.00E+02, 5.00E+01, 2.50E+01, 1.30E+01, 1.60E+00, 2.00E-01, 2.40E-02, 3.10E-03, 3.80E-04, 4.80E-05, and 6.00E-06 nM, respectively. The inhibitory effect on the target gene FXI was measured at each concentration using qPCR, and IC₅₀ values were calculated.

The IC₅₀ values and maximum inhibition rates (transfection at the highest concentration) demonstrated that the five siRNA derivatives, C00211-DV25P, C00211-DV25PInV, C00211-DV25PInVs, C00211-DV25Pd7B, and C00211-DV25P6InV, exhibited IC₅₀ values lower than that of the positive control APC-SL, indicating superior efficacy compared to the positive control.

### Example 4: Inhibitory effect of siRNA conjugates on FXI gene expression in humanized mice

In this example, transgenic mice expressing the human FXI gene were used to evaluate the inhibitory effect of the siRNA conjugates listed in Table 9 on FXI protein expression *in vivo* using ELISA. The positive control was the most active compound L10-siFXIg1-M1SP disclosed in CN113227376B.

### 4.1 Inhibitory effect of siRNA conjugates on FXI protein expression in mice

Male hFXI transgenic mice aged 6 to 8 weeks were acclimated in the animal facility for approximately one week after arrival. The mice were then grouped based on their baseline hFXI levels and body weight, with 6 mice per group, totaling 40 groups, including a vehicle control group (saline), a negative control group, a positive control group, and test compound groups (Table 14). The test compounds were administered at a dose of 1 mpk via a single subcutaneous injection. On D7, 14, 21, 28, 35, 42, and 49 post-dose, serum hFXI levels were measured using an ELISA kit, and the inhibition rate of FXI protein expression *in vivo* was calculated.

**Table 13: Inhibition rate (%) of serum hFXI protein in high-dose groups of humanized mice**

| Group No. | Test compound | D7 | D14 | D21 | D28 | D35 | D42 | D49 |
|---|---|---|---|---|---|---|---|---|
| 1 | Vehicle control | -47.1 ± 34.43 | -31.55 ± 16.40 | -11.86 ± 24.28 | -15.58 ± 20.65 | -19.78 ± 21.20 | -36.04 ± 14.16 | -41.00 ± 21.69 |
| 2 | DNC-DV25PG101 | -38.61 ± 37.45 | -37.77 ± 21.01 | -10.29 ± 25.72 | -7.93 ± 20.50 | -5.66 ± 22.01 | -21.48 ± 22.95 | -48.98 ± 35.66 |
| 3 | DPC-SL10 | 59.12 ± 10.39 | 67.56 ± 9.04 | 66.06 ± 9.78 | 61.12 ± 8.67 | 49.81 ± 9.62 | 33.05 ± 18.52 | 17.40 ± 19.05 |
| 4 | D00209-DV26PG101 | 76.33 ± 4.57 | 73.46 ± 5.83 | 67.61 ± 7.74 | 57.53 ± 14.36 | 42.99 ± 12.51 | 24.75 ± 18.87 | / |
| 5 | D00209-DV26PInVG101 | 66.64 ± 6.07 | 62.07 ± 11.09 | 46.28 ± 12.22 | 37.71 ± 9.01 | / | / | / |
| 6 | D00209-DV32PG101 | 66.33 ± 8.17 | 53.23 ± 8.80 | 36.44 ± 16.90 | 28.27 ± 13.76 | / | / | / |
| 7 | D00209-DV32Pd7BG101 | 15.55 ± 23.74 | -9.61 ± 28.02 | -9.07 ± 14.03 | -2.15 ± 29.36 | / | / | / |
| 8 | D00209-DV32PInVG101 | 65.03 ± 9.97 | 60.75 ± 7.91 | 52.71 ± 9.71 | 36.84 ± 14.64 | / | / | / |
| 9 | D00209-DV34PG101 | 67.31 ± 11.53 | 65.3 ± 9.99 | 64.95 ± 5.81 | 56.01 ± 12.85 | 39.10 ± 15.70 | / | / |
| 10 | D00209-DV34Pd7BG101 | 62.71 ± 4.65 | 37.02 ± 7.94 | 18.05 ± 10.15 | 16.94 ± 13.14 | / | / | / |
| 11 | D00209-DV34PInVG101 | 69.20 ± 6.87 | 62.73 ± 10.54 | 52.61 ± 8.70 | 49.15 ± 12.15 | 33.27 ± 16.65 | / | / |
| 12 | D00210-DV25PG101 | 65.90 ± 12.39 | 58.84 ± 14.55 | 54.12 ± 12.33 | 42.05 ± 20.04 | 30.28 ± 19.97 | / | / |
| 13 | D00210-DV25PInVG101 | 75.06 ± 5.70 | 72.07 ± 4.83 | 67.41 ± 6.05 | 58.27 ± 11.07 | 38.09 ± 10.09 | / | / |
| 14 | D00211-DV25Pd7BG101 | 65.74 ± 9.15 | 59.32 ± 12.13 | 37.87 ± 15.55 | 13.40 ± 19.30 | / | / | / |
| 15 | D00211-DV25PG101 | 86.92 ± 4.16 | 88.61 ± 3.49 | 85.78 ± 4.10 | 84.08 ± 4.15 | 81.25 ± 5.45 | 73.03 ± 10.07 | 65.65 ± 9.78 |
| 16 | D00211-DV25PInVG101 | 78.23 ± 6.14 | 81.28 ± 5.96 | 77.09 ± 8.92 | 65.92 ± 12.86 | 55.92 ± 15.04 | 43.86 ± 15.50 | 17.68 ± 14.62 |
| 17 | D00211-DV25PInVsG101 | 80.65 ± 9.76 | 80.74 ± 11.93 | 74.96 ± 14.94 | 66.65 ± 18.52 | 58.03 ± 25.96 | 45.89 ± 28.65 | 22.49 ± 34.93 |
| 18 | D00211-DV26PG101 | 80.26 ± 4.51 | 81.36 ± 3.25 | 75.14 ± 5.61 | 63.87 ± 5.57 | 54.99 ± 13.75 | 45.35 ± 18.15 | 27.20 ± 11.12 |
| 19 | D00211-DV26Pd7BG101 | 61.69 ± 15.08 | 48.93 ± 13.65 | 24.44 ± 18.67 | -13.86 ± 23.44 | / | / | / |
| 20 | D00211-DV26PInVG101 | 78.59 ± 2.02 | 76.41 ± 4.64 | 59.33 ± 6.52 | 41.39 ± 6.86 | 20.89 ± 9.92 | / | / |
| 21 | D00211-DV26PInVsG101 | 77.93 ± 8.82 | 74.76 ± 9.50 | 66.55 ± 9.95 | 48.44 ± 14.06 | 28.16 ± 18.35 | / | / |
| 22 | D00211-DV34PG101 | 90.44 ± 2.60 | 92.08 ± 2.16 | 87.58 ± 2.50 | 90.09 ± 3.51 | 79.31 ± 5.73 | 75.15 ± 5.23 | 58.22 ± 12.44 |
| 23 | D00211-DV34PInVG101 | 82.54 ± 3.81 | 85.52 ± 4.21 | 77.81 ± 5.82 | 80.58 ± 9.41 | 60.42 ± 9.60 | 42.23 ± 13.00 | 17.92 ± 21.71 |
| 24 | D00211-DV34PInVsG101 | 82.28 ± 3.13 | 87.15 ± 2.01 | 80.59 ± 3.85 | 75.76 ± 4.35 | 63.49 ± 6.54 | 46.21 ± 7.11 | 21.68 ± 17.42 |
| 25 | D00211-DV39PG101 | 64.74 ± 10.86 | 46.43 ± 14.98 | 13.42 ± 19.25 | 0.06 ± 18.93 | / | / | / |
| 26 | D00211-DV39PInVG101 | 64.46 ± 3.60 | 52.01 ± 9.67 | 25.41 ± 13.73 | 4.14 ± 18.79 | / | / | / |
| 27 | D00211-DV39PInVsG101 | 46.45 ± 5.35 | 36.64 ± 4.68 | 10.92 ± 12.60 | -13.27 ± 11.04 | / | / | / |
| 28 | D00211s-DV25SPG101 | 85.06 ± 4.93 | 87.64 ± 2.81 | 85.75 ± 1.93 | 84.53 ± 3.61 | 83.66 ± 2.60 | 77.12 ± 1.86 | 70.09 ± 5.19 |
| 29 | D00211s-DV25SPInVG101 | 72.71 ± 7.42 | 72.91 ± 4.17 | 63.42 ± 10.32 | 52.95 ± 8.09 | 48.99 ± 16.08 | 30.04 ± 9.99 | / |
| 30 | D00212-DV32PG101 | 77.69 ± 3.25 | 79.30 ± 2.58 | 75.23 ± 3.94 | 69.12 ± 5.82 | 68.02 ± 6.68 | 55.13 ± 7.21 | 40.57 ± 10.90 |
| 31 | D00212-DV32PInVG101 | 71.91 ± 2.76 | 76.17 ± 1.24 | 67.65 ± 3.95 | 58.59 ± 4.61 | 54.13 ± 4.41 | 31.98 ± 13.67 | / |
| 32 | D00216-DV34PG101 | 80.65 ± 6.52 | 87.93 ± 4.62 | 85.34 ± 7.09 | 79.93 ± 9.25 | 78.47 ± 7.53 | 65.78 ± 15.49 | 53.33 ± 18.74 |
| 33 | D00216-DV34PInVG101 | 75.09 ± 10.46 | 79.95 ± 9.91 | 76.13 ± 15.29 | 68.35 ± 14.22 | 68.73 ± 15.59 | 55.62 ± 14.81 | 30.80 ± 25.82 |
| 34 | D00217s-DV32SPG101 | 66.96 ± 8.53 | 76.99 ± 4.93 | 73.13 ± 5.58 | 69.02 ± 6.60 | 71.69 ± 9.64 | 55.77 ± 12.87 | 39.12 ± 15.02 |
| 35 | D00217s-DV32SPInVG101 | 55.44 ± 5.70 | 47.74 ± 8.87 | 26.68 ± 7.68 | 6.72 ± 6.09 | / | / | / |
| 36 | D00217s-DV32SPInVsG101 | -7.94 ± 24.10 | -17.93 ± 21.55 | -16.55 ± 25.34 | -8.09 ± 17.76 | / | / | / |
| 37 | D00404-DV25PG101 | 70.64 ± 4.46 | 80.06 ± 3.96 | 74.93 ± 6.11 | 68.61 ± 3.59 | 68.89 ± 8.71 | 51.79 ± 9.06 | 32.62 ± 13.24 |
| 38 | D00404-DV25Pd7BG101 | 66.78 ± 6.37 | 77.26 ± 9.36 | 70.45 ± 7.04 | 62.57 ± 5.41 | 51.63 ± 12.26 | 39.51 ± 16.02 | / |
| 39 | D00404-DV25PInVG101 | 62.99 ± 7.65 | 70.85 ± 6.04 | 59.70 ± 8.36 | 48.53 ± 6.88 | 35.44 ± 9.19 | / | / |
| 40 | D00404-DV25PInVsG101 | 64.36 ± 6.20 | 64.00 ± 6.36 | 52.42 ± 7.53 | 37.77 ± 12.33 | / | / | / |

| | | | | | | | | |
|---|---|---|---|---|---|---|---|---|
| Note: "/" indicates that the observation was terminated and no measurement was performed. | | | | | | | | |

As shown in Table 13, in the study in which humanized mice were treated with high-dose siRNA conjugates, the positive control DPC-SL10 exhibited an inhibition rate of 61.12% on D28. A total of 14 siRNA conjugates exhibited inhibition rates higher than that of the positive control, demonstrating their ability to suppress FXI expression *in vivo* within 28 days. Specifically, five candidate compounds, including D00211-DV34PG101, D00211s-DV25SPG101, D00211-DV25PG101, D00211-DV34PInVG101, and D00216-DV34PG101, achieved inhibition rates above 80%. On D49, the inhibition rate of the positive control DPC-SL10 decreased to 17.40%, while thirteen siRNA conjugates exhibited inhibition rates higher than that of the positive control, among which D00211s-DV25SPG101 (70.09%), D00211-DV25PG101 (65.65%), D00211-DV34PG101 (58.22%), and D00216-DV34PG101 (53.33%) exhibited inhibition rates above 50%, demonstrating strong pharmacological activity.

To further identify highly active compounds, eight sequences showing superior pharmacological activity in the high-dose study (see Table 14) were selected for a low-dose study, together with the positive control DPC-SL10 and the vehicle control group. The dosing regimen for the low-dose groups was 0.5 mpk, with 8 mice per group, totaling 10 groups.

**Table 14: Inhibition rate (%) of serum hFXI protein in low-dose groups of humanized mice**

| Group No. | Test compound | D7 | D14 | D21 | D28 | D35 | D42 | D49 |
|---|---|---|---|---|---|---|---|---|
| 1 | Vehicle control | 3.28 ± 17.80 | -9.32 ± 18.07 | -16.25 ± 10.16 | -31.80 ± 16.15 | -32.57 ± 17.70 | -15.53 ± 18.01 | -43.15 ± 13.48 |
| 2 | DPC-SL10 | 63.56 ± 5.90 | 54.84 ± 6.00 | 45.14 ± 5.79 | 34.62 ± 9.71 | 24.91 ± 12.76 | 27.85 ± 12.74 | -1.01 ± 17.12 |
| 3 | D00211-DV25PG101 | 78.66 ± 8.74 | 74.37 ± 10.25 | 65.47 ± 15.11 | 53.77 ± 20.78 | 56.71 ± 21.53 | 61.30 ± 19.73 | 40.69 ± 26.02 |
| 4 | D00211-DV25PInVG101 | 79.54 ± 4.54 | 75.19 ± 6.53 | 57.30 ± 5.70 | 41.88 ± 8.61 | 29.18 ± 11.57 | 24.97 ± 8.28 | -4.57 ± 6.33 |
| 5 | D00211-DV34PG101 | 83.06 ± 3.76 | 80.21 ± 3.42 | 72.09 ± 3.80 | 66.36 ± 5.26 | 61.96 ± 5.50 | 58.91 ± 5.31 | 44.06 ± 6.85 |
| 6 | D00211-DV34PInVsG101 | 73.55 ± 4.35 | 72.50 ± 4.61 | 55.11 ± 6.08 | 39.63 ± 11.76 | 25.00 ± 9.86 | 26.37 ± 6.18 | -9.81 ± 14.62 |
| 7 | D00211s-DV25SPG101 | 85.81 ± 3.27 | 81.20 ± 4.46 | 74.81 ± 5.49 | 72.86 ± 4.58 | 71.52 ± 5.01 | 70.21 ± 6.04 | 55.87 ± 11.03 |
| 8 | D00212-DV32PG101 | 72.47 ± 6.22 | 67.30 ± 8.82 | 52.20 ± 11.96 | 53.44 ± 10.34 | 41.64 ± 19.31 | 43.32 ± 13.35 | 22.11 ± 17.05 |
| 9 | D00216-DV34PG101 | 70.99 ± 5.14 | 65.47 ± 9.36 | 56.09 ± 8.83 | 58.48 ± 8.65 | 45.38 ± 14.63 | 50.03 ± 12.57 | 23.58 ± 15.97 |
| 10 | D00404-DV25PG101 | 54.66 ± 12.10 | 51.31 ± 6.54 | 36.53 ± 9.59 | 42.56 ± 7.72 | 19.73 ± 13.44 | 31.83 ± 9.72 | -8.81 ± 19.54 |

As shown in Table 14, in the study in which humanized mice were treated with low-dose siRNA conjugates, the lowest inhibition rates against serum FXI across all groups were observed on D7. Specifically, seven siRNA conjugates (D00211-DV25PG101, D00211-DV25PInVG101, D00211-DV34PG101, D00211-DV34PInVsG101, D00211s-DV25SPG101, D00212-DV32PG101, and D00216-DV34PG101) exhibited inhibition rates higher than that of the positive control DPC-SL10 (63.56%), demonstrating strong pharmacological activity. On D49, the serum FXI level in the positive control group returned to baseline (an inhibition rate of -1.01%), while the inhibition rates of D00211s-DV25SPG101 (55.87%), D00211-DV34PG101 (44.06%), D00211-DV25PG101 (40.69%), D00216-DV34PG101 (23.58%), and D00212-DV32PG101 (22.11%) were significantly higher than that of the positive control, demonstrating strong pharmacological activity.

### 4.2 Prolongation effect of siRNA conjugates on APTT (activated partial thromboplastin time) in humanized mice

For the eight siRNA conjugates and the positive control listed in Table 15, APTT was measured on D49 in both high-dose and low-dose studies. The results are shown in Table 15 below.

**Table 15: APTT measurement in humanized mice on D49**

| Group No. | Compound No. | High-dose APTT (s) | Low-dose APTT (s) |
|---|---|---|---|
| 1 | Vehicle control | 46.0 ± 6.8 | 48.3 ± 4.6 |
| 2 | DPC-SL10 | 57.6 ± 11.8 | 49.4 ± 5.7 |
| 3 | D00211-DV25PG101 | 59.2 ± 8.1 | 55.4 ± 9.8 (↑14.5%) |
| 4 | D00211-DV25PInVG101 | 50.2 ± 4.5 | 46.8 ± 5.5 |
| 5 | D00211-DV34PG101 | 69.8 ± 23.1 (↑51.7%) | 56.4 ± 10.8 (↑16.8%) |
| 6 | D00211-DV34PInVsG101 | 56.5 ± 9.7 | 51.7 ± 7.2 |
| 7 | D00211s-DV25SPG101 | 70.5 ± 21.1 (↑53.3%) | 54.1 ± 9.2 (↑12.0%) |
| 8 | D00212-DV32PG101 | 71.1 ± 11.9 (154.6%) | 47.6 ± 10.7 |
| 9 | D00216-DV34PG101 | 66.2 ± 13.8 | 47.6 ± 7.0 |
| 10 | D00404-DV25PG101 | 64.3 ± 7.0 | 53.9 ± 18.8 |

As shown in Table 15, compared to the vehicle control group, APTT was prolonged in all eight siRNA conjugates and the positive control in the high-dose study. Specifically, D00211-DV34PG101, D00211s-DV25SPG101, and D00212-DV32PG101 exhibited prolongation rates greater than 50%. In the low-dose study, D00211-DV25PG101, D00211-DV34PG101, and D00211s-DV25SPG101 exhibited prolongation rates greater than 10%.

### Example 5: Pharmacodynamic effect of siRNA conjugates in cynomolgus monkeys

In this example, the pharmacodynamic effects of siRNA in cynomolgus monkeys were evaluated by measuring serum FXI protein levels, hepatic FXI mRNA expression, serum FXI activity, and APTT (activated partial thromboplastin time). The positive control DPC4059-SL10 corresponds to L10-siFXIf1-M1SP disclosed in CN113227376B.

Prior to administration, the animals were grouped based on serum FXI protein levels and body weight, while also considering measurement indicators such as APTT, PT (prothrombin time), and FXI activity, with 3 male cynomolgus monkeys per group, totaling 6 groups, including five siRNA conjugate groups and one positive control group. The test compounds were administered at a dose of 1 mpk via a single subcutaneous injection (at the dorsal neck region), with a dosing volume of 1 mL/kg (corresponding to a dosing concentration of 1 mg/mL). The day of administration was recorded as D0, and the observation period lasted 84 days.

### 5.1 Measurement of serum FXI protein levels

Serum FXI protein levels were measured by ELISA on D-1 pre-dose and on D7, 14, 21, 28, 42, 56, 70, and 84 post-dose, and the inhibition rate was calculated, as shown in Table 16.

**Table 16: Inhibition rate (%) of serum FXI protein**

| Group No. | Test compound | D7 | D14 | D21 | D28 | D42 | D56 | D70 | D84 |
|---|---|---|---|---|---|---|---|---|---|
| 1 | D00211-DV34PG101 | 70.47 ± 5.31 | 87.00 ± 5.52 | 90.01 ± 4.23 | 97.52 ± 0.63 | 94.31 ± 2.56 | 94.69 ± 2.31 | 92.69 ± 3.07 | 92.09 ± 2.27 |
| 2 | D00211-DV34PInVsG101 | 66.8 ± 17.80 | 84.69 ± 9.82 | 86.47 ± 9.31 | 96.00 ± 3.21 | 88.54 ± 7.93 | 84.52 ± 8.14 | 79.70 ± 9.54 | 64.27 ± 17.92 |
| 3 | D00211s-DV25SPG101 | 59.55 ± 6.18 | 77.04 ± 6.14 | 77.49 ± 3.02 | 92.29 ± 2.58 | 88.32 ± 4.29 | 83.59 ± 2.62 | 79.75 ± 6.15 | 66.55 ± 4.64 |
| 4 | D00212-DV32PG101 | 43.65 ± 14.02 | 66.46 ± 9.17 | 67.73 ± 8.44 | 87.45 ± 3.66 | 82.29 ± 5.78 | 79.05 ± 8.65 | 72.98 ± 14.85 | 60.81 ± 15.22 |
| 5 | D00216-DV34PG101 | 69.52 ± 5.30 | 89.14 ± 6.43 | 92.75 ± 4.17 | 96.90 ± 2.86 | 94.83 ± 4.53 | 93.10 ± 7.25 | 94.39 ± 1.98 | 86.13 ± 3.57 |
| 6 | DPC4059-SL10 | 26.19 ± 7.71 | 44.12 ± 5.88 | 42.1 ± 6.30 | 80.73 ± 1.10 | 71.16 ± 8.07 | 70.97 ± 8.81 | 66.96 ± 13.55 | 61.06 ± 18.60 |

As shown in Table 16, all five siRNA conjugates and the positive control DPC4059-SL10 exhibited inhibition of serum FXI expression in cynomolgus monkeys as early as D7 post-dose. The inhibition rates reached their peak on D28, with all five siRNA conjugates showing higher inhibition rates than the positive control. At the study endpoint (D84), the inhibition rates for all groups remained above 60%.

### 5.2 Measurement of hepatic FXI mRNA expression

Liver biopsies (approximately 5 mg of tissue) were collected from cynomolgus monkeys prior to administration and on D28, 56, and 84 post-dose, and total RNA was extracted. FXI mRNA expression levels were quantified by RT-qPCR, and the inhibition rate was calculated.

**Table 17: Inhibition rate (%) of hepatic FXI mRNA expression**

| Group No. | Compound No. | D28 | D56 | D84 |
|---|---|---|---|---|
| 1 | D00211-DV34PG101 | 79.31 ± 15.11 | 91.97 ± 4.67 | 71.25 ± 10.33 |
| 2 | D00211-DV34PInVsG101 | 79.53 ± 8.27 | 78.29 ± 15.12 | 24.53 ± 11.82 |
| 3 | D00211s-DV25SPG101 | 72.61 ± 11.15 | 78.05 ± 5.83 | 27.91 ± 17.2 |
| 4 | D00212-DV32PG101 | 50.52 ± 16.31 | 62.76 ± 13.79 | 29.73 ± 22.04 |
| 5 | D00216-DV34PG101 | 76.38 ± 11.24 | 88.64 ± 7.42 | 62.43 ± 6.05 |
| 6 | DPC4059-SL10 | 47.70 ± 12.80 | 72.57 ± 5.00 | 13.26 ± 27.03 |

As shown in Table 17, the lowest FXI mRNA levels across all groups were observed on D56, among which D00211-DV34PG101, D00216-DV34PG101, D00211-DV34PInVsG101, and D00211s-DV25SPG101 exhibited higher inhibition rates against hepatic FXI mRNA expression compared to the positive control DPC4059-SL10. On D84, the inhibition rates of all five siRNA conjugates against hepatic FXI mRNA expression were higher than that of the positive control DPC4059-SL10.

### 5.3 Measurement of serum FXI activity

FXI activity in plasma was measured using a semi-automated coagulation analyzer on D-1 pre-dose and on D7, 14, 21, 28, 42, 63, 77, and 84 post-dose. Specifically, whole blood was collected into a tube containing 3.8% sodium citrate anticoagulant and mixed thoroughly (to avoid hemolysis or coagulation). The samples were centrifuged immediately at 2500 g for 15 minutes at room temperature to obtain plasma, which was then diluted 10-fold with Owren-Koller buffer. Subsequently, 50 µL of FXI-deficient plasma was mixed with 50 µL of plasma sample to be tested, followed by incubation at 37°C for 1 minute. 50 µL of APTT reagent and one magnetic bead were then added, followed by incubation at 37°C for 3 minutes. Finally, CaCl₂ solution was added, mixed immediately at 37°C, and coagulation time was measured. FXI activity and reduction rates were calculated based on a standard curve of FXI activity versus coagulation time. As shown in Table 18, all five siRNA conjugates and the positive control induced a decrease in FXI activity in cynomolgus monkeys as early as D7, which persisted until D84.

**Table 18: Decrease in FXI activity (%)**

| Group No. | Compound No. | D7 | D14 | D21 | D28 | D42 | D63 | D77 | D84 |
|---|---|---|---|---|---|---|---|---|---|
| 1 | D00211-DV34PG101 | 47.66 ± 11.33 | 78.27 ± 9.51 | 80.73 ± 9.02 | 85.88 ± 7.78 | 91.50 ± 5.25 | 88.31 ± 5.74 | 51.55 ± 14.02 | 52.90 ± 9.94 |
| 2 | D00211-DV34PInVsG101 | 63.58 ± 14.01 | 84.98 ± 7.37 | 84.06 ± 8.56 | 83.83 ± 8.64 | 93.84 ± 2.21 | 75.54 ± 12.14 | 22.01 ± 5.97 | 20.16 ± 20.63 |
| 3 | D00211s-DV25SPG101 | 39.56 ± 20.36 | 67.31 ± 1.15 | 68.28 ± 0.80 | 69.42 ± 1.42 | 83.97 ± 0.72 | 68.95 ± 6.97 | 20.77 ± 20.73 | 20.89 ± 20.98 |
| 4 | D00212-DV32PG101 | 18.34 ± 18.84 | 54.39 ± 18.09 | 64.20 ± 11.89 | 56.53 ± 19.93 | 72.18 ± 19.90 | 73.77 ± 9.65 | 24.37 ± 6.41 | 13.9 ± 24 |
| 5 | D00216-DV34PG101 | 60.69 ± 1.67 | 83.11 ± 10.41 | 87.59 ± 9.95 | 89.63 ± 6.51 | 93.50 ± 4.69 | 91.35 ± 5.88 | 56.36 ± 4.90 | 37.12 ± 2.56 |
| 6 | DPC4059-SL10 | 25.13 ± 9.65 | 47.43 ± 0 | 47.54 ± 7.81 | 46.26 ± 3.94 | 65.79 ± 12.19 | 66.14 ± 2.89 | 21.70 ± 19.58 | 34.29 ± 10.99 |

### 5.3 Measurement of APTT and PT

APTT and PT in plasma were measured using a fully automated coagulation analyzer on D-1 pre-dose and on D7, 14, 21, 28, 42, 56, 70, and 84 post-dose to evaluate the effects of siRNA conjugates on intrinsic and extrinsic coagulation pathways. Specifically, whole blood was collected into a tube containing 3.8% sodium citrate anticoagulant and mixed thoroughly (to avoid hemolysis or coagulation). The samples were transferred immediately to the clinical laboratory and centrifuged at 3500 rpm for 15 minutes at room temperature to obtain plasma, which was analyzed immediately for APTT and PT, and APTT prolongation rates relative to the pre-dose baseline (D-1) for each group of animals were calculated. The results are shown in Tables 19 to 21 below.

**Table 19: APTT (s)**

| Group | Test compound | D-1 | D7 | D14 | D21 | D28 | D42 | D56 | D70 | D84 |
|---|---|---|---|---|---|---|---|---|---|---|
| 1 | D00211-DV34PG101 | 28.77 ± 0.81 | 37.40 ± 5.05 | 55.47 ± 15.76 | 46.00 ± 8.21 | 58.23 ± 18.09 | 48.33 ± 10.56 | 45.07 ± 6.57 | 39.63 ± 3.84 | 43.10 ± 3.22 |
| 2 | D00211-DV34PInVs101 | 27.50 ± 2.61 | 33.33 ± 1.76 | 52.73 ± 7.85 | 40.23 ± 4.31 | 44.97 ± 4.76 | 37.77 ± 0.85 | 35.20 ± 2.72 | 30.57 ± 1.77 | 32.17 ± 1.85 |
| 3 | D00211s-DV25SPG101 | 25.43 ± 0.23 | 28.97 ± 2.65 | 37.87 ± 4.38 | 33.13 ± 0.80 | 35.10 ± 2.98 | 33.37 ± 1.15 | 33.97 ± 0.87 | 31.37 ± 2.01 | 31.00 ± 0.62 |
| 4 | D00212-DV32PG101 | 32.97 ± 4.22 | 35.00 ± 4.46 | 44.10 ± 4.83 | 40.73 ± 6.57 | 46.07 ± 6.66 | 38.93 ± 3.12 | 37.93 ± 3.25 | 35.07 ± 1.02 | 38.03 ± 3.27 |
| 5 | D00216-DV34PG101 | 28.70 ± 3.11 | 35.05 ± 1.06 | 48.75 ± 4.03 | 45.20 ± 5.37 | 52.45 ± 7.71 | 46.25 ± 4.60 | 46.95 ± 5.44 | 44.75 ± 6.58 | 42.10 ± 3.96 |
| 6 | DPC4059-SL10 | 28.57 ± 2.90 | 28.30 ± 0.98 | 33.90 ± 3.52 | 30.60 ± 1.25 | 32.23 ± 0.67 | 32.67 ± 1.40 | 33.97 ± 3.01 | 30.23 ± 1.96 | 33.00 ± 3.15 |

**Table 20: PT (s)**

| Group No. | Test compound | D-1 | D7 | D14 | D21 | D28 | D42 | D56 | D70 | D84 |
|---|---|---|---|---|---|---|---|---|---|---|
| 1 | D00211-DV34PG101 | 13.27 ± 0.76 | 12.20 ± 0.61 | 11.37 ± 0.60 | 11.50 ± 0.70 | 11.70 ± 0.60 | 11.37 ± 0.81 | 11.40 ± 0.53 | 11.13 ± 0.40 | 11.53 ± 0.55 |
| 2 | D00211-DV34PInVsG101 | 13.17 ± 0.25 | 12.43 ± 0.12 | 12.10 ± 0.10 | 11.63 ± 0.25 | 11.70 ± 0.26 | 11.57 ± 0.06 | 11.47 ± 0.06 | 11.37 ± 0.15 | 11.97 ± 0.31 |
| 3 | D00211s-DV25SPG101 | 13.20 ± 0.78 | 12.03 ± 0.65 | 11.43 ± 0.35 | 11.77 ± 0.46 | 11.23 ± 0.49 | 11.17 ± 0.21 | 11.37 ± 0.42 | 11.67 ± 0.35 | 11.13 ± 0.55 |
| 4 | D00212-DV32PG101 | 13.40 ± 0.75 | 12.03 ± 0.06 | 11.77 ± 0.06 | 11.57 ± 0.06 | 11.73 ± 0.06 | 11.40 ± 0.17 | 11.43 ± 0.32 | 11.70 ± 0.44 | 11.57 ± 0.21 |
| 5 | D00216-DV34PG101 | 13.85 ± 0.64 | 12.45 ± 0.21 | 12.05 ± 0.35 | 12.00 ± 0.00 | 11.90 ± 0.28 | 11.70 ± 0.28 | 11.95 ± 0.21 | 12.00 ± 0.14 | 11.60 ± 0.00 |
| 6 | DPC4059-SL10 | 13.87 ± 0.15 | 12.27 ± 0.32 | 12.30 ± 0.36 | 12.20 ± 0.53 | 12.33 ± 0.40 | 11.87 ± 0.21 | 11.87 ± 0.06 | 12.00 ± 0.40 | 12.03 ± 0.55 |

**Table 21: APTT prolongation rate relative to pre-dose (D-1)**

| Group No. | Compound No. | D7 | D14 | D21 | D28 | D42 | D63 | D77 | D84 |
|---|---|---|---|---|---|---|---|---|---|
| 1 | D00211-DV34PG101 | 29.95 ± 16.63 | 91.91 ± 48.71 | 59.48 ± 23.91 | 101.39 ± 56.37 | 67.44 ± 31.66 | 56.34 ± 18.41 | 37.68 ± 11.24 | 49.70 ± 7.11 |
| 2 | D00211-DV34PInVsG101 | 21.76 ± 10.60 | 91.14 ± 10.56 | 46.2 ± 2.24 | 63.41 ± 2.24 | 38.01 ± 10.77 | 28.18 ± 4.90 | 11.79 ± 11.68 | 17.67 ± 12.74 |
| 3 | D00211s-DV25SPG101 | 13.84 ± 9.35 | 48.81 ± 16.17 | 30.27 ± 2.28 | 37.96 ± 10.85 | 31.18 ± 3.56 | 33.54 ± 2.69 | 23.37 ± 8.82 | 21.89 ± 2.41 |
| 4 | D00212-DV32PG101 | 6.22 ± 4.04 | 34.32 ± 12.81 | 23.42 ± 9.66 | 39.72 ± 9.15 | 18.92 ± 12.98 | 15.63 ± 9.35 | 7.35 ± 11.90 | 15.89 ± 8.30 |
| 5 | D00216-DV34PG101 | 23.05 ± 17.03 | 71.63 ± 32.65 | 59.44 ± 36.01 | 85.30 ± 46.94 | 62.98 ± 33.68 | 65.59 ± 36.92 | 58.09 ± 40.05 | 48.31 ± 29.87 |
| 6 | DPC4059-SL10 | 0.46 ± 7.35 | 19.27 ± 15.44 | 7.54 ± 6.26 | 13.43 ± 8.77 | 14.87 ± 8.36 | 19.38 ± 12.20 | 6.63 ± 13.83 | 16.38 ± 17.40 |

As shown in Tables 19 and 20, all five siRNA conjugates and the positive control induced a prolongation of APTT in cynomolgus monkeys, while no significant effect on PT was observed, indicating that the compounds prolong coagulation time by modulating the intrinsic coagulation pathway, without affecting the extrinsic coagulation pathway. As shown in Table 21, all five siRNA conjugates exhibited varying degrees of APTT prolongation as early as D7. From D7 to D42, the APTT prolongation rates of all five siRNA conjugates were higher than that of the positive control DPC4059-SL10. On D84, D00211-DV34PG101 and D00216-DV34PG101 exhibited APTT prolongation rates of 49.70% and 48.31%, respectively, demonstrating strong pharmacological activity.

### Example 6: Comparison between the sequences disclosed herein and those disclosed in the prior art

In this example, the unmodified sequences B00209, B00210, B00211, B00211s, B00212, B00215, B00216, and B00404 disclosed herein were compared with similar unmodified sequences disclosed in the prior art in terms of sequence structure and *in vitro* pharmacological activity.

### 6.1 Comparison of sequence structures

The sequences disclosed in the prior art are highly similar to those disclosed herein. The table below presents information such as sequence, length, position, and terminal mutations:

**Table 22: Structural comparison between sequences disclosed in the prior art and those disclosed herein**

| Group | Cpd | Sense strand | | | | | | Antisense strand | | | | | | Source |
|---|---|---|---|---|---|---|---|---|---|---|---|---|---|---|
| | | Sequence | Len | 5' Pos | 3' Pos | Term Mut | SEQ ID NO: | Sequence | Len | 5' Pos | 3' Pos | Term Mut | SEQ ID NO: | |
| Previously disclosed similar sequences | AV05205. um | GUUCAGG AUGAUUU UCUUAUA | 21 | 102 | 122 | 5' U→G | 787 | UAUAAGAA AAUCAUCCU GAAC | 21 | 122 | 102 | 3' A→C | 799 | WO202424 0058A1 |
| | AV01122. um | GAGGAUG AUUUUCU UAUAUCA | 21 | 105 | 125 | 5' C→G | 788 | UGAUAUAA GAAAAUCA UCCUC | 21 | 125 | 105 | 3' G→C | 800 | WO202424 0058A1 |
| | siRNA4 | CAGGAUG AUUUUCU UAUAU | 19 | 105 | 123 | | 789 | AUAUAAGA AAAUCAUCC UGAA | 21 | 123 | 103 | | 801 | WO202202 8457A1 |
| | TD09 | AGGAUGA UUUUCUU AUAUC | 19 | 106 | 124 | | 790 | GAUAUAAG AAAAUCAU CCUGA | 21 | 124 | 104 | | 802 | WO202504 5194A1 |
| | TD02 | GGAUGAU UUUCUUA UAUCA | 19 | 107 | 125 | | 791 | UGAUAUAA GAAAAUCA UCCUG | 21 | 125 | 105 | | 803 | WO202504 5194A1 |
| | AV05502. um | GGGAUGA UUUUCUU AUAUCAA | 21 | 106 | 126 | 5' A→G | 792 | UUGAUAUA AGAAAAUC AUCCC | 21 | 126 | 106 | 3' U→C | 804 | WO202424 0058A1 |
| | TJ-ASID4 | UCAGGAU GAUUUUC UUAUA | 19 | 104 | 122 | | 793 | UAUAAGAA AAUCAUCCU GAAA | 21 | 122 | 102 | | 805 | WO202310 9945A1 |
| | AV05205 191.um | UCAGGAU GAUUUUC UUAUA | 19 | 104 | 122 | | 793 | UAUAAGAA AAUCAUCCU GA | 19 | 122 | 104 | | 806 | WO202424 0058A1 |
| | TD10 | GAUGAUU UUCUUAU AUCAA | 19 | 108 | 126 | | 794 | UUGAUAUA AGAAAAUC AUCCU | 21 | 126 | 106 | | 807 | WO202504 5194A1 |
| | AV01123. um | CGAUGAU UUUCUUA UAUCAAA | 21 | 107 | 127 | 5' C→G, 3' G→A | 795 | UUUGAUAU AAGAAAAU CAUCG | 21 | 127 | 107 | 5' C→U, 3' C→G | 808 | WO202424 0058A1 |
| | TD11 | AUGAUUU UCUUAUA UCAAG | 19 | 109 | 127 | | 796 | CUUGAUAU AAGAAAAU CAUCC | 21 | 127 | 107 | | 809 | WO202504 5194A1 |
| | TD12 | UGAUUUU CUUAUAU CAAGU | 19 | 110 | 128 | | 797 | ACUUGAUA UAAGAAAA UCAUC | 21 | 128 | 108 | | 810 | WO202504 5194A1 |
| | AV00124. um | GCUUCAG UUUCUGG UGAAUGA | 21 | 148 | 168 | 5' A→G, 3' U→A | 798 | UCAUUCACC AGAAACUG AAGC | 21 | 168 | 148 | 5' A→U, 3' U→C | 811 | WO202424 0058A1 |
| Sequences disclosed herein | B00209 | UUUCAGG AUGAUUU UCUUAUA | 21 | 102 | 122 | | 8 | UAUAAGAA AAUCAUCCU GAAAAG | 23 | 122 | 100 | | 114 | The present disclosure |
| | B00210 | UUCAGGA UGAUUUU CUUAUAU | 21 | 103 | 123 | | 9 | AUAUAAGA AAAUCAUCC UGAAAA | 23 | 123 | 101 | | 115 | The present disclosure |
| | B00211 | CAGGAUG AUUUUCU UAUAUCA | 21 | 105 | 125 | | 10 | UGAUAUAA GAAAAUCA UCCUGAA | 23 | 125 | 103 | | 116 | The present disclosure |
| | B00211s | AGGAUGA UUUUCUU AUAUCA | 20 | 106 | 125 | | 11 | UGAUAUAA GAAAAUCA UCCUGA | 22 | 125 | 104 | | 117 | The present disclosure |
| | B00212 | AGGAUGA UUUUCUU AUAUCAA | 21 | 106 | 126 | | 12 | UUGAUAUA AGAAAAUC AUCCUGA | 23 | 126 | 104 | | 118 | The present disclosure |
| | B00215 | UCAGGAU GAUUUUC UUAUAUA | 21 | 104 | 124 | 3' C→A | 15 | UAUAUAAG AAAAUCAU CCUGAAA | 23 | 124 | 102 | 5' G→U | 121 | The present disclosure |
| | B00216 | GGAUGAU UUUCUUA UAUCAAA | 21 | 107 | 127 | 3' G→A | 16 | UUUGAUAU AAGAAAAU CAUCCUG | 23 | 127 | 105 | 5' C→U | 122 | The present disclosure |
| | B00404 | ACUUCAG UUUCUGG UGAAUGU | 21 | 148 | 168 | | 33 | ACAUUCACC AGAAACUG AAGUAA | 23 | 168 | 146 | | 139 | The present disclosure |

### 6.2 Comparison of in vitro pharmacological activity

At the *in vitro* cellular level, the inhibitory effects on the expression of the target gene FXI were compared between unmodified sequences disclosed in the prior art and those disclosed herein. The experimental procedure was essentially the same as described in Section 3.1.1, except that the cell transfection experiments were performed in a 96-well plate at a density of 3000 cells per well, and the final siRNA transfection concentrations were 0.01 nM and 0.10 nM. After 48 hours, RNA was extracted, and the inhibition of FXI gene expression was measured by qPCR. The results are shown in Table 23. The unmodified sequences disclosed herein exhibited significantly enhanced activity compared to similar unmodified sequences disclosed in the prior art.

**Table 23: Comparison of the inhibition of FXI expression between sequences disclosed in the prior art and those disclosed herein**

| Group | Compound name | Inhibition rate (%) | |
|---|---|---|---|
| | | 0.01 nM | 0.1 nM |
| | AV05205.um | 28.3 | 59.1 |
| | AV01122.um | 58.0 | 69.3 |
| | siRNA4 | 32.0 | 58.4 |
| | TD09 | 24.4 | 58.8 |
| Previously disclosed similar sequences | TD02 | 50.2 | 68.8 |
| | AV05502.um | 7.9 | 23.5 |
| | TJ-ASID4 | 29.3 | 56.6 |
| | AV05205191.um | 24.0 | 47.5 |
| | TD10 | 25.7 | 50.9 |
| | AV01123.um | 8.4 | 55.1 |
| | TD11 | 34.3 | 62.7 |
| | TD12 | 10.6 | 44.3 |
| | AV00124.um | 20.2 | 63.4 |
| | B00209 | 48.3 | 67.9 |
| | B00210 | 25.3 | 61.5 |
| Sequences disclosed herein | B00211 | 59.2 | 66.7 |
| | B00211s | 58.2 | 72.3 |
| | B00212 | 45.1 | 61.2 |
| | B00215 | 34.4 | 60.2 |
| | B00216 | 39.3 | 66.7 |
| | B00404 | 37.7 | 61.4 |

Although specific embodiments of the present disclosure have been described in detail, the aforementioned examples and specific embodiments should not be construed as limiting the scope of the present disclosure. Those skilled in the art will understand that, without departing from the spirit and essence of the present disclosure, various modifications and substitutions can be made to those details based on all the teachings that have been disclosed, and such modifications and substitutions shall fall within the scope of the present disclosure. The full scope of the present disclosure is defined by the appended claims and any equivalents thereof.

## Claims

1. An siRNA duplex, wherein the siRNA duplex comprises a sense strand and an antisense strand that form a reverse complementary duplex region, wherein the siRNA duplex comprises any one oligonucleotide duplex selected from the following pairings of a sense strand and an antisense strand, or a combination of at least two thereof, optionally wherein the sense strand and the antisense strand each independently comprise at least one modified nucleotide:
(1) the sense strand has the sequence of SEQ ID NO: 10 or a fragment thereof; and the antisense strand has the sequence of SEQ ID NO: 116 or a fragment thereof;
(2) the sense strand has the sequence of SEQ ID NO: 11 or a fragment thereof; and the antisense strand has the sequence of SEQ ID NO: 117 or a fragment thereof;
(3) the sense strand has the sequence of SEQ ID NO: 12 or a fragment thereof; and the antisense strand has the sequence of SEQ ID NO: 118 or a fragment thereof;
(4) the sense strand has the sequence of SEQ ID NO: 16 or a fragment thereof; and the antisense strand has the sequence of SEQ ID NO: 122 or a fragment thereof;
(5) the sense strand has the sequence of SEQ ID NO: 9 or a fragment thereof; and the antisense strand has the sequence of SEQ ID NO: 115 or a fragment thereof;
(6) the sense strand has the sequence of SEQ ID NO: 8 or a fragment thereof; and the antisense strand has the sequence of SEQ ID NO: 114 or a fragment thereof;
(7) the sense strand has the sequence of SEQ ID NO: 15 or a fragment thereof; and the antisense strand has the sequence of SEQ ID NO: 121 or a fragment thereof;
(8) the sense strand has the sequence of SEQ ID NO: 17 or a fragment thereof; and the antisense strand has the sequence of SEQ ID NO: 123 or a fragment thereof;
(9) the sense strand has the sequence of SEQ ID NO: 33 or a fragment thereof; and the antisense strand has the sequence of SEQ ID NO: 139 or a fragment thereof.

2. The siRNA duplex according to claim 1, wherein the sense strand or the antisense strand comprises at least one modified nucleotide selected from a deoxy-nucleotide, a 3'-deoxythymidine nucleotide, a 2'-O-methyl modified nucleotide, a 2'-fluoro-modified nucleotide, a 2'-deoxy-modified nucleotide, a locked nucleotide, an unlocked nucleotide, a conformationally restricted nucleotide, a constrained ethyl nucleotide, an abasic nucleotide, a 2'-amino-modified nucleotide, a 2'-O-allyl-modified nucleotide, a 2'-C-alkyl-modified nucleotide, a 2'-hydroxy-modified nucleotide, a 2'-methoxyethyl-modified nucleotide, a 2'-O-alkyl-modified nucleotide, a morpholino nucleotide, a phosphoramidate, a nucleotide comprising a non-natural base, a tetrahydropyran-modified nucleotide, a 1,5-anhydrohexitol-modified nucleotide, a cyclohexenyl-modified nucleotide, a nucleotide comprising a phosphorothioate group, a nucleotide comprising a methylphosphonate group, a nucleotide comprising a 5'-phosphate group, and a nucleotide comprising a 5'-phosphate mimic; and combinations thereof.

3. The siRNA duplex according to claim 1 or 2, wherein all the modification of nucleotides on the sense strand and the antisense strand is a chemical modification at the 2' position of the ribose of the nucleotide, wherein the chemical modification at the 2' position of the ribose of the nucleotide is any one or more selected from 2'-O-methyl, 2'-methoxyethyl, 2'-fluoro, 2'-deoxy, 2'-benzyloxy, 2'-methylcarbonylamino, and 2'-pyridinylmethoxy, and a combination thereof.

4. The siRNA duplex according to claim 1, wherein 5' carbon atom of the nucleotide glycoside at the 5' end of the modified antisense strand is phosphorylated, wherein the phosphorylation of the 5' carbon atom is selected from the following 5'-phosphorylation groups: 5'-vinylphosphonate group, 5'-methylphosphonate group, 5'-C-methylphosphonate group, 5'-phosphorothioate group, and 5'-phosphate group, the structures of which are as follows: 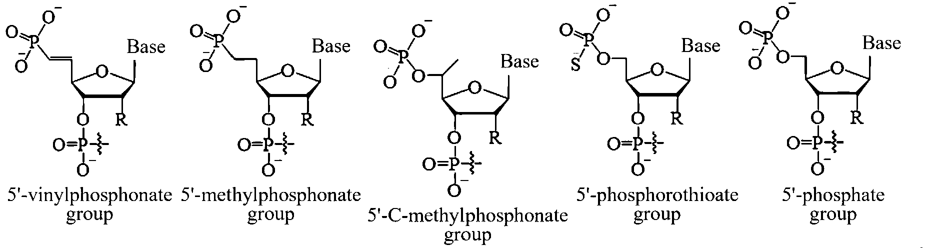
wherein R is hydrogen, hydroxyl, amino, C₁₋₄ alkyl, aryl, C₁₋₄ alkoxy, C₁₋₄ alkylcarbonylamino, or halogen;
the base is selected from adenine, guanine, cytosine, thymine, and uracil.

5. The siRNA duplex according to claim 1, wherein the antisense strand and the sense strand are modified according to the following modification patterns:
(1) the antisense strand has any one of modification patterns A to H as follows: 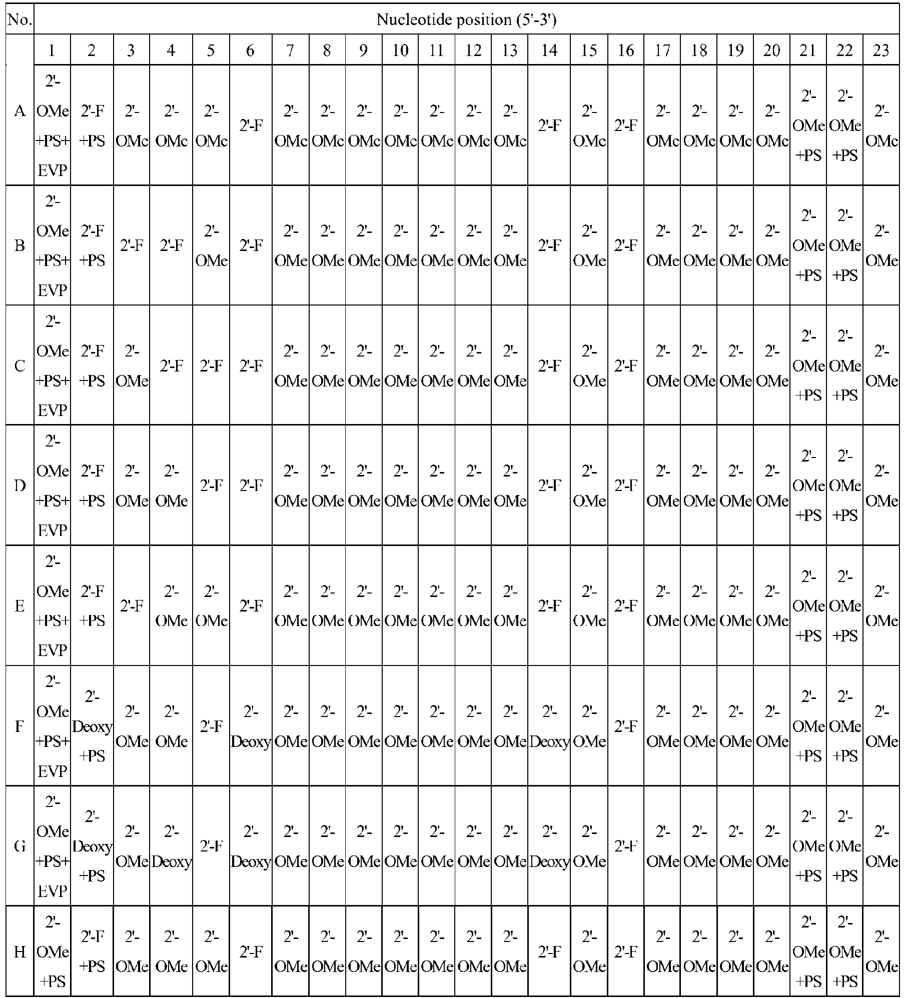
and/or, the sense strand has any one of modification patterns a to c as follows: 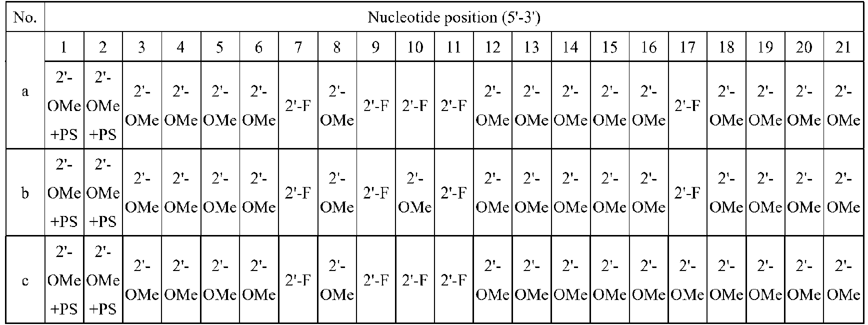
wherein 2'-OMe represents a 2'-O-methyl modification; 2'-F represents a 2'-fluoro modification; 2'-Deoxy represents a 2'-deoxy modification; PS indicates that, starting from the 5' end, the nucleotide at the position is linked to the adjacent nucleotide on the right via a phosphorothioate bond; EVP represents a 5'-vinyl-(E)-phosphonate modification;
preferably, the antisense strand is modified according to modification pattern A, and the sense strand is modified according to modification pattern a; or
the antisense strand is modified according to modification pattern B, and the sense strand is modified according to modification pattern a; or
the antisense strand is modified according to modification pattern C, and the sense strand is modified according to modification pattern a; or
the antisense strand is modified according to modification pattern C, and the sense strand is modified according to modification pattern b; or
the antisense strand is modified according to modification pattern D, and the sense strand is modified according to modification pattern b; or
the antisense strand is modified according to modification pattern E, and the sense strand is modified according to modification pattern b; or
the antisense strand is modified according to modification pattern F, and the sense strand is modified according to modification pattern b; or
the antisense strand is modified according to modification pattern G, and the sense strand is modified according to modification pattern b; or
the antisense strand is modified according to modification pattern H, and the sense strand is modified according to modification pattern c;
(2) the antisense strand has any one of modification patterns A' to G' as follows: 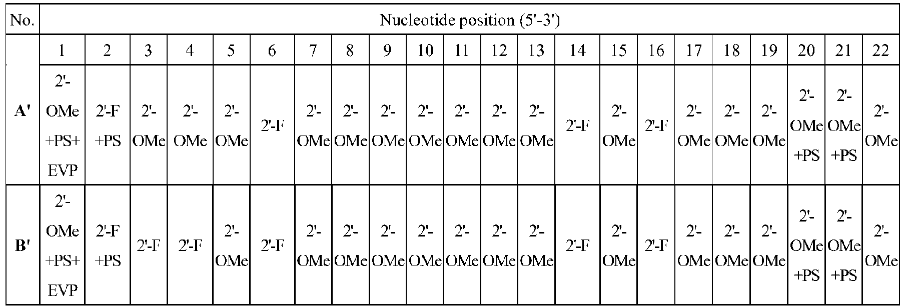 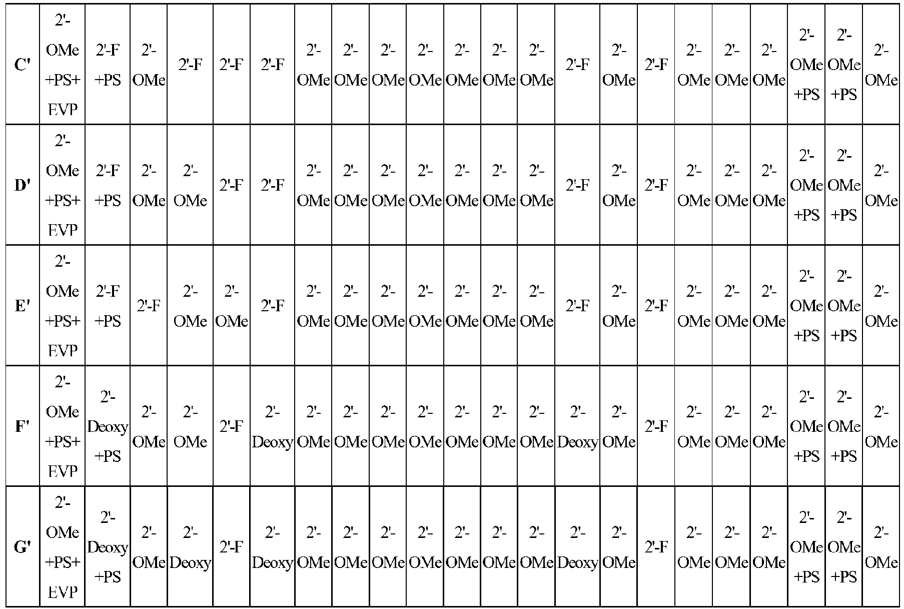
and/or, the sense strand has modification pattern a' or b' as follows: 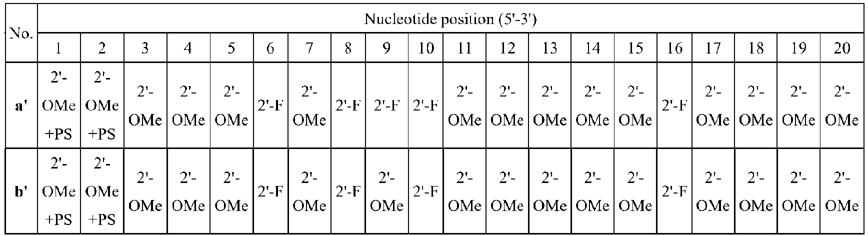
wherein 2'-OMe represents a 2'-O-methyl modification; 2'-F represents a 2'-fluoro modification; 2'-Deoxy represents a 2'-deoxy modification; PS indicates that, starting from the 5' end, the nucleotide at the position is linked to the adjacent nucleotide on the right via a phosphorothioate bond; EVP represents a 5'-vinyl-(E)-phosphonate modification.

6. The siRNA duplex according to claim 5, wherein the antisense strand is modified according to modification pattern A', and the sense strand is modified according to modification pattern a'; or
the antisense strand is modified according to modification pattern B', and the sense strand is modified according to modification pattern a'; or
the antisense strand is modified according to modification pattern C', and the sense strand is modified according to modification pattern a'; or
the antisense strand is modified according to modification pattern C', and the sense strand is modified according to modification pattern b'; or
the antisense strand is modified according to modification pattern D', and the sense strand is modified according to modification pattern b'; or
the antisense strand is modified according to modification pattern E', and the sense strand is modified according to modification pattern b'; or
the antisense strand is modified according to modification pattern F', and the sense strand is modified according to modification pattern b'; or
the antisense strand is modified according to modification pattern G', and the sense strand is modified according to modification pattern b'.

7. The siRNA duplex according to claim 1, wherein the siRNA duplex comprises any one oligonucleotide duplex selected from the following pairings of a sense strand and an antisense strand, or a combination of at least two thereof:
(1) the sense strand has the sequence of SEQ ID NO: 222; the antisense strand has the sequence of SEQ ID NO: 328;
(2) the sense strand has the sequence of SEQ ID NO: 228; the antisense strand has the sequence of SEQ ID NO: 334;
(3) the sense strand has the sequence of SEQ ID NO: 223; the antisense strand has the sequence of SEQ ID NO: 329;
(4) the sense strand has the sequence of SEQ ID NO: 224; the antisense strand has the sequence of SEQ ID NO: 330;
(5) the sense strand has the sequence of SEQ ID NO: 220; the antisense strand has the sequence of SEQ ID NO: 326;
(6) the sense strand has the sequence of SEQ ID NO: 221; the antisense strand has the sequence of SEQ ID NO: 327;
(7) the sense strand has the sequence of SEQ ID NO: 227; the antisense strand has the sequence of SEQ ID NO: 333;
(8) the sense strand has the sequence of SEQ ID NO: 229; the antisense strand has the sequence of SEQ ID NO: 335;
(9) the sense strand has the sequence of SEQ ID NO: 245; the antisense strand has the sequence of SEQ ID NO: 351;
(10) the sense strand has the sequence of SEQ ID NO: 426; and the antisense strand has the sequence of SEQ ID NO: 552;
(11) the sense strand has the sequence of SEQ ID NO: 427; and the antisense strand has the sequence of SEQ ID NO: 553;
(12) the sense strand has the sequence of SEQ ID NO: 429; and the antisense strand has the sequence of SEQ ID NO: 555;
(13) the sense strand has the sequence of SEQ ID NO: 430; and the antisense strand has the sequence of SEQ ID NO: 556;
(14) the sense strand has the sequence of SEQ ID NO: 431; and the antisense strand has the sequence of SEQ ID NO: 557;
(15) the sense strand has the sequence of SEQ ID NO: 435; and the antisense strand has the sequence of SEQ ID NO: 561;
(16) the sense strand has the sequence of SEQ ID NO: 439; and the antisense strand has the sequence of SEQ ID NO: 565;
(17) the sense strand has the sequence of SEQ ID NO: 440; and the antisense strand has the sequence of SEQ ID NO: 566;
(18) the sense strand has the sequence of SEQ ID NO: 442; and the antisense strand has the sequence of SEQ ID NO: 568;
(19) the sense strand has the sequence of SEQ ID NO: 443; and the antisense strand has the sequence of SEQ ID NO: 569;
(20) the sense strand has the sequence of SEQ ID NO: 444; and the antisense strand has the sequence of SEQ ID NO: 570;
(21) the sense strand has the sequence of SEQ ID NO: 445; and the antisense strand has the sequence of SEQ ID NO: 571;
(22) the sense strand has the sequence of SEQ ID NO: 446; and the antisense strand has the sequence of SEQ ID NO: 572;
(23) the sense strand has the sequence of SEQ ID NO: 447; and the antisense strand has the sequence of SEQ ID NO: 573;
(24) the sense strand has the sequence of SEQ ID NO: 448; and the antisense strand has the sequence of SEQ ID NO: 574;
(25) the sense strand has the sequence of SEQ ID NO: 449; and the antisense strand has the sequence of SEQ ID NO: 575;
(26) the sense strand has the sequence of SEQ ID NO: 450; and the antisense strand has the sequence of SEQ ID NO: 576;
(27) the sense strand has the sequence of SEQ ID NO: 454; and the antisense strand has the sequence of SEQ ID NO: 580;
(28) the sense strand has the sequence of SEQ ID NO: 455; and the antisense strand has the sequence of SEQ ID NO: 581;
(29) the sense strand has the sequence of SEQ ID NO: 456; and the antisense strand has the sequence of SEQ ID NO: 582;
(30) the sense strand has the sequence of SEQ ID NO: 468; and the antisense strand has the sequence of SEQ ID NO: 594;
(31) the sense strand has the sequence of SEQ ID NO: 471; and the antisense strand has the sequence of SEQ ID NO: 597;
(32) the sense strand has the sequence of SEQ ID NO: 477; and the antisense strand has the sequence of SEQ ID NO: 603;
(33) the sense strand has the sequence of SEQ ID NO: 478; and the antisense strand has the sequence of SEQ ID NO: 604;
(34) the sense strand has the sequence of SEQ ID NO: 479; and the antisense strand has the sequence of SEQ ID NO: 605;
(35) the sense strand has the sequence of SEQ ID NO: 480; and the antisense strand has the sequence of SEQ ID NO: 606;
(36) the sense strand has the sequence of SEQ ID NO: 529; and the antisense strand has the sequence of SEQ ID NO: 655;
(37) the sense strand has the sequence of SEQ ID NO: 530; and the antisense strand has the sequence of SEQ ID NO: 656;
(38) the sense strand has the sequence of SEQ ID NO: 532; and the antisense strand has the sequence of SEQ ID NO: 658;
(39) the sense strand has the sequence of SEQ ID NO: 481; and the antisense strand has the sequence of SEQ ID NO: 607.

8. The siRNA duplex according to claim 1 or 7, wherein the nucleotides at positions 2 to 8 from the 5' end of the antisense strand are each independently a modified nucleotide, wherein the modified nucleotide is an unlocked nucleic acid or a glycol nucleic acid; or, the nucleotides at positions 2 to 8 from the 5' end of the antisense strand are each independently DNA, wherein the structures of the unlocked nucleic acid and the glycol nucleic acid are as follows: 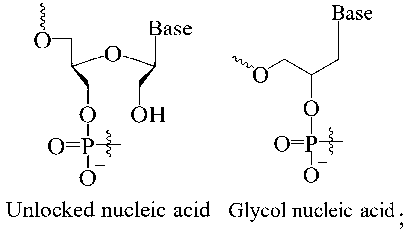
wherein the base is any one selected from adenine, guanine, cytosine, thymine, and uracil; and/or
the nucleotide at the 3' end of the sense strand or the antisense strand is an invertedly linked nucleotide, or the nucleotide at position 6 or 7 from the 5' end of the sense strand is an invertedly linked nucleotide, wherein the modified nucleotide is selected from dAinV, dTinV, dGinV, and dCinV having the following structures:

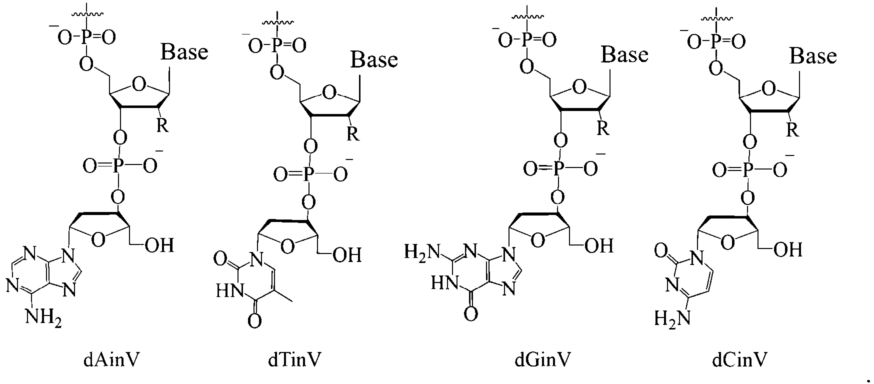

9. The siRNA duplex according to claim 8, wherein the siRNA duplex comprises any one oligonucleotide duplex selected from the following pairings of a sense strand and an antisense strand, or a combination of at least two thereof:
(1) the sense strand has the sequence of SEQ ID NO: 677; and the antisense strand has the sequence of SEQ ID NO: 686;
(2) the sense strand has the sequence of SEQ ID NO: 678; and the antisense strand has the sequence of SEQ ID NO: 687;
(3) the sense strand has the sequence of SEQ ID NO: 684; and the antisense strand has the sequence of SEQ ID NO: 693;
(4) the sense strand has the sequence of SEQ ID NO: 680; and the antisense strand has the sequence of SEQ ID NO: 689;
(5) the sense strand has the sequence of Um-Um-Um-Cm-Am-Gm-Gf-Am-Uf-Gf-Af-Um-Um-Um-Um-Cm-Uf-Um-Ams-Ums-dAinV; and the antisense strand has the sequence of UmsEVP-Afs-Uf-Af-Am-Gf-Am-Am-Am-Am-Um-Cm-Am-Uf-Cm-Cf-Um-Gm-Am-Am-Ams-Ams-Gm;
(6) the sense strand has the sequence of Ums-Ums-Um-Cm-Am-Gm-Gf-Am-Uf-Gm-Af-Um-Um-Um-dT-Cm-Uf-Um-Am-Um-Am; and the antisense strand has the sequence of UmsEVP-Afs-Um-Am-Af-Gf-dA-Am-Am-Am-Um-Cm-Am-Uf-Cm-Cf-Um-Gm-Am-Am-Ams-Ams-Gm;
(7) the sense strand has the sequence of Um-Um-Um-Cm-Am-Gm-Gf-Am-Uf-Gm-Af-Um-Um-Um-Um-Cm-Uf-Um-Ams-Ums-dAinV; and the antisense strand has the sequence of UmsEVP-Afs-Um-Am-Af-Gf-Am-Am-Am-Am-Um-Cm-Am-Uf-Cm-Cf-Um-Gm-Am-Am-Ams-Ams-Gm;
(8) the sense strand has the sequence of Ums-Ums-Um-Cm-Am-Gm-Gf-Am-Uf-Gm-Af-Um-Um-Um-dT-Cm-Uf-Um-Am-Um-Am; and the antisense strand has the sequence of UmsEVP-Afs-Uf-Am-Am-Gf-dA-Am-Am-Am-Um-Cm-Am-Uf-Cm-Cf-Um-Gm-Am-Am-Ams-Ams-Gm;
(9) the sense strand has the sequence of Um-Um-Um-Cm-Am-Gm-Gf-Am-Uf-Gm-Af-Um-Um-Um-Um-Cm-Uf-Um-Ams-Ums-dAinV; and the antisense strand has the sequence of UmsEVP-Afs-Uf-Am-Am-Gf-Am-Am-Am-Am-Um-Cm-Am-Uf-Cm-Cf-Um-Gm-Am-Am-Ams-Ams-Gm;
(10) the sense strand has the sequence of Um-Um-Cm-Am-Gm-Gm-Af-Um-Gf-Af-Uf-Um-Um-Um-Cm-Um-Uf-Am-Ums-Ams-dTinV; and the antisense strand has the sequence of AmsEVP-Ufs-Am-Um-Am-Af-Gm-Am-Am-Am-Am-Um-Cm-Af-Um-Cf-Cm-Um-Gm-Am-Ams-Ams-Am;
(11) the sense strand has the sequence of Cms-Ams-Gm-Gm-Am-Um-Gf-Am-Uf-Uf-Uf-Um-Cm-Um-dT-Am-Uf-Am-Um-Cm-Am; and the antisense strand has the sequence of UmsEVP-Gfs-Am-Um-Am-Uf-dA-Am-Gm-Am-Am-Am-Am-Uf-Cm-Af-Um-Cm-Cm-Um-Gms-Ams-Am;
(12) the sense strand has the sequence of Cms-Ams-Gm-Gm-Am-Um-Gf-Am-Uf-Uf-Uf-Um-Cm-Um-dT-Am-Uf-Am-Um-Cm-Am; and the antisense strand has the sequence of UmsEVP-Gfs-Af-Uf-Am-Uf-dA-Am-Gm-Am-Am-Am-Am-Uf-Cm-Af-Um-Cm-Cm-Um-Gms-Ams-Am;
(13) the sense strand has the sequence of Cm-Am-Gm-Gm-Am-Um-Gf-Am-Uf-Uf-Uf-Um-Cm-Um-Um-Am-Uf-Am-Ums-Cms-dAinV; and the antisense strand has the sequence of UmsEVP-Gfs-Af-Uf-Am-Uf-Am-Am-Gm-Am-Am-Am-Am-Uf-Cm-Af-Um-Cm-Cms-Ums-Gm;
(14) the sense strand has the sequence of Cm-Am-Gm-Gm-Am-Um-Gf-Am-Uf-Uf-Uf-Um-Cm-Um-Um-Am-Uf-Am-Ums-Cms-dAinV; and the antisense strand has the sequence of UmsEVP-Gfs-Af-Uf-Am-Uf-Am-Am-Gm-Am-Am-Am-Am-Uf-Cm-Af-Um-Cm-Cm-Um-Gms-Ams-Am;
(15) the sense strand has the sequence of Cm-Am-Gm-Gm-Am-Um-Gf-Am-Uf-Um-Uf-Um-Cm-Um-Um-Am-Uf-Am-Ums-Cms-dAinV; and the antisense strand has the sequence of UmsEVP-dGs-Am-Um-Af-dT-Am-Am-Gm-Am-Am-Am-Am-dT-Cm-Af-Um-Cm-Cms-Ums-Gm;
(16) the sense strand has the sequence of Cm-Am-Gm-Gm-Am-Um-Gf-Am-Uf-Um-Uf-Um-Cm-Um-Um-Am-Uf-Am-Ums-Cms-dAinV; and the antisense strand has the sequence of UmsEVP-dGs-Am-Um-Af-dT-Am-Am-Gm-Am-Am-Am-Am-dT-Cm-Af-Um-Cm-Cm-Um-Gms-Ams-Am;
(17) the sense strand has the sequence of Am-Gm-Gm-Am-Um-Gf-Am-Uf-Uf-Uf-Um-Cm-Um-Um-Am-Uf-Am-Ums-Cms-dAinV; and the antisense strand has the sequence of UmsEVP-Gfs-Am-Um-Am-Uf-Am-Am-Gm-Am-Am-Am-Am-Uf-Cm-Af-Um-Cm-Cm-Ums-Gms-Am;
(18) the sense strand has the sequence of Am-Gm-Gm-Am-Um-Gm-Af-Um-Uf-Um-Uf-Cm-Um-Um-Am-Um-Af-Um-Cms-Ams-dAinV; and the antisense strand has the sequence of UmsEVP-Ufs-Gm-Am-Uf-Af-Um-Am-Am-Gm-Am-Am-Am-Af-Um-Cf-Am-Um-Cm-Cm-Ums-Gms-Am;
(19) the sense strand has the sequence of Gm-Gm-Am-Um-Gm-Am-Uf-Um-Uf-Um-Cf-Um-Um-Am-Um-Am-Uf-Cm-Ams-Ams-dAinV; and the antisense strand has the sequence of UmsEVP-Ufs-Uf-Gm-Am-Uf-Am-Um-Am-Am-Gm-Am-Am-Af-Am-Uf-Cm-Am-Um-Cm-Cms-Ums-Gm;
(20) the sense strand has the sequence of Am-Um-Gm-Am-Um-Uf-Um-Uf-Cm-Uf-Um-Am-Um-Am-Um-Cf-Am-Ams-Gms-dTinV; and the antisense strand has the sequence of AmsEVP-Cfs-Um-Um-Gf-Af-Um-Am-Um-Am-Am-Gm-Am-Af-Am-Af-Um-Cm-Am-Ums-Cms-Cm;
(21) the sense strand has the sequence of Um-Uf-Cm-Uf-Um-Am-Um-Am-Um-Cf-Am-Ams-Gms-dTinV; and the antisense strand has the sequence of AmsEVP-Cfs-Um-Um-Gf-Af-Um-Am-Um-Am-Am-Gm-Am-Af-Am-Af-Um-Cms-Ams-Um;
(22) the sense strand has the sequence of Ams-Cms-Um-Um-Cm-Am-Gf-Um-Uf-Uf-Cf-Um-Gm-Gm-dT-Gm-Af-Am-Um-Gm-Um; and the antisense strand has the sequence of AmsEVP-Cfs-Am-Um-Um-Cf-dA-Cm-Cm-Am-Gm-Am-Am-Af-Cm-Uf-Gm-Am-Am-Gm-Ums-Ams-Am;
(23) the sense strand has the sequence of Am-Cm-Um-Um-Cm-Am-Gf-Um-Uf-Uf-Cf-Um-Gm-Gm-Um-Gm-Af-Am-Ums-Gms-dTinV; and the antisense strand has the sequence of AmsEVP-Cfs-Am-Um-Um-Cf-Am-Cm-Cm-Am-Gm-Am-Am-Af-Cm-Uf-Gm-Am-Ams-Gms-Um;
(24) the sense strand has the sequence of Am-Cm-Um-Um-Cm-Am-Gf-Um-Uf-Uf-Cf-Um-Gm-Gm-Um-Gm-Af-Am-Ums-Gms-dTinV; and the antisense strand has the sequence of AmsEVP-Cfs-Am-Um-Um-Cf-Am-Cm-Cm-Am-Gm-Am-Am-Af-Cm-Uf-Gm-Am-Am-Gm-Ums-Ams-Am;
wherein m represents a 2'-O-methyl modification; f represents a 2'-fluoro modification; s indicates that the nucleotide at the position is linked to the adjacent nucleotide on the right via a phosphorothioate bond; dA represents a 2'-deoxyadenosine-3'-phosphate; dG represents a 2'-deoxyguanosine-3'-phosphate; dT represents a 2'-deoxythymidine-3'-phosphate; EVP represents a 5'-vinyl-(E)-phosphonate modification; inV indicates that the linkage at the ends of the oligonucleotide sequence was altered from the natural 3'-5' linkage to 3'-3'.

10. A conjugate for reducing FXI expression, wherein the conjugate comprises the siRNA duplex according to any one of claims 1 to 9 and a conjugate moiety attached thereto;
preferably, the conjugate moiety is attached to the 3' end or 5' end of the sense strand of the oligonucleotide; and/or
the conjugate moiety is a GalNAc derivative attached via a bivalent or trivalent branched linker arm;
more preferably, the conjugate moiety is one selected from the following conjugate moieties:
(1) the conjugate moiety is as follows: 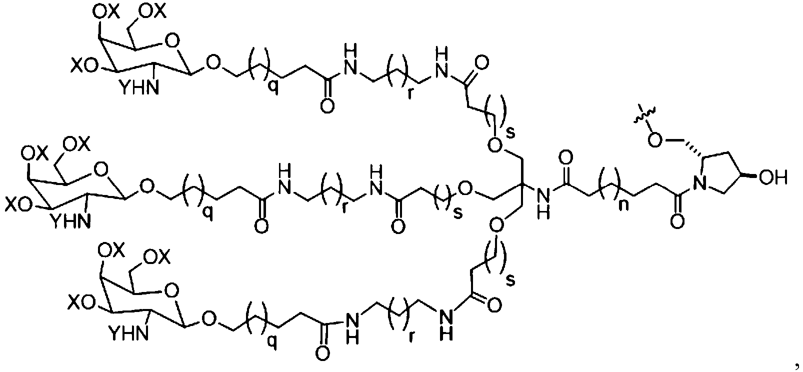
wherein X is a hydroxyl protecting group or H, the hydroxyl protecting group being selected from acetyl, benzoyl, and isobutyryl; Y is an amino protecting group or H, the amino protecting group being selected from formyl, acetyl, propionyl, n-butyryl, and isobutyryl; n is an integer from 0 to 20; q, r, and s are independently integers from 1 to 7;
(2) the conjugate moiety is as follows: 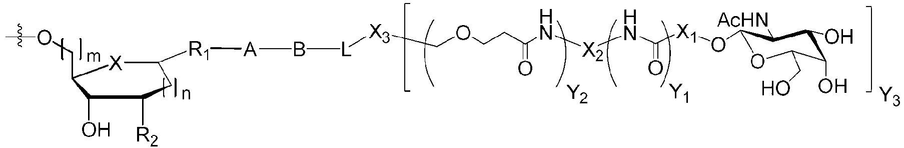
wherein X is oxygen, -N(Y)-, or sulfur;
Y is C₁₋₄ alkyl or C₆₋₁₀ aryl;
R₁ is oxygen or sulfur;
R₂ is hydrogen, -NH₂, C₁₋₄ alkyl, C₆₋₁₀ aryl, C₁₋₄ alkoxy, or halogen;
A is -(CH₂)ₐ-, -(CH₂CH₂O)_{b}-, -((CH₂)_{c}NHCO)_{d}-, or -((CH₂)_{c}CONH)_{d}-, wherein a is an integer from 1 to 15, b is an integer from 1 to 7, c is an integer from 1 to 7, and d is an integer from 1 to 5;
B is -(CH₂)ₑ-, wherein e is an integer from 0 to 7;
L is -CONH- or -NHCO-;
X₁ is -(CH₂)_{f}- or -(CH₂CH₂O)_{f}CH₂-, wherein f is an integer from 1 to 5;
X₂ is -(CH₂)_{g}-, wherein g is an integer from 1 to 6;
X₃ is oxygen or sulfur;
Y₁ is 0 or 1;
Y₂ is 0, 1, or 2;
when Y₃ is 1, X₄ is CH₂; when Y₃ is 2, X₄ is CH; when Y₃ is 3, X₃ is carbon;
m is an integer from 0 to 4;
n is an integer from 0 to 4;
(3) the conjugate moiety is as follows: 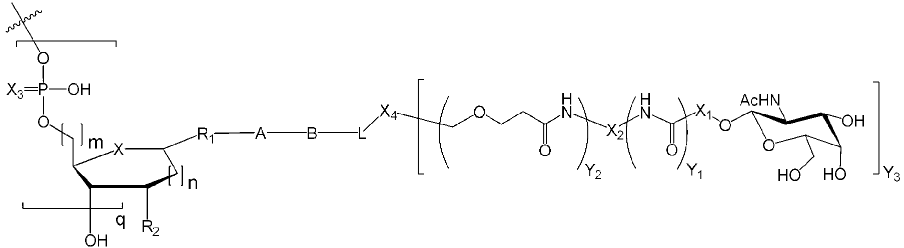
wherein X is oxygen, -N(Y)-, or sulfur;
Y is C₁₋₄ alkyl or C₆₋₁₀ aryl;
R₁ is oxygen or sulfur;
R₂ is hydrogen, -NH₂, C₁₋₄ alkyl, C₆₋₁₀ aryl, C₁₋₄ alkoxy, or halogen;
A is -(CH₂)ₐ-, -(CH₂CH₂O)_{b}-, -((CH₂)_{c}NHCO)_{d}-, or -((CH₂)_{c}CONH)_{d}-, wherein a is an integer from 1 to 15, b is an integer from 1 to 7, c is an integer from 1 to 7, and d is an integer from 1 to 5;
B is -(CH₂)ₑ-, wherein e is an integer from 0 to 7;
L is -CONH- or -NHCO-;
X₁ is -(CH₂)_{f}- or -(CH₂CH₂O)_{f}CH₂-, wherein f is an integer from 1 to 5;
X₂ is -(CH₂)_{g}-, wherein g is an integer from 1 to 6;
X₃ is oxygen or sulfur;
Y₁ is 0 or 1;
Y₂ is 0, 1, or 2;
when Y₃ is 1, X₄ is CH₂; when Y₃ is 2, X₄ is CH; when Y₃ is 3, X₃ is carbon;
m is an integer from 0 to 4;
n is an integer from 0 to 4;
q is an integer from 0 to 4.

11. The conjugate according to claim 10, wherein the conjugate moiety is as follows: 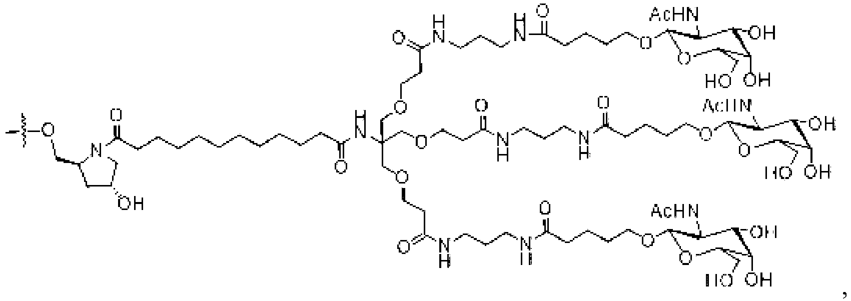 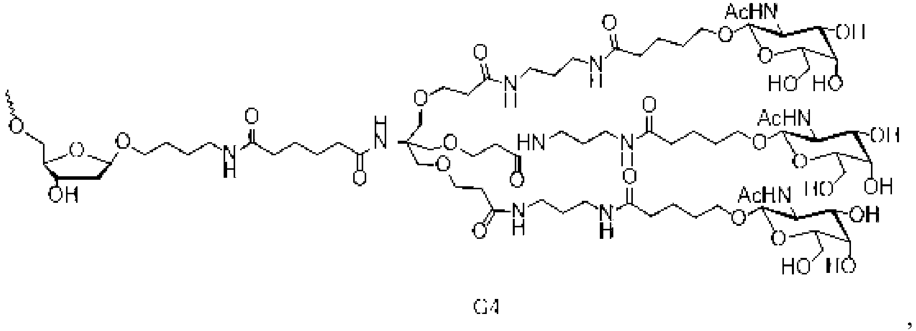 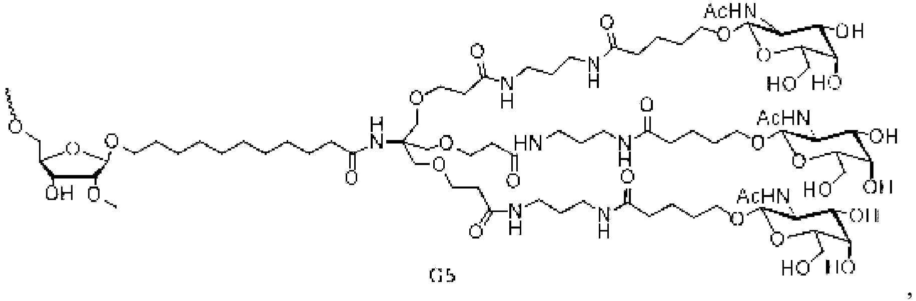 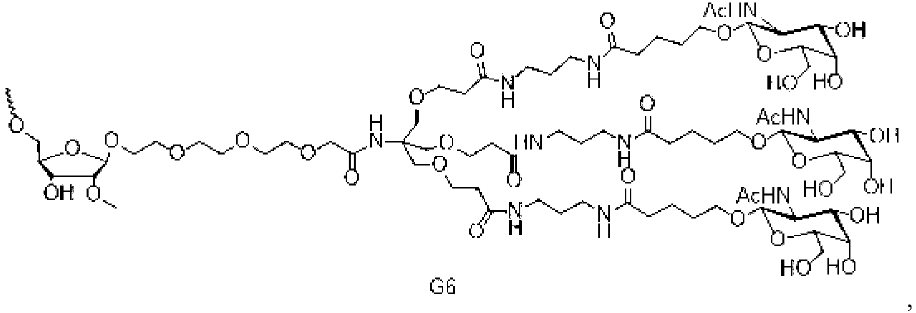 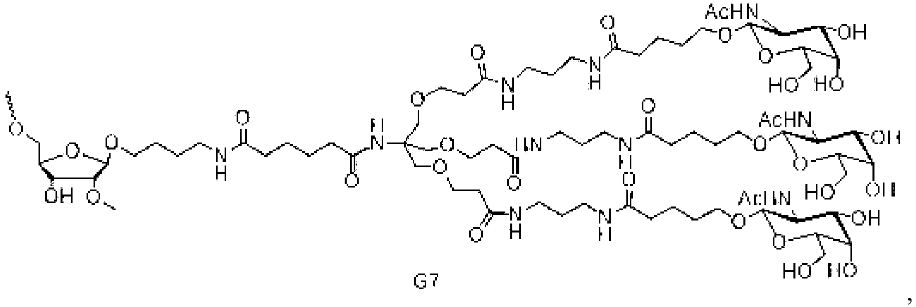 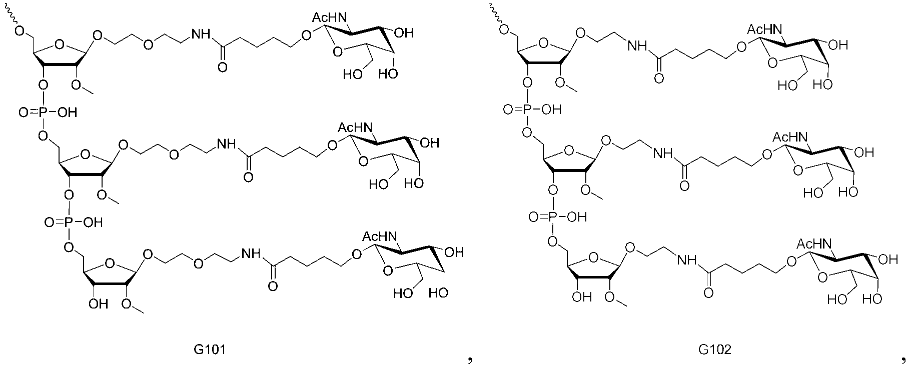 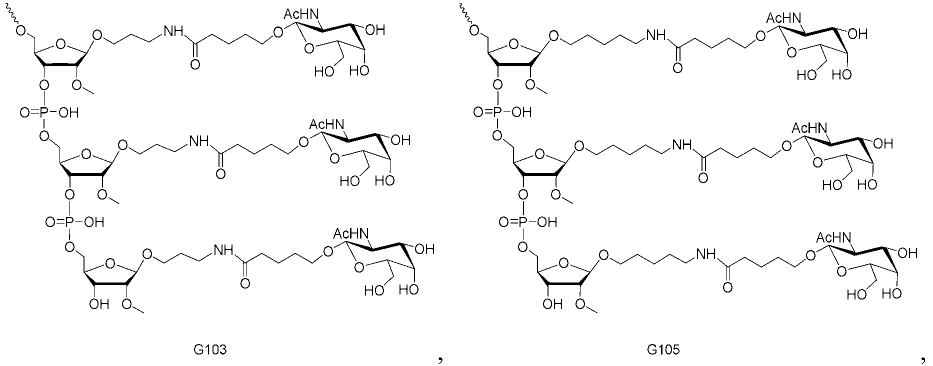 or

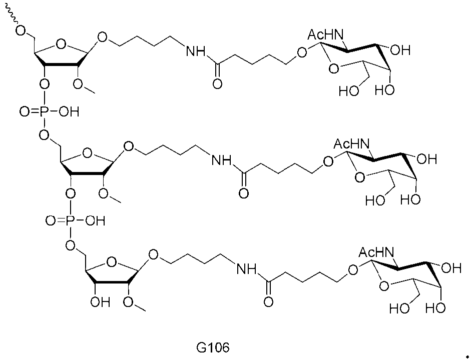

12. The conjugate according to claim 10 or 11, wherein the siRNA duplex comprises any one oligonucleotide duplex selected from the following pairings of a sense strand and an antisense strand, or a combination of at least two thereof:
(1) the sense strand has the sequence of SEQ ID NO: 447; and the antisense strand has the sequence of SEQ ID NO: 573;
(2) the sense strand has the sequence of SEQ ID NO: 480; and the antisense strand has the sequence of SEQ ID NO: 606;
(3) the sense strand has the sequence of SEQ ID NO: 450; and the antisense strand has the sequence of SEQ ID NO: 576;
(4) the sense strand has the sequence of SEQ ID NO: 456; and the antisense strand has the sequence of SEQ ID NO: 582;
(5) the sense strand has the sequence of SEQ ID NO: 426; and the antisense strand has the sequence of SEQ ID NO: 552;
(6) the sense strand has the sequence of SEQ ID NO: 427; and the antisense strand has the sequence of SEQ ID NO: 553;
(7) the sense strand has the sequence of SEQ ID NO: 429; and the antisense strand has the sequence of SEQ ID NO: 555;
(8) the sense strand has the sequence of SEQ ID NO: 430; and the antisense strand has the sequence of SEQ ID NO: 556;
(9) the sense strand has the sequence of SEQ ID NO: 431; and the antisense strand has the sequence of SEQ ID NO: 557;
(10) the sense strand has the sequence of SEQ ID NO: 435; and the antisense strand has the sequence of SEQ ID NO: 561;
(11) the sense strand has the sequence of SEQ ID NO: 439; and the antisense strand has the sequence of SEQ ID NO: 565;
(12) the sense strand has the sequence of SEQ ID NO: 440; and the antisense strand has the sequence of SEQ ID NO: 566;
(13) the sense strand has the sequence of SEQ ID NO: 442; and the antisense strand has the sequence of SEQ ID NO: 568;
(14) the sense strand has the sequence of SEQ ID NO: 443; and the antisense strand has the sequence of SEQ ID NO: 569;
(15) the sense strand has the sequence of SEQ ID NO: 444; and the antisense strand has the sequence of SEQ ID NO: 570;
(16) the sense strand has the sequence of SEQ ID NO: 445; and the antisense strand has the sequence of SEQ ID NO: 571;
(17) the sense strand has the sequence of SEQ ID NO: 446; and the antisense strand has the sequence of SEQ ID NO: 572;
(18) the sense strand has the sequence of SEQ ID NO: 448; and the antisense strand has the sequence of SEQ ID NO: 574;
(19) the sense strand has the sequence of SEQ ID NO: 449; and the antisense strand has the sequence of SEQ ID NO: 575;
(20) the sense strand has the sequence of SEQ ID NO: 454; and the antisense strand has the sequence of SEQ ID NO: 580;
(21) the sense strand has the sequence of SEQ ID NO: 455; and the antisense strand has the sequence of SEQ ID NO: 581;
(22) the sense strand has the sequence of SEQ ID NO: 468; and the antisense strand has the sequence of SEQ ID NO: 594;
(23) the sense strand has the sequence of SEQ ID NO: 471; and the antisense strand has the sequence of SEQ ID NO: 597;
(24) the sense strand has the sequence of SEQ ID NO: 477; and the antisense strand has the sequence of SEQ ID NO: 603;
(25) the sense strand has the sequence of SEQ ID NO: 478; and the antisense strand has the sequence of SEQ ID NO: 604;
(26) the sense strand has the sequence of SEQ ID NO: 479; and the antisense strand has the sequence of SEQ ID NO: 605;
(27) the sense strand has the sequence of SEQ ID NO: 529; and the antisense strand has the sequence of SEQ ID NO: 655;
(28) the sense strand has the sequence of SEQ ID NO: 530; and the antisense strand has the sequence of SEQ ID NO: 656;
(29) the sense strand has the sequence of SEQ ID NO: 532; and the antisense strand has the sequence of SEQ ID NO: 658;
(30) the sense strand has the sequence of SEQ ID NO: 677; and the antisense strand has the sequence of SEQ ID NO: 686;
(31) the sense strand has the sequence of SEQ ID NO: 678; and the antisense strand has the sequence of SEQ ID NO: 687;
(32) the sense strand has the sequence of SEQ ID NO: 683; and the antisense strand has the sequence of SEQ ID NO: 692;
(33) the sense strand has the sequence of SEQ ID NO: 680; and the antisense strand has the sequence of SEQ ID NO: 689;
(34) the sense strand has the sequence of Um-Um-Um-Cm-Am-Gm-Gf-Am-Uf-Gf-Af-Um-Um-Um-Um-Cm-Uf-Um-Ams-Ums-dAinV; and the antisense strand has the sequence of UmsEVP-Afs-Uf-Af-Am-Gf-Am-Am-Am-Am-Um-Cm-Am-Uf-Cm-Cf-Um-Gm-Am-Am-Ams-Ams-Gm;
(35) the sense strand has the sequence of Ums-Ums-Um-Cm-Am-Gm-Gf-Am-Uf-Gm-Af-Um-Um-Um-dT-Cm-Uf-Um-Am-Um-Am; and the antisense strand has the sequence of UmsEVP-Afs-Um-Am-Af-Gf-dA-Am-Am-Am-Um-Cm-Am-Uf-Cm-Cf-Um-Gm-Am-Am-Ams-Ams-Gm;
(36) the sense strand has the sequence of Um-Um-Um-Cm-Am-Gm-Gf-Am-Uf-Gm-Af-Um-Um-Um-Um-Cm-Uf-Um-Ams-Ums-dAinV; and the antisense strand has the sequence of UmsEVP-Afs-Um-Am-Af-Gf-Am-Am-Am-Am-Um-Cm-Am-Uf-Cm-Cf-Um-Gm-Am-Am-Ams-Ams-Gm;
(37) the sense strand has the sequence of Ums-Ums-Um-Cm-Am-Gm-Gf-Am-Uf-Gm-Af-Um-Um-Um-dT-Cm-Uf-Um-Am-Um-Am; and the antisense strand has the sequence of UmsEVP-Afs-Uf-Am-Am-Gf-dA-Am-Am-Am-Um-Cm-Am-Uf-Cm-Cf-Um-Gm-Am-Am-Ams-Ams-Gm;
(38) the sense strand has the sequence of Um-Um-Um-Cm-Am-Gm-Gf-Am-Uf-Gm-Af-Um-Um-Um-Um-Cm-Uf-Um-Ams-Ums-dAinV; and the antisense strand has the sequence of UmsEVP-Afs-Uf-Am-Am-Gf-Am-Am-Am-Am-Um-Cm-Am-Uf-Cm-Cf-Um-Gm-Am-Am-Ams-Ams-Gm;
(39) the sense strand has the sequence of Um-Um-Cm-Am-Gm-Gm-Af-Um-Gf-Af-Uf-Um-Um-Um-Cm-Um-Uf-Am-Ums-Ams-dTinV; and the antisense strand has the sequence of AmsEVP-Ufs-Am-Um-Am-Af-Gm-Am-Am-Am-Am-Um-Cm-Af-Um-Cf-Cm-Um-Gm-Am-Ams-Ams-Am;
(40) the sense strand has the sequence of Cms-Ams-Gm-Gm-Am-Um-Gf-Am-Uf-Uf-Uf-Um-Cm-Um-dT-Am-Uf-Am-Um-Cm-Am; and the antisense strand has the sequence of UmsEVP-Gfs-Am-Um-Am-Uf-dA-Am-Gm-Am-Am-Am-Am-Uf-Cm-Af-Um-Cm-Cm-Um-Gms-Ams-Am;
(41) the sense strand has the sequence of Cms-Ams-Gm-Gm-Am-Um-Gf-Am-Uf-Uf-Uf-Um-Cm-Um-dT-Am-Uf-Am-Um-Cm-Am; and the antisense strand has the sequence of UmsEVP-Gfs-Af-Uf-Am-Uf-dA-Am-Gm-Am-Am-Am-Am-Uf-Cm-Af-Um-Cm-Cm-Um-Gms-Ams-Am;
(42) the sense strand has the sequence of Cm-Am-Gm-Gm-Am-Um-Gf-Am-Uf-Uf-Uf-Um-Cm-Um-Um-Am-Uf-Am-Ums-Cms-dAinV; and the antisense strand has the sequence of UmsEVP-Gfs-Af-Uf-Am-Uf-Am-Am-Gm-Am-Am-Am-Am-Uf-Cm-Af-Um-Cm-Cms-Ums-Gm;
(43) the sense strand has the sequence of Cm-Am-Gm-Gm-Am-Um-Gf-Am-Uf-Uf-Uf-Um-Cm-Um-Um-Am-Uf-Am-Ums-Cms-dAinV; and the antisense strand has the sequence of UmsEVP-Gfs-Af-Uf-Am-Uf-Am-Am-Gm-Am-Am-Am-Am-Uf-Cm-Af-Um-Cm-Cm-Um-Gms-Ams-Am;
(44) the sense strand has the sequence of Cm-Am-Gm-Gm-Am-Um-Gf-Am-Uf-Um-Uf-Um-Cm-Um-Um-Am-Uf-Am-Ums-Cms-dAinV; and the antisense strand has the sequence of UmsEVP-dGs-Am-Um-Af-dT-Am-Am-Gm-Am-Am-Am-Am-dT-Cm-Af-Um-Cm-Cms-Ums-Gm;
(45) the sense strand has the sequence of Cm-Am-Gm-Gm-Am-Um-Gf-Am-Uf-Um-Uf-Um-Cm-Um-Um-Am-Uf-Am-Ums-Cms-dAinV; and the antisense strand has the sequence of UmsEVP-dGs-Am-Um-Af-dT-Am-Am-Gm-Am-Am-Am-Am-dT-Cm-Af-Um-Cm-Cm-Um-Gms-Ams-Am;
(46) the sense strand has the sequence of Am-Gm-Gm-Am-Um-Gf-Am-Uf-Uf-Uf-Um-Cm-Um-Um-Am-Uf-Am-Ums-Cms-dAinV; and the antisense strand has the sequence of UmsEVP-Gfs-Am-Um-Am-Uf-Am-Am-Gm-Am-Am-Am-Am-Uf-Cm-Af-Um-Cm-Cm-Ums-Gms-Am;
(47) the sense strand has the sequence of Am-Gm-Gm-Am-Um-Gm-Af-Um-Uf-Um-Uf-Cm-Um-Um-Am-Um-Af-Um-Cms-Ams-dAinV; and the antisense strand has the sequence of UmsEVP-Ufs-Gm-Am-Uf-Af-Um-Am-Am-Gm-Am-Am-Am-Af-Um-Cf-Am-Um-Cm-Cm-Ums-Gms-Am;
(48) the sense strand has the sequence of Gm-Gm-Am-Um-Gm-Am-Uf-Um-Uf-Um-Cf-Um-Um-Am-Um-Am-Uf-Cm-Ams-Ams-dAinV; and the antisense strand has the sequence of UmsEVP-Ufs-Uf-Gm-Am-Uf-Am-Um-Am-Am-Gm-Am-Am-Af-Am-Uf-Cm-Am-Um-Cm-Cms-Ums-Gm;
(49) the sense strand has the sequence of Am-Um-Gm-Am-Um-Uf-Um-Uf-Cm-Uf-Um-Am-Um-Am-Um-Cf-Am-Ams-Gms-dTinV; and the antisense strand has the sequence of AmsEVP-Cfs-Um-Um-Gf-Af-Um-Am-Um-Am-Am-Gm-Am-Af-Am-Af-Um-Cm-Am-Ums-Cms-Cm;
(50) the sense strand has the sequence of Am-Um-Gm-Am-Um-Uf-Um-Uf-Cm-Uf-Um-Am-Um-Am-Um-Cf-Am-Ams-Gms-dTinV; and the antisense strand has the sequence of AmsEVP-Cfs-Um-Um-Gf-Af-Um-Am-Um-Am-Am-Gm-Am-Af-Am-Af-Um-Cms-Ams-Um;
(51) the sense strand has the sequence of Um-Uf-Uf-Cf-Um-Gm-Gm-dT-Gm-Af-Am-Um-Gm-Um; and the antisense strand has the sequence of AmsEVP-Cfs-Am-Um-Um-Cf-dA-Cm-Cm-Am-Gm-Am-Am-Af-Cm-Uf-Gm-Am-Am-Gm-Ums-Ams-Am;
(52) the sense strand has the sequence of Am-Cm-Um-Um-Cm-Am-Gf-Um-Uf-Uf-Cf-Um-Gm-Gm-Um-Gm-Af-Am-Ums-Gms-dTinV; and the antisense strand has the sequence of AmsEVP-Cfs-Am-Um-Um-Cf-Am-Cm-Cm-Am-Gm-Am-Am-Af-Cm-Uf-Gm-Am-Ams-Gms-Um;
(53) the sense strand has the sequence of Am-Cm-Um-Um-Cm-Am-Gf-Um-Uf-Uf-Cf-Um-Gm-Gm-Um-Gm-Af-Am-Ums-Gms-dTinV; and the antisense strand has the sequence of AmsEVP-Cfs-Am-Um-Um-Cf-Am-Cm-Cm-Am-Gm-Am-Am-Af-Cm-Uf-Gm-Am-Am-Gm-Ums-Ams-Am;
wherein m represents a 2'-O-methyl modification; f represents a 2'-fluoro modification; s indicates that the nucleotide at the position is linked to the adjacent nucleotide on the right via a phosphorothioate bond; dA represents a 2'-deoxyadenosine-3'-phosphate; dG represents a 2'-deoxyguanosine-3'-phosphate; dT represents a 2'-deoxythymidine-3'-phosphate; EVP represents a 5'-vinyl-(E)-phosphonate modification; inV indicates that the linkage at the ends of the oligonucleotide sequence was altered from the natural 3'-5' linkage to 3'-3'.

13. The conjugate according to claim 10 or 11, wherein the conjugate comprises any one oligonucleotide duplex selected from the following pairings of a sense strand and an antisense strand, or a combination of at least two thereof:
(1) the sense strand has the sequence of SEQ ID NO: 713; and the antisense strand has the sequence of SEQ ID NO: 750;
(2) the sense strand has the sequence of SEQ ID NO: 723; and the antisense strand has the sequence of SEQ ID NO: 760;
(3) the sense strand has the sequence of SEQ ID NO: 719; and the antisense strand has the sequence of SEQ ID NO: 756;
(4) the sense strand has the sequence of SEQ ID NO: 721; and the antisense strand has the sequence of SEQ ID NO: 758;
(5) the sense strand has the sequence of SEQ ID NO: 714; and the antisense strand has the sequence of SEQ ID NO: 751;
(6) the sense strand has the sequence of SEQ ID NO: 695; and the antisense strand has the sequence of SEQ ID NO: 732;
(7) the sense strand has the sequence of SEQ ID NO: 706; and the antisense strand has the sequence of SEQ ID NO: 743;
(8) the sense strand has the sequence of SEQ ID NO: 707; and the antisense strand has the sequence of SEQ ID NO: 744;
(9) the sense strand has the sequence of SEQ ID NO: 708; and the antisense strand has the sequence of SEQ ID NO: 745;
(10) the sense strand has the sequence of SEQ ID NO: 710; and the antisense strand has the sequence of SEQ ID NO: 747;
(11) the sense strand has the sequence of SEQ ID NO: 715; and the antisense strand has the sequence of SEQ ID NO: 752;
(12) the sense strand has the sequence of SEQ ID NO: 720; and the antisense strand has the sequence of SEQ ID NO: 757;
(13) the sense strand has the sequence of SEQ ID NO: 722; and the antisense strand has the sequence of SEQ ID NO: 759;
(14) the sense strand has the sequence of SEQ ID NO: 724; and the antisense strand has the sequence of SEQ ID NO: 761;
(15) the sense strand has the sequence of SEQ ID NO: 725; and the antisense strand has the sequence of SEQ ID NO: 762;
(16) the sense strand has the sequence of SEQ ID NO: 728; and the antisense strand has the sequence of SEQ ID NO: 765;
(17) the sense strand has the sequence of SEQ ID NO: 729; and the antisense strand has the sequence of SEQ ID NO: 766.

14. A pharmaceutical composition, wherein the pharmaceutical composition comprises the siRNA duplex according to any one of claims 1 to 9, or the conjugate according to claim 12 or 13, and a pharmaceutically acceptable carrier.

15. The siRNA duplex according to any one of claims 1 to 9, the conjugate according to claim 12 or 13, or the pharmaceutical composition according to claim 14 for use in treating a disease associated with FXI gene expression, or for use in the manufacture of an anticoagulant.
